# EUROPEAN PATENT APPLICATION

(11) **EP 3 115 466 A1**
(43) Date of publication of application: **11.01.2017**
(21) Application number: 15306154.4
(22) Date of filing: 10.07.2015
(51) Int. Cl.: C12Q 1/68

(54) **LISTERIA MONOCYTOGENES CLONOGROUPING AND ASSESSMENT OF INFECTIVITY**

(71) Applicant: INSTITUT PASTEUR, 75724 Paris Cedex 15 (FR); UNIVERSITE PARIS DESCARTES, 75006 Paris (FR)
(72) Inventor: CHENAL-FRANCISQUE, Viviane, 92220 Bagneux (FR); MAURY, Mylène, 91400 Orsay (FR); LAVINA, Morgane, 94110 Arcueil (FR); TOUCHON, Marie, 92270 Bois-Colombes (FR); LECLERCQ, Alexandre, 75019 Paris (FR); LECUIT, Marc, 75006 Paris (FR); BRISSE, Sylvain, 92140 Clamart (FR)
(74) Representative: Desaix, Anne

(57) **Abstract**

The application relates to *Listerin monocytogenes,* more particularly to the determination of the clone to which a *L. monocytogenes* isolate belongs. The means of the application involve primers and/or probes, more particularly multiplex primers. The means of the application are notably useful to assess the invasivity that said *L. monocytogenes* isolate might show in a human being.

SEQUENCE LISTING

<110>  INSTITUT PASTEUR

<120>  Listeria monocytogenes clonotyping and assessment of virulence

<130>  B11296 - FP

<160>  189

<170>  PatentIn version 3.5

<210>  1
<211>  1287
<212>  DNA
<213>  Listeria monocytogenes

<400>  1
atggggaaat tgtttattaa tgcaacaaaa aattttggca ataaaataga aatagcaatc      60

agcaactcaa acggaagaat cgatggagta cactatagta tagctgatat accaatagaa     120

aaaacttttg aattgagaag tgagggtatt tacacttttc ctgaactacc tctttactat     180

ctaaccacaa aaaatgatta tattgaaaaa tatttggatc gatcaccaat taatttagag     240

gatgataaaa gctttatccc taacgaaaga aataacaagc ttatctgcat ggaaaagcat     300

attgatttat catttttaag tacgctacca ttatcaactt ttgagtattc tacaaaacgt     360

gaagaatttt taaatatggt taatacattt tatgatactt attacgaaga tgattatctt     420

atgacgccga catttatgat taataaagtc ggttcgtggg aagagattat gttgctagat     480

gttttaaaaa atacaaatga atatctaatt tctaatcaga aaagtaacgt ttttttgagt     540

atattacttg atccagtgct tttggattct tcggatattg caggtaatat cttgaactat     600

atcgacatgt atcctttgat aaacaacttc agtttaacta taattagtga tgagtcgatg     660

tattatcata cagaagataa ttataaaaat atacttattt ttataaagga gttaagagct     720

cgaaatatag aagtctactt gcaatattgt ggaattaaag atgttgtttt ttcagtgttg     780

aatataaaaa gattttcagt aggttggttt ggaagttata gaaattttga tactaattca     840

aagagaattt cagaagtgag ctccagttcc tttggaaaga gaatcagaaa aatattgaca     900

gaaaatttta tgtcagaggt gccgttaagc tatttaagtg ttttaaatga aagtgaatgt     960

aagaaatatt tttctattga aaaagataaa cttgaaacta ttgactataa ggacctggaa    1020

caaagatact ggtgtgagat gttaaaaatt atagaagaag acacttctga cgagaatctt    1080

tatactggag atgagttaat aaactataga tgtgaaagaa ttaaaataaa atttcacaat    1140

gcagtttcca atttgtcttt tttgataaga aaactaaaag aagcaggacg atttgcggat    1200

gctacgaaat tagaaaaaaa taatttgaaa catgtaaaac tttatgaaga agttttaaat    1260

gattttcagg aaaaattatt cttttaa                                        1287

<210>  2
<211>  3189
<212>  DNA
<213>  Listeria monocytogenes

<400>  2
atggttcaat attatacaga taaagaattt acagacttga cacatacact tgcaaaggca      60

gtaaaggatt ttaatctagt tgttttcgta ggagcaggaa cttctttatc acaaggatat     120

ccaaattgga atggttatat tgagaagcta attcatttct ggcagttcaa tattcaacat     180

gttgctggcg ggaaagttac agtgacaaat gagttgcttt cgcaatttga tgaaatcttg     240

aattcaaata ctacaccaaa aagaaaaatt gatttattgc acactttgtt acaggatatt     300

ttggaagaag actttaaaga ggtaaagctt aattttgaag agtatttttt caaagatgtt     360

gtaccagatt acatagaaaa tagtgtgtta gttgaaatga taaaattaga tccaatcttt     420

atcacttcga attatgattt tgaaattgaa agacattgaa aggtttcaaa acaaaaagga     480

gcattcaaac caataaataa tattcgtgag tttgtagaat caaataataa gttacgttca     540

ggcgatgttt tacatttaca tggaaccact cagggaaatt gggatttttt tgtcaattca     600

tcagttgatt attctcgaca gtatttaaaa gagtcaagcg acttcaatag tttaagtaaa     660

tggtttcaag aaagaaatcc agttgttcta tttattggaa gtagtatgga agaagaggaa     720

attttatctc tacttccagc cactactaaa aatttcgcac tgatgaaagc aaattcaaat     780

gagacacgta gttttagaaa aatctacaat aagacatatc aaaataacaa taatacaact     840

attttttggt acggagatag ctatgatgat ttacctggaa aagtatctga aattgtaaag     900

gctatcgaaa atgaattaga aattccacaa agcattgatg attggaatat actccattca     960

gtatctacta aggataaaat atataaggaa attcttgaaa agcatataga agataagaga    1020

tttttatttg atatttttaa agcagaagat cctaatttag aaaaaaagat tttaaagaat    1080

acatttagca gtcatatttt attagataaa attagtgata ttagtagttt ttggacaatg    1140

attgataaaa attttgaaac tttagatgaa gaacaagttc gatcgattat tagcattttc    1200

caaaagcaga gcttgagcgt ttattggaag gaaatcttta aagtttttga gaaattaaaa    1260

gaatcagaac ctatcggaca agatgatata aataaaatga gaagaaatct ttcccaagag    1320

caagaaatta ttgaaacagc tttctctagt gatgctgatt taatgggtta ttggttaatt    1380

gaacaacttc aaaaggaaac ttcaaatcgt agtagtattt tttatgatga tgagatactt    1440

tcaattaatt tgaaatctga aatgattcca ttaattgtta aactaatgac ggatgaaact    1500

agatatattt attggtcttt taaagaaatt atctctgatg agctgataag aattatatat    1560

gtatctcttc tgaatgacaa aatgttgttg gataataagt caatattaga taattgtcct    1620

gacttattac tagaatctca tcctttccaa agaattttag taagtattga taatgaggtt    1680

ggattgaatg attctattaa aaataagttg ataactaaaa ttgatttttc caatactacc    1740

tttggaagcg agctaaacct tttttcaaga aaacacaaag atgagattga agaattaggg 1800

atagaaattt caagagatta tcaagatatg atttttggag ttgagtctgg ttttgtacat 1860

cagaaatcat ttattgatat taatcagatt cttagtgaag atatggatgc tattttggaa 1920

attttattac ccaaacaaga tgagcttagt tcaaaaagag aagacttttt ttctgaaagc 1980

acatatcaag aaacatctaa ttttttatta agcttgttga ataaaaatga tgaagtatca 2040

aaaaaagtaa aacagataat tcttgaaaaa ggattacttt tgtatccaat atatgacaaa 2100

cttttcgttg agattttagt aaataatacc tattgtactg aattaagaaa tgaatctctg 2160

aatatcttct tagagaagtt cagtttgaag agttttagtt gggaagagaa gaaatttttc 2220

gaagcattaa ttgataaaga agaatttgag aacctagctt ttgaaaagct actaaaagta 2280

aatgttaatg agttgaacta tgattatgtt tttgtagata aaacaagacc agaattacta 2340

gaagtcaatg attttattaa cacggaacta ggaagatatt tagagatact aataaaattg 2400

aataaaaaag agtactctag gcggagtgaa attaaaaata ttatatctca agtaaattca 2460

aaaccattta aagaattttc acaaggtgct ttatcattag tgaacagttc tgtagactta 2520

gaagaaatta ctattaatac cttccaaggc tattcttatg ttgttcgtgg atttcaagaa 2580

aaaagtttgg aaaaatttaa atctgtaggt caagagttat taaaaaaagg gcttgtaaat 2640

gacttgaata aagataatct atttattctc tctctatcca tgataaatcc gagggaggaa 2700

gaaatggaga ttaattggag tgaaattaat ttttcaagga tttttgatat cattcttcaa 2760

aatgaacttg agttcgcttt tgagtcacaa tggattgaaa atataatttt aaaggatgaa 2820

gatggacaat atggggtgag tatcttatat tctatcaaaa gagagcaagc tttaatagat 2880

aagtcaaaaa agcttgttaa tatattcgaa aaagaaatta gtaattatag tggggaaata 2940

aaagttgatt tgttaccgca tgctattaaa aaacaagaca atccacaaaa aaaggattta 3000

ttgattagat tcttctttat attactagat aatgctaaac ttgctcagag ttattttgga 3060

agtgggatgt tagcagattt aatgatgcac ttgaattcta gtttacagaa aaaacttgcc 3120

aaacatccta agttatcgac tattctatca ccattagaaa ttgaaaattt gaaaagagag 3180

attgaataa 3189

<210> 3
<211> 840
<212> DNA
<213> Listeria monocytogenes

<400> 3
atgaatcaat tacaatactc tgaagatgct ttaaaaaata ggtatctctt ttttaaagaa 60

ataaatgaaa taaatatttt tgttgaagat aaaggaaaag aatatgaata tgaggaaata 120

ttgtctaaag tttttgctga tgaatatcat atacagacga tatatgcact ggggggtaaa 180

ccacaagttt tatccgcatt tagagactgt aaagaacatg tttctagcaa taataatata 240

aaaaacatat atcttttaga tggtgatttt gatcaatatt tggataatat agtaatggaa 300

tcacatccac atattattta tttgcaatca tataatattg aaaattattt tttgaatgaa 360

gaatcggtag taacttttat gcaaggaaaa atgaaaatgc tgaagaatga tgtaaggact 420

aaaatagctt attctgaatg gctttctaat acattataca atatagaaaa catttttatt 480

ttatattgta taattcaaga aaaggaatta gggattccta atgtagggga ttctgaatat 540

aaagtaatta atatgatcga tggatctatc aatatgacga gatatgagga tttgaaaaag 600

caaataagta attgtacagt aaacatagat ttagtagagc aagaaatgaa agcaaaaatt 660

cacaatataa atcctaatgt gtttgatttg atttgtggta aacatttgat ttgtagtttg 720

taccgtcatc tgttaaaaat aactaaaggt ttcagctatg atgaatttag atggcattta 780

gttcaaaatg ttgaagtatc tagacttaat tttataaagg aaagagttgc taatttgtaa 840

<210> 4
<211> 1041
<212> DNA
<213> Listeria monocytogenes

<400> 4
atgaagaaaa agaatgtatt aaatttaatt aaatatcatt cagaaaaaaa tgattttcag 60

tttagaaatg aggctatcga aattgctcgc tattttgata caataggtga cagtcaatta 120

tcggaataca tcatgagttt gttatctaca gcaaatacct ttgtgccgca aggtgaggct 180

tttgagaact tatttttcaa aaaaatagaa cctaatgtag aaccattacc actccctaaa 240

gcaattagtg aagatattcg aggtattata aatgctgtaa atcatcatgt aggaataaat 300

aaatttctct tcgagggtgc acctggaact ggaaagacag aaacggttaa acagattgct 360

cgattattag aacgccaact atatatggta gaatttagcg aattaattga tagtaaaatg 420

gggcaaacag ctaagaacat tgtagcagtg ttcaacgaaa taaatcgtgt atcaaatcca 480

tcaaaattat taatactttt cgatgaaata gatgctatag ccctagatag agtaaactct 540

aatgacttaa gagaaatggg acgggtaaca tctacaatct tgaaagagtt ggataagtta 600

aatgaagaag ttgttcttat tgccactact aatttgtatg aaaaatttga tagagcctta 660

ataagaagat ttgattcagt aattaatttt aatagatatg aaaaggaaga tttagtagaa 720

atttcagaaa tcatattgaa ttcattattg aaaaaattta aagatgctgg acgagatatg 780

aagttattta aaaaagtaat cagaaacatg gatgaaattc cgaatcctgg agaattaaaa 840

aatttgatta gggtatcatt agcttttagt gatccaacaa atgaatttga ttatctcagg 900

agattgctga ggtatgtagt tgaaaaacct ggtaatatag aattgaaaga gttacaaaac 960

atggggttta ctgttagaga gatagagata ttaactggta tatcaaaaag ccaagtctca 1020

agagagttga agggagattg a                                              1041


<210>  5
<211>  1731
<212>  DNA
<213>  Listeria monocytogenes

<400>  5
atgaggagaa atataagtag cattgattat gatattctgg aggagtgtgc tctaaaatat      60

tttgaaaaag tagaaaaaaa aacaaaatca ttagataaca aaaataaagc tagaatagga     120

ttttattact tagtttttga tttactctat aatatgaatc aggtggaaga agtgaatacg     180

tgcattacag atcagacttt cagtaatata atgagacaac aagataataa agatttaggc     240

attgatgctg tatacataga tgaagagaat aaggaagtta atttttttaa ttttaaatat     300

cgagaaaaat ttataaaagg taagactaaa gcccatgatg aattaagagc gtgtgaacca     360

tttttacttt atgttcagga tacaaatagt tttgaaaaag ataagtcaga attaagtaat     420

tttactagag aaaaattaaa tgatatagct gatgtgaaaa aagattcaca atactcatat     480

aagctatata tggtaacaaa tgatatgact actacagata ttgaggagcc acaaataaca     540

gcgtttaaaa agaactatag ttggttagaa gttgaggata tcaatttagc tactataagc     600

gaatgggtat ctattcgttc aaaggaaaat gtttctatat tagtattaga gaacgagtct     660

cttttacaac atggtacgtc atacgagaca gctaattcat atgttgcaga ggtttcttta     720

gttgaattaa taaagatgac aagtcaaaat atcgatttgc gaatgaaaga aaatattgat     780

gatcttagca ttgaagaact tgctgattta gaaattgatt tagatgtttt gtttgataat     840

gtcagagggt atctaggaaa cacaaattat aataaaaaaa ttcttgaaac tttgaaaaaa     900

gaacctggaa aattcttttt atttaacaat ggtataacaa ttactgcaga tgatattaat     960

gtgaaagaag ttagactggg taaggctagt cgaattaagc tagatcggtt tcaagttgtc    1020

aatgggggac aatccttacg aactatatat aaatacaaag atcaaaaaga ctcaccaatt    1080

gaaaatttag ctaaggcaag tgtattagtg agatttttta aaacagggca agaagaggga    1140

ttaattaata aggtatcaga atataccaat agtcagaatg ctatctcagc tattgattta    1200

aggacagttg atcaattaca attaaaaatt gagaagcact tagaactcaa gggaattcaa    1260

tatataagaa agagaagttc tgataaaact aagttgcaac tagaaaattc tattacaatg    1320

gaaaaactag gccaattgtt atttgcatac gcgggttatc ctgaaaaagc agggaataat    1380

aagaggaaaa tatttagtga atattacgag aaagtattta ctaatgaaag tgatttgatg    1440

gatagaatta tttctactat agatacttat cataaaataa tttctttata taatgatgat    1500

gactacaaat attatgagca gaaagtttat tatataattt atttaaaaag attttatcaa    1560

aataaaaatt tgaaagagtg cttagagctt tttgaggatg ttttgctcaa ttatgaaacc    1620

aaacaagaag tatccccctc tagaaagcta ttacaatcta aatttaaaga ggaaataaat 1680

agaataatta agttggatat aggagatata aagggtctaa ttaatttgta g 1731

<210> 6
<211> 1092
<212> DNA
<213> Listeria monocytogenes

<400> 6
gtgaaaaaag ttgggagaaa tgaattatgt ccatgtggta gcgggaagaa atttaaaaga 60

tgccaccttg gtgttaatga aatagatgaa gataatatgg aagactttgt taagacttat 120

aaacaattaa ataaagaaag gcgaataaaa caatgcttat atccagaaaa atctaaatgc 180

agtgataaaa taattagagc gcattctata caaaatagta aaataattca acgaatatct 240

gagaatggga atgtttatat gaacaaagca aaaccaaata atccatttgc tgtatttact 300

aagtgggggc gtaaagaagc aacgacattt actggatttt gtgattatca tgataaagtt 360

gtattccagc caattgaaga tagagacttt gagttaagta atgaacagat ctttttgcat 420

acttatagat gtttggtagc taacatgcat gataagcaag aacaagttaa gttagggaaa 480

tcaactactg aaactttttc attatccagt gaatctagtg ttggattatt gggcttagga 540

aatggcttgg cagtggatga tttcttgcga gaaaaaatga ttttggataa ggctttaata 600

aatggagact ttgatgtagt tgattcaatt gtatgggaat ttgattttcc catacagttt 660

gcaggtagcg catttgaagc tcctacacat gacttgaaaa atagaaagat acaatctctc 720

gaaaccaatg atattatagc tcatttattt attactattt ttccagaaaa tgataaatca 780

tattgtatca tttcatggct aaaaaattcg aaagagattc ttagtgagtt agttgaacaa 840

attatggggc ttagtgcgaa ggaaagaaaa gattatttaa acacaacttt agtaaaaaat 900

tcggataatc ttgtttttag tcctaaattg attgaaagaa tgtcaaaaga tgaagagcat 960

tactttgggt atacttctca gggagcctat ctattcgagg cattacttga agcgatggat 1020

gatgaacttc atattgaatc taagatccca gaggtaagtg atccaggtta tgatttattc 1080

aagcaatgtt aa 1092

<210> 7
<211> 1641
<212> DNA
<213> Listeria monocytogenes

<400> 7
atggtctcaa gtaaattaag aactacctgg tttgtaactc gtcctcaacg tgatcctcaa 60

tttttttcac aagcattaga agctttaaaa aaagcaacga gtaattttaa tgaaaaatgg 120

tcctctaata gagaaatcca taaaaaatat gagaaatttc tagcagagaa tgggctaaaa 180

aaagaaaata tttcttctga tggatccggc ggtcgaactt gggttgctat gttaagaaca 240

tatagcctgg tttatataga tccgaactct ggtaaactca tccccactaa agtaggtata 300

gctttactta acgggaaaaa aagatatgaa aatgtgacga aacaactact tactttacaa 360

atcccaaatg catattttgt ttcttcaggc tttagaccta agtatgataa aggcttctca 420

attcgtccta ttcgcttttt aataaagtta acatgtcaaa gttctttaga gtattatatc 480

actaaagaag aaattatttt ttttgctatg accgcaaaac aagattgtga gcttgatgca 540

gtaatagaaa gaattaaaga ttttaggctt agtgattcga aaaaaagaaa ttatttaaaa 600

caaagtatag ctacaattga ttatcgggaa cgatcagata aaggagccag aggttttgaa 660

gaagctaatg gagatgtagc tcacactcta atgttacaag ctagttatac agaattggtg 720

aaatactctg ctggaaaatt aataatagaa cagagcccta ttgaatttaa aaaagtgatg 780

caaacactat cagaatatga tgagaggtac ccattcgata gtcgttatga aatttctgaa 840

cccagattta gtgaacatgc tggtttagac attgatagtt ataaatctag gcctattgta 900

aatactaaag ttgccagcaa tcagcaaaaa atagacctac agttaaaaaa aatcgtgtct 960

aaatcaccta acttaattgg ccaagattat gacgagattg ttaagattct ttttgttaat 1020

tttcctccaa aaaaagcaaa ggaacttgcg cacaaaattg tagaaatgac tcctaaacac 1080

aaccaaatgg atgaggggtt tataacttct tatctaaacc aaactaatga tttagaattt 1140

gaaaaacaaa cagttgaggt acttcgagct ttgggttttg aaacggtcct tcaacctaag 1200

cctgttgtta aaggagtaag aactagtatt gaaatattaa ttcatctaga tgcgcaaaat 1260

attgctatta tagatgcaaa aaactacaaa aagaaattca ctctaacagc aaacttggcc 1320

aattatatgg ctactgaata tatccccata tatcaaggct atgatggaaa aaatgttaca 1380

cattttggat atattacagc ttctaaaatt ggcggtagta gcaatctaaa aaaaatagta 1440

catacagcta gaacatataa tgataaattc cgagttaatg gttttttaat ttcagcgcct 1500

gctttactag gtcttctaga ttattgtata gaaaatagta ttgataattc tcgaagaaaa 1560

caatatttct taacactatt aaatgataat aaagcatatg aaacctacag tgaagtatca 1620

caacgattaa atttatctta a 1641

<210> 8
<211> 765
<212> DNA
<213> Listeria monocytogenes

<400> 8
atgaaaaaaa ttcgtaatag tttaattgta ataatgagta tttttgcgtt aacatttggg 60

atgataccat caggagttgc taatgcacaa gagcctaacg aagagtctga ttttaaaatc 120

acagaccatg ttgtaactga caataaagag tcttttacaa ttgctatgaa cggtgaggaa 180

gtggattata agtcgactaa aacggaagat tcattcgaaa ttgaaactgt agaagaaaat 240

aatgaagtac atacctttac atatactaaa ggtgacgatt acatgatgat agacggcgaa 300

aaagtatctg ttgaaattga tacagaagta gaagcagaat taacagattc ttctgcacca 360

aaactattaa aagcttcggc atggacacct aagtatatgt cgacaggatc tataaagata 420

agtaagtcag taaaaacatt aggaactctg gttggagtaa ttgggggagc tatttctatt 480

gctgcactat caggggtggc aattgcaaca tcaacaattg caagtacagt aggacactgg 540

gctagtgcag taggtttagg cactatcgct gcgggttacg catttaatgg taaagttcaa 600

tttaagcagt atcgtacaac aggattagtg ccaacaaatt atggcaaaag attatatgct 660

tttagatttc aagatgtgcg tgttataggt acgcttaaag gcaaaaaaat agataaacaa 720

ctgaaaaatg taggaaattg gttctttaat tctaaaccgt attaa 765

<210> 9
<211> 1113
<212> DNA
<213> Listeria monocytogenes

<400> 9
atgagtaaat ggatttctac ttctctaggt gaggtagcaa aaataatcac tggcccattc 60

ggaacccaac ttcacaagtc agattatgtt gactctggag ttgcagttat aatgcctcaa 120

aatataggtt ctagacaagt taattatgag aagatttctt acatttctga ggagtttgca 180

acgactctaa ggcgatacaa agtattaaaa aacgatattg tttttgctcg tcgaggtgat 240

gttgaaaaac atgcatttat cacagaatct gaggaaggtg aactctgcgg aactggatgt 300

tttctcgtac gattcacaag cgaacatgtt ttaccagaat ttatttcttt gattttaagt 360

acacctttcg tgaaaaaatg gctagtctta aatgctgtcg gttcaaatat gccaaatttg 420

aatactgaaa tattaaaaaa tgttcctatt aaatttccag atttatctac tcaacaaaag 480

attttatcaa ctatttcatc ggttgaatat aaaattagaa tcaatacaaa gataaatact 540

aatttattag acatggctaa ggcaatttac atgcattctt tttttggaaa acatgagaat 600

gcaaagattt cagatatact tctggaaaac agtaagtcca acatccaagt tggtgaagca 660

agagaggctc gcggcgatta tccatttttt acaagtggtg aaacaattta tgaatgggat 720

aattatcttg tcaaagatag aaatatttat cttaatactg gtggaaatgc cgatgtcaaa 780

ttctatattg gtaaagccgc atattccact gatacatggt gcatttcagc taaaaatgat 840

tttacagact atctctatct attccttgat gcaataagac ctgaattaaa tcagaaattc 900

tttcaaggta ccgggttaaa acacctacag aaagcattag ttaaagataa agaaatatac 960

cttccttcca aagaaatact aacagaattt aactctattg tgaaaccgat gatggaacag 1020

gtatccttca atactcgtaa taaccaatac ttatctgacc tacgtgattg gctattactt 1080

gtcttgatga acgaactagc aacaatagaa tag                                    1113

<210>  10
<211>  1806
<212>  DNA
<213>  Listeria monocytogenes

<400>  10
atgaccaaga aaactaccat ttcgcatccc gtgataaaat catatgttga taactttatt        60

tctgaacatg agatatcaaa atcaaaagcc aaaaaccagc atggtatatt tgaacaatat       120

attaatgatt taattttatc aatttattca aacgatccta gtgtaactta tcaagatatg       180

gagacaggtt ctgcatttgg aattgatgga gttgctatat ttgtttcaga tcgagttgca       240

actagtacgg aagatgttga ttctataatt gaaggagtaa aaaatttgga agtaagcttt       300

ttctttacac aatcaaaaac aacagatcaa tttgatcgaa cagatatttc tgattttttt       360

acagctgtta gacgtttttt caattttaat aaatgtgaaa tacctgaact tagagaatat       420

tgggatgtcg ccaaatatat ctactctaaa ggttcaaaat tcaaacggca accaagttta       480

aatatgaaat tcattacttt atctcctaaa gaaatagatt tgactgatat ccatatgaaa       540

tcagaaatag atttgggatt gaatgattta aatgacatga gccttttcaa tgaagtgaaa       600

gttcctgact ttttaggaat caaagatatt atgtcattac atgagaaaaa cacttccggc       660

ttagaagttt ctgtgacttt aacaaaacta cccgtaccat atcctaagga tactcataat       720

aaaataaaaa atgggtatta tggattaatt aaacttgaag aatttatact tttactaaca       780

gatgaagtta ataatgaaaa gattttacga aaaggtatct tcaatgataa cattcgttac       840

tatcttggct catcagaaaa aatagaggtc aatacttcaa tgaaaaatca gctgttaggt       900

acagaagatt atttgtttgg attattaaat aatggtatta ctataattgc agatgagatt       960

tcattaagtt ctgatgactt aacacttact aattaccaaa tagtaaatgg gtgccaaaca      1020

agtaatgtta tatttgaatg tttagagtct attgagcaat ctagtgacat atatattcct      1080

attaggttga tttcaacaga ggacgaagac acaaagcact ccattataaa agctactaat      1140

agtcaaacac aattaaagcc agaacagttg gttgctttat cagcaattca gaaagctatt      1200

gagcagtact ataatacaaa ggcaaaagga aactcatgcc ctttatacta tgaacgtaga      1260

actgaacaat accgagatga aaatatacca aagacaaaaa taatcaatat accatttcaa      1320

atcaaggcta cttctgcttt attttttgat ttacctcatg aagtttctgg tcaatatggt      1380

aaagttgtta aacaaacagg tgatttgtta tttaaagata cgcatctatc ttttcttaac      1440

tcttattacg taagtggtct tgcttggtat aaagttgaaa gatttgtact gaataatgaa      1500

gatggcaaaa gattcagaag agctagatgg cacattataa tgattttaaa atatctctgc      1560

ttccctatgg acaaaataaa aatttctatt gataagcaat ctgaacaaaa tagtatgata      1620

ttagaaaaaa tactacttga tgatgaaaaa acaaatgaac ttttgaaaaa ggcaatagat 1680

attattgaga aaagtttaag tcaaaatgaa tctattgaag aagtattagc ggatagaaaa 1740

acatttgaaa gaaaagaaac tacaaccact attattgaat atgtgaaaac aaataacaat 1800

aactaa 1806

<210> 11
<211> 1518
<212> DNA
<213> Listeria monocytogenes

<400> 11
atgggtgtaa aatatgtaga aatcgaaaaa attgataact atattgaaaa tcctaggcat 60

gaagtaggtc tgaatgaatt agatacttta aaaaagttat ttgatgtttc tggatatcaa 120

aatatgatta atttggcaca agatatctat actaatggtt tggtaaatgc aagtctagtt 180

acaattgtaa aactcaataa tgccgataga tatactgtat atgaaggtaa tagacgagtt 240

gcatgtataa aactaatttt acatccagaa aaatttagtt ttttaccaaa gaaccaaatt 300

gatcgaatca aaaaaatgaa aagtgacact ccttcaaaaa taaatctctc tcaaattgaa 360

tgcttaataa cagatgaaga ggatgctttt ttcattatga ggcgtataca ctcaggagaa 420

gataaaggta ggggtcttaa atcctggaat actaaggaac aagaaatctt taaacttcga 480

actaatccaa aaaacagtac atctattgca aagattatta gtgacaaata tgaagaattt 540

tttaaagaag atattcaaga agaaatggca tatactaata tccaaagatt atttaataat 600

ttggaagtga gagagtcact aggcattgaa aaagacaata tagattcttt ttcatgtgaa 660

agattatatt taattaaagg tgttattgaa aaagtaaacc agattgctaa aattgaaaat 720

ttatctattt ctcgtaaatt taataaaagg aaaataattg aacaaatagt aatacccatt 780

atagaagaat taaaatctaa acagttggaa agtaaagcaa taaattctat acatattgaa 840

caaaaaaata aaagtaataa attatcaaat tctaaagaac aacaaacaca aaataaagaa 900

gcaacttcaa acaatggaat tagtgtacga gaagatactg gtttgaaaac tgaatataaa 960

agcatagaat ctatatcatc cactctaaaa ccaaaattac ccccaaaaat agggagcgga 1020

aaacttctaa gtagtactat gccctttaca aatttatatc attctaatgt gcgtataaat 1080

actctagtaa ttgagattaa tcagattaga tataaggatt ttcgtttagc tacacaattc 1140

ttattaaggt ctcttatgga agtatatgga catgaatata ttgattactt tgcaaattta 1200

gactatcaag atgtccagaa aatgaaggct atagcaaaag accgtataaa acgaaatcaa 1260

acagtaaatg aaatttattc agagtttatt agtaaccaca taactaaaca ttttcctaag 1320

tatgccgaac aagccgaatt aattaatata tcattatcaa aaaacaacaa tagttctcta 1380

atgaaaattt tgaattttat ggttcattct caaaatcata ttcctgatag tatcgaatta 1440

atagaagctt ggtacaaagt taaatctatc gttgaagcaa ttgataatat attgcatgag 1500

gttaagcata actactaa 1518

<210> 12
<211> 1470
<212> DNA
<213> Listeria monocytogenes

<400> 12
gtgttacaaa gaagattttg gggtattttt tgttttgcca tattcttgtt tttatttcca 60

actattggtt ctgccgaaac atctggtgac tatgaatata caataaatgg aaatgaagct 120

accattactg attatacagg acagtctact gatattacca ttcctacaac acttggtcct 180

aataatgaat ataccgttac tgctatcggg aatggcgcat tcaaatccaa gagattaact 240

aatgtaacga ttcctaatac agttataact attggcgatg gtgcttttac catcaattca 300

cttgaacaac tagtcttacc caactcagtg cagactatag ggaggaactc ttttagtgtc 360

aataaattag aaaaaataac ttattctact gctttaaaaa atattccttc ccaagcattt 420

ttagctaata atttaaaaac agtgactaca ccagccactg ttgaaagtat tgatgcttct 480

gcctttgaaa ataattttat tactaatatt acaattcaaa acccaaatct tcaaatggct 540

tatcaagctt ttgccgctca aacagtttta agtacactca ttgtaccaag caaccacatc 600

ctacctatag aaaattacat tcagtttcaa gatgcttcgg ctcatttgac tacagataat 660

ttattcataa ctgatttagc gaatggtatt acgtataatc aagccgaaaa agcattaaac 720

ttttcagcag agccactcga atctactttt tcacttttca ctgggacaaa tcgatttgat 780

tcttactatg atatttctga atatggtcct tctggaaagc catttatcta cttcaagtat 840

actaaaccag tcctagtttc ctataaagat gcgtctggga atgaattagc tacttctact 900

agattagatg gtagtattgg tgaaaattac gtcactactc ctaaaataat agatggctat 960

actttaaaag aaacacccgg aaatgcgaca ggacaatttt ctgaaacact gcaaaatgta 1020

acgtatattt atgaaaaaac tgctgtccaa aatgggacag tcactgtaaa atatcaagat 1080

gaatcgggta aaacactagc aaaagatact gttttaaccg gtgaagtaaa taatacctac 1140

caaactaaaa gcaaagacat agcaggctat aaacttcaaa aagtggaagg aaatgaatcg 1200

ggtactttta gcacaacccc tgcaactgtt acctatattt atgaaaaaat agcaaacagt 1260

gataatacaa ataccaatgg agaaatgacc gacaatacta cactgagtac taatgatact 1320

gtgatttcat ccgaagcaac aaaaaaagtt gataaaaaca cctctaatat cctcccaact 1380

actggtgatt caaaagatgc tctctttttt gcactaggaa gtttattgac gttgttatct 1440

actagcttct ttttctttaa aagaagttaa 1470

<210> 13

<211>  1332
<212>  DNA
<213>  Listeria monocytogenes

<400>  13
atggcaacgc aaaagaattt agttgattta gcaacaaaag cttatattta cggttttcct      60

ttcattttta atacacaaca aatcgagcgt tatgtaacgg tagggattgg tggaacaaag     120

caagttcctt tcaataattt tactcatgca tctcgcttgg cagaaccgtc tgataaattt     180

gtcagtgtga acaacgatac tatttattct aatgcaccaa ttgatgttag tgccggaccg     240

gtcgttttaa gtgtacccga tacaagtggt cgttattatg tacttcagtt tgttgatgca     300

tggtcaaaca attttgctta tgttggaaaa cgtgcaacgg ggacttcagc tggcaaattt     360

cttttaactc ctccaaattg gaacggtgat gttcctgctg atatgataga aattaaattt     420

cctactaata tagggattat tattggccga cttgcttgtg acggtgaagc tgatttacca     480

actgtaagag aattacaaga acaacttaag ataactcctt taaatgaagg gaaagaagtg     540

gatggttttc ctgaatatga tcgctcacta ggaaaagagc tagctttctt tgaacaatta     600

cgagtttata tggctcaatt tccaccagca gaaagagatt tggttaagca agaaagtttt     660

gcgccaattg gtttgatgga aaaaggggta tctccttata gtaacccgag tgaagaatta     720

aaaaatgccc ttatagaggg agcaaaagca ggtttagcga atatcaaaaa agcaacaacc     780

aattttaaat cagaaaatgg ctggggatta acacaacatt tatttgatta taatgctgat     840

ttttttgaaa taggaacgaa aaaatcttta gactgggtaa ttgaggaccg tgaagaagca     900

tacataattc gtgcagtttc agctattacg gcattatggg gaaaccatgg ctatgaagcg     960

gtttatttga tgacatggac agacacagac ggtaatgcct taaatggaaa aaataaatat    1020

acattggagc taaatccaat tccgccagtt gactcatttt ggtcgattac gatgtatgac    1080

ttgcctgaat actttttatg tgagaacccg attaatcgtt actcgattgg agaccgtacg    1140

ccaggtatac aatataatga tgatggcggt atcactatag tggtttcgaa agatgagcca    1200

actgacccga aagaacgtgc gaattggttg ccagctcctg atgctgagtt cagaccaatg    1260

ttccgcttat ataatccgca acaaccactc gtggatggaa gctatcattt tccgggattt    1320

gaaaaaaagt aa                                                        1332

<210>  14
<211>  1689
<212>  DNA
<213>  Listeria monocytogenes

<400>  14
atgagcaaca gtaaatttat tattacagaa ttatactggg aaagaattca actacatgta      60

aagggtaaaa tagaaaatat ttcgttagag aactatacgt tttcatttag gactttaaca     120

gatgaatttc atttttcacc aacgggaatc aaattggaag gaaacacatt tgatcttcgt     180

ttcaatattg ctattttaaa tgatgggaat tatttaccaa gtggggatta tctcttatcc 240

ttatataaca gtgaaacagc ggatgaaatt gttgcccaac cggctgctga actatataaa 300

tttccagaag atgaagatct tttagaagaa ctaaacgaag ctaaaaccga aaacgaaaaa 360

aacaatgtgc ttttagcagg tttaagaaaa gaatttaaac gcggtggggc gaatctcaag 420

tatacgtata aagtcacccc aatgatttct gcagatgtaa atgaatttgt tttctctgtt 480

tcatttacaa tccctgtacc aaaaccaaca aagtggcaag tattcaaaaa taaaattaaa 540

gaaatgtatc atactttcag ttttaatttt agaactggtt tgtttaaatc tattttctat 600

acctcacaag ccctagtacc taaaaacgga aaacgaattc ttttttcttc agattcccgt 660

gcagaaattg gtggtaattt tgagttcgtt ctaaataaaa tgaaagagat gggtattgat 720

aaagattata aaatcaaaat gacctttaaa cccaacattc gtttgagaag agcttttatt 780

gagaaattta gatttccatt tttgctaggg agttcagata ttatttttat tgatgattat 840

catccgatga tttatacaat tgacttcagc gaaaaaacac aagtaattca attatggcat 900

gcatgtgggg cttttaaaac agttggattc agtcgttcag gtaagcaagg cgggccgttc 960

tttgatgata gagcacatcg taattataca cgtgctattg ttgcttcaga tacagacata 1020

ccattttatg cagaagcttt tgggataaaa gaatcttcta ttttaccaac tggtgtacca 1080

agaacagata ttttctttga tccagagtac aaaagaaata ttgttactac gatggagcag 1140

ttgttcccag aaactcaagg caaacaagtt attttatttg ctccaacttt ccgtggaaat 1200

ggtgcgaatc aagctcatta cccatatttt aaaattaatc tggctaagtt tgctgaatac 1260

tgtcgaaaaa ataattcggt cgtaatcttt aaaatgcatc cgtttattaa aaatgaattt 1320

attattccag aacaatatgc agatgttttc attgatgcta gttcttatcg tgaagtaaat 1380

gatattctat tcattacgga tatattgatt acggattatt catctgttat tttcgaattc 1440

tctaccttac aacgcaaaat gctgttctat gcatttgact tggaagatta tgtttctacg 1500

cgtgactttt atgaagaata tgaaggtttt gtgccaggga aaattgtata tgatttcgaa 1560

gcattaatta aggcgcttga aatgaatgat tttgaacaag aaaaggtaaa accattttta 1620

gataagcact ttaaatatca agatactaat tctgcaaaac gaattgtgga agaaatcttt 1680

aaaaaataa 1689

<210> 15
<211> 1353
<212> DNA
<213> Listeria monocytogenes

<400> 15
atgaaaaagc ttagaatatt tactttgatg cttgcttttg ctctattttt attaggtgga 60

ggaattgttg cacaagcagc agaggaagcc ccaatcgatg aaaagatagt tggggaaacg 120

gtaacaaatg acggagaaga gtttatagtc gatgagatta gtgatttaga agatgttaat 180

tcaacaacgg ggacgttaga tacatctgat gaagttgagg ttgatttatc aggattaact 240

attgaaaatg aggaagccgt tctaacccca ggtttaaaaa cgatttttgg cggagatggt 300

agaaaacttg taacaaatac aactcaatat ccatattcaa cgagcgctta ccttgtaatg 360

gaatttccaa atggaaaaac ttatataggt agtggacaat tgattggtga agattctgta 420

cttacagccg ctcattgttt atatggtaaa aaagatggtg gatgggcaaa aaaagtgact 480

gtatatcctg gatataatgg cacgaaagct ccttttggaa cagcaaaagc aagaaaaatg 540

tatgttccaa aagaatggac aaaaaaagaa ccttctacag aagattatgg tgttattaaa 600

ttagataaaa atattgggac aaaaactgga acaatggggt taacaactaa tacatctggt 660

gcaattacta ttagtggtta tcatggtgac aaaaaaggga aattgtacac tcaaactgga 720

aatatctctc aagtcactgc aaataatgtt ttttatagat tagatacaac aggtggtagt 780

agtggtagtg gtgtttataa ttctaaaaaa cagattttag cagtaaacgc atatgaatat 840

ttaaatggta ccggggacaa ctttggtaca agaataacaa aagaaaaact aaataatatt 900

tatacttggg cgtttgacaa taatctttct gtaagcaaac aaaaagggat aaattacgag 960

ctccacgtcc aaagtaaggg atggatggga aacgttgcta atagtatgac ttcgggcaca 1020

gttggccttg gcttacgagc agaagcaatg aaaattagtt tatcagggat gccgtattct 1080

ggagatattc aatatcgctc acatgtacaa ggtagtggat ggcaaggttg gttgaaaaat 1140

ggggaaattt ctggaactcg tggacagtca aaacgtctgg aagctttcca agtaaaatta 1200

actggaaata tggctaaaaa ttatagtgtg cagtatagag cgcatgtcca aaataaaggt 1260

tggcaaagtt gggttaaaga tggagcaaca gcaggaacaa caggagaaag tttaagaata 1320

gaagctgtac aaatgcgact agttgcaaaa taa 1353

<210> 16
<211> 405
<212> DNA
<213> Listeria monocytogenes

<400> 16
tcagcaactc aaacggaaga atcgatggag tacactatag tatagctgat ataccaatag 60

aaaaaacttt tgaattgaga agtgagggta tttacacttt tcctgaacta cctctttact 120

atctaaccac aaaaaatgat tatattgaaa aatatttgga tcgatcacca attaatttag 180

aggatgataa aagctttatc cctaacgaaa gaaataacaa gcttatctgc atggaaaagc 240

atattgattt atcattttta agtacgctac cattatcaac ttttgagtat tctacaaaac 300

gtgaagaatt tttaaatatg gttaatacat tttatgatac ttattacgaa gatgattatc 360

ttatgacgcc gacatttatg attaataaag tcggttcgtg ggaag 405

<210>  17
<211>  20
<212>  DNA
<213>  Listeria monocytogenes

<400>  17
tcagcaactc aaacggaaga                                                    20


<210>  18
<211>  20
<212>  DNA
<213>  Listeria monocytogenes

<400>  18
cttcccacga accgacttta                                                    20


<210>  19
<211>  565
<212>  DNA
<213>  Listeria monocytogenes

<400>  19
gcattttcca aaagcagagc ttgagcgttt attggaagga aatctttaaa gtttttgaga        60

aattaaaaga atcagaacct atcggacaag atgatataaa taaaatgaga agaaatcttt       120

cccaagagca agaaattatt gaaacagctt tctctagtga tgctgattta atgggttatt       180

ggttaattga acaacttcaa aaggaaactt caaatcgtag tagtattttt tatgatgatg       240

agatactttc aattaatttg aaatctgaaa tgattccatt aattgttaaa ctaatgacgg       300

atgaaactag atatatttat tggtctttta aagaaattat ctctgatgag ctgataagaa       360

ttatatatgt atctcttctg aatgacaaaa tgttgttgga taataagtca atattagata       420

attgtcctga cttattacta gaatctcatc ctttccaaag aattttagta agtattgata       480

atgaggttgg attgaatgat tctattaaaa ataagttgat aactaaaatt gatttttcca       540

atactacctt tggaagcgag ctaaa                                             565


<210>  20
<211>  20
<212>  DNA
<213>  Listeria monocytogenes

<400>  20
gcattttcca aaagcagagc                                                    20


<210>  21
<211>  20
<212>  DNA
<213>  Listeria monocytogenes

<400>  21
tttagctcgc ttccaaaggt                                                    20

<210>  22
<211>  622
<212>  DNA
<213>  Listeria monocytogenes

<400>  22
gggggtaaac cacaagtttt atccgcattt agagactgta aagaacatgt ttctagcaat      60

aataatataa aaaacatata tcttttagat ggtgattttg atcaatattt ggataatata     120

gtaatggaat cacatccaca tattatttat ttgcaatcat ataatattga aaattatttt     180

ttgaatgaag aatcggtagt aacttttatg caaggaaaaa tgaaaatgct gaagaatgat     240

gtaaggacta aaatagctta ttctgaatgg ctttctaata cattatacaa tatagaaaac     300

atttttattt tatattgtat aattcaagaa aaggaattag ggattcctaa tgtaggggat     360

tctgaatata aagtaattaa tatgatcgat ggatctatca atatgacgag atatgaggat     420

ttgaaaaagc aaataagtaa ttgtacagta aacatagatt tagtagagca agaaatgaaa     480

gcaaaaattc acaatataaa tcctaatgtg tttgatttga tttgtggtaa acatttgatt     540

tgtagtttgt accgtcatct gttaaaaata actaaaggtt tcagctatga tgaatttaga     600

tggcatttag ttcaaaatgt tg                                              622


<210>  23
<211>  20
<212>  DNA
<213>  Listeria monocytogenes

<400>  23
gggggtaaac cacaagtttt                                                  20


<210>  24
<211>  24
<212>  DNA
<213>  Listeria monocytogenes

<400>  24
caacattttg aactaaatgc catc                                             24


<210>  25
<211>  764
<212>  DNA
<213>  Listeria monocytogenes

<400>  25
tgaggctatc gaaattgctc gctattttga tacaataggt gacagtcaat tatcggaata      60

catcatgagt ttgttatcta cagcaaatac ctttgtgccg caaggtgagg cttttgagaa     120

cttatttttc aaaaaaatag aacctaatgt agaaccatta ccactcccta aagcaattag     180

tgaagatatt cgaggtatta taaatgctgt aaatcatcat gtaggaataa ataaatttct     240

cttcgagggt gcacctggaa ctggaaagac agaaacggtt aaacagattg ctcgattatt 300

agaacgccaa ctatatatgg tagaatttag cgaattaatt gatagtaaaa tggggcaaac 360

agctaagaac attgtagcag tgttcaacga aataaatcgt gtatcaaatc catcaaaatt 420

attaatactt ttcgatgaaa tagatgctat agccctagat agagtaaact ctaatgactt 480

aagagaaatg ggacgggtaa catctacaat cttgaaagag ttggataagt taaatgaaga 540

agttgttctt attgccacta ctaatttgta tgaaaaattt gatagagcct taataagaag 600

atttgattca gtaattaatt ttaatagata tgaaaaggaa gatttagtag aaatttcaga 660

aatcatattg aattcattat tgaaaaaatt taaagatgct ggacgagata tgaagttatt 720

taaaaaagta atcagaaaca tggatgaaat tccgaatcct ggag 764

<210> 26
<211> 20
<212> DNA
<213> Listeria monocytogenes

<400> 26
tgaggctatc gaaattgctc 20

<210> 27
<211> 20
<212> DNA
<213> Listeria monocytogenes

<400> 27
ctccaggatt cggaatttca 20

<210> 28
<211> 606
<212> DNA
<213> Listeria monocytogenes

<400> 28
tcgatttgcg aatgaaagaa aatattgatg atcttagcat tgaagaactt gctgatttag 60

aaattgattt agatgttttg tttgataatg tcagagggta tctaggaaac acaaattata 120

ataaaaaaat tcttgaaact ttgaaaaaag aacctggaaa attcttttta tttaacaatg 180

gtataacaat tactgcagat gatattaatg tgaaagaagt tagactgggt aaggctagtc 240

gaattaagct agatcggttt caagttgtca atgggggaca atccttacga actatatata 300

aatacaaaga tcaaaaagac tcaccaattg aaaatttagc taaggcaagt gtattagtga 360

gattttttaa aacagggcaa gaagagggat taattaataa ggtatcagaa tataccaata 420

gtcagaatgc tatctcagct attgatttaa ggacagttga tcaattacaa ttaaaaattg 480

agaagcactt agaactcaag ggaattcaat atataagaaa gagaagttct gataaaacta 540

agttgcaact agaaaattct attacaatgg aaaaactagg ccaattgtta tttgcatacg 600

cgggtt                                                                 606

<210>  29
<211>  20
<212>  DNA
<213>  Listeria monocytogenes

<400>  29
tcgatttgcg aatgaaagaa                                                   20

<210>  30
<211>  20
<212>  DNA
<213>  Listeria monocytogenes

<400>  30
aacccgcgta tgcaaataac                                                   20

<210>  31
<211>  487
<212>  DNA
<213>  Listeria monocytogenes

<400>  31
tgccaccttg gtgttaatga aatagatgaa gataatatgg aagactttgt taagacttat      60

aaacaattaa ataaagaaag gcgaataaaa caatgcttat atccagaaaa atctaaatgc     120

agtgataaaa taattagagc gcattctata caaaatagta aaataattca acgaatatct     180

gagaatggga atgtttatat gaacaaagca aaaccaaata atccatttgc tgtatttact     240

aagtgggggc gtaaagaagc aacgacattt actggatttt gtgattatca tgataaagtt     300

gtattccagc caattgaaga tagagacttt gagttaagta atgaacagat ctttttgcat     360

acttatagat gtttggtagc taacatgcat gataagcaag aacaagttaa gttagggaaa     420

tcaactactg aaactttttc attatccagt gaatctagtg ttggattatt gggcttagga     480

aatggct                                                               487

<210>  32
<211>  20
<212>  DNA
<213>  Listeria monocytogenes

<400>  32
tgccaccttg gtgttaatga                                                   20

<210>  33
<211>  20
<212>  DNA
<213>  Listeria monocytogenes

<400>  33
agccatttcc taagcccaat                                                   20

<210>  34
<211>  327
<212>  DNA
<213>  Listeria monocytogenes

<400>  34
tttgaaacgg tccttcaacc taagcctgtt gttaaaggag taagaactag tattgaaata      60

ttaattcatc tagatgcgca aaatattgct attatagatg caaaaaacta caaaaagaaa     120

ttcactctaa cagcaaactt ggccaattat atggctactg aatatatccc catatatcaa     180

ggctatgatg gaaaaaatgt tacacatttt ggatatatta cagcttctaa aattggcggt     240

agtagcaatc taaaaaaaat agtacataca gctagaacat ataatgataa attccgagtt     300

aatggttttt taatttcagc gcctgct                                         327


<210>  35
<211>  20
<212>  DNA
<213>  Listeria monocytogenes

<400>  35
tttgaaacgg tccttcaacc                                                  20


<210>  36
<211>  20
<212>  DNA
<213>  Listeria monocytogenes

<400>  36
agcaggcgct gaaattaaaa                                                  20


<210>  37
<211>  460
<212>  DNA
<213>  Listeria monocytogenes

<400>  37
tgcgttaaca tttgggatga taccatcagg agttgctaat gcacaagagc ctaacgaaga      60

gtctgatttt aaaatcacag accatgttgt aactgacaat aaagagtctt ttacaattgc     120

tatgaacggt gaggaagtgg attataagtc gactaaaacg gaagattcat tcgaaattga     180

aactgtagaa gaaaataatg aagtacatac ctttacatat actaaaggtg acgattacat     240

gatgatagac ggcgaaaaag tatctgttga aattgataca gaagtagaag cagaattaac     300

agattcttct gcaccaaaac tattaaaagc ttcggcatgg acacctaagt atatgtcgac     360

aggatctata aagataagta agtcagtaaa aacattagga actctggttg gagtaattgg     420

gggagctatt tctattgctg cactatcagg ggtggcaatt                           460


<210>  38
<211>  20

<212>  DNA
<213>  Listeria monocytogenes

<400>  38
tgcgttaaca tttgggatga                                                    20


<210>  39
<211>  20
<212>  DNA
<213>  Listeria monocytogenes

<400>  39
aattgccacc cctgatagtg                                                    20


<210>  40
<211>  627
<212>  DNA
<213>  Listeria monocytogenes

<400>  40
aataatcact ggcccattcg gaacccaact tcacaagtca gattatgttg actctggagt        60

tgcagttata atgcctcaaa atataggttc tagacaagtt aattatgaga agatttctta       120

catttctgag gagtttgcaa cgactctaag gcgatacaaa gtattaaaaa acgatattgt       180

ttttgctcgt cgaggtgatg ttgaaaaaca tgcatttatc acagaatctg aggaaggtga       240

actctgcgga actggatgtt ttctcgtacg attcacaagc gaacatgttt taccagaatt       300

tatttctttg attttaagta cacctttcgt gaaaaaatgg ctagtcttaa atgctgtcgg       360

ttcaaatatg ccaaatttga atactgaaat attaaaaaat gttcctatta aatttccaga       420

tttatctact caacaaaaga ttttatcaac tatttcatcg gttgaatata aaattagaat       480

caatacaaag ataaatacta atttattaga catggctaag gcaatttaca tgcattcttt       540

ttttggaaaa catgagaatg caaagatttc agatatactt ctggaaaaca gtaagtccaa       600

catccaagtt ggtgaagcaa gagaggc                                           627


<210>  41
<211>  20
<212>  DNA
<213>  Listeria monocytogenes

<400>  41
aataatcact ggcccattcg                                                    20


<210>  42
<211>  20
<212>  DNA
<213>  Listeria monocytogenes

<400>  42
gcctctcttg cttcaccaac                                                    20

<210>  43
<211>  729
<212>  DNA
<213>  Listeria monocytogenes

<400>  43
aaattcaaac ggcaaccaag tttaaatatg aaattcatta ctttatctcc taaagaaata      60

gatttgactg atatccatat gaaatcagaa atagatttgg gattgaatga tttaaatgac     120

atgagccttt tcaatgaagt gaaagttcct gactttttag gaatcaaaga tattatgtca     180

ttacatgaga aaaacacttc cggcttagaa gtttctgtga ctttaacaaa actacccgta     240

ccatatccta aggatactca taataaaata aaaaatgggt attatggatt aattaaactt     300

gaagaattta tacttttact aacagatgaa gttaataatg aaaagatttt acgaaaaggt     360

atcttcaatg ataacattcg ttactatctt ggctcatcag aaaaaataga ggtcaatact     420

tcaatgaaaa atcagctgtt aggtacagaa gattatttgt ttggattatt aaataatggt     480

attactataa ttgcagatga gatttcatta agttctgatg acttaacact tactaattac     540

caaatagtaa atgggtgcca aacaagtaat gttatatttg aatgtttaga gtctattgag     600

caatctagtg acatatatat tcctattagg ttgatttcaa cagaggacga agacacaaag     660

cactccatta taaaagctac taatagtcaa acacaattaa agccagaaca gttggttgct     720

ttatcagca                                                             729


<210>  44
<211>  20
<212>  DNA
<213>  Listeria monocytogenes

<400>  44
aaattcaaac ggcaaccaag                                                  20


<210>  45
<211>  21
<212>  DNA
<213>  Listeria monocytogenes

<400>  45
tgctgataaa gcaaccaact g                                                21


<210>  46
<211>  907
<212>  DNA
<213>  Listeria monocytogenes

<400>  46
aggggtctta aatcctggaa tactaaggaa caagaaatct ttaaacttcg aactaatcca      60

aaaaacagta catctattgc aaagattatt agtgacaaat atgaagaatt ttttaaagaa     120

gatattcaag aagaaatggc atatactaat atccaaagat tatttaataa tttggaagtg     180

agagagtcac taggcattga aaaagacaat atagattctt tttcatgtga aagattatat 240

ttaattaaag gtgttattga aaaagtaaac cagattgcta aaattgaaaa tttatctatt 300

tctcgtaaat ttaataaaag gaaaataatt gaacaaatag taatacccat tatagaagaa 360

ttaaaatcta aacagttgga aagtaaagca ataaattcta tacatattga acaaaaaaat 420

aaaagtaata aattatcaaa ttctaaagaa caacaaacac aaaataaaga agcaacttca 480

aacaatggaa ttagtgtacg agaagatact ggtttgaaaa ctgaatataa aagcatagaa 540

tctatatcat ccactctaaa accaaaatta cccccaaaaa tagggagcgg aaaacttcta 600

agtagtacta tgccctttac aaatttatat cattctaatg tgcgtataaa tactctagta 660

attgagatta atcagattag atataaggat tttcgtttag ctacacaatt cttattaagg 720

tctcttatgg aagtatatgg acatgaatat attgattact ttgcaaattt agactatcaa 780

gatgtccaga aaatgaaggc tatagcaaaa gaccgtataa aacgaaatca aacagtaaat 840

gaaatttatt cagagtttat tagtaaccac ataactaaac attttcctaa gtatgccgaa 900

caagccg 907

<210> 47
<211> 20
<212> DNA
<213> Listeria monocytogenes

<400> 47
aggggtctta aatcctggaa 20

<210> 48
<211> 20
<212> DNA
<213> Listeria monocytogenes

<400> 48
cggcttgttc ggcatactta 20

<210> 49
<211> 194
<212> DNA
<213> Listeria monocytogenes

<400> 49
ggtccttctg gaaagccatt tatctacttc aagtatacta aaccagtcct agtttcctat 60

aaagatgcgt ctgggaatga attagctact tctactagat tagatggtag tattggtgaa 120

aattacgtca ctactcctaa aataatagat ggctatactt taaaagaaac acccggaaat 180

gcgacaggac aatt 194

<210> 50
<211> 20
<212> DNA

<213>  Listeria monocytogenes

<400>  50
ggtccttctg gaaagccatt                                                    20


<210>  51
<211>  20
<212>  DNA
<213>  Listeria monocytogenes

<400>  51
aattgtcctg tcgcatttcc                                                    20


<210>  52
<211>  163
<212>  DNA
<213>  Listeria monocytogenes

<400>  52
   cagaaaatgg ctggggatta acacaacatt tatttgatta taatgctgat ttttttgaaa      60
   taggaacgaa aaaatcttta gactgggtaa ttgaggaccg tgaagaagca tacataattc     120
   gtgcagtttc agctattacg gcattatggg gaaaccatgg cta                       163


<210>  53
<211>  20
<212>  DNA
<213>  Listeria monocytogenes

<400>  53
cagaaaatgg ctggggatta                                                    20


<210>  54
<211>  20
<212>  DNA
<213>  Listeria monocytogenes

<400>  54
tagccatggt ttccccataa                                                    20


<210>  55
<211>  334
<212>  DNA
<213>  Listeria monocytogenes

<400>  55
cgaagctaaa accgaaaacg aaaaaaacaa tgtgctttta gcaggtttaa gaaaagaatt      60

taaacgcggt ggggcgaatc tcaagtatac gtataaagtc accccaatga tttctgcaga     120

tgtaaatgaa tttgttttct ctgtttcatt tacaatccct gtaccaaaac caacaaagtg     180

gcaagtattc aaaaataaaa ttaaagaaat gtatcatact ttcagtttta attttagaac     240

tggtttgttt aaatctattt tctatacctc acaagcccta gtacctaaaa acggaaaacg     300

aattcttttt tcttcagatt cccgtgcaga aatt                                 334

<210>  56
<211>  20
<212>  DNA
<213>  Listeria monocytogenes

<400>  56
cgaagctaaa accgaaaacg                                                    20


<210>  57
<211>  20
<212>  DNA
<213>  Listeria monocytogenes

<400>  57
aatttctgca cgggaatctg                                                    20


<210>  58
<211>  301
<212>  DNA
<213>  Listeria monocytogenes

<400>  58
tatgacttcg ggcacagttg gccttggctt acgagcagaa gcaatgaaaa ttagtttatc        60

agggatgccg tattctggag atattcaata tcgctcacat gtacaaggta gtggatggca       120

aggttggttg aaaaatgggg aaatttctgg aactcgtgga cagtcaaaac gtctggaagc       180

tttccaagta aaattaactg gaaatatggc taaaaattat agtgtgcagt atagagcgca       240

tgtccaaaat aaaggttggc aaagttgggt taaagatgga gcaacagcag gaacaacagg       300

a                                                                       301


<210>  59
<211>  20
<212>  DNA
<213>  Listeria monocytogenes

<400>  59
tatgacttcg ggcacagttg                                                    20


<210>  60
<211>  20
<212>  DNA
<213>  Listeria monocytogenes

<400>  60
tcctgttgtt cctgctgttg                                                    20


<210>  61
<211>  2139
<212>  DNA
<213>  Listeria monocytogenes

<400>  61

atgaaaattg aactagcaac taggaagaat gtttttcaag aagataacat tgaatttaaa 60

aggaaaaact ttatatttgg aaaaaacggt tctggaaaat caacactttg cgatttgata 120

aaaatgcaaa aacaccttta tcaaactgaa atgaactcaa actacgaact agtagaagtt 180

aaagaagatg gagaatacgt tctagctaac aaccaagaag aaaaatttga tgtgcaaatt 240

tttcaggggt tcgatagtgt cataggagaa aataataact taaatgcgat tgcattgagt 300

gctggaaata aggttattga agataaatta ccaatattga atagagagct agaagcgtta 360

aatgatgaga aaaagataaa gaataatata tatttaaaag ctaaggaaaa acataacttt 420

caagaaagta aaatagataa ttggtactca gttgctgcaa aatcaatcaa acgaaatact 480

caatatcaga ttgatcctaa ttataataag cgtaagttta aagacgacat aaataaaagc 540

aaaagaattg aaaatgtaga agaattaaat agcataatta aggaaacgcc taagaatcca 600

gttgttgatt acttttttga tattcctaac ttgaaagaga ttcttaattt agtaaatgat 660

attttagtca agacagttga aattcctcaa aagtgtaacg agctaaatac ttctgagaaa 720

gagaagtttg cgaggaaagg tttaaatctt cacgaagaaa atgatgattg tttattttgt 780

ggaaataagt taaccaggga tagaatgaat aaattaaacc aacattttaa tgaaggatat 840

cagaaattag aagaagaagt tttaagcatt gaaattgaaa aaatcacatt ccagaaaata 900

aaacagcaag aattttatgc taaatttgat gttgaagaaa taaataatat aattttagaa 960

aaagaaaatg agattaatag attccttgaa aatctgctga atgcaataaa tgaaaagaaa 1020

gaagatatta ctaaaatttt taatatttta gaactagaaa tccctcaaat tacaaattta 1080

caaataaaaa ttaatgaact aatacgagaa aataatattt ttggtgaaaa tttaaataat 1140

aatattaaga ttgctaaaga gagtattaag taccatttaa ttagtattga atgtgagaaa 1200

tttaacttta atgttgaaaa aaatgaatta aacaatttaa aattacaaat tcctgattta 1260

acaaaaatta accaagaaat tgaagagaaa aataaagaaa ttcaagaatt gaaaaatcag 1320

caaaaagata catcgaaaat tgcacaatta ataaacgaaa gattgaaaaa agcaggtaag 1380

gatgatctac aacttaaaaa aatagagaat gatggatttg aaagatacga aattcaagat 1440

ggagaaaatg aagttcgtag tataaataaa atctcaacgg gagagaagaa tataattgca 1500

tttttatact ttatatactc tcttgaagat attgaaaatc aaaaaaataa acctaagata 1560

attattttcg atgaccctat gaattcaaat gatgatacta tgcaatattt aataataaca 1620

gagttacaaa agctttattc tggaattgat aaaaataagt ttaatcatga aaaagattac 1680

ttcctttgcc taactcacaa tgtacacttc tatctaaatg tacagcccca cggaaatcat 1740

aaagacagta aaggtagaac aaagtatgat aaaagtaatt tttttagaat tgaaaataaa 1800

aaattcagat taataaaaaa tgagaaggaa gatattaaaa cgaattatgc aggactgtgg 1860

atagaattaa gcgaactatg tgaacgtaat cttaggtatg caatcctaaa ctcaatgcga 1920

agaataattg aaacttttgt gaaattcaac aacttaaata cagatgattt ttatcgagag 1980

aatgcgatat ataagaagct ctttgatgtg ggctctcatt ctattgatga cttgacacat 2040

gaacaattca cagaaacacc tgcagaatta aaattaattt tttcaaatct atttgaagaa 2100

aatggatttg aggatcattt taaaaattat tggaaataa 2139

<210> 62
<211> 633
<212> DNA
<213> Listeria monocytogenes

<400> 62
atgtataaat atgaaagtga attggttgat aattttattt acttaaaaaa ctttcaaaat 60

gagtatattg aagaatgccc gtttagatgg ggtaacattg atttagtcga gtatgaaact 120

tcctctttcc gttttttaag tgaattacag ataaaagaat tatctaataa agaaagcctt 180

cttatatttt caatgttata taaaagtagg ccacatacgt ttgaatatat tttaaaaaaa 240

ttagatttga ccaaagatag attagttatg aaattgaaat ctcttgaacg gactgaaatt 300

gtcgtgaaag caggaaattt atattcgatt aataataaca tcgattttcc tgatattatt 360

gtgagagcat atgaaatgaa gttaacggat ataagaaaag ctattaatca agctgtgatt 420

aacaagcaat attgtgatta ctcttacgtg gtaatgcctg aggaaaaaag aaaactttgc 480

ctagaataca aagatatttt tcagaagtgt tcaatcggat tgttacttgt ggataacaat 540

aacattaaag aggtagtaag agcaaaacgg attaaagaag tgaactataa taagcttgca 600

tcaaaaatta aaattgtgaa tttttgtgga tga 633

<210> 63
<211> 966
<212> DNA
<213> Listeria monocytogenes

<400> 63
gtgagagata ttttagttta ttatagagaa cagattattg attatgtgaa atcagagttt 60

aatggaatta gtgatattac agatattaga attaatatgt cagcagtttt taaatgcgta 120

tataatggta gaacttgtat ttttaaatat tcacttctta aagctgataa aatgatggat 180

cgtactcttt ccgaatatct ttttatacat gctgataaaa tgattaagtt atcagaatgt 240

atagcatttc cggcgattta tgtaaaaaga actttagaga taaaaaactg tacttttcta 300

ttaactgttg aggaaatgct tccttatact tttgcacaag gaattatgaa aatgcatagt 360

aattgcggaa atgatgatct caaatgtaca tctagattct taaaagattt attgaattta 420

tatcttgaag gggaaaagta tggatttata tcccacagag atttatcatt tgataattta 480

atgtttgatg agaattataa tcttaaaatg attgatttag gtagtgcgaa atcaacaaaa 540

gctgaaacta caatgtttca tattgctgct ccaacgaaat atttttattc agcgccagag 600

tatgagaata ttcatttaaa gaataaaact gaatttgagt tgattaaggc agaaatatat 660

actattggat tactaacact atcactttta aatgcgctaa aagaaaatct ttttattgaa 720

aattctcctg tgtgttgtgg tattatgcaa tggttaagag agaatggtag acaacaagac 780

tttaatgaaa aattaactga gattaataaa cgagacttca taaatcatat tttgattaat 840

agtttttttg gagatgataa aaatgttttg tatagattgt taaaaggtat gaccgataaa 900

agtgtctctg caagaattcc agattattac tttattttga agaatataga aaagggggga 960

atataa 966

<210> 64
<211> 201
<212> DNA
<213> Listeria monocytogenes

<400> 64
atgataattc atcaaatttt tttagaagag tctgttgtag atgaagttat gctagttgat 60

gaggacgaag agctatttac tgttttatca acattaactc ctgaacatgc agataataaa 120

atcaaactga tatcgcttat taaaataata cttactttat gcattttcct ctcttatcta 180

tttattaaca attcattata a 201

<210> 65
<211> 231
<212> DNA
<213> Listeria monocytogenes

<400> 65
ttgaaattaa tattaattgc agatattgtt gattcacgtg tcaaagaata tccatattta 60

gaaggactta ccttatattg ttttgtcgat aatagtgatc tctatctaca aattacaaat 120

aatgctgata ctggtcattc gatttataaa cttttaataa acgacgatga tacgacttct 180

acgcaagata ttcgttattt aaataaaaag gatatgaaga agacgacata g 231

<210> 66
<211> 300
<212> DNA
<213> Listeria monocytogenes

<400> 66
gtggtagcta tttcaggggt ttctttggta tttgcagtgt tgggaatgaa agattatacg 60

atgagtagtg attggtggac tagtagcgat aattatgatt attcgtatga ctcttttgca 120

ggaaccatta catacattct ttttacattt attagtctag taaatttgtc tctatttaca 180

ttattcccga cttctctttt aaaaacatca gttgtggtga acggttttgt ttataaaaag 240

tactcggagg aatttagaaa agaatatgag ttcacggata gggaatggta cggtgaatga 300

<210>  67
<211>  156
<212>  DNA
<213>  Listeria monocytogenes

<400>  67
atgttaggag taaacattat tttaaaaaat gcagcttata caatagctgt ttatttgata      60

acacaaacaa ctgtcctttt tttcttttct gtttggatac cttggggaaa cccagatatg     120

actaaaacaa cagctgtttt atattgtaag ggttag                               156


<210>  68
<211>  360
<212>  DNA
<213>  Listeria monocytogenes

<400>  68
atgaacacac tatccaaatt aataaattct gtttcttttg aaaatgcttt agaaaaaaat      60

tcaatacatc gagtatatga gaatttgaat ggtacaggaa aagaactaat acttaagtcg     120

atgaaagtat attttttagc cggtttttcg tggtttatat gtttgatttc tggtattaaa     180

ggtgctggtt tcccaattgt agcctctatt gtaatcctag gcgtttttat aggatatgtt     240

aggagtaaac attattttaa aaaatgcagc ttatacaata gctgtttatt tgataacaca     300

aacaactgtc ctttttttct tttctgtttg gataccttgg ggaaacccag atatgactaa     360


<210>  69
<211>  147
<212>  DNA
<213>  Listeria monocytogenes

<400>  69
atgagcgctt tacttagttt ttatgttggt ttgtttctag ggcatttgaa tagttactta      60

atggcaattg tgggcatgcc tattcttttt attttaataa tgcagtgggc tgtggttagt     120

tttaagagag gacgtaaaac atggtag                                          147


<210>  70
<211>  156
<212>  DNA
<213>  Listeria monocytogenes

<400>  70
ttgcttaaac aaaagtatat aactgagtat ctaggcatcc ttgtcaattc tttaaagaaa      60

ttggagtttg aaggacttct tagtgtaact ctagacggtt taaaacttta ttcaaaagat     120

gaaactgttc agtgggttct agggcatcaa aaataa                               156


<210>  71
<211>  831
<212>  DNA
<213>  Listeria monocytogenes

<400>  71
atgaggtcta tcgagattga taagaaaaaa ctaggtttaa aaattaaaag catacgacta      60

acaaaagggt tgaatcaagg tgaatttggt gctttattta ctccaggagc tgacaaaagc     120

attgtttcta ggtgggagaa aggtattagt atccctagtg ctgatagatt aaaaataatt     180

agtgagattg gaaatattac tattgatgat ttaatttacg gaacaattga agaagcatta     240

agaaatttat tgcaagaagc agaaacgttc tatgaaaaaa agggttatca aaaaaatgat     300

gttcttgctg attctattac agacacagat acgtttgtcc gccttagctt attgaatact     360

atctctggat ttaaacataa tagctatttc aatacttcac gcccaaaatg ttttaagaaa     420

gcaaaagaag attggagtga aacagaatta caagaagtaa ataattacta tatgttagaa     480

gatatgaaag gttcaaaatc tataatggat atggcattag atgaagcttt ttcaaaaaac     540

ttaaaaccca ccgatgaaat tgaacttcta aaagtgcttg cttattgctc tgaactccta     600

ttcaatgatg aagcctatac taatcaaggt ctgcttaatt ttgtatccaa taatatttca     660

gaaatatttg aagaaaagct atctaatttt actcagacag caatttcaac tagtaaaggt     720

aaaaaggtag ttattcgtga aaatattaac cccgaacttg ttgaggaatt agaaatgtta     780

tataacaaaa tccatgaaaa aattgagtgt ctggaagaag aatattctta a              831


<210>  72
<211>  1056
<212>  DNA
<213>  Listeria monocytogenes

<400>  72
atgatagtca atgcggttga tctgttttgt ggtgtaggtg gtctaacttg tggagttcag      60

aagacgggga tcaatgttat tgcgggttat gatattgcta aagaatgtca gtattcatat     120

gagtataata ataaggcaag atttattcat aaagatataa aagaaatctc cgatgatgaa     180

atatcagcat tatatcctga aaatacggat ataagacttt taatgggctg tgctccgtgt     240

caaccatttt ctgcatatag tcataggtat aaagaaagcg aaacaagaaa acaaaaaatg     300

gatttattag attattttgg aaaacaagtt gaattagtac agccggaaat cgtatcaatg     360

gaaaatgttc cacaacttag aaatgatccg gtttttgaaa aatttattac tttattaaaa     420

agtaataatt attttgttga ttggaaaatc gtttatgctc cagaatatgg tgttcctcaa     480

aaaagaaaaa gacttttact gttagcatct aaaataggtg agattagttt attagagcca     540

acaagaacga aagataatta tccaacagtg agggaagcaa tttcaagatt gaagccaatt     600

aaggcaggtg aaacggataa cgaggatttt cttcataggg caagaaaatt atctgacttg     660

aatataaaaa gaattaagag ttcttttccg ggaggaactt ggaaagattg ggatgaagag     720

cttttgccta actgttataa acgaaaaagt ggtgaaagtt ataaatcagt atacggtcga     780

ttggaatggg acaagccatc gtcaactatt actactcaat ttgtgggata tgggaatggt 840

agatttgggc atcctgaaca agatagggct ttgagtttga gagaaggagc gattctacag 900

acttttccta aagattatca gtttgtagaa aaaattgagc aagcatcttt tgttaaacta 960

gctgttcaaa taggaaatgc tgtacctgtt aaattgggag aggttatagg ggaaagtata 1020

aaaatacatc tagaaaataa taaattatta tcataa 1056

<210> 73
<211> 417
<212> DNA
<213> Listeria monocytogenes

<400> 73
gtggctgtag gtattaaagt agatttttta tggttttcta tagggactag tgatttcttt 60

cattcatttt tttcaactgt atgtgtaaac ttagaaaata tgaattgggg aagcaaattc 120

cctgttgtaa tgaaagatat gtacgctggt tatttgtcaa aagaaaaagt tgaaattgct 180

ctaaaggaat tgaaagaaat taaattagga ttgggtgaaa ttcctcctga aaaaattata 240

tgggatgccg aagatttgga caagactccg ccttggggga cacatataag taaggatatt 300

actaatttag ctcaatattt tgtaacaact gatggaaaca atctaataag tgtattagaa 360

gaagctatgg aagaagctat agaagaagat gtggatttag aaataaaaga tttataa 417

<210> 74
<211> 168
<212> DNA
<213> Listeria monocytogenes

<400> 74
ttgctaatag ctagctggat tcattgtgaa aaaggagcaa ttgaattagg ttgttccttt 60

ttccacgcaa cgggattttt aaatgtaaag aagaaagaat atctttggag cgttattggt 120

tctattaagt ttaataatta tattagtgta actgatagga acatatga 168

<210> 75
<211> 405
<212> DNA
<213> Listeria monocytogenes

<400> 75
atgcgtttaa atgaaatcac ttttgtagct aaagacaatc gttattttaa aaaagttcct 60

aaccaaataa ttgatcataa cactatcaac tactcccaag ctgcaaaaca caatttacca 120

gatttagagg aaccttttta cattttctcc aaacaagaca ctggagcaat ctgccttaaa 180

accattaaaa aaagaagtga ttatactttt ccagctgtcg aatatgcttg taaatctttt 240

attgctaaag atgaagcaac tgttgaagcg caaatccaac tatttttgga agaattaaaa 300

tatcctagaa caagcatgat attatacgaa aacatctatc cttcactcgc ctatccggac 360

gcaatggttt acacaatgga agtgccgctg gaagtgccgc tatga                    405


<210>  76
<211>  336
<212>  DNA
<213>  Listeria monocytogenes

<400>  76
atgcatactc tttccagtgg cgaaatcgcg caactttttc aaatatctaa atataaaatc      60

cgtcattata ttgatgaagg tattctagtt cctaaaagaa atcctgaaaa tggctattat     120

tattttgaag aaacggatat ttaccgttta tatcaaatta ttctttttag aaaaattggc      180

ttttctattc aagaaatcaa agaaagtttg cttggtgaaa aagtgactcc catgttagag     240

caggctgaac aagatttgca acaaaaaata gaagagttag taggaattca gaaaacgatt     300

caaaaaatca tccattcgca aaaaaaatgc gtttaa                               336


<210>  77
<211>  141
<212>  DNA
<213>  Listeria monocytogenes

<400>  77
gtgttaacag gcaataaaat aagtttgact cctataggtc acttagttca attttacttg      60

ggaatattaa atgacatgaa agcactacat cgctttattt taattaaatg ttatattgag     120

ataaacaaaa agatatttta a                                               141


<210>  78
<211>  912
<212>  DNA
<213>  Listeria monocytogenes

<400>  78
atggtactat tccaaacaaa atgtaaaaaa aaacttaaaa aatatgaaga aagaaactta      60

ttagagtata ttccaggaaa agggcgaggt aatttatcga caataatttt caggaataat     120

tttcattctg aagtatataa tgtattaact aattgcattg aaaataatga tattaagttt     180

gcactccaac tatctcaatt atcaatacct caagattggt tctctccatt tttagaaaag     240

atacacaccc tttttaatga tggtagtaaa aatgaagata atattcttag atttataatt     300

aatagaaaaa tcactgtact agacccaaca actgtttcct tgcatttcga ggcttcctta     360

atcaaacaaa taagcaatac actagttaac tataatgaag aagaagatat atttacccct     420

tcagttgcaa ttgattggtc tcacaatgaa aaattcacaa catggaactt caacattaga     480

aaaaatattt tattccataa cgaaaaaaga ttaaacggta atgatattct ctttacattt     540

gaagaagcta taaaaagcag tactggaaaa tggttactgt gtaatttaaa aaatatgtat     600

tgcacctctg aattttctgt acattttgaa ttcaaaacac ctgagccagc ctttttaaaa     660

ttagtaacac attattcact tgtaattaga cctgttcttt caaacagtaa aagctttatc 720

ggctgtgggc agtttaagct tatcgaaagt aaaagtaatt acgtatgcct aaaaagtttt 780

tcaaaatatt ttaaagaact ccctcttata gatggggtag aattctggtt aatgaattct 840

aatttcaaaa aatggctaat tgtcccacag aaaaaaatat cactactaac actaatgcta 900

ttaaccccat ag 912

<210> 79
<211> 774
<212> DNA
<213> Listeria monocytogenes

<400> 79
atggctaatt gtcccacaga aaaaaatatc actactaaca ctaatgctat taaccccata 60

ggagtaacaa gatctggagt atcttattta attttcaata aaaataaagc aaatccagtt 120

gaacctttta taataaatta tttaaaagcc gcctttaatt cattgcattt tgtgtctgaa 180

ttatatgata ataaaacacc taaggcctac agttattttt ataagaattc taaaactaaa 240

gaacaagctt ccaaaaatga ttgggttgat actttcaatt tcccagatga ctcagataga 300

acaattcaat tagcagttta tgatcatcct agattcatac gtgaagcctc atggttaaaa 360

aaacaagctg cttcttttaa aatcaaatta gaaataaaaa catattcatt caatgatgat 420

tttttcacag agaaaattat acttgattct gatatagtct tagcaacaga tattcctgta 480

actgatattg aattaggcta tctcgatttt ttattaaaca aaaatttgtt atttcaaaaa 540

tttatcaata aaacacaaaa aaacactata aattcactca ctgataaata tagaacttct 600

gtttctttcg aacaaaaagc tatcttaata aatcaaattg aagattacat taatcaagaa 660

aatatactta tttatttata tcatccatta aaatactatg atatccatag cttcatcaat 720

ggggtggaat ttgatagtaa tggtaatatc gctttagata aattgtggtg gtga 774

<210> 80
<211> 1170
<212> DNA
<213> Listeria monocytogenes

<400> 80
atgattagag ttgtagattt attttctggt gcagggggtt tgacattagg atttcagaat 60

agaattataa ataacacctt tttacctatg tctgattata atattttatt tgctaatgaa 120

gtggataaac acgcatctga agccttctct ctaaatttcc cacaaattcc aatgttaaat 180

tgtagtataa cggaactaac cgaggattat ttagataatc atgagattga gtattctaat 240

attgatttag taataggtgg ccctccctgc caatccttca gtacagttgg taggaggcaa 300

tatgatgaaa aggctacgat gtataaggaa tacagaagaa tgctttcctt cttacagcct 360

aaagtattcc tttttgaaaa tgtgacaggt cttttaacga tgaaaaatga tgaaggggaa 420

cctgtacttg aagatatcag aaaagaattt ggcgattttt ctgactttga aattgattta 480

acctatgaaa ttagagaaac agttctaaat gcaaaacatt ttggtgtccc tcagaataga 540

gagagagtat ttttactagg gataagaaag gatttagaaa ttaaacaaaa ttgggaattt 600

ccagaacgat tattatttga ggatgatttt ttaacattag aagatgctat aggagattta 660

cctgcgttga gaaatggtga aaggaaggat gcttacgatg gagatccgta tactccttat 720

caaaatttaa tgaggaataa ttcgactata ttaatggacc atttcaatgg gttaaacggt 780

gaaaaaatgt tgagaattat ggaaacagtt attccgggag aggggaaaaa atatataaat 840

gagttagttg aatctggaca attagattca aagtattacc taacatctgg ttataataat 900

acatatagta aactttggtg ggatagacct agttcaacaa ttactaataa tttatccacg 960

ccatcttctt tcagatgtat acatccaata caaaatagag cattaacccc tcgagaagga 1020

gctaggatac agtcttttcc ggatacattt caatttgttg gcagtaagga aaaaattaat 1080

agccaaattg gtaatgctgt acctccttta ttagcgattg ttattgcaca agaaatatat 1140

aatttttttg aagagaacaa ttggatatag 1170

<210> 81
<211> 2142
<212> DNA
<213> Listeria monocytogenes

<400> 81
atgaacgaaa aacaaaatga aatcccattt gatttttctt attatgcttt acatttatta 60

ggtaaagggc tttataaaaa tagatggagt gcaatatctg aattaatagc taacggaata 120

gatgctcgtg caacaaaaat aagtttatat atgaatctaa tcgataaaga gaaagcagtt 180

atagaaattt ttgacaatgg gacaggtatg gactatgacg atcttgtttc gaagtatgta 240

catattggaa gaaataagcg tgatgaagaa ttggatgatg ttgaaagaaa tgctctcatg 300

gggcgcaaag ggatagggaa attagcagca ctaaatcttt cacaaaaata ttatttaatt 360

tctaaaacaa gaaatgaatc ttccctatgg tgtctggatg cgactgaagt aaataaatct 420

gacactccga agttaaaaag agtagaaagc aaatcagtag cattggaaag catagaacat 480

tggaaagaaa attcaactgg taccatgata aaattaacca atgttgatat gactggcttt 540

ggaattcaga gtatggaagg attaaaatta aaactttctg atttctactt gttaaatcaa 600

atgagctgcg aaatagaagt agcttacatt actacaaaag aagaaaaaaa taacatcaag 660

tttaaaaaag tggaaaagaa ggtagcattt aagaattttt atggtttttt tgaaaatatg 720

gaaaatgata aatataaagc ttccctagcg gatacagtta gatttccatc ggtatatgaa 780

acaattactg aaaaaccaag aaaggttctt tactttgata aacaaaattt tccggaaatt 840

aaaggaaaga gaagatttaa aaataaaaat ggaactttaa gtgagaaaga atacgaattt 900

gagttgaagg gatggatagg gatccataca tcaacaaaaa aagatgatgc agaaaggaat 960

gatattactt tctttagaaa taatacctat actcctaata aattgagact ttatatacgg 1020

gataaattga ttgttgaaga ttttatggcc cagtatattc gaagtactca agcaacttct 1080

ggatatattg aaggtgaaat tagttttgat attcttgatg tcaatgactt agaagatatt 1140

acaacatcag atagacaagg attcactcat gaggatgata gagttaagtt gctaattgat 1200

attctaaagc cgatagtaaa tttgcttatc agagagcgaa ataaaatggg gggacaaata 1260

agaaaagaag aagaagagta tcgtgaacaa gaaagagaag agattaggaa acaaaaagat 1320

gtcgaagcaa taaaaagaaa agaagccgag gatcagaaag aggctgctga aaaagcaaaa 1380

gcaaaagtaa accaggaaaa tatgattttg aagaatagga ttacgcaaaa agatatacat 1440

ttaggtagtg aaaagaaaag gaacattttt ttgaaatctt cattatctga agataaaaaa 1500

agtttctctc aaaaggcgca tatgatacga attaatgtaa aaacaattga aaatactact 1560

tcttttttag ttaatgaaat tacaaaagaa aaacctaaat ttaatataat taaagaaaaa 1620

ttgaaaataa tttctcataa tacaaacaga attaagagga ttatttcgta tgtggatagt 1680

gcgaaattta atattgataa tgaaaaaaca gaaggagatt taataggctt tttcgaggaa 1740

tatgttgtaa acattgccaa tcaagaatgg gaaaaaccgc aagggaaagt tgttaatccg 1800

gggaaatgtt ccttaataaa ggttttctct ccacaagata taggagttct tattgataac 1860

ttatttagta actcaagtaa aaataaagca agaaatataa ttatttcaat aaaagaagat 1920

aaaaaatatt gttatttgaa ttttaaagat gatggtaatg gattaccttc acatataacg 1980

gatacagctg agctttttga atttggaaaa agctatacta ttcgagggac aggggtaggt 2040

ttagcacata ttaaagatat agtgaatgat atggatggaa aagtatatat tcctaaggat 2100

aataaagaag ggtttgaagt agagatgaga ttaacaaaat ga 2142

<210> 82
<211> 993
<212> DNA
<213> Listeria monocytogenes

<400> 82
atgagagcag acttatcaat tatgtgggtt gaagatacaa cttcatggta tacagaacaa 60

aaagatcttc taaatatgga catagaaagc ttgggtatag atattgaatt tactcgtatt 120

tctacaattg atgattttta taaattggtt aggaatgata atacaggttt tagtaaatat 180

gatatttttt ttgttgatta tgcattatcc tctgatactg aacaaaaagg aagtaacata 240

attgttaagt taaaagatat gggcttaaca acagatattc ttttctattc ttcaaaaaat 300

ataagagaaa taagggaaat tgtgaaaaaa gatattgaac agtttgaggg aatatatcta 360

gctgataggg ataaagattt cagggataaa agttttcaat tagttgaaaa aaatattaaa 420

tcattatcat ctataaaaaa tattagaggt ctattaatgg atcaaacttc tgaaaacgat 480

tttatagtta aatcttatat ttcaaaagaa ataaatgaac ttttacctga acaaaaagaa 540

aaaatatata aatggtttga gaaggaagta gataaaaata tagaagaagt aacgaaaaca 600

attaaaggtt atgtagagag aaagcccagt actatagaaa aatatttaaa attaccaagt 660

tatatattac cagttaaact acaatataat ttattagaag aaattataaa tattaatgga 720

gtagagactt ttgttgattc aactattgat tattatttga aaaaagttgt gagtaataga 780

aataaattag ctcatagaaa attagttatg tgtaggcagc aacaacattt attgcagtat 840

aataatatta atacgtttct aaatgatgat tctaatgttt gtgataatca tgaaggagat 900

gtagtttcaa tagatgattg gattcaactc agagttgaag ttaaagaata tggtaagtta 960

tttgatgatt tgtgggagaa aattaagtcg tga 993

<210> 83
<211> 1362
<212> DNA
<213> Listeria monocytogenes

<400> 83
ttgagattaa ctagattaaa aattacggat ttacatagtt cttatagtta tgacatcgaa 60

tttaataaag acatcacttt tttttacggg actaatggta gcggaaaaac aacaatatta 120

aatattcttt ctagtattgt aacaggaaaa atttatcaac tattcactta tgaatttagt 180

tcaattactc tacactattg ttttgaaaga acaaaaaata aagaacaaaa aattgaaata 240

cttagaacta cacccctatg tattgaagtt acattcaatg gtcagagata tattttagaa 300

aaatttaatg aatctttgag taattatcgt caatctataa acagaaatct agaaaaagac 360

ttttttggtg aaaataaatt tttaaaagaa atagcagata tgtttaacta tgtgtacctt 420

ccactaaata gacatatttc attagataat aatatgttat atgataatag attacatcgt 480

gaaagaacaa taaatgcaat gatgcatgga gttgaggagg atgaaaatga ttactatatg 540

gaaagagata agtcgatgaa gaaggttcaa tatttaataa agacaaaagt aatgaggaat 600

aatactagaa tcaacagact aaatgataac ttcagaaatc aaatactaag atcctctata 660

gatatacaca agcttacttt tagtatagaa gaaatattta atgaggttaa aaacttaaaa 720

gtagaagaaa tcaaagatat caaaacatct tatataaaca tattgaaaaa tttgaatcaa 780

ataacccaaa ttgaagaaaa aaattatgtt aatttttttg ataattatat aagagaaatt 840

aaagagaatg aaaatattga ggataattca gtttcaattc agttgatttt aaaatataat 900

gaaatagcaa aaataaaaaa tatagtaaaa ctagccgagg atatggaaac taaaaaagca 960

aaagtagttg aatctgttaa tttattttgt gaaactatta acagtttcat atcaaccgat 1020

ggtacagaaa aaaaagaaat attaattgat gaaaacggag gtatatattt acaaaatgta 1080

attgaaaaaa aacaattaag tatatataaa ctttcttctg gggaaaagca aattgtaatt 1140

ttttatgcaa acttaatttt tggagtaaat gaaaataaaa gaggtatatt tattgttgat 1200

gaacctgaac tatcgttaca cttatcttgg caaaaattat ttattgaaaa aactatagaa 1260

ttaaataaaa atatacaatt tatatttgct actcattcgc cagaaataat aggtaaatat 1320

tataataaaa cattcgagtt aaagaaaaag atagatagat ag 1362

<210> 84
<211> 177
<212> DNA
<213> Listeria monocytogenes

<400> 84
atgaaggagg agttagggat gcaatataaa ataaaagaaa tgccactgga agaactagaa 60

cctaaagtag tagaggggct tttggaacat aaagaaagat taggttatga cattccaaaa 120

aaagacacag agtatattgg ctttgcggct ttaagtgaag taggggaaat agtttga 177

<210> 85
<211> 231
<212> DNA
<213> Listeria monocytogenes

<400> 85
gtgtctcttc tttctgttga tccaagcaca caaggatcgg gcgtgggtac agaattgatg 60

gcccaaatag aaagatatgg tagagcaaat aactgccacc atatctcctt aacaacattt 120

agttaccaag ccccaggatt ttataaaaaa tgcggtttta ctgagcttgg acgtgtaaaa 180

gattttccta taataggaga agaaaagtac ttttttatta aatatttata g 231

<210> 86
<211> 204
<212> DNA
<213> Listeria monocytogenes

<400> 86
atgacaatga ataagccaca agtaagatca aacataaacc acattattaa aatggctgac 60

tctgttctaa aggatgaaaa tacaattaac aaaaggacta gtgattcaga ttatcaaaaa 120

catatctata cacccgaagc tatcttagca aatattaaag atagtgctga aagtgcattg 180

aaagcattaa acgaatatga ataa 204

<210> 87
<211> 507
<212> DNA
<213> Listeria monocytogenes

<400> 87
atgacgcact taagacatga agcagaaaaa ctagatgact gggtacgtta cgaagccgaa 60

tacaaagggc gctatgcaca ccaactaact gacgcaatcg agaattgtaa gtcagatgaa 120

gaattgaaaa acattattgt tagttctatt ttagatcggt acggatttta ctatacaaaa 180

gaaagtaaaa aagggaaaat aaaccgtccg actatagaaa ctaaaaaaat gctagatctg 240

ttaaataata atgattttaa attcgattct ccctcaccaa gaaacaacat gttaaatcaa 300

acaataaatt atatccaaaa aaacagtggt ttatttcctt tgctttggaa agtagatgga 360

atctggggag atggaactta taaagagtta ctggagtggt tgggtgacca gtactacaat 420

agttttgaac ccaatgatga ccatatatct tggttaaata agtacaaggc actttaccaa 480

ttagagggta agccttggga aggatga 507

<210> 88
<211> 168
<212> DNA
<213> Listeria monocytogenes

<400> 88
atgattaatg atgagaagaa atttaaaaag aatcaatcca agattaggga tgttagtagc 60

aattttaatg cacaaacgat ctatatcggt aaacgtggcg aaaatatcga tgctatgctt 120

agaatttatg acaaaaaagc cgagcagtta ggaagtagta aacgatga 168

<210> 89
<211> 390
<212> DNA
<213> Listeria monocytogenes

<400> 89
gtggaaagca gaaagtctgg attagagtta ttattcgggc tggtagaaga agtagatggt 60

ggaattctaa aaaattatac aaaaggttat acgtttttag atgctccctt ctattttcgc 120

atcgcctatc atccgtttta tgtaaacatg ggtgtttcaa tatacttttc ggcttatgcg 180

tggagtgaat accgcaaaag atatgaagaa tattttaaca aaccgattca tctacacaca 240

ttttttcagt taatccattc aaaagattat tcatatagac ttagtcgtat cgatatggtc 300

attgatttta aaaatgaagg ggtagatatc gcaaagatat atcgctcttt agaggctgga 360

agaactgagg tgagatacaa ccatgattaa 390

<210> 90
<211> 108
<212> DNA
<213> Listeria monocytogenes

<400> 90
atgactgtga cagccgatat tgatcaattc acattaatac ttcagccaag atctctggca 60

tttgactttg atgattggga aaagtggaaa gcagaaagtc tggattag 108

<210> 91

<211>  903
<212>  DNA
<213>  Listeria monocytogenes

<400>  91
ttgagagtca cacaaagtaa attaaatggg ttctacgttt ttggttctac attgatgtta      60

atagttatct ggatattgct tgcatatcaa aacgagtcaa tgaaccaagt gtttaaggct     120

gtgggcataa attttagaat agaacttcaa aaagtaaata atatattttt tgttgctgtg     180

acaatgataa tatttcctac aatcttgttc tggtctttaa gaaataaaat ctgggaaggc     240

aaaagagcac tgaagcgtta ttttctgatc ttaaatttaa ggaaagaaat gatagacgca     300

aattatcgag atgaaagaca tgtgaccgaa cgtgtagtac aaatgccaac tatcaaagta     360

gaatttgata ataaagaaat gacttctgga aaattaattg tacgagattc attggaattt     420

catgaccgtc ttgcaaaagc aacatttact ccttcactaa aaggttttaa agtggaagat     480

ttctatctat cggatgatgg tgattggtgg atttataatt tctattcagt aaattcacaa     540

attcaagcag tcttcgattc attggaagaa tacttaaatt ggtcaaatga aacaacaaac     600

aaatttcaat tgagaatcga taaccgtcta tcattcgatt taaaacacac acttcttgtt     660

ggtgctacta gaagcggaaa aacttatgga ttgattggat tattgctcca aatgataaat     720

aaattgattc attacgaatt gttttttgca gacccgaaga atgaccaatt aaggaaaatt     780

ggaaactgga tcaatggcaa gaacacagct tacacaaccg aaaatattat agatttgatt     840

gatagttaca ataacggcga aaaagaccct gtttctctat ggaagcgcac gcagctccaa     900

tag                                                                   903

<210>  92
<211>  378
<212>  DNA
<213>  Listeria monocytogenes

<400>  92
atggctagat tgccaaatca gtatttattt gtgaaaacag tggtggatgc cgaaagattg      60

ttcgacgaga aaacaaaaag ttatgttgtg gttgaactaa gggatgcgta cgaatacgct     120

gacggcaaga taactgacaa tgtgatcgga aagaatgtaa cacttatgtg taccagagac     180

gaagaggatt atggagtaac aaaagctggc aaaaaaatac ccaatctcaa attcaagcaa     240

ttcgatatta aaattttgaa tggggctttt gaagtaccaa aaggcacaag agtcaaaatg     300

gatttaggga aactagaaaa ggctgttggt tacaaaacga ctttggtgaa caagtatcga     360

ttgtatttaa agatttag                                                   378

<210>  93
<211>  642
<212>  DNA
<213>  Listeria monocytogenes

<400>  93
atgcattgga tagatctgaa gtttggttac agatttgatg aaaaaaaaga taatttgatt      60

tcggaatcta catggagaaa gtatttagat gatttcttta attacaagaa caataaagaa     120

ggaactttaa ccaaatatac agatatgctt aggggcggga atagaaatag ggaatatcat     180

atcgatttta taaaggaaat tattgaattc aaaaaagacg atttaataga acatcttaat     240

agtaataaaa aaacaataaa gaatcctagt ctggaatatt tcgccgaaga aatgaactta     300

aaatttgata cccaagataa atttataaag ggaaccttta ttaaacgcgg aagagtaatc     360

aagactcaat ataaatattt caaagaaaat catatttcta tattgactga agaagaaaaa     420

gaagattata aaaaaggatt tataaatcaa ttaatcgata tattaactga taaaagtaaa     480

atagaagaag atatagagca catatttgta ggtaatgaaa ttcatcacgg tcaagcagag     540

ccaagggaaa tgatagaggg agaatttggt ccagtaggct ttaaaatgga ttggacaaat     600

tatttaaaag aaagtgtaat caatcaaata aaaaacaagt aa                        642

<210>  94
<211>  444
<212>  DNA
<213>  Listeria monocytogenes

<400>  94
atggaaacca ttttagaagt agttcgcatc gaacaaatta ttagagaagc agaggaaggt      60

gttgattata tcattcgtag cccagaagac ggagcaaaga tagcgtctcg ctttattggt     120

cgtgatgatc gtgaagtgtt ttttgttatg tgtttaaata cgaaaaataa tgttgttgca     180

gttcatcgtt gtcatgttgg ttcgttaaat tcctcgattg ttcatccacg ggaggtattt     240

aaatcagcaa tacttaataa tgtagcaagt gtcattcttg cacatcagca tccaagtggt     300

gacataaaac catcgatgga agatattaac gtaactaaga gattagtcga agctggaaag     360

ctactaggca ttgaagtgct tgaccaccta atagtaaata gtgataacag ttttactagc     420

ctgaaagaaa gaggctatat ttaa                                            444

<210>  95
<211>  219
<212>  DNA
<213>  Listeria monocytogenes

<400>  95
atgggcatca gttataaacc cttatggaaa cagttgatag ataaagatat taagaaaata      60

gatttaataa agagtgctgg tttgactaca aatgctatgg cgaatatggg gaaagataag     120

tatatttcac tccagaatct tgagaaaata tgtagggcgt tagaatgcac tcctaatgaa     180

gttattagtt ttaagacaga aaaagtgaaa aatgaataa                            219

<210>  96
<211>  1866
<212>  DNA
<213>  Listeria monocytogenes

<400>  96
atgaattttt atatgcggtt tattgggaat aaagaatcaa tggtaacaga aatatatgaa      60

ttgttgaaaa ggaaagggct tgttgataaa ggatatacct tattcgatgc ttttactgga     120

acaggtaccg tcgctgatta cttaaaggat tcttttaggt taaaagtgaa tgacttacta     180

aattggagtg ttatttactc taggggaaga attcttgcga atgaatgtac atttgcaaaa     240

ttaggttttg atcctataaa attttttaat agtaacgatg aaataataaa aggctttttc     300

tatgaaaact actctccagg tggctcggaa agaatgtatt tcaacgaaaa aaatgctggt     360

agaatagatt actttagaca aacagtggaa gaatggttta ataaagaatt gattaactac     420

aatgaatatt gctatttgct tgcgtgttta atagaatccg tttcccatgt atcaaacact     480

gccggggtat atggtgcatt cctcaagcgt tgggactcca gagcgcttaa agatatcaaa     540

tttattgagg ttatttccta tgaaaagaaa aatataaaag ctgattttta caattcaaaa     600

cttgaagata taattgaaga tgttgaatgt gacgtattat atcttgaccc tccttataca     660

caaaatcagt acggcacgca gtaccatttg ttagaaactc tggttttata tgataatccg     720

tctatcagcc ccgttaccgg atctagaaaa actgctccaa tgagatcaga ctggtcaaag     780

aactttaaat ctcatattct cctagataaa attttagcta aaacaaaggc taaatatgtt     840

gtattaagtt acaatgtgga tggttttatg tcaaaacagt ttatagaaac ttgctttaaa     900

aggtatggta aaatagagac atatcaatgt gagaaaattt catataaaaa gtatcgtaat     960

tttaaaacaa aaagggaaaa cgaacacttt gaatacctat tctttattga gaagaaggat    1020

attaatgaag ttacctatga atcgccatta aactacattg gtagtaaatc caagatgata    1080

gcagaaatga agcctttaat gccatcagaa atagataaat ttgtagactt gtttggcggt    1140

ggttttaatg ttggcattaa tattaaaagc cagaaaacaa tttataatga tactaattgg    1200

attgtaaaag acttggtaaa gtcgttcagt aaatatgaga catatgatta tttaatgtat    1260

atccaaagaa tgattaaaaa atttggactg gaagcatcca acagagaaag ttatttaaat    1320

gtacgaaatc attataattc attacctatc gctgacagag atccacgtct attgtatacc    1380

gttattttat atgggtttca gcaacagatt cgatttaata gtgattatga ttttaataat    1440

cctgttggaa tgagatggtt taacgatagg gttctagaaa aaatggtgag tttttcaaga    1500

gctattaaag aggagaattt tgaattcaca agccgtagtt tcgaagacta cattgatgag    1560

atagattcaa ataccttcgt ctatatggac cccccataca tgttaactaa cggatcttac    1620

aatgatggaa aaagaggttt cggtggttgg acaacagaac atgaacaaaa acttatggat    1680

tttgcagatg agttaaatag aagaggtata aaatttatga tttcttatgt actagagcat    1740

caaggagaag taaacgatcg aatgaaagac tggttagaat ataggcttta taaattaatt 1800

aacttaaatc ccattcctgg aagaaaaaga aaggaggttg taataataaa ctatgactta 1860

tcatga 1866

<210> 97
<211> 1191
<212> DNA
<213> Listeria monocytogenes

<400> 97
atgacttatc atgaagttcc ccatattgtt attaaaaata actctcaaaa aggtggttat 60

tttttcgagg atattttgac agaagaaatt ttaacagatg tttgtaggaa ggtaacaggt 120

acatccgaat atacagtcga atttgataat gaaggaggat ataataaagg aagactagct 180

actatttcat ataaaggtag taagatatac gtgtctttct ctcaagcagg aaaagttgaa 240

ggacgaaatt ataactttca aagtttaaca actgctctag ttaggtttta tagaggatct 300

aggcatagta gtagaatttg cttctatttt ctcccccaag aaggaaatag agagactgaa 360

tatttttctt ttatgtatag ggtgatggct acagcaggtg ttgaatttat taatgatgaa 420

cagtatttaa cacaaacgat tgaaaaattt gcaaatgttc aggacataat taatgctaga 480

gacagactta gagagggaaa aagaaataat aattcctctt atttaactaa aagtgagtat 540

ggtgtagcag agatatatgc aaaaacatat ggtgcaaaca aaaaggaagc agtacttata 600

tcgcttgcag cttcacatat ttctaaaaaa attagaatct acgagataag ggaacagaat 660

atatctgttt tgccaaaacc agacaaagaa gccttagaaa tgttaccgaa tgtagaaatt 720

ataaatactg atatgcagat agaaataaga gagtttgtag gaagaaatag cttaaggtct 780

ccaagatata tatttaactt attagatagg ttaggtccta aaaaatgcac tttatgtgat 840

tgtgagattc cagaattaat tgaaggggca catatctggc ccgttgctga tattaaagcg 900

gataaatcta taccgaatga tcaaaaactt aattatgcga ttgatggaca caatggaatt 960

tggttatgtg aaaaccatca taaaatgttc gatgaaggtt taattagaat tgaacatgac 1020

ggaacaatta gattaaaaga tgatttgaac gataatgata aaagttttat tattactaca 1080

acgacaaaca cacttttacc tgatggtgta attagtgagg aagcagaaat atatctagcc 1140

aaaagggatg aggcaagtac ttatgaagct tcaaactata ttactattta a 1191

<210> 98
<211> 150
<212> DNA
<213> Listeria monocytogenes

<400> 98
atgtcattaa gtaagattca aggttattta ttttttagcc atttagaatt tttcacaaat 60

ttgtatattg aattttctga aaagcgggac aaacaaaggt tcccactggt atataagtat 120

aaatttgttc catttacata tacggattag 150

<210> 99
<211> 195
<212> DNA
<213> Listeria monocytogenes

<400> 99
atgaagcaaa cagggatgac aataacaaac attaaaaatt ttgctgcatt acaaaagcaa 60

ggagacatga cacttccaaa tcggatagaa attttaaata atcatatgaa aaacttgtac 120

gagcaacaga aagatttggc agaaacaatt tcctttgtgg ctaaaaaagt ggatggatat 180

cgagagaagt tataa 195

<210> 100
<211> 156
<212> DNA
<213> Listeria monocytogenes

<400> 100
atgtatatta aagattttgc cattaaaacg gggctttcga ttgatacgct tcgatattac 60

gaggaagaag aattattaat tcctgctaga aatgaaaaaa attatcgtgt ttatacggaa 120

gaagattatt gctgggtaca gcttttacta aaatga 156

<210> 101
<211> 2232
<212> DNA
<213> Listeria monocytogenes

<400> 101
atgaatgata ttttacattt gaaaggtaaa ttcgaacaaa aagaacgaac aaatagacct 60

agtggtccta aattacctaa aaatgcaatt gtttctatta aggaagtaga agaactttgt 120

gatgatttgt tgaaaatgga aagtttttgg agtacgcaaa ctatttttag tggcgggttg 180

gtcagcgtat attataaaaa ggtagcagca aagagtaaca gaattagcgg ttttctatct 240

tataaaagta attcatcaaa tgatacaatt gtgggagcaa aatttacaga tggaaaaaag 300

aaaaaacata ttattactca ctatgtttca attgaagcaa ttaaagagtc tattctgaag 360

gggaaaaaag caattgaagt tctgaagtca gagttcaatg gcacaataag tgatgaagaa 420

ttgaatccaa aaggcaagct agattttatt gattttgatt attatggaat tgcaaaaaca 480

aatttccaga aaatcattgt ggactcattt tatgtagaaa aatttgatac tgaaaatagt 540

gattttgatt ctaaaaaaaa ttctattgtg acgatatatg atacgggtaa cgatacacaa 600

gaacttttta gtagactggg cataaaaata tttaatgata gaattattaa tgacactaca 660

attttattag atgaaaatta cttagaggtt cttatgcaaa aagcgccata tttgatatcc 720

atggcaacag agaatttaac cgagttggct ccgagtgatt ttatagaaac ttttgatggt 780

gaaaataata taattccaag tccaaatgat gagccgacta ttggagtaat tgatacttta 840

tttgatgaaa atgtttattt tagtgactgg gtagaatttt ataatatgat tgatactaat 900

ataccaactg ataatgaaga ttacagacat ggtacagctg ttagttcctt aattgtagat 960

ggagcaagta ttaatccctc gcttgatgat ggctgcggta gatttaaagt acggcatttt 1020

ggagtagcta caagaaaagc ctttaattct tttaccatta tccgcgcaat taaggaaata 1080

gtagcaaaaa ataaagatat ccacgtttgg aacctgtcat tgggatctaa tgaagaggtt 1140

aacagaaact ttatatctgc tgaaggagca attcttgatc agattcaatt tgaaaatgat 1200

attatttttg taattgctgg aaccaataaa aaaagtagcg aaactgataa acggataggt 1260

gcaccagctg attcaataaa ttcaatgata gtaaattctg taggactaaa taaagaacct 1320

gcaaattatt ctcgacaagg aattgtgtta tcatttttta caaaaccaga tgttagctac 1380

tatggaggaa ctgctgagga ttatatggta gtatgcgagc cactaggaaa agcatatgta 1440

acagggacat catatgcagc gccttggata gctagaaagt tatcttattt gattgatatt 1500

atcggtctaa gtaaagaggt agccaaagct ctattgattg attcagctat tggctggaaa 1560

gacaactcca aatttgatag tttggcaata aaaggacatg gagttgtgcc tataagaatt 1620

gaagatatta taaattcacc agatgatgaa attcaatttg tagtatcagg aatttcagaa 1680

aaatatgaca ccttcaatta tagcttccca gtgccagtgc ataataataa atatcctttt 1740

gttgcaaaag ctacattatg ttatttccca aaatgctcta gaaaccaagg tgttgattat 1800

acaaatacag aattggatat atactttggc cgtataaatg ataaagatgt attagatagt 1860

atcaataaaa acaaacaaag tatagaagaa gaaacgcatt atttatatga agaagatgca 1920

aggaaagctt ttcgaaaatg ggataatatc aaacatatta gtgaaaaatt cagccctaga 1980

tcacaaggga aaaaagtcta tgaaaataga ttatggggta tgagtatcaa aactaaggaa 2040

cggttaaatg ccagtgatgg agaaggatac cgctttggtg tagttgttac attgaaggaa 2100

attaatggtg ttaatagaat tgatgatttt attcaacagt gtagcctgcg tggatggcta 2160

gtgaatagaa ttaatgttga aaatagaatt gatatatatc aaactgctaa tgaaactata 2220

gactttgaat ag 2232

<210> 102
<211> 480
<212> DNA
<213> Listeria monocytogenes

<400> 102
atggcaattg gagataacat aagaaagttt cgtaagctca aaggtatgac tcaaaaagaa 60

ttaggtttag ctctaggctt tgataaaaaa acagctgata ttcgtattgc acaatatgaa 120

agtggaacaa gaaaaccaaa agaagatatg attaatgatt tatcaaaaat ttttgatatt 180

cgtaccgatg ctcttactac accaaatatt gatagctatg tcggtcttat gcatacctta 240

ttttcaattg aggatactta tggtttaaag attatagatg gtgatgatgg catttcttta 300

aagcttgata aaaattcaaa atcattccac actctgtatg attcttttct ttcttggcaa 360

aaagaagctg agaaatttga gaatgaagaa ataactttgg aagaatacaa tcactggcga 420

tttaattatc caaaagtaga agcagaaaaa acaaaatcaa aacgagatca gtcgaaataa 480

<210> 103
<211> 228
<212> DNA
<213> Listeria monocytogenes

<400> 103
ttgcccgaag cggttttaga agttaccgaa tgtgatgcgc tagaaaatat aacaaaggaa 60

atcttaatcg ataaactagt tttgtcaggc gtttggtttt cggaaaatgc caataatgag 120

ttggatttaa cattcgattt caaactatta ccagaagtta gcgatgaact tttagttgtt 180

cgttttaata caaaaggaga aattactgaa ttaacccatg aaagttaa 228

<210> 104
<211> 219
<212> DNA
<213> Listeria monocytogenes

<400> 104
atgacaaaca gaacattaac aaccaagtta ctaggaacac taagctatac gcaagaaaat 60

ggaacgatga tggacgccgt aattcctttg gatggcaaag aagtaaaggt tgattatagt 120

atttttgaaa agcttacttc agaagattat tttaaagata tcgttacttt aacggatcaa 180

attccggaac tacacctaga agcgaaaaaa ctattttag 219

<210> 105
<211> 162
<212> DNA
<213> Listeria monocytogenes

<400> 105
atggaagttg aagctaataa ggaattattg aaaatatgta aaattgaaaa tgaaaacctg 60

ataaaaaaag tgtcagcatt gaattttgaa ttagtaaaaa tgaaagagta taaagatcat 120

ccaataaaaa ctttctggaa agataagatc cgattcaaat aa 162

<210> 106
<211> 276
<212> DNA
<213> Listeria monocytogenes

<400> 106
gtgcgcgttg tattactatc tccaactata cccctccttt taatattgta tctgtcgact 60

cgattacttg aggtgcaatt ttccccaata ttaatttgct ggcctttagg aataattatt 120

tctgcttcgt acatgcgagt attcttccat ccaggtaata atgccatatc tatttttgcg 180

tctattcgac ttattgtctt ctctgaagtc acaaaacttc ctgtagactt tgcatttcca 240

ccaaatactc tctatacacc gttatttcct cagtag 276

<210> 107
<211> 180
<212> DNA
<213> Listeria monocytogenes

<400> 107
ttgatgcctg ccgttattaa tttgacaatg ggaatatgga tagggtttga ttttatattt 60

ataaagattt ttagcctatt tattgctata aattccttat taccaattcc ttacttacaa 120

accgatggct atcttatgtt taaggagata caaaaaagag ctgttttcaa aaaaaattaa 180

<210> 108
<211> 126
<212> DNA
<213> Listeria monocytogenes

<400> 108
ttgctaactc aaggtaaatt agtactacta actatccata atgaaacttt tctaaaagaa 60

gtatacaaca atctatatat tattgaaaat ggcgctacca aggaagcata tgaagcatgc 120

tactga 126

<210> 109
<211> 870
<212> DNA
<213> Listeria monocytogenes

<400> 109
atggcacaaa ttattaaaaa tccgctagta aaaccttttg taaaatgggt cggcggtaaa 60

aagcaattga tggattacat caatttatat aagcctaaat cgttcggtag atattatgaa 120

ccgtttgtgg gcggagggtc tgtttttatg acttttcaac atcctaaaac aactataaat 180

gattttaata atgaattaat aaatgtttat gaagtagtaa gggataatgt agagatttta 240

atacaagaat taaaagttca tgctgataat aattcaaaag aatattatta tgaattaaga 300

gaatgggatc gaacaggggt aatggaagaa aagaatgcaa ttgaaagagc tgctcgtttt 360

atttatttga ataaaacatg ttataatggg ttatttcgtg tgaattcaca aggacagttc 420

aatgtaccct atggacaata taaaaatcca aacattgtaa atgaagaaat attaagagca 480

gatagtaaat ttttaaaaaa gtctactata aaaataataa atggggactt tgaactagct 540

gttaaaaatg caaaaaaagg tgactttgta tactttgatc cgccatacgc tcctttggta 600

gaagatactc aaagttttgt aggttataca ttaaatggat ttggctatga agagcaagct 660

agacttagag atttgtttgt gaagcttgat aaaaaaggat gttatgtaat gttgtctaat 720

tcttcatcta agattattca tgaactatat acagattacg cagaaactac tgaaattgtt 780

ggtgctaata ggatggtcaa ctcaaaatca tcaggcaggg gtaaggtgga tgaagtattg 840

attatgaatt ataattatag agaacaataa 870

<210> 110
<211> 1290
<212> DNA
<213> Listeria monocytogenes

<400> 110
ttggctaaat taaatcagac agatgaagcc tgggaaagat tgtttgagaa atataatata 60

ttagaagtta tagaaagaga tggagtattt ataatttctg cgcagcaaat caaggaatat 120

agagaagcaa ggttaatgac taagtttgat cataaagcta atttacctaa tttatttaaa 180

aataatgact tgagcattct tccgaattca agaggaacat atgttatagg gaaatttaat 240

gcttaccagc agttagtcgt tgatgataaa aaacctattt cagtagagat cccctattat 300

atagaatcat ttgataattt atctattaca agtgaaagta ccgctctaaa tgtggcgcac 360

tttacaggaa tgattgatat ggttatggaa acaggaaatg atgaacctaa ttccgtactg 420

acgctttctg gaagaatgag ttcaggtgaa atccaatatc aaattaaagg tgagaaaaat 480

ttctcttatg attttaatgt gaataatagt caaatagaaa tagacggttc ttatgaaaat 540

cttcaaaaaa tagctttaat tgaagcgaaa agtaagcttc cattagattt tcatataagg 600

cagttatatt atccttatag agtttatagt aatttaatac aaaagaagga aattatgcct 660

gtttttttca cctttgcaga tgatattttt agttttcata tatataagtt taatgattta 720

aatgactata atagcattca aaaggtcgaa caatttagtt ttattttaaa caccgtatta 780

gatttaaaca ttaatgaggt taaaaaaata agttgtaata gtccacaatt gcatagtacc 840

gaaggagttc cgtttcctca ggcaaatagc atgggacgga ttctctctat tcttgagtat 900

ataaaaatac ctatgaataa aaatgagtta gcaaaaattt gtgagtttga tattcgtcaa 960

agcgactatt atagtaatgc tttgatctat ttgggttttg caaaaagaaa tgaaaagagt 1020

gaatttgtac acacacaact aggttctaag atttctaaaa tggcaaataa taatgagaga 1080

aacaaaataa ttattgagag tatccttaaa tttaaaattt ttaaaaagat atttgattca 1140

ataattataa atggaggaga gtttgatact gaatatattt tcaaactagt tttagttgat 1200

gggggaaata gaatctctga atcaacaatt aggcgaagaa cgagtactgt atgtgcatgg 1260

atgaggtgga ttttttctgt tatagaataa 1290

<210> 111
<211> 339

<212>  DNA
<213>  Listeria monocytogenes

<400>  111
gtgaacggaa ttctgcgttc tgaaaaaaag tatattatag atggtgatta tttctttaat      60

ttggagaaaa gactcgaaca tgcagattta gttatttgga taaaaattcc attgtttctg     120

tgtgtagcaa atatagttaa aagaagattt aaatacatga cgaatgcgcg accagatgta     180

actgaaggct gtgacgaaaa attaaattta tcatttttgc tgtatgcttt aaagtataat     240

aagcgttctg gaaaacaaac gaaagaatta ttggataatg tgtatgataa agagctgttt     300

gtaattgata gttataagaa attgaagagt tattgctga                            339


<210>  112
<211>  138
<212>  DNA
<213>  Listeria monocytogenes

<400>  112
gtgttagtat tgctaaagtt aagcaattca ttgacgaaat taactaaccc accactcggt      60

gggctttttt atgcaaaaaa acacgctaaa cataagctta gcgtaattgt tatatcaatt     120

aatttctatg gaaaataa                                                   138


<210>  113
<211>  282
<212>  DNA
<213>  Listeria monocytogenes

<400>  113
atgaccgatt taaattcgat actaataaaa agaataaaga tattgcgcaa acgtttagga      60

cttagtcaag aaaagtcatc attagcagct tcgctagatg ctagatatgt taataagtta     120

aaaaatggaa agtttaatct ttcagtccct acatttgata aactgattga agcatttgga     180

atgtcttata aagacttttt tgagattgag aaagataacg gactagagaa acttaaaaat     240

tgtctcctaa agaaaaagag gatttgctca ttcaatgtat aa                        282


<210>  114
<211>  558
<212>  DNA
<213>  Listeria monocytogenes

<400>  114
atgagtgatt tacagcaaaa agaaaaatta gagatacaac ttcgagaagc atatggacgc      60

gttacttata catatacttc acatttgaag tttatgaata ggcttaacca gaaaaataaa     120

ctcttgagat attcacaaat cactctatct gccatatcaa ctggaggctt cttaggtaca     180

ttcatatttg ataaaatcat attaaccact attgcaggtc ttgtttcagc tgttttactc     240

gcgttaaacc ttttctttaa aaactttgaa ttagacgagg agtcaaaaca acatagggtg     300

gctgctgata aattatggtt aattagagaa aaatacgtag cgttattgac ggattttgaa 360

actctctcaa taaaagaaat tgttgtaaca agagatatac ttcgggagga aacatatata 420

atctattctg agtctcctaa aacagacaag aagagttatt cagaatcaca gaaagctcta 480

aagaatgaag aagaacaatt ttttaccgaa gtggaacttg acaaaatgtt acctttacaa 540

cttagaaaaa ataaataa 558

<210> 115
<211> 816
<212> DNA
<213> Listeria monocytogenes

<400> 115
atgagtattt tagtaacaaa ttcgtatgtt acaccattag aagcaagaca aaccatagct 60

agaagatatc gtattgtgac taaagcgatt aatgtagagt tttggaattc aattagtgaa 120

acagctcata gtttttacgt aggatcttac ggacgtggaa ctgcaataag tacaagtgat 180

attgatattt tggtagaaat tcctaattca gagtatgata aattcaattc gtctactggt 240

aatggccaat cacgattatt acagtcaatt agaaaatcac ttcaagtagc ataccctcaa 300

agtgatataa gagcagatgg acaggtggtt aaaattaatt ttcatgatgg aataaaattt 360

gaaattttac ctgcttttca gaatatagat tattggggta aaaatcaggg gtatatctat 420

cccgactcaa atatgggagg gaattggaaa gcgactaatc caaaaaatga acaagaagct 480

atgaagataa agaatggtcc aacatatagt aatgggctgc tttatgcaac gtgtagacat 540

tttcgttatg ttcgtgatac ttacttcagt agttatcatc tttccggcat agtaatagat 600

agttttgttt acaatgcgat gggaaactgg agatatactg aatctggaag tagttctaat 660

gcaagtatgg gagcttatga gaacatctta ttagagtatt ttaataataa tacaatttgg 720

ggattaagtt tgaattcgcc aggtagtaat caaactgtaa gcactactaa tagtattaca 780

tgtctagaaa aagtgataaa gaagattgca acttaa 816

<210> 116
<211> 1119
<212> DNA
<213> Listeria monocytogenes

<400> 116
atggataaac gaataatact tgccgttgca ggttcaggta aaacctatca tatttgtaat 60

gagttaaaac cgctgaaaag aaatctaatt atagctttta ctaatcaaaa tatcaaaaat 120

ataaaggatg agttaattaa gatacatgga gatattccta aaaatactag ggtcatgaca 180

ttctcaaaat ttatctataa tttttatctg ctaccatatg aatcattgat tcaagagcaa 240

ttttttgcta ctgatttcaa tagtgatggg gtatatatgg cagactcacc agtaaggaga 300

ttgaagaatt ctaaggggaa agaatatacc aatcctaact atataaagca ggaagaattt 360

gagcacttcg tcaaatttat tagtaaatat aaatatcgct attatgtaga taaattttca 420

aaattagttt tgaaaaccaa agatttatat aagaaaggaa ctgacaatgt aagcttcttt 480

tttgataaac tttacattga tgagttccaa gattttagag aagacgatta taggttactc 540

gaaaaattaa taaaacgatt taataaagtt ttactagttg gagattatta tcagcattct 600

gttaatggaa aaaataatag tggaaaacca ataaagaaaa acatgaatta tagtgagtac 660

aaaatattat tagaaaaatt aggattagag gtagatgata ttagtttatc aaagtccaag 720

agatgcccag ctaatgtgtg taactacgtt tctaataaat tatcaataag tatcgaatct 780

gactctgaat ttgctggtga tggagatgtt atatttatac aaaattgtga agaggcaaga 840

aatatattga gtgactcaac tatagaaaaa ttaattttta gtggagcgaa caagtatagt 900

tttgaggcaa tcaattgggg atattcaaaa ggagacacat ataaaaatac atgtattatt 960

ctaactggga attttgaaaa cattgagaac actgatgtta agtataaagc tgacagtact 1020

ttgaataaat tatatgttgc tttgactcga acaaaaggaa acgtgtatat gctaaagaag 1080

agcatatttg atcaaataaa aaaagattat atacaatag 1119

<210> 117
<211> 1584
<212> DNA
<213> Listeria monocytogenes

<400> 117
atgaatcata ttaaaagaat gcatatcaaa ggattgaaaa aatttaagga cattgacatt 60

gagtttaacg agaagattaa tattttagtg ggtgaaaatg aaagcgggaa aagtactata 120

ctagaggcta ttaatatagt gttgaatcaa caatatagaa gtatagataa gtatatatta 180

aaagatttga tgaatataaa ttctatgaaa gactttcata aaaatccaca agtaaataat 240

ttgcctagta ttacaataga aatagaacta gaattggata accaagcgaa tgtcgatact 300

atttattttc gtggtataaa tgataaagat aagggacaat ttgaaaagtt tgggatctta 360

tttgaatgtg aatttgataa ggaattcgaa gatgaattac gaagtgaaat cgaagatggt 420

gtgattccgt ttgaatacta taaattaact tggagtacat tccagggcag ttcgtataac 480

attatgaaaa agccattgcg tacgatttcg attgataact caattgcaga ctcaaatagc 540

tcatttaatt actttaataa gtcgttgttt gataataaat actctagctc agataaaatg 600

aaagcaagaa atagttttag aaatgaagta gatttaattt ttaatgcttt aaatttggaa 660

gggctaggga ataatcgcca gtttggtatt aatagtaaaa agacaatttt ggataatgtt 720

cttacggtat atgaagaatc aatttctctt gaaaataaag ggaaaggcat ggaaaatctg 780

ataaagacag aaattgcatt aaataaatcg aaaaataagt tagatgttgt attgttggag 840

gagccagaaa atcatctttg tcattctaat cttttgaaaa tgttagatga aattaaaaag 900

aatactaatg aggcacaatt gatattaact acacatagca acatgattgc aagcagactg 960

gacttaagaa atattctttg gattggttct gatgaagcta tcagtttaaa taaaattgat 1020

caagaagatg ctattttctt tgaaaaagcc gaccaaaacg gaatcctgaa cctcctttta 1080

tcaaaaaaag taattttggt agaagggcca actgaatata tgttattgcc gattatgtat 1140

gaaaaaataa ttggaaagtc tatggaggaa gattttataa ctataatttc atgcaatggt 1200

atagcttata aaagatactt agcaattgca gagtctgcta agaaaaaagt ggcggttata 1260

actgataatg atggtaaaca gaacgtcata gattcaaagg aagaatacaa taaagataaa 1320

gttttacaaa gaatatttac tgatggtaat ctatctaatt ggacatggga aatctgtatg 1380

tatgagttga ataaaaatta ctttgaagaa catgttactg tgaataaaac ttcaaagtat 1440

ctatttaata aacaggacta tggtaaagta aaaggaaaga tgttaaataa taaggcagag 1500

atagcctatt caattctaaa tgatattgat gatttaaaaa ttcctgaata tgtaaaagga 1560

gcttttacat ggataaacga ataa 1584

<210> 118
<211> 2382
<212> DNA
<213> Listeria monocytogenes

<400> 118
atgaagatgg ataagaattt tggaagaacg attatcaatt ggttattttc ctatagtgat 60

gagaggagaa tgatgggtta taatatttta tctcttaaaa aatctaagga tgaattactg 120

aatgtagttt caaatttaga aaaagagata agaaatttaa aggaaaatca agtcaataat 180

gaaagtgaac aaaaaaaatt aagtatgctt gtgaacgaat atacaaatag atttggtact 240

attagtaata caaacaattt tgcttcagat gttgtgagga tggaagatga agatgctaat 300

atttttcatc atttagtaag tttaaatgga ggtaacgaat ttaacaaaga gcaagtaaat 360

gctattcgat ttgatatgag aacacatctt aggattatcg ctggtgctgg aagtggaaaa 420

acgcaaacaa tttgtgcaaa ggcagcttat ttagttttga aagaaaaaat tcaagaacga 480

aatatagtga tgtgcacatt ctctaaaaaa gcaagccaag aaatgaagga acgtgtcaac 540

gagtttcttg gtggcaatag tcgaatagaa gtattgactt tccatggatg gtttcaaaaa 600

gagtacaatg tacttattaa aaaatatccg tatctagaaa agtatgggat agcgggagag 660

gtacccggaa aaggctctgg aaaagatagc catgatagga ttttaatgaa tttaattaag 720

cagtatcatt tatataattt tgataaatat gaagaaaaac caattaatga acgacttagt 780

tattggcgaa atatgggata taatgatgag gaaattgttg cttttatagc aagatatttt 840

gatgacaaag aaatattaaa ggatcaacta cttagtgaag ttttttcaga gatgttaaaa 900

gaattggaac gaattaagag aagagagaaa gtcattaatt ttgatgatat gttaattaat 960

ttaaagtgtg tattggagaa cgactcaaat gctctttctt taatacaaac tcgatatcaa 1020

tatatattta ttgatgagtt tcaggatatc aatcccttgc aaaaacagat aattgaactt 1080

atttgtccac cagattacga gaagagtgtt atcaattcta ctaaattaat tattgtagga 1140

gacgatgatc aaagtattta tttcttccgt ggtgctgaac cacgttacat taaagaattt 1200

gatagacaat acaggcaaac gtcaatagag ttaatgacta actatcgttc tattgctcct 1260

gtggtacata taggaaatac tctgatatca aacaatcaac atgatagaat aaagaagtct 1320

atgatacctc acaaactaaa tgaaggtgat tgttacgcaa aagtatttca aaatgaacag 1380

ctagaagcta attggattgc aatgcggata ttaagcttgt cttcagaaga gaaaccattc 1440

caagatcaaa ttggtaaacc gaattatacg gaaacaatcg tattataccc taataaaaat 1500

caattaaaat ctatgattat tgctttacag gaaaaaggaa ttccctttgt cacaaaatcg 1560

gatgatgatt tacttggtat atttggtatt aattttttta aacgatcatt caatctatta 1620

atggatatta ttgatgcaga aacgatagaa ataaaaaagc atttatacaa gcaactcata 1680

aaaaatattt gtttatatta ttatatacca tttgcaaaaa gtacagattt tgtctcagat 1740

gagactaatt atacttcgga aaagttaact gattttatat gcaaagaaaa gaaagttagt 1800

aaaaaagaga tggcacttat taaatccata ttccgagata ttaatgaatt tataaaaacc 1860

gaagaattga atattaaagt ttttgttaat tggcttgtag aaactccgaa agtaaagaaa 1920

caattaattg atgaagagaa agattggttg cttaaagagg ttagcaatgt taaaacgcta 1980

aaagagttaa tacttaaata taaagcagcg agaaccttaa aagaagagat ggctgacaaa 2040

ttaagagaat atgataataa taactataat gcagtgtatc tattaacaat tcattcaagt 2100

aagggattgg gatataaaaa tgtttttgta aaaggtattt ataaaaactc actaccagac 2160

tatcgttcag tggataaaga aagtattgaa cttcagccgt taattgaaca agcatctcca 2220

ccaacaacaa tggaagaaca aagaagatta atgtatgtag cgctaacccg agcaaaagaa 2280

aatctttatg taacttaccc tagagaaata aatgataagc ctacacttgt gtcaccattt 2340

ataaaagaat ctaatattat gtttagtgaa gaaaaaatat ag 2382

<210> 119
<211> 450
<212> DNA
<213> Listeria monocytogenes

<400> 119
atgaatttac taagtatcaa accggcactc cttaactatc acacagcgat agttgccaaa 60

ttacagaacg accaaccagt cattctggaa aacctaagta ttgatgcaaa agtctatcaa 120

caaaattcgg ataaattact ttaccaaaca aagaaagcga atatgaaaat ggcgccaaat 180

tccaattttg attttgggat tgacttggaa aatcaacctt taaaagaagg taaatacaga 240

ttaaaaatga ctgcaacgaa tggcgtggaa acctggactt gggacgaagc gttcaccatt 300

ggtaaagaag gacaaaactt aaataaagaa gcagtaaacc tagagaaaac gaacacatgg 360

ctttacgttg cgattgttgc aggtgtggtt cttatagcac taatcatcat tttggttata 420

agaaaacgaa gacaaaaaca agaaaaataa 450

<210> 120
<211> 564
<212> DNA
<213> Listeria monocytogenes

<400> 120
ttgaagaaaa gttttcttag tttattgttc attattccgt tattagttac atgtagcaat 60

tttacagaag cacaggcggc agaaggagac gtcggttatt ccgttcaagc tcatattccc 120

gccaatcaaa tagataaaag acaaacttat tttgacctaa aaatgcagcc gaaacaaaaa 180

caaacagtaa acattgatgt aatgaatagt tcaaatgaag aaattcaagt ggaagcagca 240

atcaactacg catccaccaa tcgaactggt gtcattgatt acaccaaaaa tgatcttacg 300

aaaaaagata aaagtttaga atatccactt ccagaactag ctaaaattcc agacgaccaa 360

aaactcctaa cgataccagt agatggcaaa aagaccgtcc aagtgatgat tgaaatgcct 420

gctgaatcga ttgatggcgt tgttttgggc gcagtagaat ttaaaaagaa aaatactaac 480

gagacgaaaa aaacaaaagg tgtctcctta aaaaatgaat attcctatat tgtcggcatg 540

caactagctg aaaccgacaa ataa 564

<210> 121
<211> 453
<212> DNA
<213> Listeria monocytogenes

<400> 121
atgaataaaa taaataaatt tagcattttt tctatcacta aaccaggtat ttacacaatt 60

acaggtagta acggaagcgg aaaaacaacc tttattgaaa atgaattaaa aaataacaca 120

aataaagtaa aagatgtagc ttactttgcg caaaaaaatt ggaaatataa aactagtgta 180

gagaaatatt tacacttccc aaaaactaat ccgaacttaa ttcaaaagta ttgcgagttg 240

ttttctgtag ataactacta cttagaaaaa gatatccaat tattaagcgg cggagaattt 300

gtcaaagtag aattagtgag aacattagct ttggactctt ctataattat tctagatgaa 360

cctacaaata atctagataa caagtcctcg gagatacttg ctaatatttt aagtgaatta 420

gcaaaaacga aaattattta cttagttagt tag 453

<210> 122
<211> 627
<212> DNA

<213>  Listeria monocytogenes

<400>  122
atggctaaga ccatccaaaa caaaaccaaa gaaaccttca cacttccacc gcgtgagaaa      60

ctcgcctttg cgggaggagg cagtatcccg gaacaaagcg ccactggagc cgcctccata     120

gctgcgaaga aaaagctgaa agatatgatg cagaacatgg ataatttgaa tgtgaagggc     180

ggcggagaga gtggtacaat agaaaaaatc ggtaaaacta atgaactgaa aagcccatcg     240

tcaattgcaa ggagttggca agggggcggt aaatatccag gagtggatga ctatgaggac     300

attgtattga aagtggggga tgtcatttac agaggagagc caaatggatc agaatacttt     360

accactaagg aagtcataga aaatgctgat attagtgcga ctaaaatttt tgaaggatta     420

caagtcgaaa aacatcctat ttatggctat cggaaaagta tgacaggata taaagtgaat     480

agtgaagttg atgcagcatc tggatttacg aaagctaatc cacaatttgg tgagggtgga     540

gcacttcaag tttttgttcc aaatgtaaat gaattaatag aaaaaggcat tttaatacct     600

attgatgaga taaaattaat tgattga                                        627

<210>  123
<211>  522
<212>  DNA
<213>  Listeria monocytogenes

<400>  123
gtgaaaatag acttagagca ggtatacatt ttagaatcta ctatgagatt gaggtatagt      60

ccgttacaac ttagacataa tttgaaaata ttttttatag ctctattgtt tgttatgtgg     120

tttcaaggtt ttattatggg agtcttacca ggagattttg ctggagaaag aattaatatg     180

atattgcttg ctatattagt aattttgtat attttattat atccaaggaa aataactata     240

aaatttcaaa atttgcaata tgtgctagca atttgtgtat atcaattgac aatatttatg     300

ggaacaaaaa tattttattt tgcaatattt tatactttaa aatatgaatt tagagttgat     360

tttgcagtta gttctattaa attagcatat atagccttat ttgttatttg gtttttaacc     420

tttatagttt ttagcgttca ttttagaaga aggttaataa gggggatttt tagagaaggg     480

tcagctttac aaaaaaacgt ggtaatattg gccttaagct aa                        522

<210>  124
<211>  108
<212>  DNA
<213>  Listeria monocytogenes

<400>  124
atgtatagaa aagctaaaga gttatcagtt ttagaaaatc ttaagaaaaa gggtgatatt      60

aaaataacta aatatggaca aaaaaatgat gtttatcata gtagctga                  108

<210>  125

<211>  1143
<212>  DNA
<213>  Listeria monocytogenes

<400>  125
atgattttcc gaacagcaga actattcgct ggaccaggtg gtggtgctta tgctgctaaa      60

acagctaaat atgtagatag agaaggaaat aattggggct ttgaacatgc ttgggcaaat     120

gaatatgata aagatactgt ggaaacatat aaattaaaca ttttaggtga ccctgaagca     180

acaactgttt attgtcaaga tgttcgaaag tttgatttaa ctaatcataa gttactatct     240

gatatagatg cactaatttt tggctttcca tgtaatgatt attctttagt tggcaaacaa     300

aaaggtttag atggagagtt tggaccttta tattcatatg gatctaaagc attatctgaa     360

tttcaaccca aagtttttat tgctgaaaat gttgggggac tatcttctgc aaatgaaggc     420

gaagcattta ttcacatttt aaaagaattt cagaaagcag gttataaatt aacccctcat     480

ttctataagt ttgaacaata tggcgtccct caagcacgtc atagaataat aattgtagga     540

atcagaaatg atatatatga agaaggcata acctttagag tacctgcacc tatcttcaaa     600

gatcatacag aatataaaaa agcatatgat gctttaacta atccgcctat atccttaaac     660

gcattaaatc atgaatttac caagcataat gctaaaactg aaaaaatgct ttcttatatt     720

ccagaaggag gaaatgcttg ggtggaatct attccagaag ctttccgttt aaacgtaaaa     780

ggagctaggt taagcaatat atataaacgc ttgcatagaa acaaaccttc gtatacggtt     840

acaggttctg gcggtggagg cacacatatg tatcattgga gcgaaaatcg tgctttgaca     900

aatagagaac gtgctagact ccagactttc cctgatgact tttactttca aggaggaaaa     960

gaaagtgtac ggaaacaaat aggtatggct attcctcctg acgggttaag acctatactt    1020

ttagctgtgt taaaatcttt tgcacaagaa gattatgagt ggattgaacc tacaaaaaaa    1080

ttacaaacag atatactttt taatgaatct ggtaaaacaa ttgctactat agtaaagtca    1140

taa                                                                  1143

<210>  126
<211>  351
<212>  DNA
<213>  Listeria monocytogenes

<400>  126
atggataaat ctaatttaac aatgtttgga gaggaattca tcacttcctc gcgagataga      60

agtattcgac atttaaactc cttattaaac caagaaatca aagccccctc gctacaagac     120

attcaatata aactttcaac gatgaacgag gaggaaaaag aatttataac ccttttagga     180

gtaatgatag ttgataatac tctatttaat gtgttaacca tgtttgagca atcagaagac     240

aagttaactc tcttagcgaa tcatgaaaac attgtcgaaa ctagtgacgg acttgctggt     300

gagttgttta ctgaagacgg ttggatatca aagttcagtc aggttcaatg a              351

<210>  127
<211>  591
<212>  DNA
<213>  Listeria monocytogenes

<400>  127
ttgtacaaaa taacaggtga gatcaaagga aatctcctta tcgatacaaa ttccccgttg      60

gcgctcggga ccccttttaa ggcatttacc tttgctactc caaaaaaact gatagacagc     120

tttatagaag ctaagacgaa ggagaatacg gcgacattaa cgatgtcaga aacaaataat     180

aagacaacga cagctggagg cgttacaaca acttctgtcg cttatgaatt accgcatatt     240

caagtcgaaa gacagacggg tcatttgttc tcggactatc aagtaaaagc aaaagataaa     300

cgaattcagt ttgcgatgcg tgacggtggc cacaaatttt atttaccgat ctggatggat     360

cttggcaagt atgacacgac cacagctact ggaaaacttg ggacacatgc cattcaagtg     420

aattggaaag atcaaatcaa tgtagaagca tttatgtacc ttacaatgga ctctaccacc     480

aaagataaag atgcgattat gttaaagcca gtcaatggag ataacccatt tccggaggga     540

ttaccaaagg gctggagcaa agcggaagaa aactggatca aaagtcatta a              591


<210>  128
<211>  132
<212>  DNA
<213>  Listeria monocytogenes

<400>  128
atgagagtac ctagtggtaa tgaaccagga gggttcacaa gtggtggaat acctgaagct      60

gttgtaaata caattaaaag ggatgaatat attgttaaaa atatatttaa atgcttgggg     120

gataaattgt aa                                                         132


<210>  129
<211>  135
<212>  DNA
<213>  Listeria monocytogenes

<400>  129
gtgattttgg aatggtatac aatttttttg tttcaaaagt tgaaaaagtt aacaaaagaa      60

aatgaagttt gtgaaaaaca catcttttta tatagaaatg ttgttatgga aagaccaatt     120

atcaaggctt tgtag                                                      135


<210>  130
<211>  405
<212>  DNA
<213>  Listeria monocytogenes

<400>  130
atgaataaat ttaaaatcct aggtgaatat aaagactggt gcgagattta taaagatgga      60

accctcattc ataatggttc atctttaggg attgtaagcc aagtagagtc tgaactttgt 120

ttaaggctaa attatggatc aaataaacat ttctattcgg tattgaaaaa acgtggtgat 180

tttatagtag ctgtccctaa gaaagtagag tttttaaaag ctgagtacaa atatgaacct 240

attattttta ataaacaaga atttgatgaa tttattgatt gtatttatgt tgataaaaat 300

ttgatttcta gtgtacttca aataagtaag gaagatcttt tgaatatatg gttcgtatca 360

aatccgcagt ataaaaccta tatcaatgaa atggaaatgc aataa 405

<210> 131
<211> 306
<212> DNA
<213> Listeria monocytogenes

<400> 131
atgaagtggt cggaatgcat ctgtatactt tttagtgatg accggattat ttgtcggaat 60

ttcgctccac tatttgacat taatgaagaa gctgcgactg ggacctctaa tggtgctttg 120

gcctgttatc tttatcaaaa tcaatacttg caaaaagaac tttatgtatt tgaacaagga 180

cactcactac actctccttc cgagatttta gttaaattag atgtaaatgt tcatagcgaa 240

atagcccgtg tatatgtagg tggaaatggg tattattgtg aaactaaata tttaagtatt 300

gaatag 306

<210> 132
<211> 456
<212> DNA
<213> Listeria monocytogenes

<400> 132
atgtataaac ataatcctga ttttcgtgtt ctcactaaca aatccgaaca tccaattcca 60

attaaatata tgtttaagtt cttaaaaaga aatgtcctgt ttcaaaatca aaatacatta 120

aaatattctt atatttacta ttgtcaagcc ttcttatcag agaccaataa cgcttccgtc 180

cttagactat cattcaaatg tccattgaca gtagataatt taactattta tccatcttta 240

attgtttcca aagctcatat tgaaaatgag tatcctgata tttatgatca atttgttagt 300

ggaatagaaa cagaatttga agttttcaca acattacctt ttttaaaaaa gtacgtttca 360

cctagtaaaa tatacatcaa tttttcttct tttcaagaat cggctaatgt cgatcctttt 420

agtgatgaat tattttacaa cttatacatt aattaa 456

<210> 133
<211> 507
<212> DNA
<213> Listeria monocytogenes

<400> 133
ttggcccact taattacaac agcaattcca actgatgaag tagcaatttc atttctaaaa 60

tcaaatcaag ttaaatatcc aagtattgat ataactccat ttgttgcaaa agaattgtac 120

aatatttata ttttcagtaa acgtcatttt atttgtccta ctgatgggtg tgatgcacca 180

gtcacttgtc gatcaattga tccatctagt aaaaataaac ctacatttgt taatcagtca 240

ctttcaacta ataaacattc atctaattgc ccttatcatc cgaataacta cctctcagta 300

gtcacaaatg aagctccttc tggtgagtcg ttcaaatatt taaatagtgg cgaaatcatt 360

tctgatcttt ctttggctca tgggttccaa ccattggata atactacaac tacaagaaca 420

tcaagttcgc ctgtattcac atcgcctact ccaaaaaaat cgaaaaaaac aacatcttca 480

aacagtacta aaaaagaaaa aaagtaa 507

<210> 134
<211> 1095
<212> DNA
<213> Listeria monocytogenes

<400> 134
atgtattctg aaatactaaa aattattgag ggtggcatct cacatgataa acaaaaggtc 60

attaattatt caaaaaggtt agctgagtac ttaattaaag atggccaaaa aaatgttgga 120

aataagatta ataagcttat tgcagaaaat aatacgagtt taacaacttt ggacggatta 180

agttcaaagc cttttgataa agatactaaa gttgatatgg ttgatatttt tataccaact 240

gaatctaaag agagtttgat atttaatgaa attgtggaaa atgaagtact agattttatc 300

aattcatatc tgaatagaga taagcttata atggcaggaa ttgattttaa taatcatttg 360

cttctgtatg gaaaaccagg aactgggaaa acatcattgg ctaaatttat cagtttgcaa 420

acaggattac cacttatcac agcgagacta gatggaattg tttcatcact tttaggcagt 480

acagcaaaaa atattcgtaa agtttttgat tatgcttctc aacgtccatg tattttattt 540

ttagatgaat ttgatgtttt agctaaaatt cgagatgata agaatgagtt aggtgaattg 600

aagcgagtgg taaatagctt actacaaaat atggataatt ttaatgagga cagtattttg 660

attgctgcga caaatcatga gaaactgctt gatgaagcag tttggcgaag atttgataaa 720

attattaaat tagatttacc agataaaaaa ttaagaaaag acttaatcct tgagtattcc 780

agcgttatgg aaactaattt ttctgatgat caaaaaaaat tgacagaatt agcagatgtt 840

acagatggat attcgccagc agatataaaa aatgtagttc tatcagctgc aaaaaaaaca 900

ttaatacgta atgatgaaat tttaaactat tcacaactct tatgtgaaat cttccttaga 960

gagcatattg gagaggattc tatggatgaa cctataaaat acttgctaca acacaaagta 1020

tcacaaaaag aagtatcagc tactttaaat gtttcattaa ggaaaataag aaatttatta 1080

aaggaggagg agtaa 1095

<210> 135

<211>  2337
<212>  DNA
<213>  Listeria monocytogenes

<400>  135
atgtcagata aacatccaat tattctcttt caacagagag aagtagacaa tggaaggact      60

ccaggaggtg gggattccaa aaagccaaag tgggtattaa caggagcgga actgcaagcc     120

catagccaag aacttagttt agaaatagat tcaataaata gttcatggga tttgaatgag     180

gagataggtg taccaaaagt tgtcagagtc gaattcatta aaaaggcaaa agcaaaatca     240

catcaatcta aaattgttga tttattctca gtcactgaaa atttagcgca aatagggatg     300

gattctgaat attcaatact tttgaaaatt ccagattcag aaaatatgaa catgattcat     360

tctcgaattt tggatattga aagaaatgat attacgattt cagctattga tacaatttca     420

gagtttaaac cacgtttgga agctcataca agacaagctc aacataaatt gactttttta     480

gattttcgta atcaaaagtt aaatttaaat gcaaaaaatt atactttaaa aatgttagaa     540

aaaaatagta ttccgtttaa atttgaaaaa tatggtagtt cagacatagt tttgataaga     600

gaaagtaatt ttgataaact tcagttcatt aaaaatctac cagttaaaag cattgaacca     660

ttggaagaaa cgttgcctcc ctttcaattt ttaagcgttg atttttcggt aaaggaaaat     720

tttgttagtt ataaaaatga taaaatctac ccagttgtgg gtttgctaga ttcaggtgtt     780

gaactaaatg attttacgaa ggagtgggta actaaaggaa atggctcaaa ttattcagag     840

aatgatctta atacggatca tggcacatat atagcatctt tgcttattca cggtgataaa     900

ttgaatggtt atcatgattc agttgtaatg ggatgtaagg ttgtggatgt tcctgtagtt     960

cctaataagc ccatatctgg tcctgaactt attaaaaata ttgaagatgc aattaaaggt    1020

aatcctgatg ttaacatttg gaacttatct attagtttag ctggtgaaat tcaaaatgat    1080

caattttcgg attttgcagt tgaattagac cgcattcaaa gtgagtatcg agttttaatt    1140

tttaaatctg caggaaatga tgcttcattt tattcaggag gaatagctgg taaattaagt    1200

atcggtgctg acagtgttag agctgttact gtaggttctg ctaatagaaa ttcagatgaa    1260

tttggttata cactagcaga gtatccttct ccatattcta ggataggtag agggccagcg    1320

catataatta aacctgatgt agttggttat ggtggtgatg tatttgcatt aaaagaaaat    1380

ccatcagcaa aaaatgaatt tgaagtgatt ggagagcttg gcttttcagg tgattgtgta    1440

gaagaaagac aggtaggtac aagtttttct actccgaaaa ttgcgaaaac agcagctgag    1500

ataagtatac ttttagatca agaatttgat ccacttctaa tcaaagcttt acttgttcac    1560

tcagcaggtt acagcgaatc agcaatgatt aaatgggaag aaaaattaca aaaacttggg    1620

tatggaaagc ctaaaaatgc agaatttatc cttgaagaag aagatcattc cgctaccctt    1680

ttacttcgag gaaatcttaa aaaaggtcgg aatattgata ttatggattt tccgtttcca    1740

gagaatttag ttgtagacgg gaagtttatt ggtcgaataa aggctacgtt ggtatacgat 1800

ccaattctaa gttcggggtt aggcgatgaa tattgtcaat caaatttatc tttaaaattt 1860

gggacgtttt cagaaaaggt tgatagggaa ggaaggtatc agctgtttaa cccaattggg 1920

agattggata caaagaattt attgaatact gatttatatg ccaaaaataa aatgaagcaa 1980

aacatttcat atggaactga acgattacaa attaagtatg gggataaata ttatcctgta 2040

aaaaaatatg attgtgattt gagtgaacta acggatgcaa atgaaaaata tttaagtttt 2100

gaaaggaatt ggtacttatt tttagaggga caatatagag attttgtgac caaatattct 2160

aaaaaaaata gtgaaacttt aagtattgac tattgtttaa taatttctat tattgatcct 2220

acaaacgaaa aagatgttta tagtgaaaca atcagaagct tggataaata taacttcatt 2280

cacgaaagta ttaaaactga aattgatact cacattaata acgggctaga gtcctaa 2337

<210> 136
<211> 162
<212> DNA
<213> Listeria monocytogenes

<400> 136
gtgaattcag aatcaccaat aagaattatt ccatgttcaa cacaaagaaa tacgttgatt 60

attctgttta atcatatgga tactctcatt gacccattat ataagctatc ctttgagtta 120

aaatacctag aagaaatatt gttctctatt tttgataatt ga 162

<210> 137
<211> 159
<212> DNA
<213> Listeria monocytogenes

<400> 137
ttgttcttcc tatttaattg ttttttaaat ttccgtaaat tgatttataa agttaagttt 60

cctgcctttt tttcagataa aaaagtatgt tataataaaa aatataaaag tacggcgttt 120

atttgggtac tcgtgaatag taaggttgtt gaattttga 159

<210> 138
<211> 288
<212> DNA
<213> Listeria monocytogenes

<400> 138
ttgaaaaata tacagaaaaa aaacccatta attcaaacga ctattttcga tgcttatcca 60

attataacta aaaaatctaa gtttaaagtg tatagagctc ataaagaaat atattttaaa 120

tttgatggag agaaaatatt tactgttatg aaaatgtcta atcatgttgc tttatttcta 180

aacgaagata ataagattgt aggaatggaa ttttctggtt ttacagaggg tgagtggaaa 240

gaacttgatg aaagcatgag atgtaaatca ggcttaaaaa gcaactaa 288

<210>  139
<211>  312
<212>  DNA
<213>  Listeria monocytogenes

<400>  139
atgaaaattc aaacttacga aaataaactg gttaaagatg agctagaaat agactactat      60

gtaccaatca caataacttt tcccgaaaat gcagataaat atagactagg agaactctat     120

tactttttat ttatcaatga acatgactca cttgttgaag ttaaaatcaa tgctatcaca     180

caaaagattg tggaattggt tgtcgtatcc attaatgata tagaagatag caatttggtt     240

attgaaaaat atacagaaaa aaaacccatt aattcaaacg actattttcg atgcttatcc     300

aattataact aa                                                         312


<210>  140
<211>  195
<212>  DNA
<213>  Listeria monocytogenes

<400>  140
atgcttgatt ctaacagtga agacccagcg gcagtagccg acgccgatgc tttctttgaa      60

aaagtgagca gctcaggaag agtgacagtg acgttacctt ttgaagagca attttggggc     120

gggaaaatgg gacaattcgt cgatgagtac ggaatttcct ggatgattca tacgcaacca     180

tattctaaat tataa                                                      195


<210>  141
<211>  240
<212>  DNA
<213>  Listeria monocytogenes

<400>  141
atggcaaaaa tgtacccgta tttggctttt gaaaatgcaa aagaggcttt aggatattat      60

gaagaagtgt tcggagcaac gaatatttca aggcttccag taagtgagga acaaagcgaa     120

atgtttggct taccaaaaga aaacttagag aacacaactg ttcacggtgg ctttaccgtg     180

cttggtgcaa atttattttg ttcagattca ttcggcaaag aagtaaaacc atccaactaa     240


<210>  142
<211>  177
<212>  DNA
<213>  Listeria monocytogenes

<400>  142
gtgagtagaa ttgacatcgg agaaatacaa gcctttttat accaattacg tgcagccaat      60

gaatcgggaa ggaaaactat ccaaaacaat agtgaaatag tatgtggaga taatattaaa     120

aaggagggta gtttatgcat cgaaaaaata ttcccaaatg cttcattttt cttctag        177

<210>  143
<211>  789
<212>  DNA
<213>  Listeria monocytogenes

<400>  143
atgaactggg atttgtacgc tttaataata attatatcgt tttttatgtt atataaattt      60

tgtgaaaaat tttctgaaac aatgatagta aatattattt tttcagtttc tgttttcctt     120

cttttagcaa atgtactaag tacttcgaat ttttttcata aaagtgctgt taaaacctac     180

gtagaaacag taaagaatat gatacagatt ataattatat ttgctgtagt aattttaatt     240

atatatgcag ttgtaggttt ctggggaaaa agatattctc tgcgtgtaga taactttaat     300

ataggaggta taaatgtatt ttttgataaa tcaagtgaaa tatttgtgaa agctgtagga     360

ggattcatag cttctaaaag aacattattt aaatttaata aagaaagaga taatattagt     420

gaggttttgg attcctacta cgcaacatat aagtttatca tggagaactt agagttactg     480

gacagcgaaa aagatgagga gttgtacact acagctgtaa gtatattaca aaaattaaac     540

aattttctta ctaaacatca gaatgattac agacgttggt atgtgaaaat aatgagagag     600

gatattatca aagtatctga gcacgaagag gacattatcg tgcatcagac gacaatcgaa     660

gtggttcaag aatgttacta tagatatgag gaactacagg aagattttaa agcgcttaat     720

aaatttttta ttgaaagtga tattagaaaa atattaaaaa ttaatagtta cgaatggaga     780

attgattaa                                                             789

<210>  144
<211>  624
<212>  DNA
<213>  Listeria monocytogenes

<400>  144
atgcataaaa cctttattag ctaccatcat gctggggagc aagatttaaa agaagcaata      60

attcgcaaag gcgttgcggg aggagagttt attgacaaat cagtatctga tggggatatt     120

aatacagatc tatcagaaga tacaattatg aggaaaataa gaagtgaata tattcaagat     180

tccacagtag ttgtagtttt gattggagag gatacaaccc agcgacccta tgtgaattct     240

gaaattcagg ctgctctatg gagtaaccct actggattaa ttggagtagt tagggatgaa     300

ttatatgata aagtgtacac taaaaaaaaa tgtaattacg aaggttgcag ttgtggagct     360

tcattacgtt cacctactta tgaatttaat aataaaattc cctttttggt gcgtatgaat     420

cactatgctt tagaatataa taaatcaaca tatccgcatt tcgaagattc ggaatctttt     480

tgtggcattt ataaatattc ttattttatc aataatatgg agacattaat agatgaagca     540

tttgataaga gaactaaaag ctatgaacta agaaagagaa atggtgaagg tgttagaact     600

ataactaatc cttatggcta ttag                                            624

<210>  145
<211>  501
<212>  DNA
<213>  Listeria monocytogenes

<400>  145
atgaaaacga gaaattcctt caaaacacca cgaaaccaca gaacggaagt gttaactatc      60

aacggaaatt ttactacaaa tgaaaaaaat cagcggacat ttttattccc gatgaaaggg     120

tttaagcggc ttgtattagg aatgattgtt atgacgatga ttttcacgct attaacgtat     180

ctagttccga acccggacaa aatcaccatt ccgaaatcag tcgagctgat taatgatata     240

agaatcgttg cattccttag catttgcgta tggatgttgt taagaattga gtatattttc     300

cgtacgaaaa aaccttggaa atatgggtta gagtatagta tttattttca tttagtacct     360

tgtctaatag caatttcaac gatatttttt gtatggattg cctcgattac tttacttgat     420

agtaattatt tattttttat attgttccat agctacgagt gttatggtgg tattatcatg     480

cacttttat tttgttttta a                                                501


<210>  146
<211>  183
<212>  DNA
<213>  Listeria monocytogenes

<400>  146
atgtcgatta atagtaattc tactattgcc agtgggattt cagcctcatt tagtcaatca      60

gctagtgcat taaatagaat atccgtttct gtcagctcta gacaaatcaa tgtggctggt     120

aatagcattg cgattgaaaa tttttcaatc tctcaatcta gtctaaaagg tgtctctaac     180

tga                                                                   183


<210>  147
<211>  555
<212>  DNA
<213>  Listeria monocytogenes

<400>  147
atggaggagc agcgcctccc attgtcggaa gagctaaagg ttatacaggc tcaaaaacaa      60

gaaacaatcg taaaaaatga gaaattaacg ttattgttag aagaaaaccc agaattgtta     120

gactcgttaa aagaacgttt agaagaactg catacattac tacgagaaaa acagattcga     180

gaaaatgagc tcttaaccat tttaagttat caaggtaaga aaatagaggt agacaacgtg     240

actgacattg ttattgacat tgataaacta gtaagcaatc aagaaaagca acagataaaa     300

cagctataca gaacgtttat tcaaaagatt aactttacgc cagatactaa agaccgctta     360

caattgacga tgtattttga ccaatccatt attgatcaac ttaacaagca ttatagagaa     420

accgtatccc attcagagga tacggttctt tttgccttaa caacaacctt tcgattcaac     480

tgtataggag caatgatgaa tcgcattaaa tgtgatcgtc ctcgtaacaa tcccctaacg 540

gttcataatc gctag 555

<210> 148
<211> 1620
<212> DNA
<213> Listeria monocytogenes

<400> 148
atgaatgaat catacacaac gattgttaag caattagttg atgacttaaa agcaatatcc 60

gctaatgcag gattaggtgg cgaagcaggt gaatacaatc tggttacaca gtcattttta 120

tataagtttt taaatgataa gtttttattt gaaataaaaa aattacaccc aaattatgat 180

tatgaaaaat tagctagtct tagtgaagac gaatatacaa tgttacttga ttttgagtta 240

ggaactaatg cggcacattt acatccacgc catttaattg aagacttgta taaacatcag 300

aacgaggatg actttgctaa aacttttgat gatacgttga atgatatcgc tgtggaaaat 360

aattcgattt tttcggtcca taccgctgga aattctgata ttcgtttatt tgaagaacgg 420

cttatcaatg atatcgttcg tgacagtagt atgcggaatg atgttgctaa gcaaatcata 480

gctaaactag cgttggttaa atttgatgag gtcatttttg atcaagggtt cgattttttc 540

tcaactattt tcgaatatat gattaaagat tataacaaag atggtggagg aaaatatgcc 600

gaatactata cgccacattc agttgctaag atcatgtcgg aaattttgat cggtgacgac 660

gaaccaaaat cagttaaagc atatgatcca agtgctggat caggaacact tttgatgaat 720

gtcgcaagta aaatcgggac agataaggta tctatttatt ctcaagatat ctctcagaag 780

tcatcaaact tacttagatt gaacttaatt ttaaataatt tgagccattc aattaataat 840

attgttcaag gtaatacaat tattgaaaat agacatgctg ataagaaaat ggattatata 900

gtatctaatc cacctttcaa actagatttt tcagaatggc gagaacaaat aacaacatta 960

cctgaattta ctgagcgatt ttttgctggt gttcctaata ttccaaactc tgctaaagat 1020

aagatggcta tttatctgtt attcatacaa catatcatct atagtcttaa caatacaggt 1080

aaagcagcag tggtcgtccc gacagggttt attacagctc aatcaggaat tgaaaaaaaa 1140

atccggaaac atttgattga taatagatgg ttaaaggggg ttgttagcat gccttctaac 1200

atatttgcga cgactggtac aaatgtcagt gtaattttta ttgacaaaac taataatact 1260

gacgatgcta aagtagttct ggttgatgca agtaaattag gaactaaagt taaagacgga 1320

aaaagtcaaa aaacattact aagttcagaa gatgaaaaac aaattatcca agcttttcag 1380

atgcaagaag cacgagatga tttttctgtt actgttactt ataacgaaat taaagagaaa 1440

aaatattcat tatctgccag ccaatatttc gatgtgaaaa ttgaatatgt tgagcttaca 1500

caggaagaat ttaataatca aatgaagaaa tttcaatgca ctttacaatc tcttttttta 1560

gaggataaca aactacaaac agatatttta gaacaattga aaaaggtgaa atatgagtaa 1620

<210> 149
<211> 3039
<212> DNA
<213> Listeria monocytogenes

<400> 149
atgacagcga ataagaaatt tcatgaactt acccgtgttc agattcctgc aatggcgcat 60

ctaacacgtt taagttatga gtatctttct ttgaaaaact taaatattga tgaagacacg 120

gggattataa cagatgtttt caagagtcaa tttactagat ttaatcctga agcagatttt 180

gaaaatgaat ttaaaaatat aaaatcagag cttgatcaag atgatttagg tcgctctttc 240

tatgagcggc ttacaaatgg agaacacaaa ttaattgatt ttatccagcc aaataaaaat 300

gtgtggcaaa ttgctgcaga agtagaacat gaacgtgatg atgaaaaatt tcgaccagac 360

atcaccattt ttattaatgg attacccctc gtatttgtag aagttaaaca gccaaacgta 420

atacgtgatg gacttacagg aattaagtca gagtctaaaa gaacgataac tcgtctcaaa 480

aagaaagcat ttcgtcgttt caacaatatc actcaactaa tgatttttag tgataacatg 540

ccttatgatt ccacattagg acaattacaa ggggcatatt atacaacacc taatttaagt 600

aacttgatgt ttaactcttt taaggaacaa gaagcttcag tatttaatgt tactccactt 660

gatgatataa aagttgattg tattttaaaa gacttgaaca aacaaatcct taaaaacagt 720

cgagaattcc aaactaattt ggaaagtacg acacctacca ataaaatttt atcgtcactt 780

ttaacgaaag agcgaatctt attctttatc caatatggtt tagtttatgt taacgaattt 840

gataaatcta atggttcatt agatatccaa aaacatgtga tgcgttatcc tcaatttttt 900

gcaactaaag ccattgaaaa taaattagaa gaaggattta agaagggcgt tatttggcat 960

actcaaggat ctggtaaaac tgcactagcg tatcacaata ttagatatct taaagatgtt 1020

tatcaaaaga aagatattat tcctcacttc tattttgttg tggatcgatt agatttggct 1080

gaccaggcag ccgaagaatt tatgaagcgt ggattaactg tgaagaaaat atcttcctct 1140

tacgaattga gtcagccatt cacacatgat gttgcagtag taaatataca aaaatttaaa 1200

aacgaaacca gtttcatcaa taaatctggt tatgaattac gtgaacaaaa tatttacttc 1260

attgatgaag cacaccgttc atataatcct aaaggatctt acttagctaa tctttacaac 1320

gctgataaag actcaattaa aatcgcactg acaggaacac cactaatcgc tcctaaaaaa 1380

tttgattctt ctggtgaaga agttcttgaa aatgcagatt taaagactac tcgtaatatt 1440

tttggagagt atattcataa gtattattac aatgattcca ttgcagatgg attcactttg 1500

cgtttgattc gtgaagatat tgaaactcaa tataaagcag aattacactc aaaacttcaa 1560

gaaatacagg ttcaacaagg ggctctaaat aagaaaatga tctatgccca tcctaagttt 1620

gttgatccta tgcttaaata tatcctaacc gaccttaaaa ggtttcgtat tgctcagggt 1680

gatgaatcta taggagctat gatcgtggca agttcttctg aacaggctaa agaattattc 1740

aaacaattta atgaaaaata cgctgcaact tatggatata cagctgagtt agtcttacac 1800

gatcaagaag aaaataaaag aaaggttaag acatttaaat caggaagtgt tgatttctta 1860

tttgtatatt ctatgttact cacaggattt aatgctcctc gactaaaaaa actataccta 1920

ggacggaaaa ttaaagctca taatttactg caaaccttaa cacgggtcaa tcgtccatat 1980

cgtgattata agcaaggata cgtcgttgat tttgctgata tatcacatga attcgataaa 2040

acaaatcaag catatcttga ggagctaaca agggaatacg acacaggtat tactggagaa 2100

gatgctaaaa atattttcgg aagtctattc gtatcaaccg atgaaatcaa ccaaaaatta 2160

gatgaggcgg atagcgtgtt gattgactac cctaccgaca atttggaggt ttttagtcaa 2220

accatatcgg atattactga tcgtaaaacg ttgaatgaat tacgcatggc attgataact 2280

attaaagata attttcaact agctcgtcta ttagggcaca atgaaatact acagagaatt 2340

gacttaacac tagtatctaa aatgctaact atgttgatac aacgaatttc tactctaaat 2400

cagctagaaa atgcagatga tacttctggt gaagagttat taaatttggt aatggatgat 2460

gctgatttca tctttgtgaa aaccggtgaa gccgaacttc agttggccgc taacgacttg 2520

caagaaacga aaaagaaggt agccagtggt ctaaaagaaa attgggataa aaaggacccc 2580

gaatatatct cacttttaga agaattcaag cggattatga tgaagcaaaa tatgtctgaa 2640

aataatgatt ggcagtttat caaaacaatg gatgaagaat ataagaaaat acttcgtcaa 2700

attggtgaat tgaatcgtaa aaataggcaa cttgcagcta acttttctgg tgatcataaa 2760

tatgctcgtg ttgcaaaacg tcttaactta agaaaaccta gtgacaatat aggattatac 2820

aatgttttga aaggagcaaa agaaggtatt gataaagtta tcgttaataa cgaaaacatc 2880

cttgataatg acaaattttt tgaaagcaaa accaaagaaa ttgttggtga aacatggcgt 2940

aatctgaacg gtaaaataaa tgtttcacca caaattttag taaagatatc tagtctagcg 3000

acaaaagaat atattaatga atttgaagga gcactgtaa 3039

<210> 150
<211> 198
<212> DNA
<213> Listeria monocytogenes

<400> 150
atgaaaaaaa cctcaatcat ttttattttt tgcacattac tctttgtaac agcatgctca 60

acctccgaaa accaaacaca agccaccgaa gcaaccggca caatagaaca agttgaaacg 120

gatgcaattc taattaataa tttcaaagaa aaaaagataa gtataatccg gaagccgcaa 180

tacgctttga catcgtag 198

<210>  151
<211>  129
<212>  DNA
<213>  Listeria monocytogenes

<400>  151
ttgccgaaaa ctagctacca agatatctat caaaataaaa atggacaata ctattacgag      60

gtaagcttag gtacggacaa aaacactggg aaacgtatca agcgtaaagg tcacaagtca     120

caattttga                                                             129


<210>  152
<211>  177
<212>  DNA
<213>  Listeria monocytogenes

<400>  152
gtgttagttg ttttaaaact tctcgggaac agttcgccag aaattacgct taaacactat      60

gcgcatttgt tacgtaggat tgatgatgat gtcgccgaat cgatgacagg cattatcaaa     120

attgaaactg caacgagtaa gcaatttaca tttgtcggta atcaagcggt gaaataa        177


<210>  153
<211>  228
<212>  DNA
<213>  Listeria monocytogenes

<400>  153
atggctttaa atgcaaaaag tatcactata ggactaccag taagtgattt ggagaaatct      60

gctagttggt atgaaaaact atttatgagt gatgaaaaat taacaccagt tgaaggcgtt     120

gttgaatatc aaattggttc agtttggatt caactttttg aagaaaaaat aaatgtttca      180

gaaaacgttt tacgcatagg ggtagaagat tttagatgtg gaatttga                  228


<210>  154
<211>  129
<212>  DNA
<213>  Listeria monocytogenes

<400>  154
atggaactac tgagtgttgc tatactcatt gttattatta cctttgtttt agacttcatt      60

cattctgatc attctatctt tataataact ttgaagttgc acacaaattg gacatattct     120

ctcatatga                                                             129


<210>  155
<211>  306
<212>  DNA
<213>  Listeria monocytogenes

<400>  155
ttgcttgacc cgtatgttat tctgaagaag cccaaagaca gtgaaaattt agctattgat      60

attttaacat tcttaacagg aatcttttct cgagatagtg ataaattccc tatcttaata 120

caaaacaacc aacttgactt attgttagtc attgataaac cacggagcga aaactcaatc 180

atcagtaata gtattgctga ccaatcgaaa gttttgtgga ttacgatttt gcacaattac 240

tttttagtga tggtactctt gcttaatcaa ttagcttcga aaatcaactc aatattattc 300

aagtag 306

<210> 156
<211> 780
<212> DNA
<213> Listeria monocytogenes

<400> 156
atgaataaag aaaccgaacg aaagcttgct gcgtcactca cttctcaatc aatagaaata 60

ttaccgttta ttccgtattt tttacaagat ttttttgaat tgggtagtag tccgaaagat 120

attacttatt taattaagca acatatgccg ttatcagctg aaagtaattt tttggattta 180

gcttgcggaa aaggcgctgt ttccattggt atcgctaagg aactaggaaa tcctgtcaaa 240

ggaatagatt taattccggc atttatagaa gaagcaaaat gcaaagcgaa agaagcgcaa 300

gtagagtcac tgtgccaatt tgaagtagga gatgtaaata aatcggttca aaacgaaaaa 360

aattatgatg ccgttatatt tggtgcagca gcggatatac ttggaaatcc agcagaaacg 420

ttggaaaaat tgcagggaac tgttaaagaa ggcggttata ttattataga tgaagcctat 480

gtgcctgaat tagcgcacaa taaccaggtg aagtatcaaa attatgaata tctcactcga 540

aacgaatggc tgaatttatt tgaacagaat caattacaat tggtggaaga gctagaggga 600

acagcagaag tggattttga actggaaaag caacacttat taacaagagc caatgaatta 660

attcaacaat atccagagaa aaaagccctt tttgaaggat accttaagag ccaatggagc 720

gaatacgatg acttagagga acatcttatt gctgttacct ggattttaca taaaaagtaa 780

<210> 157
<211> 264
<212> DNA
<213> Listeria monocytogenes

<400> 157
ttgacaagta agtatgtcaa aagattgttt tttaggttag ttcataaatt ctgcgttact 60

gatatacttc aaaagagtat taagctaatt tttgaacaga tttatacttt aaatatagaa 120

agtgaaaata ataataaata tgtttcacgt gaaacaaaca atcaattaag cgattttgtc 180

aatatatata agggaatgtt taatttattt gcactaatta attcacaaaa tatacaattt 240

gcagagagcg gaatgatgct ataa 264

<210> 158
<211> 126

<212>  DNA
<213>  Listeria monocytogenes

<400>  158
gtgaacttta agtcaagcgt aactaattta tttttaagag catcagacag tttaataaaa      60

gcgatgatta attttatttt attgattgag gcttattctt tgctcttagt aggaacaatt     120

gcatga                                                                126


<210>  159
<211>  120
<212>  DNA
<213>  Listeria monocytogenes

<400>  159
ttgttaaagg aggatataat ttatgaaaaa aatgggagtc aaaattggtt tgtgcatcct      60

tttaataatg ccatttacta tatcattttc cgcgaacgtc tcagcggaag aaaatattag     120


<210>  160
<211>  1005
<212>  DNA
<213>  Listeria monocytogenes

<400>  160
atgaaaaaaa catccataaa agatatcgct aaattaagtg gcgtctccgt tgcaactgta      60

tctcgtgtca tcaatgataa tggccgcttt tcagaagaaa cacgggaaaa agtgctggct     120

gttattaaag aaaccaacta ccaaatgaat tttagcgcaa aaagcttacg catgaataaa     180

tctttttctg tcggaatttt agttccggat attagcaact attttttctc tagcgttgtt      240

cagcaaattg aagctatttt attcgatcaa ggctactcta ctattatttg taatacaggc     300

cgtaatttag ataaagaaat ggcatattta aacatgttag agagcaaaat ggtggatggg     360

ttgattgtta tttcaggcgc agatgaattt ggctttaaat ataccaacgc cgaaaatggt     420

attccttatg tatgtattga ccgccaacca aaagataaaa aagatacaat tttcatttca     480

tcaaatcact accaaggagc ttttgaagct acggaagcat taattcatgc cggagtgaaa     540

tcccctgtta tatttatgca tagcaggcaa tcgtcttcag ctaaagaacg tctcaaaggt     600

tttcaagacg ccttaaagaa aaataatatt cgctacgacc ctgacgtttc caaatttaca     660

gtagatttac aaatacacga ttatcaaaaa agcattatta cctttgtcaa tgaagtaaca     720

acaatggatg gtatttttgc gattaatgat aatatcgcgc ttgagctatt gaacctcttg     780

ccaactatcg gaaaaaaaat accaaatgac attaaagtaa tcggattcga tgataccccg     840

cagtgcaatt acactgttcc caaattgagt agcgtgaaac aaaatattcc taaaatcgct     900

caaataaccg tagataattt aattactatt attaaaaatc ctaagcaaaa gaaacgcatt     960

acagagattg ttcccgttga attatcttta aaagactcaa tttaa                    1005

<210>  161
<211>  672
<212>  DNA
<213>  Listeria monocytogenes

<400>  161
atgagcaaaa aactgatttg tccttctatg atgtgtgctg atttttctgg cctagaaagg      60

gaagtggaag aattagacaa agctggaagc gatgtttttc atgttgatat tatggacggc     120

gcttttgtac caagctttgg tatggggtta caagattttg aagaaattcg tcaaaaaact     180

caaaagttag ttgacgtcca tttgatgatc atgaatccag gtgattatgt ggaaatgttt     240

gctgataaag gtgcagatat tatttacatt catccagaag ctgatttaca tccagctaga     300

acattagaca aaatccgaaa taaggggaag aaagttggta ttgcgattaa tccgggaacg     360

gcgattgcaa caattaaaga acttttacca ttagttgata atgtgatggt gatgacagtt     420

aatccaggtt ttgcaggaca aaaatatttg gactatgtag atgtgaaaat cgcagaactt     480

gtcgaattga aaaaggatta tacttttgaa attatggtgg atggggcaat atctccagaa     540

aaaatcagca aactttctca gttaggtgtt aaggggtttg tgctaggaac atccgcatta     600

ttcggaaaaa aagaaagtta tgctgaaata atcaaaaagt taaaacaaga aaagatggag     660

gaattacgat ga                                                         672

<210>  162
<211>  933
<212>  DNA
<213>  Listeria monocytogenes

<400>  162
atggagcaag aagtaacacg actaattagt gcttctaaac aagattttga aaagatgaac      60

ggcagagact taaaacgctc catttttcaa tcagaaggta gagtaattat ggcgcaacat     120

cttttatttg caagtcaggg tcttgtccga ggggttacga atacggaatt attagcagca     180

ttcggatcgg atatgattat gctcaataca tttaacctag aagatgaaaa aagtaattta     240

gggcttcaag gacttaccct cgaagatttg aaaaaacgcg tgaacatccc tttaggaatt     300

tatcttggtt gtccaggtga aaataagact tcagaaaaca ttatttatga tgcagcagga     360

atgttagcta ccgatgaaca tttattaaga gcaaaagaaa ttggcgcaga ttttattgtt     420

ttaggtggaa atcctggttc aggaacttct attcaagata tcattgaaac aacaaaacga     480

gcacggaagt tgctaggtaa cgatgtctta attttcgctg gaaaatggga agatggtatt     540

gatgagaaag tgttgggcga tccacttgct aaacaagatg caaaagaagt gattaagcaa     600

ttgattgatg caggcgcgga tgtgattgat ttacctgctc caggatcaag acacggtata     660

agtgtgcgta tgattcaaga gttagtgcag tttattcatt tatacaaacc aggtacactc     720

gcaatgacat tcttaaatag ttcagttgaa ggggcggatc aagatacaat tcgtttgatt     780

gcattgatga tgaaagaaac cggtgccgat attcacgcga ttggtgatgg tggtttttct 840

ggctgtacga caccggaaaa tgttatgcaa ttatctattt cacttaaagg aaaaccttat 900

acgtacttta gaatggcaag cagaaatcgt taa 933

<210> 163
<211> 663
<212> DNA
<213> Listeria monocytogenes

<400> 163
atggaaaaaa tactcattgc agaagatgat tcagctattt taggagttat tacagcattc 60

ttaaccgaag cagggtatca agtaatgaca gctaaaaatg gaatagaagc atatcatcta 120

tttcaaaagg aaacatttga tttaattatt atggatatta tgatgccgag tatggacggg 180

tacacactaa cagaactgat tcgttctact tcaaccacac cgatacttat gatgacggcg 240

ctctctgaag aagatgacga gttgaaaggc tttgatctag gggcggacga ctatatacaa 300

aaaccattct cttatttagt attattaaaa cgagtgcaag tacttttgag acgagtgaac 360

caaagtgaaa caaagaagca aatactacga tgcggaaata tttctgtaga taccgaaacc 420

tttgaagtga tttcagctgg agaaacaatc gaattaacga aaaaagaatt tgatatttta 480

gtactcctga ttaaaaatca aaagaaggtg attactcgtg aaatgatatt gtcacaagca 540

tgggatttcg cagctattgt ggatacgtcc attatcaata cgcatatgaa aaatctacgc 600

aaaaaactgc acactgaaaa aattcaaacg attagaggtg ctggctataa aatggatgaa 660

taa 663

<210> 164
<211> 1446
<212> DNA
<213> Listeria monocytogenes

<400> 164
gtgctggcta taaaatggat gaataaagtg agaaccagaa ttagttggaa aattttctta 60

atatttgtga gtatcatttt actttttacg gctctagtct atgccatgct aatctttttc 120

ttaccacaat tttattatga ttataaaata aaacaaatta atgattatgc aaatgaagtc 180

gtaaaagcag gcgaagcgga tgatattgaa ggtgtggaaa aagcactaaa tgattttatg 240

gaaaatacaa atattttacc aattttaata gatagtaatg gaaatattgt ttatatacca 300

aatgtgaata tggcatccat taaatcagca acatcgactg ttgtagacgg cacgaaagca 360

gaaggtggta caactgcaac ggattattca ggtggaacag aaacaaaaaa taaacctatt 420

tctatcaatg gtcaaaccta caatctcacc tatacgatta acatccaaaa aattaatgat 480

atttcgaccg tcctattaca atttgctcct tactttattt tatttgcatt tatattaagt 540

ttattaaccg cctacttttt ctcccgcaga atggtgagac ctcttttacg aatgaataaa 600

gttgcctcca aaatggcgaa tttaaacttt acagaagtac ttccgattac atccaaagat 660

gaaattggtc agctttcggg aaatttaaac gaaatggcaa ttaacctaga aaaaacgatg 720

cttgaattaa atgaagtaaa taaaaaatta caaaaagaaa tagaaaaaga gcgtcaactc 780

gaggaaatgc gaaaaagttt tgttacagct atttctcatg aattaaaatc ccctcttgct 840

gcagtaatgg gtcaagtaga agcaatgcgt tataatgtag ctccatatga caatcatcca 900

aaatacttac aagagtcata ccagattttg gaaaagatgt ctcagatgat acaagaaatg 960

ttagacgtat ccaaagttga tcaaacaaac tatatgatag agaaaacttc tttttcctta 1020

actgaaatgg tggagaaaat tatctatagt aaaaaaatgg taccacatcg tctgtctcaa 1080

aaagttgttt ttcaagtgaa ggaagatgtt acagtaacag caaattatgc ctttatcgaa 1140

aaagcaatcg aaaatatttt tgagaatgct tttaattatt ctaaagaaga aacaaccatt 1200

ttagttgaaa ttcaaaatcg ggaaaaaggt ttctattttt ctgtgcataa taaagctact 1260

agtattcccg aagaagaatt gaacaaacta tttgagccgt tctatcgcct tgagaaatca 1320

ggtaacaaaa aaacaggtgg aaatggttta gggttgttta ttattgcaaa atttttagat 1380

atccaaagaa tccactacaa acttattaat gaaaaagacg gtgttacttt tcaaatatgg 1440

caataa 1446

<210> 165
<211> 1422
<212> DNA
<213> Listeria monocytogenes

<400> 165
atggatttta tcagaagagc ctttattagt attaatgcta agaaagggaa atcgcttgta 60

ttatttacgc tattattagt cattttcagt ttagttttta caggttttgc cattcaggaa 120

tctactaaac aaagtgctga atcagcaaga aaacaacttg gagcaaacgt tactctacaa 180

atgaatcaag aggaaataat gaaaaaggcg caaagcgggg aagaaataaa ggaagaagat 240

ttatcgattc caaccgagga tgtaaataaa attaaaaaat tgccacaggt tgacaattat 300

acaattagct cagaaggttc cgcatcaaaa ggtgatttaa caccaatccc tgctaaaaaa 360

caagaaggtg gccaaggggg catgacccaa gttgctggaa cagatacaaa tgggaaaaag 420

gtagaaaccc caagtttttc agtcaaagca gtaaaccaaa cagctacttt aaacagtttc 480

aaagataaaa cagataaaat tatttctggg aagccattaa catcagatga caaagaaaat 540

acagcgctta ttgaaaaaca attggctaaa aaaaacaatc ttaaagtagg cgacaccttt 600

caactgacga atgataaaaa gaaaaaagtg aagtttgttg tcaaaggtat ttatcaaagc 660

aatcaaaaaa tagaacctca actagaatca tttcaaatga tgctccctgg aaataaaatc 720

tatgccaata taaaaggagc aaaagatttt ctttggagtg gaggcattga aaaagctgaa 780

tttaatttaa aagatccaaa agaaataaat tcatttatct ctgaagcaaa aaaattaacg 840

gatgtagata atggagatat gttccaattt gatgcacaaa acagtgctta caaaaaaatg 900

attggaccga ttgagcgagt ggcatctttt agtaatatta ttgtgatgat tacacttctg 960

gctggtggct taatccttgc gcttatcgta ttactatcca ttcgtgagcg taaatttgaa 1020

atgggtgtgc ttttatcact tggggaaagt aaaacaaaat taatgagtca atttttagta 1080

gaagtattaa ttattgcagc acttgctttt tctttttctt gtgcattggc taatccaatt 1140

ggccaagcta tctctaacca aatgctttcg actgaagtca cgaaagaagc aaataaagag 1200

aatgaaactt ccacacaaga aagcatggca atagctctag gtggggaaga gaaaactaag 1260

gttgatgcgg agccgattga taaaatagat gtcgtgatta caagcaatat aatggggcaa 1320

gttggtgggc ttggttttat tttaatattc ctagcgacaa cgataccgtg tttatttatc 1380

attcgactac aacccaaaat gctatttact cagaaagatt aa 1422

<210> 166
<211> 159
<212> DNA
<213> Listeria monocytogenes

<400> 166
ttggatattt ataaaaacat taataccccg gttaaaacac ttgataatac aatcgcgatt 60

aacattaaaa taacgacaac gcgaactact tttttattag tcattgcgga agaaaactcc 120

tctcataaag catttcagat atatatatct tacttataa 159

<210> 167
<211> 744
<212> DNA
<213> Listeria monocytogenes

<400> 167
atggtgcaat cagccaacaa aataataaac atgacacgag atgaagtgga aaaagcgata 60

actgattttc cagtcgcaat tttaccactc ggagcaacag agcaacatgg acatcattta 120

ccacttggag ttgatatatt tttagcagac catttagcag aattagtatc ggaaaaaaca 180

ggagcagtag tcattccacc aatgccattt ggctattctt gggtatggcg tgatattcca 240

ggaacgatct ctattcaaca acatcatgtc gaagcaatta ttaaagatgt cgcacacagt 300

gtttctcgct atggtgtcaa aatgcttgtc ttaataaatg gacatgattc taacaataac 360

tcgatgaaat atgctgttag agaattagca gatgagttag aaatgcctgt tatgtatcta 420

ttctatccat ctctttctga agtaatggaa aagaattgtg agtctggtac atggcacgga 480

atgattcatg cctgtgaatt tgaaacctct ttaatgcttg cgactaaaaa agaattagtg 540

caaatgaaaa aggcagttag agaatatccc gaaaaaccaa ctctttatgg gaaaacaact 600

atttcgctag gtgatttaag caaaagtggt gtatatggtg atgctagtct tgccacagag 660

gagaaagggg aacagatgat agaactgttt gcagaaaaga tgacagcact tcttttagaa 720

ggttatggtt atttaactaa ataa 744

<210> 168
<211> 1269
<212> DNA
<213> Listeria monocytogenes

<400> 168
atgcttactt tcatcacaga aattttttct acgccagctt ttattttagg aattatagcc 60

gctattgggt taattgcctt aaggaaaacc ggttctgata ttgtaaaagg aacattaaaa 120

acgatttttg gttttattat gctacaacag ggatccgcga ttattgttgg aagtttaatt 180

ccatttagca caatgtttga aaatgctttt caacttaaag gggtggttgc agaagataat 240

gctattgcag ctgctgtcca gcaaatttta ggaacggaaa cgtctttaat tcttgtattt 300

ggttttatta ttaatgttat tcttgctaga acaaccagat ggaaatatat tttcttaaca 360

gggcatatga tgttttcctt cgcagctaca atggccatag tattcgatca aatgggcttt 420

aatagctggc aaattatttt gtttggttct atcgcacagg gtatttcgag tattgttttt 480

ccagcactcg cacaaccttt tgtacgcaaa gtgacaggaa atgatcaagt cggttttggc 540

ttttggggca gttcccttgt ctgggcgacc ggttggcttg gagggaaatt aggaaataaa 600

gctaattcta cagaagactt aaaagtacct aaaaaactag attttttgaa agaaatgtct 660

gttttaatgg ggattgttat ggcagtgatt tatatattta cttctttctt tgtggatgaa 720

ccggtgatga aagaattagc tggcacacaa agtaatgtat tattcgcaat attgcaatca 780

ttcacttttg ttgtcggaat attgatacta cttcaagggg tccggatgtt tttaggtgaa 840

ttagttccag cgtttaaagg tattagcgag aaaattgttc ccggcgcaat tccagctctt 900

gatgttcccg ttttctattc attcgcacct actgcaacga ccattggttt tttatgcgca 960

gtagttggag ggcttggagc gacactagtt tctaccatgc ttcccgttgt cgtacttccg 1020

tctgttatcg gattgttttt tatgggcgga gcggctggtg tatttggaaa tgcaatgggt 1080

ggtagacgag gagccattat tgctggttta ttattaggat ttatttttca aatgattgta 1140

gcaattgctt atccaatttt aggattagat ggttatggca ttcaaggatt atggtttgct 1200

tcgacggatg caatcattgt tatcgtcatt attcgattaa ttggagcatt atttggaata 1260

gcactttaa 1269

<210> 169
<211> 444
<212> DNA
<213> Listeria monocytogenes

<400>  169
ttgttaccaa ttactttaga acaaattgaa ttggatgtgg aggtagaaag tccggtagat      60

gctatcagag ctgccggaaa tctcttagta aaaactgaaa gcgcttcatc tgattatgta     120

gatgcaatgg tagctagtta tgaaaaatta ggtccttata tagtcctagc accacatatt     180

gcaattccac atgcgcggcc cgaacacggg gtgaaaaaac aatgcatgtc aattgttcgt     240

ttgaaaaaac cgattaaatt tgggcaccca gccaatgacg atgtgagtct ggttatcgca     300

attggaggtg tagataccaa tttccacatc aaaatgctcc aagcgcttag tgaattatta     360

accgatgaag ataaattaaa aagattaaaa acaagtaccg ataaagcgga aatcttatgc     420

accattaata aggaggaaaa atag                                            444


<210>  170
<211>  750
<212>  DNA
<213>  Listeria monocytogenes

<400>  170
atgaaaaaat taaataaatc atcttctaca ccactttata tgcaactgaa aaatcaacta      60

caagagaaaa ttgaacaaga tatgtctgtt ggagatttac ttttgacaga aacagaaatt     120

gaaaagatgt atgatgttag caggatgaca gttcgaaaag cgatagaaga gctggtaaat     180

gagggaattg taagtaaaca acaaggaaaa ggaacgtttg tgagtcaaac aaagactaca     240

caaggacttg gacggatttt tagttggtca gaggaaatga aactacaaca taaagaatct     300

aagactatca gtgtagaaat ttctgcagca atcccgtcaa aaaaaattcg tacagcactc     360

caattaggca agcatgaaga agtaacttgt attagacgcg tgagagaaat tgatggtgaa     420

cctgttgcaa ttatgattaa ttatttacgc gccaaatatg ttcctggttt actagagaat     480

ggtttgaaat cagattccct ttaccatgat ttggaagaaa tttatcatat tagtttagta     540

catgcagatg aaattattac agcaagatta tctaccccga ttgaatctgc cttgcttgca     600

gttccagaag actctgcggt gcttcatgtg cgacgtactt cctatatgaa aggcaatacc     660

ccagtggaag tggtggatat ggtagctcgc ggagataggt accaatattt ggctgaatta     720

gaaggtagaa ctaaaaaaca tatcatatag                                      750


<210>  171
<211>  84
<212>  DNA
<213>  Listeria monocytogenes

<400>  171
gtgttaaact atttaccgaa tacgaaaaaa agacaaataa atgaggttgt gaaaaatgat      60

atttcaacgg cttttgaaaa ctag                                             84


<210>  172

<211>  1719
<212>  DNA
<213>  Listeria monocytogenes

<400>  172
atgattaaaa aagtttttca ttttatactt gtactgatgt taagtgtaag tataatacct      60

ctattccatg ctaaagcagc tgaaacaact aatggagtag atagtagtga acaggaagat     120

aataccgaag ttgcgcgtga agaaatgcct ccagagtctg aggaaccagt attctcgctt     180

gaacaaaata gagatgatgc tatggctgct ttagtcgtgc ctcaaactag aaatagtttt     240

ttgagagcag ctagtactcc gacatttcaa caaacattta taaattccat ttccacgcaa     300

gcaatggatt tatgtaaaaa gtacaattta tatccatctg taatgattgc tcaagcggct     360

cttgagagta actggggaag aagtgaatta gggaaggccc ctaattacaa cttgtttgga     420

ataaaaggat catataatgg caaaagtgta acaatgaaaa cttgggaata tagtgattcc     480

aaaggttggt atcaaatcaa cgcaaacttt gccaaatacc catcacacaa agaatcttta     540

gaagacaatg cgaaaaaact tagaaatggc ccaagttggg actctagtta ttacaaaggt     600

gcatggcgcg agaatgcaaa aacatacaaa gatgcgactg catggttaca gggacgttat     660

gcaacggaca acacatatgc ttctaagcta aatacgctaa tttcttcata taatttgact     720

caatatgata ctctgtacga tacgattaaa caacaaaaaa atgtttctga agatgctaaa     780

gtagttaaag cagatgggca tggtgtttat agtggaattt acaatacgtc tgcagccagt     840

gcgaaaaagt tatctactgg agcgccttac aacaataaag atgtaaaaat tttaaaagaa     900

ggcactacaa gcagaggaac gtgggtccaa ttttctctca ataataaagt aatcggctgg     960

atggataaac gcgcatttgt ctattatcca aaagcaacaa atgtaaaaac gcttaaccta    1020

acaggtaaaa tcactgctgg atctactaat ggtttatggt ctgaggttcc aggtacagtg    1080

aatgcgaaaa aattagctac aacagctggt gcttaccaaa ataaagatgc taaaattatt    1140

aagcaaggtc aaattagtgg ccgaacttac tatcaattcc aagtaggcgg taaaacaatt    1200

ggttggttgg acgctcgtgc atttcatgtt tatgataaaa tccaaagcca gtcaaatgta    1260

aattggaatc gaactatttt aaatgccgat aagcacggtg tgtactcggg tgtttataat    1320

acttcatcaa gtagtatgaa taaactaagt acaggcgcga aatataataa taaaaaagta    1380

aaagttatta agcaagccaa gaccgcgcgt ggaacttggt atcaattcca agtaaatggc    1440

aaaacagttg gttggatgga ttatcgtgca ttctttgaca caattacctc ccaaaaaaca    1500

atgaataaaa cagttactgt gggtaatgcc acgaatcatg gcgtttttga tggggtttat    1560

cgaacctcgc caacggtaaa acgaatttct ttggggaaac catacaataa taaaaaagta    1620

aaagtgctca aagaagctgt aacagaccat gccacatggg ttcaatttaa atacggtaac    1680

acaactgcat ggatggacaa aaaagcattt aaatattaa                           1719

<210>  173
<211>  2859
<212>  DNA
<213>  Listeria monocytogenes

<400>  173
atggaagatg ttagaaaaaa atggaaaaag gtacagtggg ataatgaaat tacgtataaa      60

acattcctta catatagtgt tctctttgtc ttagtaacaa taagtattta cttacttttt     120

tggttaagag gttattcatt tatttggtct catgatggtt ttaaccaaca ctatccgatt     180

ttaaaagaat ttagaaatat gttagtagat ttcctgaaac acccaactca agtgccggaa     240

ttatggtctt ggcaaattgg aatgggaggc gatgtggtcg gtagttttgg ctactatgtt     300

ctaggtgata tttttgctta tttagtagta ctatttccag cagatcagat ggaacttgca     360

tacacaacat taattctatt acgcttgttt tgtgtaggta ttgccttctt agctctagcg     420

aaaaacttta aaattaatca tattgctgca gttacaggtt cattagtcta tgtgttctct     480

ggttatttat ttgtttcggc aacaaggcat ccatttttta ttacgccgat gattctttta     540

ccgctacttt gtctttgtgt agagagagtg cttcaaaaga aatcaccagt tccgttaatt     600

cttgtgattt gttggacatt agttagtaac ttctattttg catatatgct tgcgattatg     660

gtttgtatct atacagttat tagatacttt actcattaca aaaagcaagg aattccgcta     720

ctaagtacta ttggaaaatt agcggtctat gcaattactg gctttttaat ggcatgtatt     780

ttatttttac ctaatttaat cgccttttta ggttcttcca gggcaaatgg agagtttgcg     840

aatgggctat ggttctatga tttgtcctat tacttatcgc ttggaaaaat gtacattaca     900

accgaaagcg ctggttattg ggcaaactta ggtttcgcag caattgctgt gtttgtactt     960

ccgtttattt ggcagtatcg aaaaaaatat ccggttgtat ttattagttt ggttttagga    1020

cttggcatga tgctatttcc gtttttcggg gcattattta atggtttaag tagtccttcc    1080

aatcgttgga tgtttgcagt tgctcttcct gtaagtattg gagtttcctt tttattaaca    1140

gactataaaa cattaacgaa aaaagatatg cgtaattttc ttattacact tattatcttt    1200

atcgttgttt catggtgggg accgaacttc aatatttatc aacctatctt gttagttcct    1260

ttaacattaa tgcttttaac cttttttgtt ttctttttac aatttattaa tttgaattat    1320

attaagaaga aagcttataa tggcttgttt tactttttga ttataatgtt agtatgtgtc    1380

aatttagctt atatgggtaa ttatatgttt agtaaaaacg gggctaataa agtacaaggt    1440

caattaaatt ctggggttgt tgaacgtaaa ataactgaac tatatggggg agcagataaa    1500

aagattgata agagctcttt ctatcgtacc agcctttcta gtgggtataa tggattttat    1560

gataattcag atgcaaacat tttgttagat gtaaatacgg tgaattcatt ttattcgatt    1620

actaatggtg cagtagctga cttagcttct gaattaaata atagtcagtt tcgcatgacg    1680

ttgccgcttg ggcaaatgga taataggact atttttacga atatacttgg aacgacttac 1740

ttatttgcac gtgatgatca agctggcaga ataccatatg gctttaaaaa aattgattct 1800

gctgaaattg aacgagtaga taaaccaggc attagccata cagatgtata tgaatctgaa 1860

aatagtgttc cattgatgta tatgaactac catacggaag atagagaatc atatgaaaag 1920

ctctctccgc ttgaaaaaga ggaagcactt tcttataatg ctatcattga taataaagac 1980

acatccaaac gtgagtataa atcgaatatt aaagaacttg aatttgaaac gagtatacct 2040

aaagttaatg gcaaagctcc tgcaaaatta acgaaagaca aaatagaggt ttctaatgct 2100

gaaaatagca ttcaatttaa attgaaaaac ccggaagaaa caaaaaatgc agaactttac 2160

ttttatattg atggtttaga tttcacgcca tacactttca aacaaagaaa agatattgcg 2220

tttgctaaag atgagtataa aacaaaaaat atgcgttata attattaccg gaacaacctt 2280

accaaacgta taaaagaaga ctatacatta acagccaaaa catctaaccg tgtagtttca 2340

gcgagccaag tggataaaag tgagctttct gggtatttta aacgggacaa catgctttta 2400

aatggtggtt tttctgaaaa agctcggaaa caagtatcta ttaatttgag cggtcttggt 2460

acgtacgact atgataatat caaaatttac gcggttccat ttgatgattc ttatacgaaa 2520

agaatgcaag aactaaaaaa acaagctgca acagacttga aatttgatac aaacaaagtg 2580

tcggctaagg tttcagcaaa acaagatggt gtgttagtta caacaattcc atatacaaaa 2640

ggttggaaag taaaagtaga tggaaaagaa gtatcaacag aaaaagtcaa tacaggattt 2700

attggttttt ccttagataa aggactccat acaatcgaaa tgaattatga aacgccaatg 2760

cttaaagctg gcatggtggc gagtggctta ggtgtcatcc tttttgctgg aattatcgtg 2820

gtgtatcatc ttagaaagcg caaaaataaa acacaataa 2859

<210> 174
<211> 1872
<212> DNA
<213> Listeria monocytogenes

<400> 174
atgagaaatt taaaagatag agtactcaat tcattaaaag gtaataaaaa agatattaaa 60

attagtgttg ttgtacctac atataataca gaattagaag gtcttaaaaa cttaatggcg 120

tcaattgata agcaaacaat gaatccagat gagtatgagc ttgtttttgt agatgatgga 180

tctacaacag atacatacga acgtttgcaa gagtttgctg aaacacgtcc aaatatgacc 240

gttaaacaaa ttgaaaactc tggttgggga agcagaccaa gaaatattgc aactaaaatg 300

gctaaaggag aatatatcct ttatttagac catgatgaca cagttttccc tgaaacattt 360

gaacgcgtat ataatttcgg aaaagaaaac aatttagatg tagtgagcgg gaaagaagta 420

agaacaaatg gttggtcatg ggatggaaag caattctctg agaacaatcc gcatgcagaa 480

gaaatgggta ttgaatgttt acttccaatg actccacaca aattttataa aagagaattc 540

ttgttagaga atgatattac atttgatgat ggagctcgag ttctttggga agatgtatat 600

tttaactcta aggcgttcat tcacggtgca aaagttggta ttttagccga ttatcctacg 660

tactattgga ttgcaactgg tgcaaataat tccagtagtt tcggacgtga tccgcatgaa 720

aaatggaacc aaattaataa attatttaat ttctttaaag acaatatcaa agagcaacgt 780

gacttagatt ttatgctaac tcattggtat cgctcacgag ttcttgggat tttagggcaa 840

tggctactaa aaaacaacaa tgaacgtatt gatattgaat ttaactatgc aaaaaaactt 900

gccgaagaat taataccagc ctatattagc gaaaacttag ataaaaacaa tcaagtaaaa 960

gattatttat taagacaagg tgatttagat tcattaaaaa aacttgctca aatcgatgct 1020

ggaattacag cgcttagcta tgtggaagat gcttacttta aagaagacaa actattcttc 1080

aaaacaagta caaaaatgac atatgaagac aaagaagatt tctttattga aaaaacagca 1140

gatcgaatgg aacgtatttt accagaagaa atcaaatcga aattgccaaa agaattcttt 1200

gattatagtg atgatttagc tgaatttacg tatgagccaa gtattaaagg acgtaattca 1260

agagctacct ggaaaatcga cggttctact agtaatgtag aagttgttaa taaaaaagca 1320

aatttgtata aaatagaagg tgaaatgtct ttttctgttc aaattaatga ttatatatta 1380

gatgcagctg ataaaaaaca accatgggat attgccacac gctttaccgg ccttgggtat 1440

acaagtcacc gagcattaac cattggtaaa attcttatca aaacagcgtt aataaataac 1500

aaaacaatga ttgtttataa aaatgcatct ggtttaataa gtctggatgt tggaagtagc 1560

gttcgttcaa tcgtggaaga ttctggtgtg aaacgtgaac aaattcttat tgataaaaca 1620

tctggaaaag taactattcc attaaatgaa attcatgtgt ttggtgaaag ccttattgaa 1680

ggtaatgcag aattaaaacc ggtgggaatt agtgatgcgg atccaattaa tgtaaaagct 1740

aaattgattg gtgaagctaa taaagcaaga gtagaagttc ttttgggtga tgaaaaacta 1800

tctggtgaat atcatttagt aactaatatc caaggtaaaa aagataaaca gcaaattaaa 1860

ataacgcttt aa 1872

<210> 175
<211> 867
<212> DNA
<213> Listeria monocytogenes

<400> 175
atgaaaggta taattttagc aggaggaagt ggaactagac tttatccact tacaaaagca 60

atttcgaaac aaatgttacc aatttatgat aagccgatga tttattatcc gctatcgatc 120

ctaatgttag caggtattaa agatatttta attatttcaa ccccagaaga tactccacgc 180

tttgagcaat tattagctga tagcgatcaa ctgggtatta atatttctta tgcggttcaa 240

gaaaaaccgg aaggattagc acaagcgttt attattgctg aagattttat tggggatgat 300

tcggtttcgc ttatacttgg agataatatt tattacggac aaggtttatc caaaatgctt 360

caaagagcat ctgctaaaaa agctggagct actgtatttg gctaccacgt gaatgatcct 420

gaaagatttg gagttgtgga gtttgatgaa tcaatgaaag ctatttccat tgaagaaaag 480

cctactgaac caaaaagtaa ttatgcggta actggtcttt atttctacga taatcgcgta 540

gtggaaattg cgaaaagcat taaaccatct gaacgtggtg agttagaaat cactgatgtt 600

aataagcgtt accttgaact gggagaacta gatgtagaac ttatgggcag aggatttgct 660

tggttagata caggaacaca tgaatctctt cttgaagcgt ctacctttat tgaaacaatt 720

gaaagaagac aaaacttgaa aatcgcttgt cttgaggaaa ttgcttatcg aatggggtat 780

attgatgagg ctgcagttga aaaattggca gaaccactta agaaaaatgc gtatgggcaa 840

tatttaatga aattaattaa taaataa 867

<210> 176
<211> 561
<212> DNA
<213> Listeria monocytogenes

<400> 176
atggaaattg ttgaaagtaa actagctggt gttaaattag taaaaccaaa agtgtttgga 60

gatcatcgtg gttttttcat ggaaagctat aatgaaaaag cattccatga agctggaatc 120

gatatgaagt ttattcaaga taaccactct ctttctgtgg aagcgggtgt acttcggggt 180

atgcattatc aaacgaaccc aaaagcacag acaaaattaa ttcgtgttgc gacaggcgca 240

atttatgatg tagttgttga tatgcgtaaa ggttctccta catacggtga gtgggaaggt 300

tatattttaa gcgaagcaaa caaacatcaa cttctagtac cacaaggatt tgctcatggc 360

ttttgtacga ttaccgaaaa tgtgaatgtg ctttataaag tagatcaatt atattcccct 420

gaaaacgatg cgggaattgc ctacgatgat ccggatttaa atattacttg gccaacagat 480

aaattaattt tatcggacaa ggatacgaaa catccacgtt taaaagacgc tgaaaataat 540

tttgtatggg agaataaata a 561

<210> 177
<211> 987
<212> DNA
<213> Listeria monocytogenes

<400> 177
atgaatttat tagtaacagg tggggcaggt tttattggca gtaatttcgt acatcatatt 60

ttaaataagc atgatgatta taaagtcgta aacttagatt tgctgactta tgcaggcaca 120

atgagcaatt tagaggatat caaagaaaat cctaatcatg ttttcgtaga aggaaatatt 180

tgtgattatg atctagtgaa aaaactagta acagatcata aaattgatac tattgttaac 240

ttcgcagcag aatctcatgt agaccgtagt attatcaatc caggtatctt tatcgaaaca 300

aatgtacaag gaacgctaaa tcttttaaat gttgcgaaag aattaaatgt cgcaaaatat 360

ctacaagtat ctactgatga agtgtatggt tcacttggag aaactggata tttcactgaa 420

gaaactccaa ttgctccaaa tagtccatac tctgcaagta aagcttcagc agacttatta 480

gttcgttcat attttgaaac atacggctta aatgtaaata ttactcgttg ttctaataat 540

tatggaccac atcatttccc agaaaaatta atcccgctaa tgatcacaaa tggtcttgac 600

ggggaaaatt tacctattta tggtgatggt aaaaatattc gcgattggtt acatgtgtca 660

gatcactgtg cagcaattga tttagtaatc cataatggta aatctggtga agtttataac 720

gttggtggac ataatgaacg tacgaataat gaaatcgtgc atatcattgt tgacgatttg 780

aatctttcta aagataaaat tgtatatgta gaagatcgtc ttggtcatga tttacgttat 840

gctattgacc ctaagaaaat cgaaacagag cttggttggg aaccaaaata tacatttgat 900

acaggaatta aagaaactat cgaatggtat gtaaataatg aagcttggtg gagaccacta 960

aaatcacgcg caaagttggg tgaataa 987

<210> 178
<211> 831
<212> DNA
<213> Listeria monocytogenes

<400> 178
atgagtattt tagtaacagg agcaaatggg cagctcggaa cggagctagt tcaattatta 60

aaagaacata atttaaccgt gactgagtgg gacaaagatt ctgtagatat tgtggataaa 120

gcagctgtaa aaaaagcaat gcttgattta aagcctgaat ggattatcca ttgtgccgct 180

tttactaatg tagaagcagc agaagatgaa ttaaaaaacg ttaactggga agtaaacgtg 240

gatgggactg aaaatatcag tgaagctgca gaaatagttg gagctaaact tgtatatatc 300

agtacagatt atgttttcga tggtactaaa aaagaagcat atttaccaga tgataaaaca 360

aatccactta atcaatatgg aattgctaaa ttagcaggag aaaaagttgc tttagaaaaa 420

aatagccaaa cttatgttat tcgtacttca tgggtgtttg gtaaatatgg taataatttt 480

gtatacagta tgcttaaact agctgaaacg cataaagaac taaaagtagt aaatgatcaa 540

ttaggtcgcc caacatacac atatgattta gctgacttca ttcgttttgt tattgagaag 600

aatccggcat atggtattta ccaattctct aatagtggaa ctgcgacatg gtttgagttt 660

gcaacagaaa ttttgaaaga taaagatgtt actgtgaatc catgtacttc agatgaattt 720

ccacaaaaag ctgaacgtcc aaaaacatct attatgagtt tggaaaaagt agagaaacta 780

ggttttaaca ttccaacttg gcaagatgct ttagttcgtt ttaaaaaata a 831

<210>  179
<211>  2091
<212>  DNA
<213>  Listeria monocytogenes

<400>  179
atgcattcgt ataattgttt ttttatatct ttatggggtt tatttgttta taatagtaat      60

gtgtataaat tactctatga acaaaaggag ccggatattt tgaacgattc aagggactta     120

cattccattt gttttgtttc tacagaagtg gaagataaga acaataacgt acattttata     180

gcagaatctg cagatttatt ggcagattgc ttagtgctat tagagaaaga gaataaacac     240

tatttaactt ttatgaaaga aaatttgaat acaattgcgt tagccgagaa aatcgaatat     300

gcaccagata tcatttgccc aaataaagtt ttgaaaggta gtttgtatac aaatcctgaa     360

tgcatagata acagccttgc tggtagtact ttctccaaag atttacttaa gagagtagca     420

aatgaaatga agcaagaagt atttgaatta acaagcttta attattattg ttatcatttg     480

gcggagaaga ttggtcattt gggagaagaa gctagctatt cgctctcttt ggcggactta     540

gatgactcgg ttacacgttt acttgatatt aaagataatc attaccgaaa agattggaca     600

attttatatt atgtaaataa gtttttaatg atgcatttag aaaatttacc gaaaattttt     660

gaagaatcag atgaaaaatt tcaaattaat tatttagaaa aacttactga acttattgcg     720

aaactgccgg atgaggttat ttttgagtta gatgcgctcg attatttggt tcaaaatata     780

ttaaatgatt catttgaatg gattaaatac gaagcatatc cagcttgttt aactttagct     840

gaaataataa gtcaccgagc agcaccaaat aaattcacgg atactaaatg gctgtgttta     900

gaaaaatgtg tccgaaaagg ttctgtaaat ccgctaagaa acaaaaaaaa tccaactgtt     960

ttgacaccaa taaaaaagaa atttgtttct ttttttagcc gtaaagtggt gtttacaatt    1020

gcaaaactgt tacctgttgc gaaagataat gttttgttag tttcgaataa aattagtgat    1080

ttagatgcta ctttcatgcc gatttaccag acaattaagc aaaagcatcc tgaaaagtct    1140

gtgcgtgcgt acatgaaaat gcgaccagaa gataatcatg caatgtatta tttaacatat    1200

ttttggaatc ttggaagagc aaagtatgtg tttttagatg actattatta tcaattgtat    1260

tctattagaa tgaggaaaga agcggaagtt attcagcttt ggcatgcagc aggagcgttt    1320

aaacgattcg gtcatagttc tctaggattt aaagactcca tatcgcttga ttttgaaaca    1380

agagcgcacc aaaattacac aacgagtgtc atatcatcca gtgatttaaa accgatttat    1440

gcagaagcct tccatatgga tgaaaccaaa attgaagcat ttggtctacc acgtttatgg    1500

aagttatttg atcaagacta caaggaattc agactagatt atttccaaaa aatgtacccg    1560

cttcttaaag ggaaaaaagt gattacttat gcaccaactt ttcgagggaa ttcagaagag    1620

cgaaaagcat tccaactcga attaaattta cgtaaaatga aagaggaact aggacaagaa    1680

tatgtagtag taattaaact gcatccatta gtaagcgttg aaacgcaagt tccagaagat    1740

ttagctgatt ttgtacttga ttttagtgga attgaaatga atgacgttct aatcgtaaca 1800

gatattctaa taactgatta ttcgtctgtt atttatgatt actctatctt gaatagaccc 1860

attattttct acgcatatga tcttgaagcc tattcattag aacgtaattt ttatcatgat 1920

tatttggaat ttgttccagg tccagtggtt gctacaactg aagaagcaat taaacttatt 1980

caaacaaatc aaatgataac agggaagtta caggactttg cagagaaagc atttgaatat 2040

catgatggtg atgccgcagc tcgaattatt gattatgtaa tgcaagattg a 2091

<210> 180
<211> 1854
<212> DNA
<213> Listeria monocytogenes

<400> 180
gtgttaaata aaaaaagagt tatagttggg gttttgctac tattactaag tttaatttct 60

attagctatt attcagaaac ctttaaacta actttaagct ggttgttatt tgcagtagtt 120

ttaacggttc tttattttag acaagagaaa aactttcaat ttcaatggtc aacattgata 180

acctcactaa taattagttt attttggatg gcttcatcat tcaatggtgg accatatggc 240

gggaattttg tttttaatag tattatttta gcaggaactt ttcttgtaat cactatcttt 300

gttttacttt tattacttga aatgcgaacg gaatataaag caagaccgaa tcgcaaagta 360

aaatggccat ttttcgcact atttacgagt attccattcg tggtctggat gatttcattt 420

ctagcctatt atccagcaaa aatgacattt gactcctatt accaatgggg aatggctcac 480

ggtattcgcc aatatagtca gtggcatccg cttttacaca ctttgtggat agaaacaaca 540

agtgcgattt acgactcacc ttcgagttac attttttctc aaataattgt tgtttcatta 600

atcgttggct ttgctattta tactcttgta aaaatgggcg cgcatatttg gattggtgtt 660

tgtatttcaa tcggctatgc catttaccct gcagcaatgt tttattctgc aacagcatgg 720

aaagattttc catttgcagc ctttatatta cttttcaccg ttttaatttt aaaaatagta 780

caatctaatg gaatgtggct gaaaaattgg tggcacctta tcgcttttgt tttagtagct 840

tttgtttgta taaatttacg aaacaatgga atgatgatta tcatcgtatc gcttctgtgc 900

ttgcttattt tcatgaaaaa ctttcgtctt attattaccg gtattcttgt tggaaccttg 960

ggactgaatt ttttatttgg tctggttatg acaaacgggc ttaatgcgca acctaatcca 1020

ttaaaccaag cgctagcaat tccttcccaa caaattgggg ctacttttta caatgatgga 1080

aactttactc ctgaattaaa agagtatttc acttccatat tacctgaaga aaattggaaa 1140

aaagattaca acccttatac tgtagaccca attaagcatg ataccaaata caattcatcc 1200

gtcattgaag atgattttgg actatacatt aaaaattggt tcaaactctt aacggctaat 1260

ttcggtactt atgtaggggc ttatttagat caaacagcag tcatttggca attctattct 1320

ccagaaaatt ataaagtatt ctttgatact tcagcgaata ttcaagatac aagatatgat 1380

gtgagagcat tcgccaaatt cttcccagaa ggtttatcgg aagaagagat taataaatta 1440

ggatatgaag tctatcaaaa tgaatacaaa aatgcaactg gaaaagatgc tgttagctac 1500

aatgagtata agagacggat tgatgactct actaatccac ttatttcaat atctaaagct 1560

ccaagtctga agaaaataac agatagcatt tatgcaaaaa caacaaatga gtggcaaaat 1620

tatttattaa aaggagccat tccattagta ttgctcataa tagcaattgc tgccgtctgc 1680

ctccaacgtc ctaaaaagaa acttcttatt tttgcacctg tagtaatggc acttattact 1740

atagcaatcg caatgccagc aacagacttt agatattctt atagttttat tttcaccgtg 1800

cctattgtct tttttgcaac taaattaaaa aattacaaag aaaatcaatt ttaa 1854

<210> 181
<211> 948
<212> DNA
<213> Listeria monocytogenes

<400> 181
atgggaattc taaatgagaa agtagctgta ctcttgcctt gttataacga ggagcttaca 60

attggtaagg taattgatga ttttaagaaa gaattaccaa atgcggatat ttatgtgtac 120

gacaataatt ctaaagataa aacctttgaa atagcgaaag atcatggtgc tatcgttcga 180

aaagaaatgc gccaaggtaa aggtaatgta gtacgttcta tgttcgcgga tatagatgct 240

gattactatt taatggtcga tggtgacgat acctatccag cagaatactg ccatgaaata 300

ttagaggtgc ttcgcaataa ggaagctaat atggttattg gtgatcgtct gagtaatggt 360

acctacactg aagaaaataa aagaaatttt catgactttg gtaactcact agtacgtaat 420

acaattaatc gtatcttcaa aagtaatttg agagatatca tgacaggcta ccgtggcttt 480

gatcgttatt ttgttaagac tatgccagtt ttaagccctg gttttgagat tgaaactgag 540

atgagcattc acgcattgga aaatcgcttt ttagtgaaag aaattgaaat tgattaccgt 600

gatcgtccag aaggtagtga atcaaaacta aacacttttt ctgatggttt caaagtaatt 660

atgacgattg taagattatt taaaaatagt cgtccgtttt tatttttcaa tttattagcc 720

tctttgtttg tgcttgtagg agttctagtt ggtttgccag tcataattca gtttgctcaa 780

attggcttgg tactaaaatt tccgagtgca ttacttgcaa ctggtttaat cataatgggt 840

atgctgttct tcatttgtgg attaatcctt gatacgatag ctcatagaag cagacaaagc 900

tacttcttag aacttgttaa ataccgcgaa agaaatccat tgaactaa 948

<210> 182
<211> 20
<212> DNA
<213> Listeria monocytogenes

<400>  182
agggcttcaa ggacttaccc                                                    20


<210>  183
<211>  20
<212>  DNA
<213>  Listeria monocytogenes

<400>  183
acgatttctg cttgccattc                                                    20


<210>  184
<211>  20
<212>  DNA
<213>  Listeria monocytogenes

<400>  184
agcaaaatgc caaaactcgt                                                    20


<210>  185
<211>  21
<212>  DNA
<213>  Listeria monocytogenes

<400>  185
catcactaaa gcctcccatt g                                                  21


<210>  186
<211>  21
<212>  DNA
<213>  Listeria monocytogenes

<400>  186
agtggacaat tgattggtga a                                                  21


<210>  187
<211>  21
<212>  DNA
<213>  Listeria monocytogenes

<400>  187
catccatccc ttactttgga c                                                  21


<210>  188
<211>  22
<212>  DNA
<213>  Listeria monocytogenes

<400>  188
gctgaagaga ttgcgaaaga ag                                                 22


<210>  189
<211>  22
<212>  DNA
<213>  Listeria monocytogenes

<400> 189
caaagaaacc ttggatttgc gg 22

## Description

### TECHNICAL FIELD

The application relates to *Listeria monocytogenes*, more particularly to *L. monocytogenes,* clonogrouping, *i.e.,* to the determination of the clone to which a *L. monocytogenes* isolate belongs. The means of the application involve primers and/or probes, more particularly primers suitable for use in multiplex amplification. The means of the application are notably useful to assess the infectivity that a *L. monocytogenes* isolate might show in a human being.

### BACKGROUND

*Listeria monocytogenes* (*L. monocytogenes*) is a major human foodborne pathogen and causes infections in animals as well. It is responsible for 360 human invasive infection cases in average per year in France. The Centers for Disease Control and prevention (CDC) in the U.S.A. estimate that approximately 1,600 illnesses and 260 deaths due to listeriosis occur annually in the U.S.A., and similar numbers of listeriosis are estimated to occur in Europe. *L. monocytogenes* infects mostly the elderly, the immunocompromised individuals and pregnant women. Besides gastroenteritis, three types of invasive infections can be caused by *L. monocytogenes*: bacteremia, central nervous system infection and materno-fetal infections that often lead to abortion. These invasive infections are characterized by a high fatality rate (estimated between 20 to 30 % of cases) and infections always require hospitalization.

Food industry is strongly concerned by contamination with *L. monocytogenes,* and regulations are in place in Western countries on the acceptable levels of food contamination, which implies intense monitoring, including quantification of levels of *L. monocytogenes,* contamination of food products and strain typing activities in food industry, national reference centers and food safety reference laboratories. In the food chain, food business operators and competent authorities manage the *L. monocytogenes* hazard by its detection and/or enumeration in samples of food chain and/or food environment, followed by its characterization at the serotype or genoserotype level. When contamination is above a defined level (assessed for example by the number of colony forming units per gram of food product), the products have to be very rapidly recalled and withdrawn from the market, the production plant has to be treated for elimination of the bacteria, and consumers have to be immediately alerted and informed.

A *L. monocytogenes* strain can be classified in lineages, serogroups, serotypes, clonal complexes (CCs, which are further refered as clones), or in PFGE types that are determined by Pulsed-Field Gel Electrophoresis (PFGE) using one or two restriction enzymes (generally *AscI and*/*or ApaI*) (*cf. e.g*., Brosch *et al.* 1994, Graves *et al.* 2001).

Lineage is usually determined by sequence-based studies (Ward *et al.* 2008). There are four lineages, *i.e*., lineage I, lineage II, lineage III and lineage IV.

Serogroup (also called PCR serogroup or genoserogroup in the literature) is determined by the reference multiplex PCR method of Doumith *et al.* 2004, which involves the PCR analysis of particular target regions in four marker genes and one control gene. The four marker genes are *lmo1118* (906 bp fragment), *lmo0737* (691 bp fragment), *ORF2110* (597 bp fragment) and *ORF2819* (471 bp fragment), within which the targeted regions are of 906 bp, 691 bp, 597 bp and 471 bp, respectively. The control gene is *prs,* within which the targeted region is of 370 bp. There are four serogroups, *i.e.,* serogroup IVb (including mostly serotype 4b strains, but also strains of serotypes 4d and 4e), serogroup IIb (including mostly serotype 1/2b strains, but also strains of serotypes 3b and 7), serogroup IIa (including mostly serotype 1/2a strains, but also strains of serotype 3a) and serogroup IIc (including mostly serotype 1/2c strains, but also strains of serotype 3c).

Serotype is determined by serotyping of somatic and flagellar antigens (*cf. e.g.,* Seeliger and Hohne 1979). Examples of serotypes notably include serotypes 4b, 1/2b, 1/2a and 1/2c.

Clone identity is determined by the Multilocus Sequence Typing (MLST) method of Ragon *et al.* 2008, which involves the PCR and sequencing analysis of internal regions of seven housekeeping genes, *i.e., acbZ* (ABC transposter), *bglA* (beta-glucosidase), *cat* (catalase), *dapE* (succinyl diaminopimelate desuccinylase), *dat* (D-amino acid aminotransferase), *ldh* (lactate dehydrogenase) and *lhkA* (histidine kinase)). Examples of clones include clone CC1, clone CC2, clone CC3, clone CC4, clone CC5, clone CC6, clone CC7, clone CC8, clone CC16, clone CC9, clone CC121 and clone CC155. Worldwide distribution of these clones has been confirmed in Chenal-Francisque *et al.* in 2011.

Serotypes reflect the antigenic properties of lipoteichoic acids and the flagellin at the surface of bacteria. The other methods reflect the phylogenetic origin of the strains. There is a strong concordance between the different phylogenetic categories. For example,
- clones CC1, CC2, CC4 and CC6 are mainly of serotypes 4b, 4d and 4e, are of serogroup IVb and of lineage I;
- clones CC3 and CC5 are mainly of serotypes 1/2b and 3b, are of serogroup IIb and of lineage I;
- clones CC7, CC8, CC16, CC121 and CC155 are mainly of serotypes 1/2a and 3a, are of serogroup IIa and of lineage II; and
- clone CC9 is mainly of serotypes 1/2c, 1/2a and 3c, is of serogroups IIc and IIa and of lineage II. The method, which food business operators and risk managers currently follow to evaluate the severity of a *L. monocytogenes* contamination, relies essentially on the epidemiological finding that serotypes 1/2a, 1/2b, 1/2c and 4b are the serotypes that are the most frequently observed in human infections or food sources. There is currently neither stratification of infectivity levels of clones nor classification of the clones according to their frequency in human cases, their specific clinical forms or their lethality rate.

Infectivity (or infectiousness) corresponds to the potentiality of a pathogen of causing an invasive infection. Here, infectivity is assessed as the proportion of humans that become infected when exposed to the pathogen.

Serogrouping is relatively easy to implement but has limited discrimination, therefore providing limited resolution for epidemiological typing. Indeed, in order to ensure efficient industrial process contamination analyses and epidemiological surveillance of listeriosis, it is of first importance to accurately identify the clones. However, the MLST method classically used to identify the clones is time-consuming and too expensive for a routine use in most industrial, private or public laboratories involved in *L. monocytogenes* typing.

A further impediment is that there was not sufficient typing and genome data to cover the whole diversity of *L. monocytogenes* clones, and that there was not sufficient clinical and bacteriological data to identify those clones, which have the highest potentiality of causing an invasive infection in a human.

There is still a need for means allowing for easy and efficient identification of the *L. monocytogenes* clones, more particularly of those clones which have the potentiality, more particularly a high potentiality, of causing an invasive infection in a human.

### SUMMARY OF THE APPLICATION

The means of the application allow detecting that a product contains a *L. monocytogenes* isolate, which has a high probability of having a potentiality, more particularly a high potentiality, of causing an invasive infection in a human. The means of the application notably comprise methods, oligonucleotides (primers and/or probes, more particularly primers), sets of oligonucleotides (comprising primers and/or probes, more particularly primers), kits, computer program products and computer devices.

By contrast to the prior art, the inventors adopted a clone-specific approach.

They identified the *L. monocytogenes* clones, which are the most relevant to assess a high probability of having a potentiality of causing an invasive infection in a human. The relevant clones identified by the inventors comprise the CC1, CC6, CC2, CC4, CC8-CC16, CC5, CC3, CC7 and CC155 clones, and may further comprise the CC121 and CC9 clones. The potentiality of causing invasive infection in a human is lower for clones CC121 and CC9 than for clones CC1, CC6, CC2, CC4, CC8-CC16, CC5, CC3, CC7 and CC155 clones. Therefore, *L. monocytogenes,* isolates of clones CC1, CC6, CC2, CC4, CC8-CC16, CC5, CC3, CC7 and CC155 have a high probability of having a potentiality, more particularly a high potentiality for clones CC1, CC2, CC4, CC6 and CC7, of causing an invasive infection in a human, whereas *L. monocytogenes* isolates of clones CC121 and CC9 have a low probability of having a potentiality, more particularly a high potentiality, of causing an invasive infection in a human.

A potentiality of a *L. monocytogenes,* isolate of causing an invasive infection in a human can be considered to be a high potentiality when the isolate belongs to a clone that is significantly more associated to clinical origin than to food origin.

Conversely, a potentiality of a *L. monocytogenes* isolate of causing an invasive infection in a human can be considered to be a low potentiality when the isolate belongs to a clone that is significantly more associated to food origin than to clinical origin.

The inventors identified genes that are specific of each one of said relevant clones (genes of SEQ ID NOs: 1, 61-71, 2, 72-79, 3, 80-85, 4, 86-106, 5, 107-111, 6, 112-122, 7, 123-128, 8, 129-141, 9, 142-149, 10, 150-155 and 11).

To achieve this result, the inventors had to overcome several technical impediments, such as the absence in the prior art of sufficient typing and genome data to cover the whole diversity of *L. monocytogenes* clones, and the absence of the *L. monocytogenes* pan-genome.

Furthermore, the finding that clone specificity can be achieved with a single marker gene, *i.e.,* that no gene combination is needed to achieve clone specificity, is highly surprising in view of the prior art knowledge, *e*.*g*., in view of the occurrence of horizontal gene transfer among *L. monocytogenes,* lineages.

The inventors further identified genes, which are specific of each of said relevant clones, and which allow clone identification by multiplex PCR (genes of SEQ ID NOs: 1, 2, 3, 4, 5, 6, 7, 8, 9, 10 and 11).

Examples of multiplex PCR implementation are shown in Table 9 below (*cf.* example 3), which shows three distinct multiplex, *i.e.,* multiplex IVb, multiplex IIb and multiplex IIaIIc. The three multiplex can be implemented simultaneously, since they can share the same thermocycling conditions.

### BRIEF DESCRIPTION OF THE FIGURES

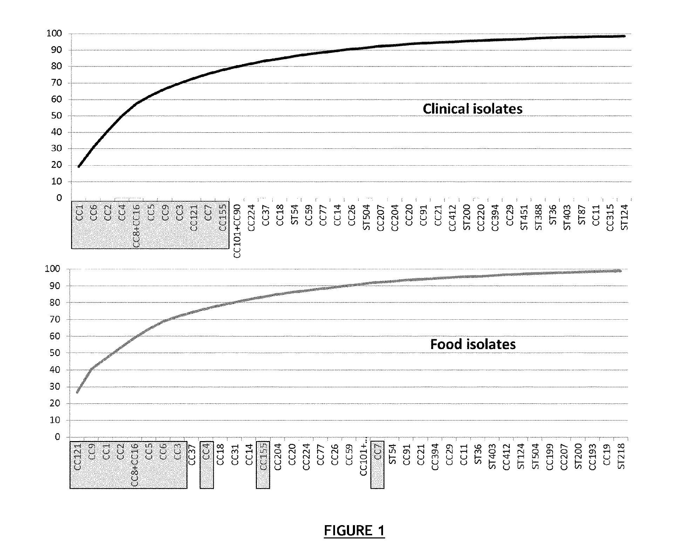

**Figure 1****.** Cumulative frequency of *L. monocytogenes* clones in clinical and food isolates.

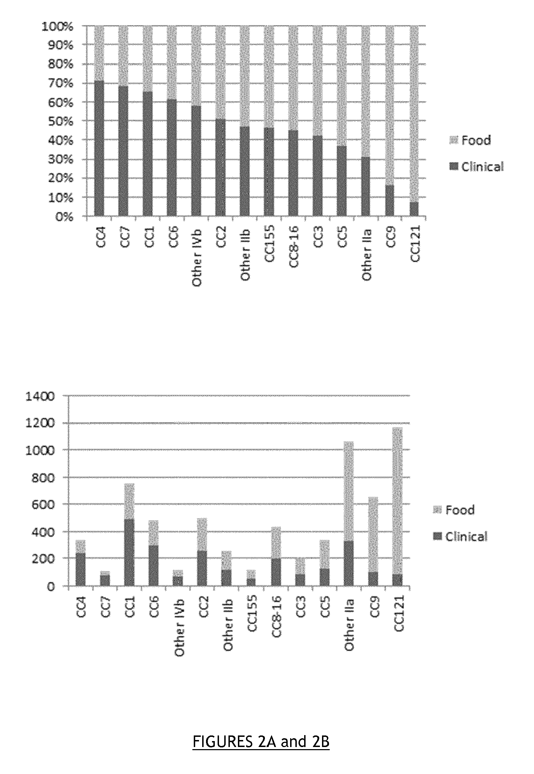

**Figures 2A** **(top) and 2B (bottom)**. Relative proportions (A) and numbers (B) of *L. monocytogenes* isolates from clinical and food sources per clone.

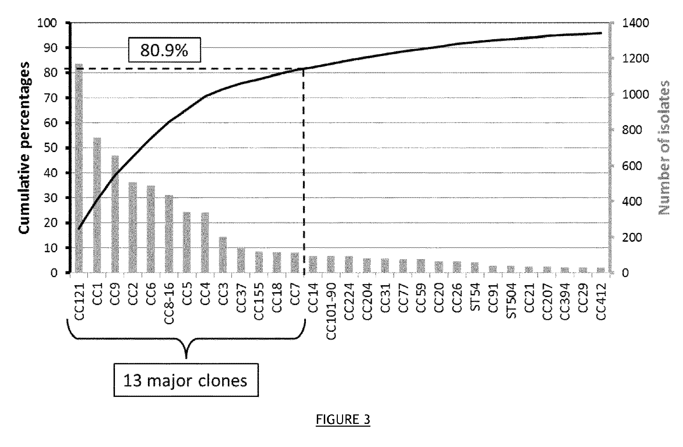

**Figure 3****.** Result of the analysis of 2005-2013 *L. monocytogenes* isolates: nearly 80% of them were represented by 13 MLST clones only. Eleven of them are the most frequent clones for clinical isolates, *i.e*., clones CC1, CC2, CC4, CC6, CC3, CC5, CC121, CC155, CC7, CC8-16 and CC9 (*cf. e.g*., Figure 1, upper panel).

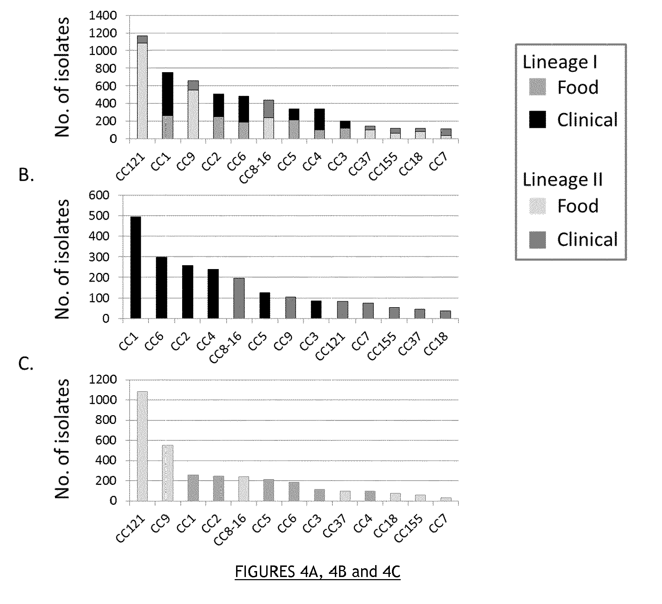

**Figures 4A** **(top), 4B (middle) and 4C (bottom).** Result of the analysis of 2005-2013 *L. monocytogenes* isolates: distribution of these isolates according the source from which they have been isolated (Figure 4A: food + clinical sources; Figure 4B: clinical sources; Figure 4C: food source). Clones CC1, CC2, CC4 and CC6 represented almost half of the clinical isolates, and clones CC9 and CC121 represent more than 40% of the food isolates.

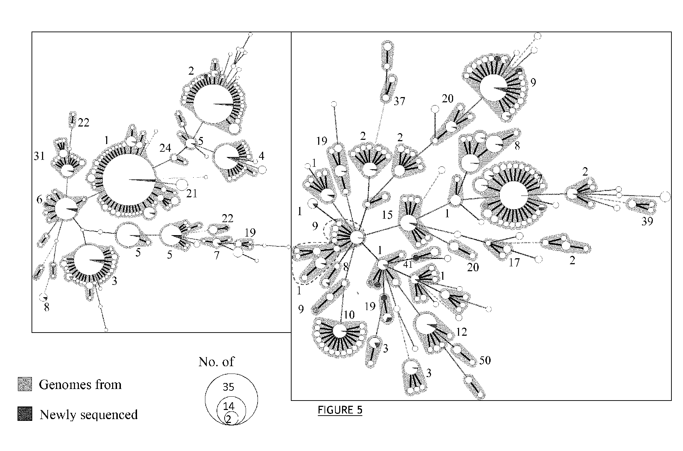

**Figure 5****.** Minimum spanning tree showing that the (more than one hundred) *L. monocytogenes,* strains analyzed in genomic comparisons are representative of a large diversity of *L. L. monocytogenes,* strains.

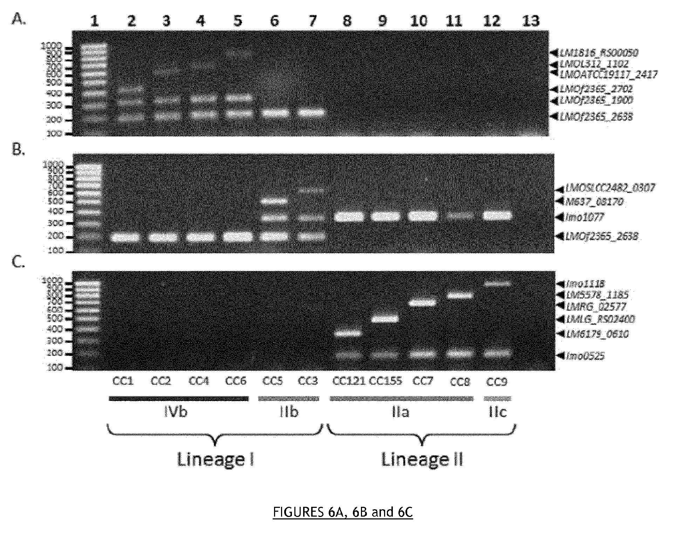

**Figures 6A** **(top), 6B (middle) and 6C (bottom).** PCR amplification patterns obtained for representatives of eleven target clones. Agarose gel electrophoresis of PCR products generated by (A) IVb clonogrouping multiplex PCR, (B) IIb clonogrouping multiplex PCR and (C) IIaIIc clonogrouping multiplex PCR. Lanes 2 to 12 correspond to *L. monocytogenes* strains of clones CC1 (CLIP2013/00068), CC2 (CLIP2012/00799), CC4 (CLIP2011/00181), CC6 (CLIP2010/01520), CC5 (CLIP2011/00140), CC3 (CLIP2011/00787), CC121 (CLIP2012/00189), CC155 (2010/01410), CC7 (CLIP2012/01275), CC8 (CLIP2012/01049) and CC9 (CLIP2010/01314), respectively. Lane 13: negative control. Lane 1 is the Smart Ladder small fragment (EUROGENTEC). Genes corresponding to the amplified fragments are indicated on the right. Molecular sizes are given (in base pairs) on the left.

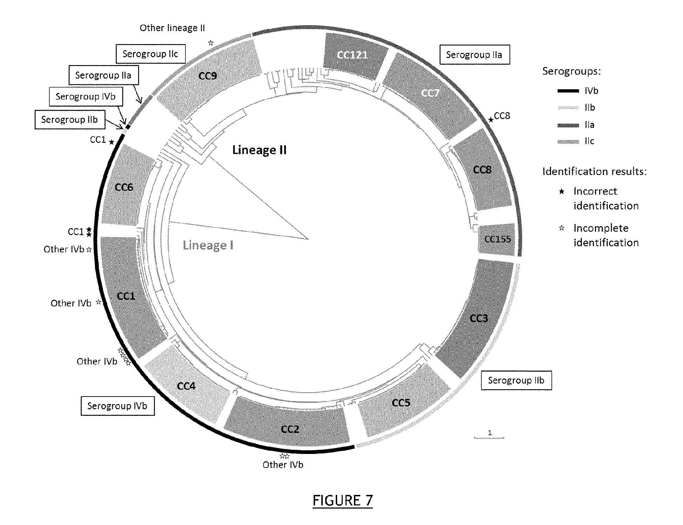

**Figure 7****.** Phylogenetic tree based on the concatenation of the seven MLST gene sequences of the 282 strains that were tested using the three multiplex PCRs of the application. Branches of lineage I are in grey and branches of lineage II are in black. Clones defined based on MLST are indicated with areas at the leaves. Circular arc areas around the tree indicate the serogroups based on the reference PCR serogrouping method (Doumith *et al.* 2004). Incomplete identifications are indicated by white stars, incorrect identifications are indicated by black stars.

### DETAILED DESCRIPTION

In the application, unless specified otherwise or unless a context dictates otherwise, all the terms have their ordinary meaning in the relevant field(s).

For exemple, an isolate is defined as a clonal population of bacteria isolated from a specific source at a given time, *e*.*g*. an isolate from a patient or a food sample, and a strain refers to an isolate which has been characterized by some phenotypic or genotypic method(s). Unlike an isolate, a strain is preserved as a sample of the diversity of a bacterial specie but its original source is not necessarily known because of the potential loss of this information. In the application, the terms "isolate" and "strain" are used interchangeably.

For example, Clonal Complex (CC) identity is determined by the Multilocus Sequence Typing (MLST) method of Ragon *et al.* 2008, which involves the PCR analysis of seven housekeeping genes, *i.e*., *acbZ* (ABC transposter), *bglA* (beta-glucosidase), *cat* (catalase), *dapE* (succinyl diaminopimelate desuccinylase), *dat* (D-amino acid transferase), *ldh* (lactate dehydrogenase) and *lhkA* (histidine kinase)). PCR conditions and primers are described in the section "Multilocus Sequence Typing" and in Table 1 of Ragon *et al.* 2008. The *L. monocytogenes* MLST data base is available from: http://www.pasteur.fr/mlst.

Examples of clones include clone CC1, clone CC2, clone CC3, clone CC4, clone CC5, clone CC6, clone CC7, clone CC8, clone CC16, clone CC9, clone CC121 and clone CC155. Worldwide distribution of these clones has been confirmed in Chenal-Francisque *et al.* 2011.

A *L. monocytogenes* strain belongs to a particular Clonal Complex (CC) [*e*.*g*., to CC1], if the sequence type (ST) of this strain differs from the other members of the CC [*e*.*g*., from the other members of CC1] by only one of said seven housekeeping genes.

For example, serogroups (IVb, IIb, IIa and IIc) of *L. monocytogenes* refers to the serogroups as defined in Doumith *et al*. 2004, the content of which is herein incorporated by reference.

Briefly, Doumith *et al.* 2004 describes a multiplex PCR serogrouping method, which involves four marker genes, *i*.*e*., *lmo0737*, *lmo1118*, ORF2819 and ORF2110. The primer pairs that are used to amplify target regions from said genes are shown in Table 1 of Doumith *et al.* 2004 (SEQ ID NOs: 182-183, 47-48, 184-185 and 186-187 in the appended sequence listing, respectively).

The multiplex PCR serogrouping method of Doumith *et al.* 2004 may further involve a control gene, *e.g*., *prs.* The size of the target region in *prs* is of 370 bp. The primer pair for amplification of this *prs* target region may *e.g.,* the primer pair of SEQ ID NO: 188 (GCTGAAGAGATTGCGAAAGAAG) and SEQ ID NO: 189 (CAAAGAAACCTTGGATTTGCGG), or any other primer pair, which amplifies the same 370 bp target region.

The amplification reactions are performed as described in Doumith *et al.* 2004. Briefly, the amplification reactions of the multiplex PCR serogrouping method of Doumith *et al.* 2004 can be performed in a 96-well plate (ABGENE) in a final volume of 100 µl containing 2 U of Taq DNA polymerase (ROCHE, BOEHRINGER), 0.2 mM deoxynucleoside triphosphates (PERKIN-ELMER), and 50 mM Tris-HCl-10 mM KCl-50 mM (NH₄)₂SO₄-2 mM MgCl₂, pH 8.3. The primer sets can be added at the following final concentrations: 1 µM for the *lmo0737*, ORF2819, and ORF2110 primers; 1.5 µM for the *lmo1118* primers (0.2 µM for the control *prs* primers). PCR can be performed with an initial denaturation step at 94°C for 3 min; 35 cycles of 94°C for 0.40 min, 53°C for 1.15 min, and 72°C for 1.15 min; and one final cycle of 72°C for 7 min in a thermocycler (ICYCLER; BIO-RAD LABORATORIES). Five microliters of the reaction mixture can then be mixed with 3 µl of loading buffer and separated on a 2% agarose gel in a TBE buffer (90 mM TRIZMA base, 90 mM boric acid, 2 mM EDTA, pH 8.3). The PCR product can be visualized by ethidium bromide staining.

The means of the application notably allow determining or detecting that a product, more particularly a product intended for administration to a human (*e*.*g*., a product intended for administration to a human *via* the oral or intra-gastric route or the intra-peritoneal route, more particularly *via* the oral or intra-gastric route, such as a food or beverage intended for human consumption) is pathogenic (*i*.*e*., toxic) to human. Indeed, the means of the application allow detecting that said product contains a *L. monocytogenes* isolate, which has a high probability of having a potentiality, more particularly a high potentiality, of causing an invasive infection in a human. More particularly, the means of the application allow detecting that said product is highly pathogenic (*i*.*e*., toxic) to human, *i.e.,* that said product contains a *L. monocytogenes* isolate, which has a high probability of having a potentiality, more particularly a high potentiality, of causing an invasive infection in a human.

The expression "*invasive infection"* is intended in accordance with its ordinary meaning in the field. It notably encompasses a life-threatening infection or an infection where said *L. monocytogenes* isolate is present in a normally sterile body tissue (*e.g*., blood), more particularly an infection where said *L. monocytogenes* isolate is present in a normally sterile body tissue (*e.g*., blood). Life-threatening infections or infections where said *L. monocytogenes* isolate is present in a normally sterile body tissue notably include (*Listeria-induced*) diseases or conditions, such as listeriosis in pregnancy, listeriosis of the Central Nervous System, bloodstream infection, brain abscess, glandular listeriosis, eye infection, hepatitis or liver disease, peritonitis or abdominal infection, lung infection, joint infection, arthritis, heart disease, bone infection, and gallbladder infection.

Said potentiality of causing an invasive infection is particularly high, where the human to whom the product is intended to be administered, is an immunocompromised human, a pregnant woman, a fetus, a neonate, an infant, a child, or an elderly.

To achieve this advantageous result, the inventors had to overcome a number of prejudices and impediments.

Indeed, by contrast to the prior art and common general approach in the field, the inventors decided to follow a clone-specific approach. Achieving a clone-specific approach required to overcome several technical impediments.

The prior art data and material did not provide sufficient typing and genome data to cover the whole diversity of *L. monocytogenes* clones, and did not provide sufficient clinical and bacteriological data to identify those clones, which have a potentiality, more particularly a high potentiality, of causing an invasive infection in a human. Therefore, the inventors built a set of *L. monocytogenes,* genomes, which covers *L. monocytogenes,* diversity (*cf.* example 1 below; *cf.* Figure 5). This set notably required *L. monocytogenes,* strains to be newly isolated by the inventors. The resulting set built by the inventors consisted of 104 L. monocytogenes genomes, which were determined for strains that were representative of the diversity of 6,000 *L. monocytogenes,* strains, which cover the world-wide diversity of *L. monocytogenes,* clones.

The inventors collected clinical and bacteriological data for these 6,000 strains set, and searched for those *L. monocytogenes* clones, which have a potentiality, more parlicularly a high potentiality, of causing an invasive infection in a human. The relevant clones identified by the inventors are the CC1, CC6, CC2, CC4, CC8-CC16, CC5, CC3, CC7 and CC155 clones (clones CC8 and CC16 have very similar Pulsed Field Electrophoresis (PFGE) profiles and therefore have been merged to constitute one epidemiologic group, *i.e.,* clone CC8-CC16). The relevant clones identified by the inventors may further comprise clones CC9 and CC121 which have a low potentiality of causing an invasive infection in a human. Please see example 1 below as well as Figures 1, 2A, 2B, 4A, 4B and 4C.

The identification of those *L. monocytogenes,* clones, which have a potentiality, more particularly a high potentiality, of causing an invasive infection in a human allowed the inventors to search whether some marker genes or some marker gene combinations could be found to specifically detect each of these relevant clones. The inventors therefore built a pan-genome of *L. monocytogenes,,* based on a total of 104 genomes covering the *L. monocytogenes,* clones. Please note that a pan-genome of *L. monocytogenes,* strains representing this diversity of clones was not available in the prior art.

The inventors identified genes that are specific of each of said relevant clones. These genes are identified in Table 2 below (*cf.* example 2): SEQ ID NOs: 1, 61-71, 2, 72-79, 3, 80-85, 4, 86-106, 5, 107-111, 6, 112-122, 7, 123-128, 8, 129-141, 9, 142-149, 10, 150-155 and 11. Each of these genes is specific of one *L. monocytogenes* clone, *i.e.,* of one of the CC1, CC2, CC4, CC6, CC3, CC5, CC121, CC155 CC7, CC8-CC16, and CC9 clones. The finding that clone specificity can be achieved with a single marker gene, *i.e*., that no gene combination is needed to achieve clone specificity, is highly surprising in view of the prior art knowledge, *e*.*g*., in view of the diversity of clones and the dynamics of gene transfer in bacterial species, including *L. monocytogenes.*

The inventors further identified control genes, which are specific of a *L. monocytogenes* lineage (lineage I or II), of a *L. monocytogenes,* serogroup (serogroup IVb), or of a *L. monocytogenes,* serogroup and lineage (serogroup IIb and lineage II). These control genes are identified in Table 3 below (*cf.* example 2): SEQ ID NOs: 12, 156-157, 13, 158-171, 14, 172-179, 15 and 180-181.

The inventors further achieved a selection of particular genes within the sets of genes they identified. Indeed, they selected genes that allowed clone identification by multiplex PCR (and further identified control genes that are suitable for implementation in multiplex in said multiplex PCR clone identification). Each of the selected genes achieves clone specificity as single marker gene and allows implementation in multiplex PCR. These (clone-specific) genes are identified in Table 4 below (*cf.* example 2). The genes that allow multiplex PCR identification of clones CC1, CC6, CC2, CC4, CC8-CC16, CC5, CC121, CC3, CC7, CC155 and CC9 clones notably comprise the genes of SEQ ID NOs: 1-11 (*cf.* Table 4 below; *cf.* example 2 below and the herewith enclosed sequence listing). The genes that allow multiplex PCR identification of the control genes notably comprise the genes of SEQ ID NOs: 12-15 (*cf.* Table 5 below; *cf.* example 2 below and the herewith enclosed sequence listing). Examples of regions that can be targeted within said genes and examples of primers that are suitable for multiplex PCR implementation comprise those identified in Tables 6 and 7 below (*cf.* example 3: target regions and primers of SEQ ID NOs: 16-48 for the marker genes; target regions and primers of SEQ ID NOs: 49-60 for the control genes). Examples of multiplex PCR implementation are shown in Table 9 below (*cf.* example 3), which shows three distinct multiplex, *i.e.,* multiplex IVb, multiplex IIb and multiplex IIaIIc. The three multiplex can be implemented separately or sequentially. They can even be implemented simultaneously in a single PCR instrument, since they may share the same thermocycling conditions.

Each of the three multiplex PCR of the application can be implemented separately, *i.e*., on its own without implementation of the other two multiplex PCR, or can be implemented sequentially or simultaneously with one or the two other multiplex PCR of the application.

Furthermore, each of the three multiplex PCR of the application can be implemented sequentially in combination with a serogrouping method, such as the serogrouping method of Doumith *et al.* 2004. More particularly, a serogrouping method, such as the serogrouping method of Doumith *et al.* 2004, can be implemented to determine whether the isolate under test is of serogoup IVb, IIb, IIa or IIc; the multiplex PCR of the application that corresponds to the (IVb, IIb or IIaIIc) serogroup thus determined can then be implemented (*i.e*.,: multiplex IVb of the application when the serogroup is IVb, multiplex IIb of the application when the serogroup is IIb, multiplex IIaIIc of the application when the serogroup is IIa or IIc).

The performance of the means of the application was demonstrated on 282 *L. monocytogenes* strains representing the whole diversity of the (MLST) clones of *L. monocytogenes,* including strains of target clones as well as other clones. At least 95.4% of the strains could be identified accurately (*cf.* example 3; *cf.* Tables 10 and 11 below).

This result confirmed that the means of the application are suitable for implementation on *L. monocytogenes,* isolates, *e.g.*, isolates from food or human samples, and that they are reliable to identify the clone, to which the isolate belongs or to assess the infectivity risk that this isolate might show in a human.

The means of the application further show a high specificity with respect to the *L. monocytogenes,* species. Therefore they allow the identification of the *L. monocytogenes* species. They have the advantage that the *L. monocytogenes* species can be identified without requiring any other or further assay, such as any further biochemical assay.

Compared to the prior art MLST clonogrouping method, the means of the application shows the advantage of allowing clone identification on a large scale basis. For example, the PCR means of the application allow at least 96 samples to be processed simultaneously. Compared to MLST, the means of the invention are faster to perform, with results obtainable within *e.g*., 4 to 6 hours or even less. Furthermore, the means of the application require less nucleic acid material in the initial sample to be analyzed, and are highly reproducible. The results obtained with the means of the application are easy to interpret, and need no highly specialized expertise.

Throughout the application, the expressions "high probability" and "low probability" are intended in accordance with their respective ordinary meanings in the field.

For example, a high probability is a probability of at least 80%, or at least 85%, or at least 90%, or at least 95%, more particularly of at least 95%.

For example, a low probability is a probability of less than 95%, or less than 90%, or less than 85%, or less than 80%, more particularly of less than 95%.

For example, a high probability is a probability of at least 80% and a low probability is a probability of less than 80%.

For example, a high probability is a probability of at least 85% and a low probability is a probability of less than 85%.

For example, a high probability is a probability of at least 90% and a low probability is a probability of less than 90%.

For example, a high probability is a probability of at least 95% and a low probability is a probability of less than 95%.

More particularly, a high probability of having, being, belonging, causing (or any similar verb) a certain feature encompasses the meaning of having, being, belonging, causing (or any similar verb) said feature.

Similarly, a low probability of having, being, belonging, causing (or any similar verb) a certain feature encompasses the meaning of not having, not being, not belonging, not causing (or any similar verb) said feature.

The means of the application notably comprise methods, oligonucleotides (primers and/or probes, more particularly primers), sets of oligonucleotides (comprising primers and/or probes, more particularly primers), kits, computer program products and computer devices.

The methods of the application comprise:
- an *in vitro* method for determining that an isolate of *Listeria monocytogenes* has a high probability of belonging to clone CC1, to clone CC2, to clone CC4, to clone CC6, to clone CC7, to clone CC3, to clone CC5, to clone CC8 or CC16, or to clone CC155 of *L. monocytogenes* (wherein a high probability of belonging to one of these clones is indicative that said *L. monocytogenes* isolate has a high probability of having a potentiality, more particularly a high potentiality, of causing invasive infection in a human);
- an *in vitro* method for determining that a *Listeria monocytogenes* isolate has a potentiality, more particularly a high potentiality, of causing invasive infection in a human;
- an *in vitro* method for determining that a *Listeria monocytogenes* isolate, which belongs to one of serogroups IVb, IIb, IIa and IIc, more particularly to serogroup IVb or to serogroup IIb or to one of serogroups IIa and IIc, has a high probability of having a potentiality, more particularly a high potentiality, of causing invasive infection in a human;
- an *in vitro* method for determining whether a *Listeria monocytogenes,* isolate has a high or low probability of having a potentiality, more particularly a high potentiality, of causing invasive infection in a human;
- an *in vitro* method for detecting or determining that a product intended for administration to a human (*e.g*., *via* the oral or intra-gastric route or the intra-peritoneal route, more particularly *via* the oral or intra-gastric route, such as a food or beverage intended for human consumption) is pathogenic *(i.e.,* toxic) to human (due to the presence of *Listeria monocytogenes*), more particularly pathogenic to an immunocompromised human, a pregnant woman, a fetus, a neonate, an infant, a child, or an elderly; and
- an *in vitro* method for detecting or determining that a line of production of a product intended for administration to a human is contaminated (e.g., at the surface of an element of the production line, or in the environment of said production line) by an isolate of *Listeria monocytogenes* that is pathogenic to human.

Said *L. monocytogenes* isolate may more particularly belong to lineage I or II of *L. monocytogenes,* more particularly to one of serogroups IVb, IIb, IIa and IIc of *L. monocytogenes.*

Throughout the application, the expression "*a high probability of having a potentiality*, *more particularly a high potentiality*, *of causing invasive infection in a human*" encompasses the meaning of "*having a potentiality*, *more particularly a high potentiality*, *of causing invasive infection in a human*".

Similarly, the expression "*a low probability of having a potentiality, more particularly a high potentiality*, *of causing invasive infection in a human*" encompasses the meanings of "*having a low potentiality of causing invasive infection in a human*" and "*not having a potentiality, more particularly a high potentiality*, *of causing invasive infection in a human*".

The oligonucleotides (primers and/or probes, more particularly primers), the sets of oligonucleotides (comprising primers and/or probes, more particularly primers), the kits, the computer program products and the computer devices of the application are especially adapted to the implementation of the methods of the application.

Each feature or combination of features, which is described in the context of a method, equally applies to the products as such, and conversely, each feature or combination of features, which is described in the context of a product, equally applies to the methods.

The methods of the application involve detecting that one of genes i.-ix. is present in said *Listeria monocytogenes*, isolate, more particularly that one of genes i.-iv., or one of genes vi. and vii., or one of genes v., viii. and ix. is present in said *L. monocytogenes,* isolate.

Said genes i.-ix. are as follows, respectively:
i. a gene, which is specific of *L. monocytogenes* clone CC1, wherein said CC1-specific gene is *LMOf2365_2702* (SEQ ID NO: 1) or one of the genes of SEQ ID NOs: 61-71,
ii. a gene, which is specific of *L. monocytogenes* clone CC2, wherein said CC2-specific gene is *LMOATCC19117_2417* (SEQ ID NO: 2) or one of the genes of SEQ ID NOs: 72-79,
iii. a gene, which is specific of *L. monocytogenes* clone CC4, wherein said CC4-specific gene is *LMO312_1102* (SEQ ID NO: 3) or one of the genes of SEQ ID NOs: 80-85,
iv. a gene, which is specific of *L. monocytogenes* clone CC6, wherein said CC6-specific gene is *LM1816_RS00050* (SEQ ID NO: 4) or one of the genes of SEQ ID NOs: 86-106,
v. a gene, which is specific of *L. monocytogenes* clone CC7, wherein said CC7-specific gene is *LMRG_02577* (SEQ ID NO: 9) or one of the genes of SEQ ID NOs: 142-149,
vi. a gene, which is specific of *L. monocytogenes* clone CC3, wherein said CC3-specific gene is *LMOSLCC2482_0307* (SEQ ID NO: 5) or one of the genes of SEQ ID NOs: 107-111,
vii. a gene, which is specific of *L. monocytogenes* clone CC5, wherein said CC5-specific gene is *M637*_*08170*(SEQ ID NO: 6) or one of the genes of SEQ ID NOs: 112-122,
viii. a gene, which is specific of *L. monocytogenes* clone(s) CC8 and/or CC16, more particularly which is specific of *L. monocytogenes* clones CC8 and CC16, wherein said CC8-and/or (and) CC16-specific gene of vii. is *LM5578_1185* (SEQ ID NO: 10) or one of the genes of SEQ ID NOs: 150-155, and
ix. a gene, which is specific of *L. monocytogenes* clone CC155, wherein said CC155-specific gene is *LMLG_RS02400* (SEQ ID NO: 8) or one of the genes of SEQ ID NOs: 129-141. The sequences of these genes are described in the examples below, as well as in the appended sequence listing, the content of which is herein incorporated by reference.

More particularrly, said genes i.-ix. are as follows, respectively:
i. a gene, which is specific of *L. monocytogenes* clone CC1, wherein said CC1-specific gene is *LMOf2365_2702* (SEQ ID NO: 1),
ii. a gene, which is specific of *L. monocytogenes* clone CC2, wherein said CC2-specific gene is *LMOATCC19117_2417* (SEQ ID NO: 2),
iii. a gene, which is specific of *L. monocytogenes* clone CC4, wherein said CC4-specific gene is *LM0312_1102* (SEQ ID NO: 3),
iv. a gene, which is specific of *L. monocytogenes* clone CC6, wherein said CC6-specific gene is *LM1816_RS00050* (SEQ ID NO: 4),
v. a gene, which is specific of *L. monocytogenes* clone CC7, wherein said CC7-specific gene is *LMRG_02577* (SEQ ID NO: 9),
vi. a gene, which is specific of *L. monocytogenes* clone CC3, wherein said CC3-specific gene is *LMOSLCC2482_0307* (SEQ ID NO: 5),
vii. a gene, which is specific of *L. monocytogenes* clone CC5, wherein said CC5-specific gene is *M637_08170* (SEQ ID NO: 6),
viii. a gene, which is specific of *L. monocytogenes* clone(s) CC8 and/or CC16, more particularly which is specific of *L. monocytogenes* clones CC8 and CC16, wherein said CC8-and/or CC16-specific gene of vii. is *LM5578_1185* (SEQ ID NO: 10), and
ix. a gene, which is specific of *L. monocytogenes* clone CC155, wherein said CC155-specific gene is *LMLG_RS02400* (SEQ ID NO: 8).

The presence of said gene i. is indicative that said *L. monocytogenes* isolate has a high probability of belonging to clone CC1 of *L. monocytogenes.* The absence of said gene i. is indicative that said *L. monocytogenes* isolate has a low probability of belonging to clone CC1 of *L. monocytogenes.* The presence of said gene ii. is indicative that said *L. monocytogenes* isolate has a high probability of belonging to clone CC2 of *L. monocytogenes.* The absence of said gene ii. is indicative that said *L. monocytogenes* isolate has a low probability of belonging to clone CC2 of *L. monocytogenes.* The presence of said gene iii. is indicative that said *L. monocytogenes* isolate has a high probability of belonging to clone CC4 of *L. monocytogenes.* The absence of said gene iii. is indicative that said *L. monocytogenes* isolate has a low probability of belonging to clone CC4 of *L. monocytogenes.*

The presence of said gene iv. is indicative that said *L. monocytogenes* isolate has a high probability of belonging to clone CC6 of *L. monocytogenes.* The absence of said gene iv. is indicative that said *L. monocytogenes* isolate has a low probability of belonging to clone CC6 of *L. monocytogenes.*

The presence of said gene v. is indicative that said *L. monocytogenes* isolate has a high probability of belonging to clone CC7 of *L. monocytogenes.* The absence of said gene v. is indicative that said *L. monocytogenes* isolate has a low probability of belonging to clone CC7 of *L. monocytogenes.* The presence of said gene vi. is indicative that said *L. monocytogenes* isolate has a high probability of belonging to clone CC3 of *L. monocytogenes.* The absence of said gene vi. is indicative that said *L. monocytogenes* isolate has a low probability of belonging to clone CC3 of *L. monocytogenes.*

The presence of said gene vii. is indicative that said *L. monocytogenes* isolate has a high probability of belonging to clone CC5 of *L. monocytogenes.* The absence of said gene vii. is indicative that said *L. monocytogenes* isolate has a low probability of belonging to clone CC5 of *L. monocytogenes.*

The presence of said gene viii. is indicative that said *L. monocytogenes* isolate has a high probability of belonging to clone CC8 or CC16 of *L. monocytogenes.* The absence of said gene viii. is indicative that said *L. monocytogenes* isolate has a low probability of belonging to clone CC8 or CC16 of *L. monocytogenes.*

The presence of said gene ix. is indicative that said *L. monocytogenes,* isolate has a high probability of belonging to clone CC 155 of *L. monocytogenes.* The absence of said gene ix. is indicative that said *L. monocytogenes* isolate has a low probability of belonging to clone CC155 of *L. monocytogenes.*

The application relates to an *in vitro* method for *Listeria monocytogenes* clonogrouping. More particularly, the application relates to an *in vitro* method for determining that an isolate of *Listeria monocytogenes* has a high probability of belonging to clone CC1, to clone CC2, to clone CC4, to clone CC6, to clone CC7, to clone CC3, to clone CC5, to clone CC8 or CC16, or to clone CC155 of *L. monocytogenes,* wherein a high probability of belonging to one of these clones is indicative that said *L. monocytogenes* isolate has a high probability of having a potentiality, more particularly a high potentiality, of causing invasive infection in a human, wherein said method comprises detecting that one of the said genes i.-ix. is present in said *L. monocytogenes* isolate, wherein the presence of one of said genes i.-ix. is indicative that said *L. monocytogenes* isolate has a high probability of belonging to the clone(s) that is(are) recited in i.-ix., respectively.

Prior to detecting one of said genes i.-ix., said method may comprise determining whether said *L. monocytogenes* isolate does or not belong to one of serogroups IVb, IIb, IIa and IIc, more particularly to serogroup IVb or to serogroup IIb or to one of serogroups IIa and IIc. Said method may then comprise:
when said *L. monocytogenes* isolate belongs to serogroup IVb, detecting that one of said genes i.-iv. is present in said *L. monocytogenes* isolate;
when said *L. monocytogenes* isolate belongs to serogroup IIb, said method comprises detecting that one of said genes vi. and vii. is present in said *L. monocytogenes* isolate; and
when said *L. monocytogenes* isolate belongs to serogroup IIa or IIc, said method comprises detecting that one of said genes v., viii. and ix. is present in said *L. monocytogenes* isolate.

Detecting that one of said genes is present can be performed by determining
the presence or absence of at least one of said genes i.-ix., more particularly the presence or absence of at least one of said genes i.-iv. and/or of at least one of said genes vi.-vii. and/or at least one of said genes v., viii. and ix.,
more particularly the presence or absence of a plurality (or several) of said genes i.-ix., more particularly the presence or absence of said genes i.-iv. and/or of said genes vi.-vii. and/or said genes v., viii. and ix.,
more particularly the presence or absence of each of said genes i.-ix.

Determining the presence or absence of one or several genes can be achieved by any means that the person of average skill in the art may find appropriate.

For example, said determination can be performed by sequencing part or all of the genome of the *L. monocytogenes,* isolate and by *in silico* searching for the presence or absence of said gene(s) (*e.g*., by using computer means to analyze the nucleic acid sequence).

Alternatively or complementarily, said determination can be performed by nucleic acid amplification (using primers or using primers and probes) and/or by nucleic acid hybridization (using nucleic acid probes).

The application thus relates to an *in vitro* method for determining that a *Listeria monocytogenes* isolate has a high probability of having a potentiality, more particularly a high potentiality, of causing invasive infection in a human, wherein said method comprises determining by the (clonogrouping) method of the application that said *L. monocytogenes* isolate has a high probability of belonging to clone CC1, or to clone CC2, or to clone CC4, or to clone CC6, or to clone CC7, or to clone CC3, or to clone CC5, or to clone CC8 or CC16, or to clone CC155 of *L. monocytogenes,* wherein the determination that said *L. monocytogenes* isolate has a high probability of belonging to clone CC1, or to clone CC2, or to clone CC4, or to clone CC6, or to clone CC7, or to clone CC3, or to clone CC5, or to clone CC8 or CC16, or to clone CC155 of *L. monocytogenes,* is indicative that said *L. monocytogenes* isolate has a high probability of having a potentiality, more particularly a high potentiality, of causing invasive infection in a human.

Detecting that one of said genes is present is performed by determining the presence or absence of a plurality of said genes, more particularly by determining the presence or absence of a plurality of genes comprising said genes i.-iv. and/or said genes vi.-vii. and/or said genes v., viii. and ix.

The application also relates to an *in vitro* method for detecting or determining that a product intended for administration to a human, *e.g*., *via* the oral or intra-gastric route or the intra-peritoneal route, more particularly *via* the oral or intra-gastric route, such as a food or beverage intended for human consumption is pathogenic to human (due to the presence of *Listeria monocytogenes*), which comprises determining that said product contains at least one isolate of *Listeria monocytogenes*, which has a high probability of having a potentiality, more particularly a high potentiality, of causing invasive infection in a human in accordance with the *in vitro* (clonogrouping) method of the application.

Said potentiality of causing an invasive infection is particularly high, where the human to which the product is intended to be administered, is an immunocompromised human, a pregnant woman, a fetus, a neonate, an infant, a child, or an elderly.

The application also relates to an *in vi*tro method for detecting or determining that a line of production of a product intended for administration to a human is contaminated (e.g., at the surface of an element of the production line or at a surface in the environnement surrounding the production line) by an isolate of *Listeria monocytogenes,* that is pathogenic to human, which comprises collecting a (biological) sample from said production line (*e.g.*, from the surface of an element of said production), wherein said sample is a sample of
- the raw material(s) that is(are) implemented as starting product(s) of the production process of said product intended for administration to a human and/or of
- the intermediate product(s) that are produced in the course of said production process, and/or of
- the final product(s) that are produced by said production process,
and/or wherein said sample is
- the sample of a (starting, intermediate or final) product contact surface (*e*.*g*., the brine cooling system, casing or wiener peeling equipment (peelers, table tops, hoppers), product conveyors, the device power supply of the slicer, the blade of the slicer, the packing machines, the supports, the carriages, the containers, the balances, and knives) and/or
- the sample of a surface surrounding the production line (*e*.*g*., the ground or floor onto which said production line is positioned or built),
   and determining that said sample contains at least one isolate of *Listeria monocytogenes*, which has a high probability of having a potentiality, more particularly a high potentiality, of causing invasive infection in a human in accordance with the *in vitro* (clonogrouping) method of the application,
   wherein the determination that said sample contains at least one isolate of *Listeria monocytogenes,* which has a high probability of having a potentiality, more particularly a high potentiality, of causing invasive infection in a human is indicative that said production line is contaminated with an isolate of *Listeria monocytogenes* that is pathogenic to human.

Said potentiality of causing an invasive infection is particularly high, where the human to which the product is intended to be administered, is an immunocompromised human, a pregnant woman, a fetus, a neonate, an infant, a child, or an elderly.

More particularly, the application relates to an *in vitro* method for determining that a *Listeria monocytogenes* isolate, which belongs to one of serogroups IVb, IIb, IIa and IIc, more particularly to serogroup IVb or to serogroup IIb or to one of serogroups IIa and IIc, has a high probability of having a potentiality, more particularly a high potentiality, of causing invasive infection in a human, wherein said method comprises
when said *L. monocytogenes*, isolate belongs to serogroup IVb, detecting that one of genes i.-iv. is present in said *L. monocytogenes,* isolate,
when said *L. monocytogenes,* isolate belongs to serogroup IIb, detecting that one of genes vi. and vii. is present in said *L. monocytogenes,* isolate,
when said *L. monocytogenes,* isolate belongs to one of serogroups IIa and IIc, detecting that one of genes v., viii. and ix. is present in said *L. monocytogenes,* isolate;
said genes i.-iv., said genes vi. and vii., and said genes v., viii. and ix. being as above-defined, wherein detecting that one of said genes i.-iv., or of said genes vi. and vii., or of said genes v., viii. and ix., respectively, is present in said *L. monocytogenes* isolate is indicative that said *L. monocytogenes* isolate has a high probability of having a potentiality, more particularly a high potentiality, of causing invasive infection in a human.

In addition to detecting that one of genes v., viii. and ix. is present in said *L. monocytogenes* isolate when said *L. monocytogenes* isolate belongs to one of serogroups IIa and IIc, said method may further comprise determining whether one or none of the further following genes is or are present:
x. a gene, which is specific of *L. monocytogenes* clone CC121, wherein said CC121-specific gene is *LM6179_0610* (SEQ ID NO: 7) or one of the genes of SEQ ID NOs: 123-128, more particularly *LM6179_0610* (SEQ ID NO: 7); and
xi. a gene, which is specific of *L. monocytogenes* clone CC9, wherein said CC9-specific gene is *lmo1118* (SEQ ID NO: 11),
wherein detecting that one of said genes i.-iv., or of said genes vi. and vii., or of said genes v., viii. and ix., respectively, is present in said *L. monocytogenes,* isolate is indicative that said *L. monocytogenes,* isolate has a high probability of having a potentiality, more particularly a high potentiality, of causing invasive infection in a human, and
wherein detecting that one of said genes x. and xi., is present in said *L. monocytogenes* isolate is indicative that said *L. monocytogenes* isolate has a low probability of having a potentiality, more particularly a high potentiality, of causing invasive infection in a human.

The application also relates to an *in vitro* method for determining whether a *Listeria monocytogenes* isolate has a high or low probability of having a potentiality, more particularly a high potentiality, of causing invasive infection in a human, wherein said method comprises determining whether one or none of genes i.-ix. is or are present in said *L. monocytogenes* isolate, said genes i.-ix. being as above-defined; and
when none of genes i.-ix. are present in said *L. monocytogenes* isolate, determining whether said *L. monocytogenes,* isolate does or not belong to (lineage I or II of L. monocytogenes, more particularly to) one of serogroups IVb, IIb, IIa and IIc, more particularly to serogroup IVb or to serogroup IIb or to one of serogroups IIa and IIc;
wherein determining that one of said genes i.-ix. is present in said *L. monocytogenes,* isolate is indicative that said *L. monocytogenes,* isolate has a high probability of having a potentiality , more particurly a high potentiality, of causing invasive infection in a human,
wherein determining that none of said genes i.-ix. are present in said *L. monocytogenes,* isolate and determining that said *L. monocytogenes* isolate belongs to (lineage I of L. monocytogenes, more particularly to) one of serogroups IVb and IIb, is indicative that said *L. monocytogenes* isolate has a high probability of having a potentiality, more particurlarly a high potentiality, of causing invasive infection in a human,
wherein determining that none of said genes i.-ix. are present in said *L. monocytogenes* isolate and determining that said *L. monocytogenes* isolate belongs to (lineage II of L. monocytogenes, more particularly to) one of serogroups IIa and IIc, is indicative that said *L. monocytogenes* isolate has a low probability of having a potentiality, more particularly a high potentiality, of causing invasive infection in a human, and
wherein determining that none of said genes i.-ix. are present in that said *L. monocytogenes* isolate and determining that said *L. monocytogenes,* isolate does not belong to (lineage I or II of L. monocytogenes, more particularly to) any of serogroups IVb, IIb, IIa and IIc is indicative that said *L. monocytogenes* isolate has a low probability of having a potentiality, more particurly a high potentiality, of causing invasive infection in a human.

In addition to determining whether one or none of said genes i.-ix. is or are present, said method may further comprise determining whether one or none of the further following genes is or are present:
x. a gene, which is specific of *L. monocytogenes,* clone CC121, wherein said CC121-specific gene is *LM6179_0610* (SEQ ID NO: 7) or one of the genes of SEQ ID NOs: 123-128, more particularly *LM6179_0610* (SEQ ID NO: 7); and
xi. a gene, which is specific of *L. monocytogenes* clone CC9, wherein said CC9-specific gene is *lmo1118* (SEQ ID NO: 11),
wherein determining that one of said genes i.-ix. is present in said *L. monocytogenes* isolate is indicative that said *L. monocytogenes* isolate has a high probability of having a potentiality, more particurly a high potentiality, of causing invasive infection in a human,
wherein determining that none of said genes i.-xi. are present in said *L. monocytogenes* isolate and determining that said *L. monocytogenes* isolate belongs to (lineage I of L. monocytogenes, more particularly to) one of serogroups IVb and IIb, is indicative that said *L. monocytogenes* isolate has a high probability of having a potentiality, more particurly a high potentiality, of causing invasive infection in a human,
wherein determining that one of said genes x.-xi. is present in said *L. monocytogenes* isolate is indicative that said *L. monocytogenes,* isolate has a low probability of having a potentiality, more particurly a high potentiality, of causing invasive infection in a human,
wherein determining that none of said genes i.-xi. are present in said *L. monocytogenes,* isolate and determining that said *L. monocytogenes,* isolate belongs to (lineage II of L. monocytogenes, more particularly to) one of serogroups IIa and IIc, is indicative that said *L. monocytogenes* isolate has a low probability of having a potentiality, more particurly a high potentiality, of causing invasive infection in a human, and
wherein determining that none of said genes i.-xi. are present in that said *L. monocytogenes* isolate and determining that said *L. monocytogenes* isolate does not belong to (lineage I or II of L. monocytogenes, more particularly to) any of serogroups IVb, IIb, IIa and IIc is indicative that said *L. monocytogenes* isolate has a low probability of having a potentiality, more particurly a high potentiality, of causing invasive infection in a human.

Determining whether said *L. monocytogenes* isolate does or not belong to one of serogroups IVb, IIb, IIa and IIc, more particularly to serogroup IVb or to serogroup IIb or to one of serogroups IIa and IIc, can be performed as described in Doumith *et al.,* 2004, the content of which is herein incorporated by reference.

Hence, determining whether said *L. monocytogenes,* isolate does or not belong to one of serogroups IVb, IIb, IIa and IIc, more particularly to serogroup IVb, or to serogroup IIb, or to one of serogroups IIa and IIc, can be performed by determining the presence or absence of serogroup marker genes, said serogroup marker genes comprising *L. monocytogenes,* genes *lmo0737*, *lmo1118* (SEQ ID NO: 11), ORF2819 and ORF2110.

Determining the presence or absence of said serogroup marker genes is performed by nucleic acid amplification using "serogroup" primers for (specific) nucleic acid amplification from each of said serogroup marker genes. Said "serogroup" primers can be primers, which produce
from marker gene *lmo0737* an amplification product of 691bp,
from marker gene *lmo1118* (SEQ ID NO: 11) an amplification product of 906 bp,
from marker gene ORF2819 an amplification product of 471 bp, and
from marker gene ORF2110 an amplification product of 597 bp.

Said "serogroup" primers may comprise
- the primer pair of SEQ ID NO: 182 and 183 (for nucleic acid amplification from marker gene *lmo0737*), or a primer pair, which anneals to the marker gene *lmo0737* at the same positions as said primer pair of SEQ ID NO: 182 and 183 and which amplifies from this marker gene the same target region as said primer pair of SEQ ID NO: 182 and 183,
- the primer pair of SEQ ID NOs: 47 and 48 (for nucleic acid amplification from marker gene *lmo1118* (SEQ ID NO: 11)), or a primer pair, which anneals to the marker gene *lmo1118* (SEQ ID NO: 11) at the same positions as said primer pair of SEQ ID NOs: 47 and 48 and which amplifies from this marker gene the same target region as said primer pair of SEQ ID NOs: 47 and 48,
- the primer pair of SEQ ID NOs: 184 and 185 (for nucleic acid amplification from marker gene ORF2819), or a primer pair, which anneals to the marker gene at the same positions as said primer pair of SEQ ID NOs: 184 and 185 and which amplifies from this marker gene the same target region as said primer pair of SEQ ID NOs: 184 and 185, and
- the primer pair of SEQ ID NOs: 186 and 187 (for nucleic acid amplification from marker gene ORF2110), or a primer pair, which anneals to the marker gene ORF2110 at the same positions as said primer pair of SEQ ID NOs: 186 and 187 and which amplifies from this marker gene the same target region as said primer pair of SEQ ID NOs: 186 and 187.

A primer pair, which anneals to the marker gene *lmo0737* at the same positions as said primer pair of SEQ ID NO: 182 and 183 and which amplifies from this marker gene the same target region as said primer pair of SEQ ID NO: 182 and 183, may consist of a primer pair, wherein a first oligonucleotide is of the same length as the primer of SEQ ID NO: 182 and is at least 95% (more particularly at least 96%, at least 97%, at least 98% or at least 99%) identical to the primer of SEQ ID NO: 182, and wherein the second oligonucleotide is of the same length as the primer of SEQ ID NO: 183 and is at least 95% (more particularly at least 96%, at least 97%, at least 98% or at least 99%) identical to the primer of SEQ ID NO: 183.

A primer pair, which anneals to the marker gene *lmo1118* (SEQ ID NO: 11) at the same positions as said primer pair of SEQ ID NOs: 47 and 48 and which amplifies from this marker gene the same target region as said primer pair of SEQ ID NOs: 47 and 48, may consist of a primer pair, wherein a first oligonucleotide is of the same length as the primer of SEQ ID NO: 47 and is at least 95% (more particularly at least 96%, at least 97%, at least 98% or at least 99%) identical to the primer of SEQ ID NO: 47, and wherein the second oligonucleotide is of the same length as the primer of SEQ ID NO: 48 and is at least 95% (more particularly at least 96%, at least 97%, at least 98% or at least 99%) identical to the primer of SEQ ID NO: 48.

A primer pair, which anneals to the marker gene ORF2819 at the same positions as said primer pair of SEQ ID NOs: 184 and 185 and which amplifies from this marker gene the same target region as said primer pair of SEQ ID NOs: 184 and 185, may consist of a primer pair, wherein a first oligonucleotide is of the same length as the primer of SEQ ID NO: 184 and is at least 95% (more particularly at least 96%, at least 97%, at least 98% or at least 99%) identical to the primer of SEQ ID NO: 184, and wherein the second oligonucleotide is of the same length as the primer of SEQ ID NO: 185 and is at least 95% (more particularly at least 96%, at least 97%, at least 98% or at least 99%) identical to the primer of SEQ ID NO: 185.

A primer pair, which anneals to the marker gene ORF2110at the same positions as said primer pair of SEQ ID NOs: 186 and 187 and which amplifies from this marker gene the same target region as said primer pair of SEQ ID NOs: 186 and 187, may consist of a primer pair, wherein a first oligonucleotide is of the same length as the primer of SEQ ID NO: 186 and is at least 95% (more particularly at least 96%, at least 97%, at least 98% or at least 99%) identical to the primer of SEQ ID NO: 186, and wherein the second oligonucleotide is of the same length as the primer of SEQ ID NO: 187 and is at least 95% (more particularly at least 96%, at least 97%, at least 98% or at least 99%) identical to the primer of SEQ ID NO: 187.

Said "serogroup" marker genes may further be used with a *Listeria* genus marker gene, such as the *L. monocytogenes prs* gene. Determining the presence or absence of said *L. monocytogenes prs* marker gene can be performed by nucleic acid amplification using primers for nucleic acid amplification from gene *prs.* The *prs* primers may amplify a target region of 370 bp from marker *gene prs.* The *prs* primers may comprise the primer pair of SEQ ID NO: 188 and 189 for nucleic acid amplification from marker gene *prs,* or a primer pair, which anneals to the marker gene *prs* at the same positions as said primer pair of SEQ ID NO: 188 and 189 and which amplifies from this marker gene the same target region as said primer pair of SEQ ID NO: 188 and 189.

The application thus involve
detecting that one of said genes i.-ix., or said genes i.-x., or said genes i-xi., or said genes i.-ix. and xi., more particularly of genes i.-iv. and/or genes vi.-vii. and/or genes v., viii. and ix., is present in said *L. monocytogenes* isolate, or
determining whether one or none of said genes i.-ix., or said genes i.-x., or said genes i-xi., or said genes i.-ix. and xi., more particularly of genes i.-iv. and/or genes vi.-vii. and/or genes v., viii. and ix., is or are present in said *L. monocytogenes* isolate.

Detecting that one of said genes is present or determining whether one or none of said genes is or are present can be performed by determining the presence or absence of a plurality of said genes, more particularly by determining the presence or absence of a plurality of genes comprising said genes i.-iv. and/or said genes vi.-vii. and/or said genes v., viii. and ix.

In the application, a plurality (or several) of genes encompasses at least 2, at least 3, at least 4, at least 5, at least 6, at least 7, at least 8, the 9 of said genes i.-ix., more particularly at least 2 of said genes i.-ix., at least 3, at least 4, more particularly 2, 3 or 4 of said genes i.-ix. For example, a plurality (or several) of genes encompasses at least 2, 3 or the 4 of said genes i.-iv. For example, a plurality (or several) of genes encompasses at least 2 or the 3 of said genes v., viii. and ix. For example, a plurality (or several) of genes encompasses the two of said genes vi. and vii.

Hence, said plurality of genes
comprises said genes i.-iv., or
comprises said genes vi.-vii., or
comprises said genes v., viii. and ix., or
comprises said genes v., and viii.-xi., or
comprises said genes i.-iv. and said genes vi.-vii., or
comprises said genes i.-iv. and said genes v., viii. and ix., or
comprises said genes i.-iv. and said genes v., and viii.-xi., or
comprises said genes vi.-vii. and said genes v., viii. and ix., or
comprises said genes vi.-vii. and said genes v., and viii.-xi., or
comprises said genes i.-iv., said genes vi.-vii. and said genes v., viii. and ix., or comprises said genes i.-iv., said genes vi.-vii. and said genes v., and viii.-xi.

The presence or absence of each gene of said plurality can be determined simultaneously and/or sequentially.

Detecting that one of said genes is present (or determining whether one or none of said genes is or are present) can be performed by simultaneously and/or sequentially (more particularly simultaneously) determining the presence or absence of at least one of said genes i.-ix., more particularly of at least one of said genes i.-iv. and/or of at least one of said genes vi.-vii. and/or at least one of said genes v., viii. and ix., more particularly of several of said genes i.-ix., more particularly of each of said genes i.-ix.

Said determination can be performed simultaneously and/or sequentially (more particularly simultaneously). Each and every combination of simultaneous determination and/or sequential determination is explicitly encompassed in the application.

For example, the presence or absence of several (e.g., 2, 3 or 4) of said genes i.-ix. may be determined simultaneously, and the presence or absence of the other of said genes i.-ix. may be determined sequentially, *i.e.,* before or after (more particularly after) said simultaneous detection. The presence or absence of said other genes i.-ix. may be in turn determined by one or several other simultaneous detection(s) (e.g., two separate simultaneous detections).

For example:
- the presence or absence of said genes i.-iv. can be determined simultaneously,
- the presence or absence of said genes vi.-vii. can be determined simultaneously,
- the presence or absence of said genes v., viii. and ix. can be determined simultaneously, and
- each of these three simultaneous determinations can be performed simultaneously or sequentially with respect to each other.

Said determination can be performed in separate or common containers, *e*.*g*., in separate or common
- tube(s) or multiwell plate(s) suitable for nucleic acid amplification *(i.e.,* suitable for receiving a reaction mixture containing oligonucleotides, *e*.*g*., primers, or primers and probes, for nucleic acid amplification), or
- solid support(s), such as array(s) (e.g., macro-array(s), micro-array(s) or nano-array(s)), chip(s) or microbeads, wherein said solid support(s) is(are) suitable for nucleic acid hybridization or for sequencing (and wherein said solid support(s) may contain oligonucleotides, *e*.*g*., probes, (covalently) linked thereto).

More particularly, the presence or absence of each gene of said plurality is determined in or on the same container. For example, the simultaneous determination of the presence or absence of said genes i.-iv. can be performed in or on a first container (e.g., a first tube suitable for nucleic acid amplification), which is distinct from a second container (e.g., a second tube suitable for nucleic acid amplification) in or on which the simultaneous determination of the presence or absence of said genes vi.-vii. is performed, and which is also distinct from a third container (e.g., a third tube suitable for nucleic acid amplification) in or on which the simultaneous determination of the presence or absence of said genes v., viii. and ix. is performed.

Said container(s) may contain reagents (e.g., primers, or probes or primers and probes) suitable for determining the presence or absence of each of said genes.

Primers and/or probes can be used to determine the presence or absence of each gene of said plurality. These primers and/or probes advantageously are
- primer pairs,
- primer pairs and probes, or
- probes,
more particularly primer pairs,
wherein each primer pair, or primer pair and probe, or probe, is specific of a (different) gene of said plurality, thereby providing a set of primer pairs, or primer pairs and probes, or probes, which (specifically) detects the whole range of genes of said plurality.

When the presence or absence of each gene of said plurality is determined in or on the same container, said container may thus contain these primers and/or probes.

Said primers may comprise at least one primer pair suitable for nucleic acid amplification from one of genes i.-ix., more particularly at least one first primer pair suitable for nucleic acid amplification from one of genes i.-ix. and at least one second primer pair suitable for nucleic acid amplification from another of genes i.-ix.

Said primers may comprise several primer pairs, wherein each primer pair is suitable for nucleic acid amplification from different genes, said different genes being selected from genes i.-ix.

More particularly, said primers may comprise several primer pairs, wherein each primer pair is suitable for nucleic acid amplification from different genes, said different genes being selected from genes i.-iv. and/or from genes vi.-vii. and/or from genes v., viii. and ix.

More particularly, said primers may comprise several primer pairs, wherein each primer pair is suitable for nucleic acid amplification from different genes, said different genes being each one of genes i.-iv. and/or each one of genes vi.-vii. and/or each one of genes v., viii. and ix.

More particularly, said primers may comprise several primer pairs, wherein each primer pair is suitable for nucleic acid amplification from different genes, said different genes being each of genes i.-ix.

Said nucleic acid amplification is specific of each of said genes, more particularly of each of said genes i.-iv. and/or each of said genes vi.-vii. and/or each of said genes v., viii. and ix. Hence, the sequences of a primer pair are such that the presence of an amplification product (*i.e.,* of an amplicon produced using the primer pair in an amplification reaction) is indicative that said gene is present and, conversely, the absence of amplification product is indicative that said gene is absent. In other words, the primer pair is specific of the gene.

Advantageously, said genes i.-iv. are the genes of SEQ ID NOs: 1-4 and/or of said genes vi.-vii. are the genes of SEQ ID NOs: 5-6 and/or said genes v., viii. and ix. are the genes of SEQ ID NOs: 9, 10 and 8, respectively.

The typical length of a primer is 15-30 nucleotides. In the application, a primer consists of 15-30 nucleotides, more particularly 19-25 nucleotides, more particularly of 20-24 nucleotides, *i.e.,* of 20, 21, 22, 23 or 24 nucleotides.

The Guanine + Cytosine (G+C) content of a primer typically is of 40-60%.

For example, for specific amplification from said gene i. of SEQ ID NO: 1 (clone CC1), said primers may comprise
- the primer pair of SEQ ID NO: 17 and 18, or
- a primer pair, which still is specific of gene i. of SEQ ID NO: 1 and which anneals to gene i. of SEQ ID NO: 1 at positions that differ by ± 5 nucleotides (more particularly by ± 4 nucleotides, by ± 3 nucleotides, by ± 2 nucleotides, by ± 1 nucleotides or by 0 nucleotide) from the positions at which the primer pair of SEQ ID NOs: 17 and 18 anneals to gene i. of SEQ ID NO: 1; or
- a primer pair, which still is specific of gene i. of SEQ ID NO: 1, and which anneals to gene i. of SEQ ID NO: 1 to amplify
   a fragment from gene i. of SEQ ID NO: 1, wherein said fragment comprises the (target) sequence SEQ ID NO: 16 that is amplified from gene i. of SEQ ID NO: 1 by the primer pair of SEQ ID NOs: 17 and 18 (*i.e*., the target sequence of SEQ ID NO: 16) and is at most 10 nucleotides longer than said target sequence (more particularly at most 9, 8, 7, 6, 5, 4, 3, 2, 1 or 0 nucleotide(s) longer than said target sequence); or
   a fragment from gene i. of SEQ ID NO: 1, wherein said fragment consists of the (target) sequence that is amplified from gene i. of SEQ ID NO: 1 by the primer pair of SEQ ID NOs: 17 and 18 (*i.e*., the sequence of SEQ ID NO: 16) but deleted from at most 10 nucleotides (more particularly at most 9, 8, 7, 6, 5, 4, 3, 2, 1 or 0 nucleotide(s)); or
- a primer pair, which anneals to gene i. of SEQ ID NO: 1 to specifically amplify (from said gene) the same target sequence as said primer pair of SEQ ID NOs: 17 and 18 (*i.e*., the sequence of SEQ ID NO: 16), wherein the first primer of said primer pair is at least 95% (more particularly at least 96%, at least 97%, at least 98% or at least 99%) identical to the primer of SEQ ID NO: 17 and the second primer of said primer pair is at least 95% (more particularly at least 96%, at least 97%, at least 98% or at least 99%) identical to the primer of SEQ ID NO: 18.

More particularly, for specific amplification from said gene i. of SEQ ID NO: 1, said primers may comprise a primer pair for specifically amplifying (from said gene) the same target sequence as the primer pair of SEQ ID NOs: 17 and 18 (*i.e*., the target sequence of SEQ ID NO: 16).

More particularly, for specific amplification from said gene i. of SEQ ID NO: 1, said primers may comprise a primer pair for specifically amplifying (from said gene) the same target sequence as the primer pair of SEQ ID NOs: 17 and 18 (*i.e*., the target sequence of SEQ ID NO: 16), wherein said primer pair is
- the primer pair of SEQ ID NOs: 17 and 18, or
- a primer pair, which anneals to gene i. of SEQ ID NO: 1 to specifically amplify (from said gene) the same target sequence as said primer pair of SEQ ID NOs: 17 and 18 (*i.e*., the target sequence of SEQ ID NO: 16), wherein the first primer of said primer pair is at least 95% (more particularly at least 96%, at least 97%, at least 98% or at least 99%) identical to the primer of SEQ ID NO: 17 and the second primer of said primer pair is at least 95% (more particularly at least 96%, at least 97%, at least 98% or at least 99%) identical to the primer of SEQ ID NO: 18.

Each of the first and second primers of the primer pair may independently consist of 15-30 nucleotides, more particularly 19-25 nucleotides, more particularly of 20-24 nucleotides, *i.e*., of 20, 21, 22, 23 or 24 nucleotides. More particularly, the first nucleotide of the primer pair may be of the same nucleotide length as the primer of SEQ ID NO: 17 and the second nucleotide may be of the same nucleotide length as the primer of SEQ ID NO: 18. The GC content of each of said first and second primers may independently be of 40-60%.

For example, for specific amplification from said gene ii. of SEQ ID NO: 2 (clone CC2), said primers may comprise
- the primer pair of SEQ ID NO: 20 and 21, or
- a primer pair, which still is specific of gene ii. of SEQ ID NO: 2 and which anneals to gene ii. of SEQ ID NO: 2 at positions that differ by ± 5 nucleotides (more particularly by ± 4 nucleotides, by ± 3 nucleotides, by ± 2 nucleotides, by ± 1 nucleotides or by 0 nucleotide) from the positions at which the primer pair of SEQ ID NOs: 20 and 21 anneals to gene ii. of SEQ ID NO: 2; or
- a primer pair, which still is specific of gene ii. of SEQ ID NO: 2, and which anneals to gene ii. of SEQ ID NO: 2 to amplify
   a fragment from gene ii. of SEQ ID NO: 2, wherein said fragment comprises the (target) sequence SEQ ID NO: 19 that is amplified from gene ii. of SEQ ID NO: 2 by the primer pair of SEQ ID NOs: 20 and 21 (*i.e*., the target sequence of SEQ ID NO: 19) and is at most 10 nucleotides longer than said target sequence (more particularly at most 9, 8, 7, 6, 5, 4, 3, 2, 1 or 0 nucleotide(s) longer than said target sequence); or
   a fragment from gene ii. of SEQ ID NO: 2, wherein said fragment consists of the (target) sequence that is amplified from gene ii. of SEQ ID NO: 2 by the primer pair of SEQ ID NOs: 20 and 21 (*i*.*e*., the sequence of SEQ ID NO: 19) but deleted from at most 10 nucleotides (more particularly at most 9, 8, 7, 6, 5, 4, 3, 2, 1 or 0 nucleotide(s)); or
- a primer pair, which anneals to gene ii. of SEQ ID NO: 2 to specifically amplify (from said gene) the same target sequence as said primer pair of SEQ ID NOs: 20 and 21 (*i.e*., the sequence of SEQ ID NO: 19), wherein the first primer of said primer pair is at least 95% (more particularly at least 96%, at least 97%, at least 98% or at least 99%) identical to the primer of SEQ ID NO: 20 and the second primer of said primer pair is at least 95% (more particularly at least 96%, at least 97%, at least 98% or at least 99%) identical to the primer of SEQ ID NO: 21.

More particularly, for specific amplification from said gene ii. of SEQ ID NO: 2, said primers may comprise a primer pair for specifically amplifying (from said gene) the same target sequence as the primer pair of SEQ ID NOs: 20 and 21 (*i*.*e*., the target sequence of SEQ ID NO: 19).

More particularly, for specific amplification from said gene ii. of SEQ ID NO: 2, said primers may comprise a primer pair for specifically amplifying (from said gene) the same target sequence as the primer pair of SEQ ID NOs: 20 and 21 (*i.e*., the target sequence of SEQ ID NO: 19), wherein said primer pair is
- the primer pair of SEQ ID NOs: 20 and 21, or
- a primer pair, which anneals to gene ii. of SEQ ID NO: 2 to specifically amplify (from said gene) the same target sequence as said primer pair of SEQ ID NOs: 20 and 21 (*i.e*., the target sequence of SEQ ID NO: 19), wherein the first primer of said primer pair is at least 95% (more particularly at least 96%, at least 97%, at least 98% or at least 99%) identical to the primer of SEQ ID NO: 20 and the second primer of said primer pair is at least 95% (more particularly at least 96%, at least 97%, at least 98% or at least 99%) identical to the primer of SEQ ID NO: 21.

Each of the first and second primers of the primer pair may independently consist of 15-30 nucleotides, more particularly 19-25 nucleotides, more particularly of 20-24 nucleotides, *i.e*., of 20, 21, 22, 23 or 24 nucleotides. More particularly, the first nucleotide of the primer pair may be of the same nucleotide length as the primer of SEQ ID NO: 20 and the second nucleotide may be of the same nucleotide length as the primer of SEQ ID NO: 21. The GC content of each of said first and second primers may independently be of 40-60%.

For example, for specific amplification from said gene iii. of SEQ ID NO: 3 (clone CC4), said primers may comprise
- the primer pair of SEQ ID NO: 23 and 24, or
- a primer pair, which still is specific of gene iii. of SEQ ID NO: 3 and which anneals to gene iii. of SEQ ID NO: 3 at positions that differ by ± 5 nucleotides (more particularly by ± 4 nucleotides, by ± 3 nucleotides, by ± 2 nucleotides, by ± 1 nucleotides or by 0 nucleotide) from the positions at which the primer pair of SEQ ID NOs: 23 and 24 anneals to gene iii. of SEQ ID NO: 3; or
- a primer pair, which still is specific of gene iii. of SEQ ID NO: 3, and which anneals to gene iii. of SEQ ID NO: 3 to amplify
   a fragment from gene iii. of SEQ ID NO: 3, wherein said fragment comprises the (target) sequence SEQ ID NO: 22 that is amplified from gene iii. of SEQ ID NO: 3 by the primer pair of SEQ ID NOs: 23 and 24 (*i*.*e*., the target sequence of SEQ ID NO: 22) and is at most 10 nucleotides longer than said target sequence (more particularly at most 9, 8, 7, 6, 5, 4, 3, 2, 1 or 0 nucleotide(s) longer than said target sequence); or
   a fragment from gene iii. of SEQ ID NO: 3, wherein said fragment consists of the (target) sequence that is amplified from gene iii. of SEQ ID NO: 3 by the primer pair of SEQ ID NOs: 23 and 24 (*i*.*e*., the sequence of SEQ ID NO: 22) but deleted from at most 10 nucleotides (more particularly at most 9, 8, 7, 6, 5, 4, 3, 2, 1 or 0 nucleotide(s)); or
- a primer pair, which anneals to gene iii. of SEQ ID NO: 3 to specifically amplify (from said gene) the same target sequence as said primer pair of SEQ ID NOs: 23 and 24 (*i*.*e*., the sequence of SEQ ID NO: 22), wherein the first primer of said primer pair is at least 95% (more particularly at least 96%, at least 97%, at least 98% or at least 99%) identical to the primer of SEQ ID NO: 23 and the second primer of said primer pair is at least 95% (more particularly at least 96%, at least 97%, at least 98% or at least 99%) identical to the primer of SEQ ID NO: 24.

More particularly, for specific amplification from said gene iii. of SEQ ID NO: 3, said primers may comprise a primer pair for specifically amplifying (from said gene) the same target sequence as the primer pair of SEQ ID NOs: 23 and 24 (*i*.*e*., the target sequence of SEQ ID NO: 22).

More particularly, for specific amplification from said gene iii. of SEQ ID NO: 3, said primers may comprise a primer pair for specifically amplifying (from said gene) the same target sequence as the primer pair of SEQ ID NOs: 23 and 24 (*i.e.,* the target sequence of SEQ ID NO: 22), wherein said primer pair is
- the primer pair of SEQ ID NOs: 23 and 24, or
- a primer pair, which anneals to gene iii. of SEQ ID NO: 3 to specifically amplify (from said gene) the same target sequence as said primer pair of SEQ ID NOs: 23 and 24 *(i.e.,* the target sequence of SEQ ID NO: 22), wherein the first primer of said primer pair is at least 95% (more particularly at least 96%, at least 97%, at least 98% or at least 99%) identical to the primer of SEQ ID NO: 23 and the second primer of said primer pair is at least 95% (more particularly at least 96%, at least 97%, at least 98% or at least 99%) identical to the primer of SEQ ID NO: 24.

Each of the first and second primers of the primer pair may independently consist of 15-30 nucleotides, more particularly 19-25 nucleotides, more particularly of 20-24 nucleotides, *i.e.,* of 20, 21, 22, 23 or 24 nucleotides. More particularly, the first nucleotide of the primer pair may be of the same nucleotide length as the primer of SEQ ID NO: 23 and the second nucleotide may be of the same nucleotide length as the primer of SEQ ID NO: 24. The GC content of each of said first and second primers may independently be of 40-60%.

For example, for specific amplification from said gene iv. of SEQ ID NO: 4 (clone CC6), said primers may comprise
- the primer pair of SEQ ID NO: 26 and 27, or
- a primer pair, which still is specific of gene iv. of SEQ ID NO: 4 and which anneals to gene iv. of SEQ ID NO: 4 at positions that differ by ± 5 nucleotides (more particularly by ± 4 nucleotides, by ± 3 nucleotides, by ± 2 nucleotides, by ± 1 nucleotides or by 0 nucleotide) from the positions at which the primer pair of SEQ ID NOs: 26 and 27 anneals to gene iv. of SEQ ID NO: 4; or
- a primer pair, which still is specific of gene iv. of SEQ ID NO: 4, and which anneals to gene iv. of SEQ ID NO: 4 to amplify
   a fragment from gene iv. of SEQ ID NO: 4, wherein said fragment comprises the (target) sequence SEQ ID NO: 25 that is amplified from gene iv. of SEQ ID NO: 4 by the primer pair of SEQ ID NOs: 26 and 27 (*i*.*e*., the target sequence of SEQ ID NO: 25) and is at most 10 nucleotides longer than said target sequence (more particularly at most 9, 8, 7, 6, 5, 4, 3, 2, 1 or 0 nucleotide(s) longer than said target sequence); or
   a fragment from gene iv. of SEQ ID NO: 4, wherein said fragment consists of the (target) sequence that is amplified from gene iv. of SEQ ID NO: 4 by the primer pair of SEQ ID NOs: 26 and 27 (*i*.*e*., the sequence of SEQ ID NO: 25) but deleted from at most 10 nucleotides (more particularly at most 9, 8, 7, 6, 5, 4, 3, 2, 1 or 0 nucleotide(s)); or
- a primer pair, which anneals to gene iv. of SEQ ID NO: 4 to specifically amplify (from said gene) the same target sequence as said primer pair of SEQ ID NOs: 26 and 27 (*i*.*e*., the sequence of SEQ ID NO: 25), wherein the first primer of said primer pair is at least 95% (more particularly at least 96%, at least 97%, at least 98% or at least 99%) identical to the primer of SEQ ID NO: 26 and the second primer of said primer pair is at least 95% (more particularly at least 96%, at least 97%, at least 98% or at least 99%) identical to the primer of SEQ ID NO: 27.

More particularly, for specific amplification from said gene iv. of SEQ ID NO: 4, said primers may comprise a primer pair for specifically amplifying (from said gene) the same target sequence as the primer pair of SEQ ID NOs: 26 and 27 *(i.e.,* the target sequence of SEQ ID NO: 25).

More particularly, for specific amplification from said gene iv. of SEQ ID NO: 4, said primers may comprise a primer pair for specifically amplifying (from said gene) the same target sequence as the primer pair of SEQ ID NOs: 26 and 27 (*i*.*e*., the target sequence of SEQ ID NO: 25), wherein said primer pair is
- the primer pair of SEQ ID NOs: 26 and 27, or
- a primer pair, which anneals to gene iv. of SEQ ID NO: 4 to specifically amplify (from said gene) the same target sequence as said primer pair of SEQ ID NOs: 26 and 27 *(i.e.,* the target sequence of SEQ ID NO: 25), wherein the first primer of said primer pair is at least 95% (more particularly at least 96%, at least 97%, at least 98% or at least 99%) identical to the primer of SEQ ID NO: 26 and the second primer of said primer pair is at least 95% (more particularly at least 96%, at least 97%, at least 98% or at least 99%) identical to the primer of SEQ ID NO: 27.

Each of the first and second primers of the primer pair may independently consist of 15-30 nucleotides, more particularly 19-25 nucleotides, more particularly of 20-24 nucleotides, *i.e.,* of 20, 21, 22, 23 or 24 nucleotides. More particularly, the first nucleotide of the primer pair may be of the same nucleotide length as the primer of SEQ ID NO: 26 and the second nucleotide may be of the same nucleotide length as the primer of SEQ ID NO: 27. The GC content of each of said first and second primers may independently be of 40-60%.

For example, for specific amplification from said gene vi. of SEQ ID NO: 5 (clone CC3), said primers may comprise
- the primer pair of SEQ ID NO: 29 and 30, or
- a primer pair, which still is specific of gene vi. of SEQ ID NO: 5 and which anneals to gene vi. of SEQ ID NO: 5 at positions that differ by ± 5 nucleotides (more particularly by ± 4 nucleotides, by ± 3 nucleotides, by ± 2 nucleotides, by ± 1 nucleotides or by 0 nucleotide) from the positions at which the primer pair of SEQ ID NOs: 29 and 30 anneals to gene vi. of SEQ ID NO: 5; or
- a primer pair, which still is specific of gene vi. of SEQ ID NO: 5, and which anneals to gene vi. of SEQ ID NO: 5 to amplify
   a fragment from gene vi. of SEQ ID NO: 5, wherein said fragment comprises the (target) sequence SEQ ID NO: 28 that is amplified from gene vi. of SEQ ID NO: 5 by the primer pair of SEQ ID NOs: 29 and 30 (*i*.*e*., the target sequence of SEQ ID NO: 28) and is at most 10 nucleotides longer than said target sequence (more particularly at most 9, 8, 7, 6, 5, 4, 3, 2, 1 or 0 nucleotide(s) longer than said target sequence); or
   a fragment from gene vi. of SEQ ID NO: 5, wherein said fragment consists of the (target) sequence that is amplified from gene vi. of SEQ ID NO: 5 by the primer pair of SEQ ID NOs: 29 and 30 (*i*.*e*., the sequence of SEQ ID NO: 28) but deleted from at most 10 nucleotides (more particularly at most 9, 8, 7, 6, 5, 4, 3, 2, 1 or 0 nucleotide(s)); or
- a primer pair, which anneals to gene vi. of SEQ ID NO: 5 to specifically amplify (from said gene) the same target sequence as said primer pair of SEQ ID NOs: 29 and 30 *(i.e.,* the sequence of SEQ ID NO: 28), wherein the first primer of said primer pair is at least 95% (more particularly at least 96%, at least 97%, at least 98% or at least 99%) identical to the primer of SEQ ID NO: 29 and the second primer of said primer pair is at least 95% (more particularly at least 96%, at least 97%, at least 98% or at least 99%) identical to the primer of SEQ ID NO: 30.

More particularly, for specific amplification from said gene vi. of SEQ ID NO: 5, said primers may comprise a primer pair for specifically amplifying (from said gene) the same target sequence as the primer pair of SEQ ID NOs: 29 and 30 (*i*.*e*., the target sequence of SEQ ID NO: 28).

More particularly, for specific amplification from said gene vi. of SEQ ID NO: 5, said primers may comprise a primer pair for specifically amplifying (from said gene) the same target sequence as the primer pair of SEQ ID NOs: 29 and 30 (*i*.*e*., the target sequence of SEQ ID NO: 28), wherein said primer pair is
- the primer pair of SEQ ID NOs: 29 and 30, or
- a primer pair, which anneals to gene vi. of SEQ ID NO: 5 to specifically amplify (from said gene) the same target sequence as said primer pair of SEQ ID NOs: 29 and 30 *(i.e.,* the target sequence of SEQ ID NO: 28), wherein the first primer of said primer pair is at least 95% (more particularly at least 96%, at least 97%, at least 98% or at least 99%) identical to the primer of SEQ ID NO: 29 and the second primer of said primer pair is at least 95% (more particularly at least 96%, at least 97%, at least 98% or at least 99%) identical to the primer of SEQ ID NO: 30.

Each of the first and second primers of the primer pair may independently consist of 15-30 nucleotides, more particularly 19-25 nucleotides, more particularly of 20-24 nucleotides, *i.e*., of 20, 21, 22, 23 or 24 nucleotides. More particularly, the first nucleotide of the primer pair may be of the same nucleotide length as the primer of SEQ ID NO: 29 and the second nucleotide may be of the same nucleotide length as the primer of SEQ ID NO: 30. The GC content of each of said first and second primers may independently be of 40-60%.

For example, for specific amplification from said gene vii. of SEQ ID NO: 6 (clone CC5), said primers may comprise
- the primer pair of SEQ ID NO: 32 and 33, or
- a primer pair, which still is specific of gene vii. of SEQ ID NO: 6 and which anneals to gene vii. of SEQ ID NO: 6 at positions that differ by ± 5 nucleotides (more particularly by ± 4 nucleotides, by ± 3 nucleotides, by ± 2 nucleotides, by ± 1 nucleotides or by 0 nucleotide) from the positions at which the primer pair of SEQ ID NOs: 32 and 33 anneals to gene vii. of SEQ ID NO: 6; or
- a primer pair, which still is specific of gene vii. of SEQ ID NO: 6, and which anneals to gene vii. of SEQ ID NO: 6 to amplify
   a fragment from gene vii. of SEQ ID NO: 6, wherein said fragment comprises the (target) sequence SEQ ID NO: 31 that is amplified from gene vii. of SEQ ID NO: 6 by the primer pair of SEQ ID NOs: 32 and 33 (*i.e*., the target sequence of SEQ ID NO: 31) and is at most 10 nucleotides longer than said target sequence (more particularly at most 9, 8, 7, 6, 5, 4, 3, 2, 1 or 0 nucleotide(s) longer than said target sequence); or
   a fragment from gene vii. of SEQ ID NO: 6, wherein said fragment consists of the (target) sequence that is amplified from gene vii. of SEQ ID NO: 6 by the primer pair of SEQ ID NOs: 32 and 33 (*i*.*e*., the sequence of SEQ ID NO: 31) but deleted from at most 10 nucleotides (more particularly at most 9, 8, 7, 6, 5, 4, 3, 2, 1 or 0 nucleotide(s)); or
- a primer pair, which anneals to gene vii. of SEQ ID NO: 6 to specifically amplify (from said gene) the same target sequence as said primer pair of SEQ ID NOs: 32 and 33 (*i.e*., the sequence of SEQ ID NO: 31), wherein the first primer of said primer pair is at least 95% (more particularly at least 96%, at least 97%, at least 98% or at least 99%) identical to the primer of SEQ ID NO: 32 and the second primer of said primer pair is at least 95% (more particularly at least 96%, at least 97%, at least 98% or at least 99%) identical to the primer of SEQ ID NO: 33.

More particularly, for specific amplification from said gene vii. of SEQ ID NO: 6, said primers may comprise a primer pair for specifically amplifying (from said gene) the same target sequence as the primer pair of SEQ ID NOs: 32 and 33 (*i*.*e*., the target sequence of SEQ ID NO: 31).

More particularly, for specific amplification from said gene vii. of SEQ ID NO: 6, said primers may comprise a primer pair for specifically amplifying (from said gene) the same target sequence as the primer pair of SEQ ID NOs: 32 and 33 (*i.e*., the target sequence of SEQ ID NO: 31), wherein said primer pair is
- the primer pair of SEQ ID NOs: 32 and 33, or
- a primer pair, which anneals to gene vii. of SEQ ID NO: 6 to specifically amplify (from said gene) the same target sequence as said primer pair of SEQ ID NOs: 32 and 33 (*i.e*., the target sequence of SEQ ID NO: 31), wherein the first primer of said primer pair is at least 95% (more particularly at least 96%, at least 97%, at least 98% or at least 99%) identical to the primer of SEQ ID NO: 32 and the second primer of said primer pair is at least 95% (more particularly at least 96%, at least 97%, at least 98% or at least 99%) identical to the primer of SEQ ID NO: 33.

Each of the first and second primers of the primer pair may independently consist of 15-30 nucleotides, more particularly 19-25 nucleotides, more particularly of 20-24 nucleotides, *i.e*., of 20, 21, 22, 23 or 24 nucleotides. More particularly, the first nucleotide of the primer pair may be of the same nucleotide length as the primer of SEQ ID NO: 32 and the second nucleotide may be of the same nucleotide length as the primer of SEQ ID NO: 33. The GC content of each of said first and second primers may independently be of 40-60%.

For example, for specific amplification from said gene v. of SEQ ID NO: 9 (clone CC7), said primers may comprise
- the primer pair of SEQ ID NO: 41 and 42, or
- a primer pair, which still is specific of gene v. of SEQ ID NO: 9 and which anneals to gene v. of SEQ ID NO: 9 at positions that differ by ± 5 nucleotides (more particularly by ± 4 nucleotides, by ± 3 nucleotides, by ± 2 nucleotides, by ± 1 nucleotides or by 0 nucleotide) from the positions at which the primer pair of SEQ ID NOs: 41 and 42 anneals to gene v. of SEQ ID NO: 9; or
- a primer pair, which still is specific of gene v. of SEQ ID NO: 9, and which anneals to gene v. of SEQ ID NO: 9 to amplify
   a fragment from gene v. of SEQ ID NO: 9, wherein said fragment comprises the (target) sequence SEQ ID NO: 40 that is amplified from gene v. of SEQ ID NO: 9 by the primer pair of SEQ ID NOs: 41 and 42 (*i*.*e*., the target sequence of SEQ ID NO: 40) and is at most 10 nucleotides longer than said target sequence (more particularly at most 9, 8, 7, 6, 5, 4, 3, 2, 1 or 0 nucleotide(s) longer than said target sequence); or
   a fragment from gene v. of SEQ ID NO: 9, wherein said fragment consists of the (target) sequence that is amplified from gene v. of SEQ ID NO: 9 by the primer pair of SEQ ID NOs: 41 and 42 (*i*.*e*., the sequence of SEQ ID NO: 40) but deleted from at most 10 nucleotides (more particularly at most 9, 8, 7, 6, 5, 4, 3, 2, 1 or 0 nucleotide(s)); or
- a primer pair, which anneals to gene v. of SEQ ID NO: 9 to specifically amplify (from said gene) the same target sequence as said primer pair of SEQ ID NOs: 41 and 42 *(i.e.,* the sequence of SEQ ID NO: 40), wherein the first primer of said primer pair is at least 95% (more particularly at least 96%, at least 97%, at least 98% or at least 99%) identical to the primer of SEQ ID NO: 41 and the second primer of said primer pair is at least 95% (more particularly at least 96%, at least 97%, at least 98% or at least 99%) identical to the primer of SEQ ID NO: 42.

More particularly, for specific amplification from said gene v. of SEQ ID NO: 9, said primers may comprise a primer pair for specifically amplifying (from said gene) the same target sequence as the primer pair of SEQ ID NOs: 41 and 42 (*i*.*e*., the target sequence of SEQ ID NO: 40).

More particularly, for specific amplification from said gene v. of SEQ ID NO: 9, said primers may comprise a primer pair for specifically amplifying (from said gene) the same target sequence as the primer pair of SEQ ID NOs: 41 and 42 (*i*.*e*., the target sequence of SEQ ID NO: 40), wherein said primer pair is
- the primer pair of SEQ ID NOs: 41 and 42, or
- a primer pair, which anneals to gene v. of SEQ ID NO: 9 to specifically amplify (from said gene) the same target sequence as said primer pair of SEQ ID NOs: 41 and 42 (*i.e*., the target sequence of SEQ ID NO: 40), wherein the first primer of said primer pair is at least 95% (more particularly at least 96%, at least 97%, at least 98% or at least 99%) identical to the primer of SEQ ID NO: 41 and the second primer of said primer pair is at least 95% (more particularly at least 96%, at least 97%, at least 98% or at least 99%) identical to the primer of SEQ ID NO: 42.

Each of the first and second primers of the primer pair may independently consist of 15-30 nucleotides, more particularly 19-25 nucleotides, more particularly of 20-24 nucleotides, *i.e*., of 20, 21, 22, 23 or 24 nucleotides. More particularly, the first nucleotide of the primer pair may be of the same nucleotide length as the primer of SEQ ID NO: 41 and the second nucleotide may be of the same nucleotide length as the primer of SEQ ID NO: 42. The GC content of each of said first and second primers may independently be of 40-60%.

For example, for specific amplification from said gene viii. of SEQ ID NO: 10 (clone CC8 and/or (and) CC16)), said primers may comprise
- the primer pair of SEQ ID NO: 44 and 45, or
- a primer pair, which still is specific of gene viii. of SEQ ID NO: 10 and which anneals to gene viii. of SEQ ID NO: 10 at positions that differ by ± 5 nucleotides (more particularly by ± 4 nucleotides, by ± 3 nucleotides, by ± 2 nucleotides, by ± 1 nucleotides or by 0 nucleotide) from the positions at which the primer pair of SEQ ID NOs: 44 and 45 anneals to gene viii. of SEQ ID NO: 10; or
- a primer pair, which still is specific of gene viii. of SEQ ID NO: 10, and which anneals to gene viii. of SEQ ID NO: 10 to amplify
   a fragment from gene viii. of SEQ ID NO: 10, wherein said fragment comprises the (target) sequence SEQ ID NO: 43 that is amplified from gene viii. of SEQ ID NO: 10 by the primer pair of SEQ ID NOs: 44 and 45 (*i*.*e*., the target sequence of SEQ ID NO: 43) and is at most 10 nucleotides longer than said target sequence (more particularly at most 9, 8, 7, 6, 5, 4, 3, 2, 1 or 0 nucleotide(s) longer than said target sequence); or
   a fragment from gene viii. of SEQ ID NO: 10, wherein said fragment consists of the (target) sequence that is amplified from gene viii. of SEQ ID NO: 10 by the primer pair of SEQ ID NOs: 44 and 45 (*i*.*e*., the sequence of SEQ ID NO: 43) but deleted from at most 10 nucleotides (more particularly at most 9, 8, 7, 6, 5, 4, 3, 2, 1 or 0 nucleotide(s)); or
- a primer pair, which anneals to gene viii. of SEQ ID NO: 10 to specifically amplify (from said gene) the same target sequence as said primer pair of SEQ ID NOs: 44 and 45 (*i*.*e*., the sequence of SEQ ID NO: 43), wherein the first primer of said primer pair is at least 95% (more particularly at least 96%, at least 97%, at least 98% or at least 99%) identical to the primer of SEQ ID NO: 44 and the second primer of said primer pair is at least 95% (more particularly at least 96%, at least 97%, at least 98% or at least 99%) identical to the primer of SEQ ID NO: 45.

More particularly, for specific amplification from said gene viii. of SEQ ID NO: 10, said primers may comprise a primer pair for specifically amplifying (from said gene) the same target sequence as the primer pair of SEQ ID NOs: 44 and 45 (*i.e*., the target sequence of SEQ ID NO: 43).

More particularly, for specific amplification from said gene viii. of SEQ ID NO: 10, said primers may comprise a primer pair for specifically amplifying (from said gene) the same target sequence as the primer pair of SEQ ID NOs: 44 and 45 (*i*.*e*., the target sequence of SEQ ID NO: 43), wherein said primer pair is
- the primer pair of SEQ ID NOs: 44 and 45, or
- a primer pair, which anneals to gene viii. of SEQ ID NO: 10 to specifically amplify (from said gene) the same target sequence as said primer pair of SEQ ID NOs: 44 and 45 (*i.e*., the target sequence of SEQ ID NO: 43), wherein the first primer of said primer pair is at least 95% (more particularly at least 96%, at least 97%, at least 98% or at least 99%) identical to the primer of SEQ ID NO: 44 and the second primer of said primer pair is at least 95% (more particularly at least 96%, at least 97%, at least 98% or at least 99%) identical to the primer of SEQ ID NO: 45.

Each of the first and second primers of the primer pair may independently consist of 15-30 nucleotides, more particularly 19-25 nucleotides, more particularly of 20-24 nucleotides, *i.e*., of 20, 21, 22, 23 or 24 nucleotides. More particularly, the first nucleotide of the primer pair may be of the same nucleotide length as the primer of SEQ ID NO: 44 and the second nucleotide may be of the same nucleotide length as the primer of SEQ ID NO: 45. The GC content of each of said first and second primers may independently be of 40-60%.

For example, for specific amplification from said gene ix. of SEQ ID NO: 8 (clone CC155), said primers may comprise
- the primer pair of SEQ ID NO: 38 and 39, or
- a primer pair, which still is specific of gene ix. of SEQ ID NO: 8 and which anneals to gene ix. of SEQ ID NO: 8 at positions that differ by ± 5 nucleotides (more particularly by ± 4 nucleotides, by ± 3 nucleotides, by ± 2 nucleotides, by ± 1 nucleotides or by 0 nucleotide) from the positions at which the primer pair of SEQ ID NOs: 38 and 39 anneals to gene ix. of SEQ ID NO: 8; or
- a primer pair, which still is specific of gene ix. of SEQ ID NO: 8, and which anneals to gene ix. of SEQ ID NO: 8 to amplify
   a fragment from gene ix. of SEQ ID NO: 8, wherein said fragment comprises the (target) sequence SEQ ID NO: 37 that is amplified from gene ix. of SEQ ID NO: 8 by the primer pair of SEQ ID NOs: 38 and 39 (*i.e*., the target sequence of SEQ ID NO: 37) and is at most 10 nucleotides longer than said target sequence (more particularly at most 9, 8, 7, 6, 5, 4, 3, 2, 1 or 0 nucleotide(s) longer than said target sequence); or
   a fragment from gene ix. of SEQ ID NO: 8, wherein said fragment consists of the (target) sequence that is amplified from gene ix. of SEQ ID NO: 8 by the primer pair of SEQ ID NOs: 38 and 39 (*i.e*., the sequence of SEQ ID NO: 37) but deleted from at most 10 nucleotides (more particularly at most 9, 8, 7, 6, 5, 4, 3, 2, 1 or 0 nucleotide(s)); or
- a primer pair, which anneals to gene ix. of SEQ ID NO: 8 to specifically amplify (from said gene) the same target sequence as said primer pair of SEQ ID NOs: 38 and 39 (*i.e*., the sequence of SEQ ID NO: 37), wherein the first primer of said primer pair is at least 95% (more particularly at least 96%, at least 97%, at least 98% or at least 99%) identical to the primer of SEQ ID NO: 38 and the second primer of said primer pair is at least 95% (more particularly at least 96%, at least 97%, at least 98% or at least 99%) identical to the primer of SEQ ID NO: 39.

More particularly, for specific amplification from said gene ix. of SEQ ID NO: 8, said primers may comprise a primer pair for specifically amplifying (from said gene) the same target sequence as the primer pair of SEQ ID NOs: 38 and 39 (*i.e*., the target sequence of SEQ ID NO: 37).

More particularly, for specific amplification from said gene ix. of SEQ ID NO: 8, said primers may comprise a primer pair for specifically amplifying (from said gene) the same target sequence as the primer pair of SEQ ID NOs: 38 and 39 (*i.e*., the target sequence of SEQ ID NO: 37), wherein said primer pair is
- the primer pair of SEQ ID NOs: 38 and 39, or
- a primer pair, which anneals to gene ix. of SEQ ID NO: 8 to specifically amplify (from said gene) the same target sequence as said primer pair of SEQ ID NOs: 38 and 39 (*i.e*., the target sequence of SEQ ID NO: 37), wherein the first primer of said primer pair is at least 95% (more particularly at least 96%, at least 97%, at least 98% or at least 99%) identical to the primer of SEQ ID NO: 38 and the second primer of said primer pair is at least 95% (more particularly at least 96%, at least 97%, at least 98% or at least 99%) identical to the primer of SEQ ID NO: 39.

Each of the first and second primers of the primer pair may independently consist of 15-30 nucleotides, more particularly 19-25 nucleotides, more particularly of 20-24 nucleotides, *i.e*., of 20, 21, 22, 23 or 24 nucleotides. More particularly, the first nucleotide of the primer pair may be of the same nucleotide length as the primer of SEQ ID NO: 38 and the second nucleotide may be of the same nucleotide length as the primer of SEQ ID NO: 39. The GC content of each of said first and second primers may independently be of 40-60%.

For example, for specific amplification from said gene x. of SEQ ID NO: 7 (clone CC121), said primers may comprise
- the primer pair of SEQ ID NO: 35 and 36, or
- a primer pair, which still is specific of gene x. of SEQ ID NO: 7 and which anneals to gene x. of SEQ ID NO: 7 at positions that differ by ± 5 nucleotides (more particularly by ± 4 nucleotides, by ± 3 nucleotides, by ± 2 nucleotides, by ± 1 nucleotides or by 0 nucleotide) from the positions at which the primer pair of SEQ ID NOs: 35 and 36 anneals to gene x. of SEQ ID NO: 7; or
- a primer pair, which still is specific of gene x. of SEQ ID NO: 7, and which anneals to gene x. of SEQ ID NO: 7 to amplify
   a fragment from gene x. of SEQ ID NO: 7, wherein said fragment comprises the (target) sequence SEQ ID NO: 34 that is amplified from gene x. of SEQ ID NO: 7 by the primer pair of SEQ ID NOs: 35 and 36 (*i.e*., the target sequence of SEQ ID NO: 34) and is at most 10 nucleotides longer than said target sequence (more particularly at most 9, 8, 7, 6, 5, 4, 3, 2, 1 or 0 nucleotide(s) longer than said target sequence); or
   a fragment from gene x. of SEQ ID NO: 7, wherein said fragment consists of the (target) sequence that is amplified from gene x. of SEQ ID NO: 7 by the primer pair of SEQ ID NOs: 35 and 36 (*i.e*., the sequence of SEQ ID NO: 34) but deleted from at most 10 nucleotides (more particularly at most 9, 8, 7, 6, 5, 4, 3, 2, 1 or 0 nucleotide(s)); or
- a primer pair, which anneals to gene x. of SEQ ID NO: 7 to specifically amplify (from said gene) the same target sequence as said primer pair of SEQ ID NOs: 35 and 36 (*i.e*., the sequence of SEQ ID NO: 34), wherein the first primer of said primer pair is at least 95% (more particularly at least 96%, at least 97%, at least 98% or at least 99%) identical to the primer of SEQ ID NO: 35 and the second primer of said primer pair is at least 95% (more particularly at least 96%, at least 97%, at least 98% or at least 99%) identical to the primer of SEQ ID NO: 36.

More particularly, for specific amplification from said gene x. of SEQ ID NO: 7, said primers may comprise a primer pair for specifically amplifying (from said gene) the same target sequence as the primer pair of SEQ ID NOs: 35 and 36 (*i.e*., the target sequence of SEQ ID NO: 34).

More particularly, for specific amplification from said gene x. of SEQ ID NO: 7, said primers may comprise a primer pair for specifically amplifying (from said gene) the same target sequence as the primer pair of SEQ ID NOs: 35 and 36 (*i.e*., the target sequence of SEQ ID NO: 34), wherein said primer pair is
- the primer pair of SEQ ID NOs: 35 and 36, or
- a primer pair, which anneals to gene x. of SEQ ID NO: 7 to specifically amplify (from said gene) the same target sequence as said primer pair of SEQ ID NOs: 35 and 36 (*i.e*., the target sequence of SEQ ID NO: 34), wherein the first primer of said primer pair is at least 95% (more particularly at least 96%, at least 97%, at least 98% or at least 99%) identical to the primer of SEQ ID NO: 35 and the second primer of said primer pair is at least 95% (more particularly at least 96%, at least 97%, at least 98% or at least 99%) identical to the primer of SEQ ID NO: 36.

Each of the first and second primers of the primer pair may independently consist of 15-30 nucleotides, more particularly 19-25 nucleotides, more particularly of 20-24 nucleotides, *i.e.,* of 20, 21, 22, 23 or 24 nucleotides. More particularly, the first nucleotide of the primer pair may be of the same nucleotide length as the primer of SEQ ID NO: 35 and the second nucleotide may be of the same nucleotide length as the primer of SEQ ID NO: 36. The GC content of each of said first and second primers may independently be of 40-60%.

For example, for specific amplification from said gene xi. of SEQ ID NO: 11 (clone CC9), said primers may comprise
- the primer pair of SEQ ID NO: 47 and 48, or
- a primer pair, which still is specific of gene xi. of SEQ ID NO: 11 and which anneals to gene xi. of SEQ ID NO: 11 at positions that differ by ± 5 nucleotides (more particularly by ± 4 nucleotides, by ± 3 nucleotides, by ± 2 nucleotides, by ± 1 nucleotides or by 0 nucleotide) from the positions at which the primer pair of SEQ ID NOs: 47 and 48 anneals to gene xi. of SEQ ID NO: 11; or
- a primer pair, which still is specific of gene xi. of SEQ ID NO: 11, and which anneals to gene xi. of SEQ ID NO: 11 to amplify
   a fragment from gene xi. of SEQ ID NO: 11, wherein said fragment comprises the (target) sequence SEQ ID NO: 46 that is amplified from gene xi. of SEQ ID NO: 11 by the primer pair of SEQ ID NOs: 47 and 48 (*i.e*., the target sequence of SEQ ID NO: 46) and is at most 10 nucleotides longer than said target sequence (more particularly at most 9, 8, 7, 6, 5, 4, 3, 2, 1 or 0 nucleotide(s) longer than said target sequence); or
   a fragment from gene xi. of SEQ ID NO: 11, wherein said fragment consists of the (target) sequence that is amplified from gene xi. of SEQ ID NO: 11 by the primer pair of SEQ ID NOs: 47 and 48 (*i.e*., the sequence of SEQ ID NO: 46) but deleted from at most 10 nucleotides (more particularly at most 9, 8, 7, 6, 5, 4, 3, 2, 1 or 0 nucleotide(s)); or
- a primer pair, which anneals to gene xi. of SEQ ID NO: 11 to specifically amplify (from said gene) the same target sequence as said primer pair of SEQ ID NOs: 47 and 48 (*i.e*., the sequence of SEQ ID NO: 46), wherein the first primer of said primer pair is at least 95% (more particularly at least 96%, at least 97%, at least 98% or at least 99%) identical to the primer of SEQ ID NO: 47 and the second primer of said primer pair is at least 95% (more particularly at least 96%, at least 97%, at least 98% or at least 99%) identical to the primer of SEQ ID NO: 48.

More particularly, for specific amplification from said gene xi. of SEQ ID NO: 11, said primers may comprise a primer pair for specifically amplifying (from said gene) the same target sequence as the primer pair of SEQ ID NOs: 47 and 48 *(i.e*., the target sequence of SEQ ID NO: 46).

More particularly, for specific amplification from said gene xi. of SEQ ID NO: 11, said primers may comprise a primer pair for specifically amplifying (from said gene) the same target sequence as the primer pair of SEQ ID NOs: 47 and 48 (*i.e*., the target sequence of SEQ ID NO: 46), wherein said primer pair is
- the primer pair of SEQ ID NOs: 47 and 48, or
- a primer pair, which anneals to gene xi. of SEQ ID NO: 11 to specifically amplify (from said gene) the same target sequence as said primer pair of SEQ ID NOs: 47 and 48 (*i.e*., the target sequence of SEQ ID NO: 46), wherein the first primer of said primer pair is at least 95% (more particularly at least 96%, at least 97%, at least 98% or at least 99%) identical to the primer of SEQ ID NO: 47 and the second primer of said primer pair is at least 95% (more particularly at least 96%, at least 97%, at least 98% or at least 99%) identical to the primer of SEQ ID NO: 48.

Each of the first and second primers of the primer pair may independently consist of 15-30 nucleotides, more particularly 19-25 nucleotides, more particularly of 20-24 nucleotides, *i.e*., of 20, 21, 22, 23 or 24 nucleotides. More particularly, the first nucleotide of the primer pair may be of the same nucleotide length as the primer of SEQ ID NO: 47 and the second nucleotide may be of the same nucleotide length as the primer of SEQ ID NO: 48. The GC content of each of said first and second primers may independently be of 40-60%.

In addition to determining the presence or absence of a plurality of said genes, more particularly the presence or absence of a plurality of genes comprising said genes i.-iv. and/or said genes vi.-vii. and/or said genes v., viii. and ix., a method of the application may further comprise determining the presence or absence of control marker gene(s), which is(are)
- a control marker gene or a combination of control marker genes, which is specific of *L. monocytogenes* serogroup IVb, and/or
- a control marker gene or a combination of control marker genes, which is specific of *L. monocytogenes* serogroup IIb, and/or
- a control marker gene or a combination of control marker genes, which is specific of *L. monocytogenes* serogroups IIa and IIc or of *L. monocytogenes,* lineage II.

The control marker gene, which is specific of *L. monocytogenes* serogroup IVb, may be the *L. monocytogenes,* gene of SEQ ID NO: 15 or of any one of SEQ ID NOs: 180-181, more particularly the *L. monocytogenes,* gene of SEQ ID NO: 15.

The combination of control marker genes, which is specific of *L. monocytogenes* serogroup IIb, may be the combination of
- a control marker gene, which is specific of *L. monocytogenes,* lineage I, and of
- a control marker gene, which is specific of serogroup IIb and lineage II of *L. monocytogenes.* The control marker gene, which is specific of *L. monocytogenes* lineage I, may be the *L. monocytogenes* gene of SEQ ID NO: 12 or of any one of SEQ ID NOs: 156-157, more particularly the *L. monocytogenes* gene of SEQ ID NO: 12.

The control marker gene, which is specific of serogroup IIb and lineage II of *L. monocytogenes,* may be the *L. monocytogenes* gene of SEQ ID NO: 14 or of any one of SEQ ID NOs: 172-179, more particularly the *L. monocytogenes* gene of SEQ ID NO: 14.

The control marker gene, which is specific of *L. monocytogenes* serogroups IIa and IIc or of *L. monocytogenes* lineage II, may be the *L. monocytogenes* gene of SEQ ID NO: 13 or of any one of SEQ ID NOs: 158-171, more particularly the *L. monocytogenes* gene of SEQ ID NO: 13.

Primers and/or probes can be used to determine the presence or absence of said control marker gene(s).

Primers to determine the presence or absence of said control marker gene(s) may comprise primers for determining the presence or absence of the control marker gene of SEQ ID NO: 15 (which is specific of *L. monocytogenes,* serogroup IVb), wherein said primers for determining the presence or absence of the control marker gene of SEQ ID NO: 15 are a primer pair for specific amplification from the control marker gene of SEQ ID NO: 15, and wherein said primer pair is
- the primer pair of SEQ ID NOs: 59 and 60, or
- a primer pair, which anneals to the control marker gene of SEQ ID NO: 15 to specifically amplify the same target sequence as said primer pair of SEQ ID NOs: 59 and 60 (*i.e*., the target sequence of SEQ ID NO: 58), wherein the first primer of said primer pair is at least 95% identical to the primer of SEQ ID NO: 59 and the second primer of said primer pair is at least 95% identical to the primer of SEQ ID NO: 60.

Primers to determine the presence or absence of said control marker gene(s) may comprise primers for determining the presence or absence of the control marker gene of SEQ ID NO: 12 (which is specific of *L. monocytogenes* lineage I), wherein said primers for determining the presence or absence of the control marker gene of SEQ ID NO: 12 are a primer pair for specific amplification from the control marker gene of SEQ ID NO: 12, and wherein said primer pair is
- the primer pair of SEQ ID NOs: 50 and 51, or
- a primer pair, which anneals to the control marker gene of SEQ ID NO: 12 to specifically amplify the same target sequence as said primer pair of SEQ ID NOs: 50 and 51 (*i.e*., the target sequence of SEQ ID NO: 49), wherein the first primer of said primer pair is at least 95% identical to the primer of SEQ ID NO: 50 and the second primer of said primer pair is at least 95% identical to the primer of SEQ ID NO: 51.

Primers to determine the presence or absence of said control marker gene(s) may comprise primers for determining the presence or absence of the control marker gene of SEQ ID NO: 14 (which is specific of serogroup IIb and lineage II of *L. monocytogenes),* wherein said primers for determining the presence or absence of the control marker gene of SEQ ID NO: 14 are a primer pair for specific amplification from the control marker gene of SEQ ID NO: 14, and wherein said primer pair is
- the primer pair of SEQ ID NOs: 56 and 57, or
- a primer pair, which anneals to the control marker gene of SEQ ID NO: 14 to specifically amplify the same target sequence as said primer pair of SEQ ID NOs: 56 and 57 (*i.e*., the target sequence of SEQ ID NO: 55), wherein the first primer of said primer pair is at least 95% identical to the primer of SEQ ID NO: 56 and the second primer of said primer pair is at least 95% identical to the primer of SEQ ID NO: 57.

Primers to determine the presence or absence of said control marker gene(s) may comprise primers for determining the presence or absence of the control marker gene of SEQ ID NO: 12 (which is specific of *L. monocytogenes,* lineage I), wherein said primers for determining the presence or absence of the control marker gene of SEQ ID NO: 12 are a primer pair for specific amplification from the control marker gene of SEQ ID NO: 12, and wherein said primer pair is
- the primer pair of SEQ ID NOs: 50 and 51, or
- a primer pair, which anneals to the control marker gene of SEQ ID NO: 12 to specifically amplify the same target sequence as said primer pair of SEQ ID NOs: 50 and 51 (*i.e*., the target sequence of SEQ ID NO: 49), wherein the first primer of said primer pair is at least 95% identical to the primer of SEQ ID NO: 50 and the second primer of said primer pair is at least 95% identical to the primer of SEQ ID NO: 51,
and may further comprise primers for determining the presence or absence of the control marker gene of SEQ ID NO: 14 (which is specific of serogroup IIb and lineage II of *L. monocytogenes*), wherein said primers for determining the presence or absence of the control marker gene of SEQ ID NO: 14 are a primer pair for specific amplification from the control marker gene of SEQ ID NO: 14, and wherein said primer pair is
- the primer pair of SEQ ID NOs: 56 and 57, or
- a primer pair, which anneals to the control marker gene of SEQ ID NO: 14 to specifically amplify the same target sequence as said primer pair of SEQ ID NOs: 56 and 57 (*i.e*., the target sequence of SEQ ID NO: 55), wherein the first primer of said primer pair is at least 95% identical to the primer of SEQ ID NO: 56 and the second primer of said primer pair is at least 95% identical to the primer of SEQ ID NO: 57.

Primers to determine the presence or absence of said control marker gene(s) may comprise primers for determining the presence or absence of the control marker gene of SEQ ID NO: 13 (which is specific of *L. monocytogenes* serogroups IIa and IIc or of *L. monocytogenes* lineage II), wherein said primers for determining the presence or absence of the control marker gene of SEQ ID NO: 13 are a primer pair for specific amplification from the control marker gene of SEQ ID NO: 13, and wherein said primer pair is
- the primer pair of SEQ ID NOs: 53 and 54, or
- a primer pair, which anneals to the control marker gene of SEQ ID NO: 13 to specifically amplify the same target sequence as said primer pair of SEQ ID NOs: 53 and 54 (*i.e*., the target sequence of SEQ ID NO: 52), wherein the first primer of said primer pair is at least 95% identical to the primer of SEQ ID NO: 53 and the second primer of said primer pair is at least 95% identical to the primer of SEQ ID NO: 54.

Advantageously, said primers for specific amplification from said gene i. of SEQ ID NO: 1, more particularly the primers of SEQ ID NOs: 17 and 18, can be used in mixture with said primers for specific amplification from said gene ii. of SEQ ID NO: 2, more particularly the primers of SEQ ID NOs: 20 and 21,
with said primers for specific amplification from said gene iii. of SEQ ID NO: 3, more particularly the primers of SEQ ID NOs: 23 and 24, and
with said primers for specific amplification from said gene iv. of SEQ ID NO: 4, more particularly the primers of SEQ ID NOs: 26 and 27,
thereby forming a multiplex of oligonucleotides (comprising four primer pairs in mixture).

The application thus relates to a multiplex of oligonucleotides, which comprises at least two, at least three or the four of said primer pairs, thereby forming a multiplex of primers for specific amplification from at least two, at least three or the four of said genes i.-iv.. The application also relates to a container (such as a tube suitable for nucleic acid amplification, or such as a kit), which contains this multiplex of oligonucleotides or primers.

Advantageously, said (multiplex of) primers comprise
- a primer pair for specifically amplifying the sequence of SEQ ID NO: 16 from said gene i. of SEQ ID NO: 1,
- a primer pair for specifically amplifying the sequence of SEQ ID NO: 19 from said gene ii. of SEQ ID NO: 2,
- a primer pair for specifically amplifying the sequence of SEQ ID NO: 22 from said gene iii. of SEQ ID NO: 3; and
- a primer pair for specifically amplifying the sequence of SEQ ID NO: 25 from said gene iv. of SEQ ID NO: 4.

Each primer may independently consist of 15-30 nucleotides, more particularly 19-25 nucleotides, more particularly of 20-24 nucleotides, *i.e*., of 20, 21, 22, 23 or 24 nucleotides Advantageously, said (multiplex of) primers comprise
- a primer pair for specifically amplifying the sequence of SEQ ID NO: 16 from said gene i. of SEQ ID NO: 1, which is
   - the primer pair of SEQ ID NOs: 17 and 18, or
   - a primer pair, (which anneals to gene i. of SEQ ID NO: 1 to specifically amplify (from said gene) the sequence of SEQ ID NO: 16,) wherein the first primer of said primer pair is at least 95% identical to the primer of SEQ ID NO: 17 and is of the same nucleotide length as SEQ ID NO: 17, and wherein the second primer of said primer pair is at least 95% identical to the primer of SEQ ID NO: 18 and is of the same nucleotide length as SEQ ID NO: 18;
- a primer pair for specifically amplifying the sequence of SEQ ID NO: 19 from said gene ii. of SEQ ID NO: 2, which is
   - the primer pair of SEQ ID NOs: 20 and 21, or
   - a primer pair, (which anneals to gene ii. of SEQ ID NO: 2 to specifically amplify (from said gene) the sequence of SEQ ID NO: 19,) wherein the first primer of said primer pair is at least 95% identical to the primer of SEQ ID NO: 20 and is of the same nucleotide length as SEQ ID NO: 20, and wherein the second primer of said primer pair is at least 95% identical to the primer of SEQ ID NO: 21 and is of the same nucleotide length as SEQ ID NO: 21;
- a primer pair for specifically amplifying the sequence of SEQ ID NO: 22 from said gene iii. of SEQ ID NO: 3, which is
   - the primer pair of SEQ ID NOs: 23 and 24, or
   - a primer pair, (which anneals to gene iii. of SEQ ID NO: 3 to specifically amplify (from said gene) the sequence of SEQ ID NO: 22,) wherein the first primer of said primer pair is at least 95% identical to the primer of SEQ ID NO: 23 and is of the same nucleotide length as SEQ ID NO: 23, and wherein the second primer of said primer pair is at least 95% identical to the primer of SEQ ID NO: 24 and is of the same nucleotide length as SEQ ID NO: 24; and
- a primer pair for specifically amplifying the sequence of SEQ ID NO: 25 from said gene iv. of SEQ ID NO: 4, which is
   - the primer pair of SEQ ID NOs: 26 and 27, or
   - a primer pair, (which anneals to gene iv. of SEQ ID NO: 4 to specifically amplify (from said gene) the sequence of SEQ ID NO: 25,) wherein the first primer of said primer pair is at least 95% identical to the primer of SEQ ID NO: 26 and is of the same nucleotide length as SEQ ID NO: 26, and wherein the second primer of said primer pair is at least 95% identical to the primer of SEQ ID NO: 27 and is of the same nucleotide length as SEQ ID NO: 27.

An example of such a multiplex of primers is shown in the examples below (multiplex IVb of the application).

Advantageously, said primers for specific amplification from said gene vi. of SEQ ID NO: 5, more particularly the primers of SEQ ID NOs: 29 and 30, can be used in mixture with said primers for specific amplification from said gene vii. of SEQ ID NO: 6, more particularly the primers of SEQ ID NOs: 32 and 33, thereby forming a multiplex of oligonucleotides (comprising said two primer pairs in mixture).

The application thus relates to a multiplex of oligonucleotides, which comprises the two of said primer pairs, thereby forming a multiplex of primers for specific amplification from each of said genes vi. and vii. The application also relates to a container (such as a tube suitable for nucleic acid amplification, or such as a kit), which contains this multiplex.

Advantageously, said (multiplex of) primers comprise
- a primer pair for specifically amplifying the sequence of SEQ ID NO: 28 from said gene vi. of SEQ ID NO: 5; and
- a primer pair for specifically amplifying the sequence of SEQ ID NO: 31 from said gene vii. of

SEQ ID NO: 6.

Each primer may independently consist of 15-30 nucleotides, more particularly 19-25 nucleotides, more particularly of 20-24 nucleotides, *i.e.,* of 20, 21, 22, 23 or 24 nucleotides Advantageously, said (multiplex of) primers comprise
- a primer pair for specifically amplifying the sequence of SEQ ID NO: 28 from said gene vi. of SEQ ID NO: 5, which is
   - the primer pair of SEQ ID NOs: 29 and 30, or
   - a primer pair, (which anneals to gene vi. of SEQ ID NO: 5 to specifically amplify (from said gene) the sequence of SEQ ID NO: 28), wherein the first primer of said primer pair is at least 95% identical to the primer of SEQ ID NO: 29 and is of the same nucleotide length as SEQ ID NO: 29, and wherein the second primer of said primer pair is at least 95% identical to the primer of SEQ ID NO: 30 and is of the same nucleotide length as SEQ ID NO: 30; and
- a primer pair for specifically amplifying the sequence of SEQ ID NO: 31 from said gene vii. of SEQ ID NO: 6, which is
   - the primer pair of SEQ ID NOs: 32 and 33, or
   - a primer pair, (which anneals to gene vii. of SEQ ID NO: 6 to specifically amplify (from said gene) the sequence of SEQ ID NO: 31), wherein the first primer of said primer pair is at least 95% identical to the primer of SEQ ID NO: 32 and is of the same nucleotide length as SEQ ID NO: 32, and wherein the second primer of said primer pair is at least 95% identical to the primer of SEQ ID NO: 33 and is of the same nucleotide length as SEQ ID NO: 33.

An example of such a multiplex of primers is shown in the examples below (multiplex IIb of the application).

Advantageously, said primers for specific amplification from said gene v. of SEQ ID NO: 9, more particularly the primers of SEQ ID NOs: 41 and 42, can be used in mixture with said primers for specific amplification from said gene viii. of SEQ ID NO: 10, more particularly the primers of SEQ ID NOs: 44 and 45,
with said primers for specific amplification from said gene ix. of SEQ ID NO: 8, more particularly the primers of SEQ ID NOs: 38 and 39, and
thereby forming a multiplex of oligonucleotides (comprising said three primer pairs in mixture).

The application thus relates to a multiplex of oligonucleotides, which comprises at least two or the three of said primer pairs, thereby forming a multiplex of primers for specific amplification from at least two or the three of said genes v., viii. and ix. The application also relates to a container (such as a tube suitable for nucleic acid amplification, or such as a kit), which contains this multiplex of primers.

Advantageously, said (multiplex of) primers comprise
- a primer pair for specifically amplifying the sequence of SEQ ID NO: 40 from said gene v. of SEQ ID NO: 9;
- a primer pair for specifically amplifying the sequence of SEQ ID NO: 43 from said gene viii. of SEQ ID NO: 10; and
- a primer pair for specifically amplifying the sequence of SEQ ID NO: 37 from said gene ix. of SEQ ID NO: 8.

Each primer may independently consist of 15-30 nucleotides, more particularly 19-25 nucleotides, more particularly of 20-24 nucleotides, *i.e.,* of 20, 21, 22, 23 or 24 nucleotides Advantageously, said (multiplex of) primers comprise
- a primer pair for specifically amplifying the sequence of SEQ ID NO: 40 from said gene v. of SEQ ID NO: 9, which is
   - the primer pair of SEQ ID NOs: 41 and 42, or
   - a primer pair, (which anneals to gene v. of SEQ ID NO: 9 to specifically amplify (from said gene) the sequence of SEQ ID NO: 40,) wherein the first primer of said primer pair is at least 95% identical to the primer of SEQ ID NO: 41 and is of the same nucleotide length as SEQ ID NO: 41, and wherein the second primer of said primer pair is at least 95% identical to the primer of SEQ ID NO: 42 and is of the same nucleotide length as SEQ ID NO: 42;
- a primer pair for specifically amplifying the sequence of SEQ ID NO: 43 from said gene viii. of SEQ ID NO: 10, which is
   - the primer pair of SEQ ID NOs: 44 and 45, or
   - a primer pair, (which anneals to gene viii. of SEQ ID NO: 10 to specifically amplify (from said gene) the sequence of SEQ ID NO: 43,) wherein the first primer of said primer pair is at least 95% identical to the primer of SEQ ID NO: 44 and is of the same nucleotide length as SEQ ID NO: 44, and wherein the second primer of said primer pair is at least 95% identical to the primer of SEQ ID NO: 45 and is of the same nucleotide length as SEQ ID NO: 45; and
- a primer pair for specifically amplifying the sequence of SEQ ID NO: 37 from said gene ix. of SEQ ID NO: 8, which is
   - the primer pair of SEQ ID NOs: 38 and 39, or
   - a primer pair, (which anneals to gene ix. of SEQ ID NO: 8 to specifically amplify (from said gene) the sequence of SEQ ID NO: 37,) wherein the first primer of said primer pair is at least 95% identical to the primer of SEQ ID NO: 38 and is of the same nucleotide length as SEQ ID NO: 38, and wherein the second primer of said primer pair is at least 95% identical to the primer of SEQ ID NO: 39 and is of the same nucleotide length as SEQ ID NO: 39.

Said (multiplex of) primers may further comprise
- a primer pair suitable for nucleic acid amplification from gene x., *i.e.,* from a gene, which is specific of *L. monoccytogenes* clone CC121, wherein said CC121-specific gene is *LM6179*_*0610* (SEQ ID NO: 7) or one of the genes of SEQ ID NOs: 123-128, more particularly *LM6179*_*0610* (SEQ ID NO: 7) *(cf.* above for the description of primers for specific amplification from said gene x. of SEQ ID NO: 7 (clone CC121)), and/or
- a primer pair suitable for nucleic acid amplification from gene xi., *i.e.,* from a gene, which is specific of *L. monoccytogenes* clone CC9, wherein said CC9-specific gene is *lmo1118* (SEQ ID NO: 11)) *(cf.* above for the description of primers for specific amplification from said gene xi. of SEQ ID NO: 11 (clone CC9)),
   thereby forming a multiplex of 4 or 5 primer pairs suitable for (specific) nucleic acid amplification from genes v., viii., ix. and from genes x. and/or xi.

An example of such a multiplex of primers is shown in the examples below (multiplex IIaIIc of the application).

Each of the three of the above-mentioned multiplex of oligonucleotides, *i.e.*
- the multiplex of oligonucleotides comprising primers for specific amplification from at least two, at least three or the four of said genes i.-iv.,
- the multiplex of oligonucleotides comprising for specific amplification from the two of said genes vi.-vii., and
- the multiplex of oligonucleotides comprising for specific amplification from at least two or the three of said genes v., viii., ix. (and optionally from one or the two of genes x. and/or xi.), may further comprise at least one *(e.g.,* one or two) primer pair for specific amplification from a control marker gene or a combination of *(e.g.,* two) control marker genes.

For example, the multiplex of primers for specific amplification from at least two, at least three or the four of said genes i.-iv. [multiplex IVb in the examples below] may further comprise a primer pair for specific amplification from a control marker gene, which is specific of *L. monocytogenes* serogroup IVb.

Said control marker gene may be the *L. monocytogenes* gene of SEQ ID NO: 15 or of any one of SEQ ID NOs: 180-181, more particularly the *L. monocytogenes* gene of SEQ ID NO: 15.

A primer pair for specific amplification from the control marker gene of SEQ ID NO: 15 may *e.g.,* be:
- the primer pair of SEQ ID NOs: 59 and 60, or
- a primer pair, which anneals to the control marker gene of SEQ ID NO: 15 to specifically amplify the same target sequence as said primer pair of SEQ ID NOs: 59 and 60 *(i.e.,* the target sequence of SEQ ID NO: 58), wherein the first primer of said primer pair is at least 95% identical to the primer of SEQ ID NO: 59 and the second primer of said primer pair is at least 95% identical to the primer of SEQ ID NO: 60.

For example, the multiplex of primers for specific amplification from the two of said genes vi.-vii. [multiplex IIb in the examples below] may further comprise primer pairs for specific amplification from a combination of two control marker genes, wherein the first of said control marker gene is specific of *L. monocytogenes,* lineage I and the second of said control marker gene is specific of serogroup IIb and lineage II of *L. monocytogenes.*

Said combination of control marker genes may comprise
- the *L. monocytogenes* gene of SEQ ID NO: 12 or of any one of SEQ ID NOs: 156-157 (which is specific of *L. monocytogenes* lineage I), more particularly the *L. monocytogenes* gene of SEQ ID NO: 12, and
- the *L. monocytogenes* gene of SEQ ID NO: 14 or of any one of SEQ ID NOs: 172-179 (which is specific of serogroup IIb and lineage II of *L. monocytogenes*), more particularly the *L. monocytogenes* gene of SEQ ID NO: 14.

Primer pairs for specific amplification from the combination of the control marker gense of SEQ ID NOs: 12 and 14 may *e.g.,* be:
- a first primer pair, which is
   - the primer pair of SEQ ID NOs: 50 and 51, or
   - a primer pair, which anneals to the control marker gene of SEQ ID NO: 12 to specifically amplify the same target sequence as said primer pair of SEQ ID NOs: 50 and 51 (*i.e.,* the target sequence of SEQ ID NO: 49), wherein the first primer of said primer pair is at least 95% identical to the primer of SEQ ID NO: 50 and the second primer of said primer pair is at least 95% identical to the primer of SEQ ID NO: 51,
   and
- a second primer pair, which is
   - the primer pair of SEQ ID NOs: 56 and 57, or
   - a primer pair, which anneals to the control marker gene of SEQ ID NO: 14 to specifically amplify the same target sequence as said primer pair of SEQ ID NOs: 56 and 57 (*i.e.,* the target sequence of SEQ ID NO: 55), wherein the first primer of said primer pair is at least 95% identical to the primer of SEQ ID NO: 56 and the second primer of said primer pair is at least 95% identical to the primer of SEQ ID NO: 57.

For example, the multiplex of the multiplex of primers for specific amplification from at least two or the three of said genes v., viii., ix. (and optionally from one or the two of genes x. and/or xi.) [multiplex IIaIIc in the examples below] may further comprise a primer pair for specific amplification from a control marker gene, which is specific of *L. monocytogenes* serogroups IIa and IIc or of *L. monoccytogenes* lineage II.

Said control marker gene may be the *L. monoccytogenes* gene of SEQ ID NO: 13 or of any one of SEQ ID NOs: 158-171, more particularly the *L. monoccytogenes* gene of SEQ ID NO: 13.

A primer pair for specific amplification from the control marker gene of SEQ ID NO: 13 may *e.g.,* be:
- the primer pair of SEQ ID NOs: 53 and 54, or
- a primer pair, which anneals to the control marker gene of SEQ ID NO: 13 to specifically amplify the same target sequence as said primer pair of SEQ ID NOs: 53 and 54 (*i.e.,* the target sequence of SEQ ID NO: 52), wherein the first primer of said primer pair is at least 95% identical to the primer of SEQ ID NO: 53 and the second primer of said primer pair is at least 95% identical to the primer of SEQ ID NO: 54.

Advantageously, the three of the above-mentioned multiplex of primers, *i*.*e*.,
- the multiplex of oligonucleotides comprising primers for specific amplification from at least two, at least three or the four of said genes i.-iv. (and optionally from the control marker gene that is specific of serogroup IVb),
- the multiplex of oligonucleotides comprising primers for specific amplification from the two of said genes vi.-vii. (and optionally from the combination of a control marker gene that is specific of lineage I and of a control marker gene that is specific of serogroup IIb and lineage II), and
- the multiplex of oligonucleotides comprising primers for specific amplification from at least two or the three of said genes v., viii., ix. and optionally from one or the two of genes x. and/or xi. (and optionally from the control marker gene that is specific of serogroups IIa and IIc, or of lineage II),
can be implemented under the same DNA amplification thermocycling conditions, more particularly under the same PCR thermocycling conditions. The three of of the above-mentioned multiplex of primers can thus be implemented simultaneously on the same DNA amplification plate, more particularly on the same PCR plate.

Examples of such PCR thermocycling conditions include an (initial) denaturation step at 94°C for 30 s followed by 25 cycles of 30 s at 94°C, 30 s at 55°C and 75 s at 72°C, and a (final) elongation step at 72°C for 10 min (*cf.* the examples below).

The primers of the application may thus comprise:
- the multiplex of primers for specific amplification from at least two, at least three or the four of said genes i.-iv. (and optionally from the control marker gene that is specific of serogroup IVb), and
- the multiplex of primers for specific amplification from the two of said genes vi.-vii. (and optionally from the combination of a control marker gene that is specific of lineage I and of a control marker gene that is specific of serogroup IIb and lineage II).

Said primers can be implemented simultaneously under the same DNA amplification (more particularly PCR) thermocycling conditions.

Examples of such PCR thermocycling conditions include an (initial) denaturation step at 94°C for 30 s followed by 25 cycles of 30 s at 94°C, 30 s at 55°C and 75 s at 72°C, and a (final) elongation step at 72°C for 10 min (*cf.* the examples below).

The primers of the application may thus comprise:
- the multiplex of primers for specific amplification from the two of said genes vi.-vii. (and optionally from the combination of a control marker gene that is specific of lineage I and of a control marker gene that is specific of serogroup IIb and lineage II), and
- the multiplex of primers for specific amplification from at least two or the three of said genes v., viii., ix. and optionally from one or the two of genes x. and/or xi. (and optionally from the control marker gene that is specific of serogroups IIa and IIc, or of lineage II).

Said primers can be implemented simultaneously under the same DNA amplification (more particularly PCR) thermocycling conditions.

Examples of such PCR thermocycling conditions include an (initial) denaturation step at 94°C for 30 s followed by 25 cycles of 30 s at 94°C, 30 s at 55°C and 75 s at 72°C, and a (final) elongation step at 72°C for 10 min (*cf.* the examples below).

The primers of the application may thus comprise:
- the multiplex of primers for specific amplification from at least two, at least three or the four of said genes i.-iv. (and optionally from the control marker gene that is specific of serogroup IVb), and
- the multiplex of primers for specific amplification from at least two or the three of said genes v., viii., ix. and optionally from one or the two of genes x. and/or xi. (and optionally from the control marker gene that is specific of serogroups IIa and IIc, or of lineage II).

Said primers can be implemented simultaneously under the same DNA amplification (more particularly PCR) thermocycling conditions.

Examples of such PCR thermocycling conditions include an (initial) denaturation step at 94°C for 30 s followed by 25 cycles of 30 s at 94°C, 30 s at 55°C and 75 s at 72°C, and a (final) elongation step at 72°C for 10 min (*cf.* the examples below).

The primers of the application may thus comprise:
- the multiplex of primers for specific amplification from at least two, at least three or the four of said genes i.-iv. (and optionally from the control marker gene that is specific of serogroup IVb),
- the multiplex of primers for specific amplification from the two of said genes vi.-vii. (and optionally from the combination of a control marker gene that is specific of lineage I and of a control marker gene that is specific of serogroup IIb and lineage II), and
- the multiplex of primers for specific amplification from at least two or the three of said genes v., viii., ix. and optionally from one or the two of genes x. and/or xi. (and optionally from the control marker gene that is specific of serogroups IIa and IIc, or of lineage II).

Said primers can be implemented simultaneously under the same DNA amplification (more particularly PCR) thermocycling conditions.

Examples of such PCR thermocycling conditions include an (initial) denaturation step at 94°C for 30 s followed by 25 cycles of 30 s at 94°C, 30 s at 55°C and 75 s at 72°C, and a (final) elongation step at 72°C for 10 min (*cf.* the examples below).

In accordance with the application, one or several probes can be implemented alternatively to or complementarily to said primers or to said multiplex of oligonucleotides or primers.

Said one or several probes may comprise at least one probe suitable for nucleic acid hybridization on one of genes i.-ix., i.-xi., i.-x., i.-ix. and xi., more particularly at least one probe suitable for nucleic acid hybridization on one of genes i.-ix., more particularly for at least one probe suitable for nucleic acid hybridization on one of genes i.-iv., or one of genes vi. and vii., or one of genes v., viii. and ix.

Advantageously, said genes i.-ix. are the genes of SEQ ID NOs: 1-6, 9, 10 and 8, respectively.

Advantageously, said genes x. and xi. are the genes of SEQ ID NOs: 7 and 11, respectively.

Advantageously, said at least one probe, which hybridizes to genes i., hybridizes to a (target) sequence, which is the sequence of SEQ ID NO: 16 or the complementary sequence sequence thereof.

Advantageously, said at least one probe, which hybridizes to genes ii., hybridizes to a (target) sequence, which is the sequence of SEQ ID NO: 19 or the complementary sequence sequence thereof.

Advantageously, said at least one probe, which hybridizes to genes iii., hybridizes to a (target) sequence, which is the sequence of SEQ ID NO: 22 or the complementary sequence sequence thereof.

Advantageously, said at least one probe, which hybridizes to genes iv., hybridizes to a (target) sequence, which is the sequence of SEQ ID NO: 25 or the complementary sequence sequence thereof.

Advantageously, said at least one probe, which hybridizes to genes vi., hybridizes to a (target) sequence, which is the sequence of SEQ ID NO: 28 or the complementary sequence sequence thereof.

Advantageously, said at least one probe, which hybridizes to genes vii., hybridizes to a (target) sequence, which is the sequence of SEQ ID NO: 31 or the complementary sequence sequence thereof.

Advantageously, said at least one probe, which hybridizes to genes v., hybridizes to a (target) sequence, which is the sequence of SEQ ID NO: 40 or the complementary sequence sequence thereof.

Advantageously, said at least one probe, which hybridizes to genes viii., hybridizes to a (target) sequence, which is the sequence of SEQ ID NO: 43 or the complementary sequence sequence thereof.

Advantageously, said at least one probe, which hybridizes to genes ix., hybridizes to a (target) sequence, which is the sequence of SEQ ID NO: 37 or the complementary sequence sequence thereof.

Advantageously, said at least one probe, which hybridizes to genes x., hybridizes to a (target) sequence, which is the sequence of SEQ ID NO: 34 or the complementary sequence sequence thereof.

Advantageously, said at least one probe, which hybridizes to genes xi., hybridizes to a (target) sequence, which is the sequence of SEQ ID NO: 46 or the complementary sequence sequence thereof.

The sequence of a probe is such that the presence of hybridization is indicative that said gene is present and, conversely, the absence of hybridization is indicative that said gene is absent. Advantageously, said at least one probe is a probe, which specifically hybridizes to said gene or target sequence.

The sequence of said probe may comprise or consist of a hybridization sequence of 15-60 nucleotides (more particularly of 20-55 nucleotides, more particularly of 30-50 nucleotides, more particularly of 35-45 nucleotides). The hybridization sequence may be covalently linked to other nucleotide sequence(s) (*e.g.,* directly at the 5' end and/or at the 3' end of the hybridization sequence), wherein these other nucleotide sequence(s) do not (directly) contribute to, or improve, the hybridization of the probe to the gene or target sequence, but which contribute to the detection of the probe, such as *e.g.,* beacon arm(s). These other nucleotide sequence may *e.g.,* consist of a total of 5-10 nucleotides (more particularly of 5, 6, 7, 8, 9 or 10 nucleotides).

The sequence of said at least one probe may thus consist of a total of 25-70 nucleotides (more particularly of 25-65 nucleotides, more particularly 35-60 nucleotides, more particularly of 40-55 nucleotides).

Said hybridization sequence may *e.g.,* be at least 90% identical (more particularly at least 91% or at least 92% or at least 93% identical or at least 94% identical or at least 95% identical or at least 96% or at least 97% or at least 98% or at least 99% or 100% identical) to the sequence that is complementary to a fragment of the gene or target sequence to which said probe (specifically) hybridizes, wherein said fragment is of the same length as said hybridization sequence. Said fragment advantageously is internal to said gene or target sequence e.g., said fragment does not comprise the first 10 nucleotides and the last 10 nucleotides at the 5' and 3' ends respectively of said gene or target sequence.

The application more particularly encompasses the implementation of several of such probes, as well as a set comprising several of such probes.

Said probes may comprise several probes, wherein each probe is suitable for nucleic acid hybridization on different genes, said different genes being selected from genes i.-ix., i.-xi., i.-x. or i-ix. and xi., more particularly from genes i.-ix., more particularly from genes i.-iv. and/or from genes vi.-vii. and/or from genes v., viii. and ix.

Said probes may comprise at least two probes suitable for nucleic acid hybridization on one of genes i.-ix., more particularly at least one first probe suitable for nucleic acid hybridization on one of genes i.-ix. and at least one second probe suitable for nucleic acid hybridization on another of genes i.-ix.

More particularly, said probes may comprise several probes, wherein each probe is suitable for nucleic acid hybridization on different genes, said different genes being each one of genes i.-iv. and/or each one of genes vi.-vii. and/or each one of genes v., viii. and ix.

More particularly, said probes may comprise several probes, wherein each probe is suitable for nucleic acid hybridization on different genes, said different genes being each one of genes i.-ix.

The application more particularly relates to a set of probes comprising
- probes, which specifically hybridize to each one of genes i.-iv., and/or
- probes, which specifically hybridize to each one genes vi. and vii., and/or
- probes, which specifically hybridize to each one of genes v., viii. and ix.

Advantageously, said probes, which specifically hybridize to each one of genes i.-iv., specifically hybridizes to the (target) sequence of SEQ ID NO: 16 or to the complementary sequence thereof, to the (target) sequence of SEQ ID NO: 19 or the complementary sequence thereof, to the (target) sequence of SEQ ID NO: 22 or the complementary sequence thereof, and to the (target) sequence of SEQ ID NO: 25 or the complementary sequence thereof.

Advantageously, said probes, which specifically hybridize to each one genes vi. and vii., specifically hybridizes to the (target) sequence of SEQ ID NO: 28 or the complementary sequence thereof and to the (target) sequence of SEQ ID NO: 31 or the complementary sequence thereof. Advantageously, said probes, which specifically hybridize to each one of genes v., viii. and ix., specifically hybridizes to the (target) sequence of SEQ ID NO: 40 or the complementary sequence thereof, to to the (target) sequence of SEQ ID NO: 43 or the complementary sequence thereof, and to the (target) sequence of SEQ ID NO: 37 or the complementary sequence thereof.

For example, said probes may comprise:
- a probe for specific hybridization to said gene i. of SEQ ID NO: 1, wherein said probe specifically hybridizes to SEQ ID NO: 1, more particularly to SEQ ID NO: 16, and wherein the sequence of said probe consists of 25-70 nucleotides;
- a probe for specific hybridization to said gene ii. of SEQ ID NO: 2, wherein said probe specifically hybridizes to SEQ ID NO: 2, more particularly to SEQ ID NO: 19, and wherein the sequence of said probe consists of 25-70 nucleotides;
- a probe for specific hybridization to said gene iii. of SEQ ID NO: 3, wherein said probe specifically hybridizes to SEQ ID NO: 3, more particularly to SEQ ID NO: 22, and wherein the sequence of said probe consists of 25-70 nucleotides; and
- a probe for specific hybridization to said gene iv. of SEQ ID NO: 4, wherein said probe specifically hybridizes to SEQ ID NO: 4, more particularly to SEQ ID NO: 25, and wherein the sequence of said probe consists of 25-70 nucleotides.

For example, said probes may comprise:
- a probe for specific hybridization to said gene vi. of SEQ ID NO: 5, wherein said probe specifically hybridizes to SEQ ID NO: 5, more particularly to SEQ ID NO: 28, and wherein the sequence of said probe consists of 25-70 nucleotides; and
- a probe for specific hybridization to said gene vii. of SEQ ID NO: 6, wherein said probe specifically hybridizes to SEQ ID NO: 6, more particularly to SEQ ID NO: 31, and wherein the sequence of said probe consists of 25-70 nucleotides.

For example, said probes may comprise:
- a probe for specific hybridization to said gene v. of SEQ ID NO: 9, wherein said probe specifically hybridizes to SEQ ID NO: 9, more particularly to SEQ ID NO: 40, and wherein the sequence of said probe consists of 25-70 nucleotides;
- a probe for specific hybridization to said gene viii. of SEQ ID NO: 10, wherein said probe specifically hybridizes to SEQ ID NO: 10, more particularly to SEQ ID NO: 43, and wherein the sequence of said probe consists of 25-70 nucleotides; and
- a probe for specific hybridization to said gene ix. of SEQ ID NO: 8, wherein said probe specifically hybridizes to SEQ ID NO: 8, more particularly to SEQ ID NO: 37, and wherein the sequence of said probe consists of 25-70 nucleotides.

Advantageously, said probe for specific hybridization on gene i. of SEQ ID NO: 1 can be in fluidic contact (*e.g.,* on a solid support or array, such as a micro- or nano-array)
with said probe for specific hybridization on gene ii. of SEQ ID NO: 2,
with said probe for specific hybridization on gene iii. of SEQ ID NO: 3, and
with said probe for specific hybridization on gene iv. of SEQ ID NO: 4.

The application relates to a solid support or array, such as a micro- or nano-array, which comprises at least one, at least two, at least three or the four of said probes (covalently) linked thereto.

Advantageously, said probe for specific hybridization on gene vi. of SEQ ID NO: 5 can be in fluidic contact (*e.g.,* on a solid support or array, such as a micro- or nano-array) with said probe for specific hybridization on gene vii. of SEQ ID NO: 6,

The application relates to a solid support or array, such as a micro- or nano-array, which comprises at least one or the two of said probes (covalently) linked thereto.

Advantageously, said probe for specific hybridization on gene v. of SEQ ID NO: 9 can be in fluidic contact (*e.g.,* on a solid support or array, such as a micro- or nano-array)
with said probe for specific hybridization on gene viii. of SEQ ID NO: 10, and
with said probe for specific hybridization on gene ix. of SEQ ID NO: 8.

The application relates to a solid support or array, such as a micro- or nano-array, which comprises at least one, at least two or the three of said probes (covalently) linked thereto.

Advantageously, said probe for specific hybridization on gene x. of SEQ ID NO: 7 and/or said probe for specific hybridization on said gene xi. of SEQ ID NO: 11 can be in fluidic contact (*e.g.,* on a solid support or array, such as a micro- or nano-array)
with said probe for specific hybridization on gene v. of SEQ ID NO: 9,
with said probe for specific hybridization on gene viii. of SEQ ID NO: 10, and
with said probe for specific hybridization on gene ix. of SEQ ID NO: 8.

The application relates to a solid support or array, such as a micro- or nano-array, which comprises at least one, at least two, at least three of the four of said probes (covalently) linked thereto.

Said probes may further comprise at least one probe, which specifically hybridizes to said serogroup IVb marker gene, more particularly to any one of SEQ ID NOs: 15 and 180-181, more particularly to said serogroup IVb marker gene of SEQ ID NO: 15.

Said probes may further comprise at least one probe, which specifically hybridizes to said lineage I marker gene, more particularly to any one of SEQ ID NOs: 12 and 156-157, more particularly to said lineage I marker gene of SEQ ID NO: 12, and at least one probe, which specifically hybridizes to said serogroup IIb and lineage II marker gene, more particularly to any one of SEQ ID NOs: 14 and 172-179, more particularly to said serogroup IIb and lineage II marker gene of SEQ ID NO: 14.

Said probes may further comprise at least one probe, which specifically hybridizes to said lineage II marker gene, more particularly to any one of SEQ ID NOs: 13 and 158-171, more particularly to said lineage II marker gene of SEQ ID NO: 13.

A probe may further comprise at least one detection label, for example at least one fluorophore (*e.g.,* 6-carboxyfluorescein, or tetrachlorofluorescein).

Various formats (types) of probes, including TAQMAN™ probes (hydrolysis probes), beacon probes (*cf. e.g.,* Tyagi S. and Kramer F.R. 1996. Nat. Biotech. 14:303-308) and SCORPION™ probes (*cf. e.g.,* Whitcomb et al., 1999, Nat. Biotech. 17:804-807) and the adjacent hybridization probes (*cf. e.g.,* Wittwer, CT. et al., 1997, BioTechniques 22:130-138), are known in the art.

In addition to the fluorophore, a probe may be covalently linked to a quencher (*e.g.,* a carboxytetramethylrhodamine fluorescent dye).

In addition to the hybridization sequence, the probe may comprise beacon arms, more particularly a 5' beacon arm and a 3' beacon arm, which impart a hairpin-configuration to the probe when unhybridized (*e.g.,* the 3' beacon arm is complementary to the 5' beacon arm). A beacon arm may *e.g.,* consist of 5-10 nucleotides, more particularly of 5, 6, 7, 8, 9 or 10 nucleotides. Hence, a probe in beacon format typically consists of 25-70 nucleotides, more particularly of 25-65 nucleotides, more particularly 35-60 nucleotides, more particularly of 40-55 nucleotides.

When used in real-time with a primer pair, the Tm of the probe may be 4-10°C higher than the Tm of the primer pair.

Primers and/or probes can be in mixture (*i.e.,* mixed together), *e.g.,* a multiplex of oligonucleotides comprising primers, or probes, or primers and probes, can be contained in the same amplification tube.

For example, the primers, or the primers and probes, for the determination the presence or absence of each of said genes i.-iv., can be contained in mixture in a first container, *e.g.,* a first tube suitable for nucleic acid amplification.

For example, the primers, or the primers and probes, for the determination the presence or absence of each of said genes vi.-vii. can be contained in mixture in a second container, *e.g.,* a second tube suitable for nucleic acid amplification.

For example, the primers, or the primers and probes, for the determination the presence or absence of each of said genes v., viii. and ix. can be contained in mixture in a third container, *e.g.,* a third tube suitable for nucleic acid amplification.

Alternatively, said primers, or probes, or primers and probes can be separate (*i.e.,* not mixed together) but contained in the same container. More particularly, they can be in fluidic contact, *i.e.,* without any material or physical barrier that would prevent a single drop of fluid (or of liquid) to contact all the probes (*e.g.,* probes, which are attached at separate locations in the same spot of a micro- or nano-array).

More particularly, said primers are in mixture in a container (such as a tube suitable for nucleic acid amplification, or such as a kit).

More particularly, said probes are in fluidic contact (*e.g.,* on a solid support or array, such as a micro- or nano-array, to which they are (covalently) linked)

The means of the application allow detecting that a product, more particularly a product intended for administration to a human (*e.g.,* a product intended for administration to a human *via* the oral or intra-gastric route or the intra-peritoneal route, more particularly *via* the oral or intra-gastric route, such as a food or beverage intended for human consumption) is pathogenic to human. Indeed, the means of the application allow detecting that said product contains a *L. monocytogenes* isolate, which has a high probability of having a potentiality, more particularly a high potentiality, of causing invasive infection in a human. The means of the applications also allow detecting that the line of production of said product is contaminated with an isolate of *Listeria monocytogenes* that is pathogenic to human.

More particularly, the means of the application allow detecting that said product is highly pathogenic to human, *i.e.,* that said product contains a *L. monocytogenes* isolate, which has a high probability of having a potentiality, more particularly a high potentiality, of causing invasive infection in a human, *i.e.,* a *L. monocytogenes* isolate, which has a high probability of causing a life-threatening infection or an infection where said *L. monocytogenes* isolate is present in a normally sterile body tissue.

Said potentiality of causing an invasive infection is particularly high, where the human to which the product is intended to be administered, is an immunocompromised human, a pregnant woman, a fetus, a neonate, an infant, a child, or an elderly.

In the application, the term "neonate" encompasses a human of 1-day old to less than 4-week old, the term "infant" encompasses a human of 4-week old to less than 1-year old, the term "child" encompasses a human of 1-year old to less than 12-year old, the term "elderly" encompasses a human above 60-year old, more particularly above 65-year old, more particularly above 70-year old, more particularly above 75-year old, more particularly above 80-year old.

Said isolate of *Listeria monocytogenes* may *e.g.,* be present or detected in a product intended for administration to a human, *e.g., via* the oral or intra-gastric route or the intra-peritoneal route, more particularly *via* the oral or intra-gastric route. Said isolate of *Listeria monocytogenes* may *e.g.,* be present or detected in a food or beverage product intended for human consumption.

More particularly, said isolate of *Listeria monocytogenes* may be present or detected as contaminant microorganism in a food or beverage intended for human consumption.

Said isolate of *Listeria monocytogenes* may *e.g.,* be present or detected in a tissue or body fluid of a human, more particularly of an immunocompromised human, a pregnant woman, a fetus, a neonate, an infant, a child, or an elderly, or in a sample of said tissue or body fluid. Said tissue or body fluid can *e.g.,* blood, plasma, a tissue or fluid from the gastrointestinal tract, a tissue or fluid from the brain, a tissue or fluid from the spinal cord or a tissue or fluid from the placenta.

Detecting that gene(s) is(are) present in said *L. monocytogenes* isolate may *e.g.,* be performed on a sample or non-naturally occurring sample containing nucleic acid material, which has been (previously) isolated or extracted from said isolate or which has been (previously) purified from a lysis extract of said isolate.

Nucleic acid isolation or extraction as well as nucleic acid purification are well known in the art, and include any means that the person of average skill in the art may find appropriate for nucleic acid isolation, extraction or purification from *L. monocytogenes,* such as a lysis buffer for DNA extraction, which contains Sodium Dodecyl Sulfate (SDS) and optionally sodium hydroxide.

The application also relates to oligonucleotide products as such.

The application relates to each primer, primer pair, combination of primers, combination of primer pairs, more particularly to each set or multiplex of primers or of primer pairs, which is herein described. The application relates more particularly to a set of oligonucleotides, which comprises at least two, three or four primer pairs of the application.

The application relates to each probe, combination or set of probes, which is herein described.

The application relates to each combination or set of oligonucleotides comprising at least one primer pair and at least one probe of the application, more particularly at least two primer pairs and at least one probe of the application, more particularly at least two primer pairs and at least two probes of the application.

More particularly, the application relates to a set of oligonucleotides (for detection of genes i.-iv.), which comprises:
- said primers for specific amplification from said gene i. of SEQ ID NO: 1, more particularly the primers of SEQ ID NOs: 17 and 18,
- said primers for specific amplification from said gene ii. of SEQ ID NO: 2, more particularly the primers of SEQ ID NOs: 20 and 21,
- said primers for specific amplification from said gene iii. of SEQ ID NO: 3, more particularly the primers of SEQ ID NOs: 23 and 24, and
- said primers for specific amplification from said gene iv. of SEQ ID NO: 4, more particularly the primers of SEQ ID NOs: 26 and 27,
and/or which comprises
- said probe for specific hybridization on gene i. of SEQ ID NO: 1,
- said probe for specific hybridization on gene ii. of SEQ ID NO: 2,
- said probe for specific hybridization on gene iii. of SEQ ID NO: 3, and
- said probe for specific hybridization on gene iv. of SEQ ID NO: 4.

Said set may comprise said primers and/or probes, more particularly said primers, in mixture together in said set.

Said set may further comprises at least one primer pair or probe for the specific detection of a marker control gene, more particularly of said serogroup IVb marker gene.

More particularly, the application relates to a set of oligonucleotides (for detection of genes vi.-vii.), which comprises:
- said primers for specific amplification from said gene vi. of SEQ ID NO: 5, more particularly the primers of SEQ ID NOs: 29 and 30, and
- said primers for specific amplification from said gene vii. of SEQ ID NO: 6, more particularly the primers of SEQ ID NOs: 32 and 33,
and/or which comprises
- said probe for specific hybridization on gene vi. of SEQ ID NO: 5, and
- said probe for specific hybridization on gene vii. of SEQ ID NO: 6,

Said set may comprise said primers and/or probes, more particularly said primers, in mixture together in said set.

Said set may further comprises at least one primer pair or probe for the specific detection of a marker control gene or of a combination of control marker genes, more particularly at least one primer pair or probe for the specific detection of said lineage I marker gene and at least one primer pair or probe for the specific detection of said serogroup IIb and lineage II marker gene.

More particularly, the application relates to a set of oligonucleotides (for detection of genes v., viii. and ix.), which comprises:
- said primers for specific amplification from said gene v. of SEQ ID NO: 9, more particularly the primers of SEQ ID NOs: 41 and 42,
- said primers for specific amplification from said gene viii. of SEQ ID NO: 10, more particularly the primers of SEQ ID NOs: 44 and 45, and
- said primers for specific amplification from said gene ix. of SEQ ID NO: 8, more particularly the primers of SEQ ID NOs: 38 and 39,
and/or which comprises
- said probe for specific hybridization on gene v. of SEQ ID NO: 9,
- said probe for specific hybridization on gene viii. of SEQ ID NO: 10, and
- said probe for specific hybridization on gene ix. of SEQ ID NO: 8,

Said set may comprise said primers and/or probes, more particularly said primers, in mixture together in said set.

Said set may further comprise (for the further detection of genes x. and xi.)
- said primers for specific amplification from said gene x. of SEQ ID NO: 7, more particularly the primers of SEQ ID NOs: 35 and 36, and/or
- said primers for specific amplification from said gene xi. of SEQ ID NO: 11, more particularly the primers of SEQ ID NOs: 47 and 48,
and/or may further comprises
- said probe for specific hybridization on gene x. of SEQ ID NO: 7, and/or
- said probe for specific hybridization on gene xi. of SEQ ID NO: 11.

Said set may further comprises at least one primer pair or probe for the specific detection of a marker control gene, more particularly of said lineage II marker gene.

The application also relates to products, such as a container or kit, which comprise at least one oligonucleotide product of the application.

Said container may *e.g.,* be
- a tube or a multiwell plate suitable for nucleic acid amplification (*i.e.,* suitable for receiving a reaction mixture containing oligonucleotides, e.g., primers, or primers and probes, for nucleic acid amplification); or
- a solid support such as an array (*e.g.,* a macro-array, a micro-array or a nano-array), a chip or microbeads, suitable for nucleic acid hybridization or for nucleic acid sequencing (and further suitable for containing oligonucleotides, *e.g.,* probes, (covalently) linked thereto).

The oligonucletides or primers of a set (or multiplex) of the application may be contained in mixture in said tube or multiwell plate.

Said solid support may *e.g.,* be formed from (or comprise or essentially consist of) polymer(s) and/or plastic (in particular polystyrene), or glass, or silicon.

Said at least one oligonucleotide product of the application (*e.g.,* a set of oligonucleotides or of primers of the application) may be (covalently) linked to said solid support, *e.g.,* through a molecular linker. The oligonucleotides or primers of a set (or multiplex) of the application, which are linked to said solid support, may be in fluidic contact with each other.

A kit of the application comprises at least one oligonucleotide product of the application (more particularly, at least one set or multiplex of oligonucleotides of the application, more particularly, at least one set or multiplex of primers of the application), or comprises a container of the application (*e.g.,* a tube, a multiwell plate or a solid support of the application).

More particularly, a kit of the application comprises at least two different sets or multiplex of oligonucleotides of the application, more particularly, at least two different sets or multiplex of primers of the application, or comprises a container (*e.g.,* a tube, a multiwell plate or a solid support of the application), which contains said at least two different sets (or multiplex) of oligonucleotides (or of primers) of the application

More particularly, a kit of the application comprises at least two or the three of the three following sets of oligonucleotides:
- a set of oligonucleotides for detection of genes i.-iv. as above-described,
- a set of oligonucleotides for detection of genes vi.-vii. as above-described,
- a set of oligonucleotides for detection of genes v., viii. and ix. as above-described (or a set of oligonucleotides for detection of genes v., viii. and ix. and for the further detection of genes ix. and x. as above-described).

More particularly, a kit of the application comprises three sets of oligonucleotides, wherein
- a first set is the set of oligonucleotides for detection of genes i.-iv. as above-described,
- a second set is the set of oligonucleotides for detection of genes vi.-vii. as above-described,
- a third set is the set of oligonucleotides for detection of genes v., viii. and ix. as above-described (or a set of oligonucleotides for detection of genes v., viii. and ix. and for the further detection of genes ix. and x. as above-described).

The oligonucleotides of a set can be mixed together in the set.

Each set of oligonucleotides may be separate or distinct from another set of oligonucleotides.

A kit of the application may further comprise an instruction leaflet and/or at least one reagent
- for determining that an isolate of *Listeria monoccytogenes* has a high probability of belonging to clone CC1, to clone CC2, to clone CC4, to clone CC6, to clone CC7, to clone CC3, to clone CC5, to clone CC8 or CC16, or to clone CC155 of *L*. *monoccytogenes,* or
- for determining that a *Listeria monocytogenes* isolate has a high probability of having a potentiality, more particularly a high potentiality, of causing invasive infection in a human, or
- for determining that a *Listeria monocytogenes* isolate, which belongs to one of serogroups IVb, IIb, IIa and IIc, more particularly to serogroup IVb or to serogroup IIb or to one of serogroups IIa and IIc, has a high probability of having a potentiality, more particularly a high potentiality, of causing invasive infection in a human, or
- for determining whether a *Listeria monocytogenes* isolate has a high or low probability of having a potentiality, more particularly a high potentiality, of causing invasive infection in a human, or
- for detecting or determining that a product intended for administration to a human (*e.g., via* the oral or intra-gastric route or the intra-peritoneal route, more particularly *via* the oral or intra-gastric route, such as a food or beverage intended for human consumption) is pathogenic to human (due to the presence of *Listeria monocytogenes*), more particularly pathogenic to an immunocompromised human, a pregnant woman, a fetus, a neonate, an infant, a child, or an elderly, or
- for implementation of a method or product of the application.

Said at least one reagent may *e.g.,* be at least one reagent suitable for DNA nucleic acid amplification, more particularly for DNA nucleic acid amplification from *L. monocytogenes.*

Said kit may *e.g.,* further comprise one or several reagents selected from
- a (DNA) polymerase from a microorganism other than *L. monocytogenes, e.g.,* a (DNA) polymerase from *Thermus aquaticus,*
- free dNTPs,
- a buffer solution suitable for nucleic acid amplification, more particularly for PCR amplification, such as a Tris-HCl buffer suitable for nucleic acid amplification, more particularly a Tris-HCl buffer suitable for nucleic acid amplification, which is at pH 8-8.5, more particularly a Tris-HCl buffer suitable for nucleic acid amplification, which is at pH 8-8.5 and contains KCl and MgCl₂,
- a cell lysis buffer for DNA extraction, such as a cell lysis buffer containing SDS or SDS and NaOH, more particularly a cell lysis buffer suitable for DNA extraction from *L. monocytogenes.*

The application also relates to
- a computer program product, for storage in a memory of a processing unit or on a removable memory support for cooperation with a reader of said processing unit, characterized in that it comprises instructions for carrying out a method of the application;
- an apparatus for carrying out a method of the application, more particularly a computer device, comprising a processing unit in the memory of which is stored the computer program product of the application, and measurement values for the presence or absence of genes as defined in the application; and
- a computer readable medium comprising software code adapted to cause the apparatus or computer device to perform a method of the application.

The term "comprising", which is synonymous with "including" or "containing", is open-ended, and does not exclude additional, unrecited element(s), ingredient(s) or method step(s), whereas the term "consisting of" is a closed term, which excludes any additional element, step, or ingredient which is not explicitly recited.

The term "essentially consisting of" is a partially open term, which does not exclude additional, unrecited element(s), step(s), or ingredient(s), as long as these additional element(s), step(s) or ingredient(s) do not materially affect the basic and novel properties of the invention.

The term "comprising" (or "comprise(s)") hence includes the term "consisting of" ("consist(s) of"), as well as the term "essentially consisting of" ("essentially consist(s) of"). Accordingly, the term "comprising" (or "comprise(s)") is, in the present application, meant as more particularly encompassing the term "consisting of" ("consist(s) of"), and the term "essentially consisting of" ("essentially consist(s) of").

In an attempt to help the reader of the present application, the description has been separated in various paragraphs or sections. These separations should not be considered as disconnecting the substance of a paragraph or section from the substance of another paragraph or section. To the contrary, the present description encompasses all the combinations of the various sections, paragraphs and sentences that can be contemplated.

Each of the relevant disclosures of all references cited herein is specifically incorporated by reference. The following examples are offered by way of illustration, and not by way of limitation.

### EXAMPLES

### Example 1: Identification of hyper- and hypo-virulent clones of L. monocytogenes

The French National Reference Center of Listeria (NRCL) receives 99% of the isolates involved in clinical infections in France as well as 80% of the food isolates from official controls and own-checks by Industry that do not comply with European regulations. Consequently, the NRCL's strain collection contains a pool of strains representative of the *L. monocytogenes* isolates in contact with humans and of the isolates that really infect patients.

The MLST clone for over 6,000 surveillance isolates isolated between 2005 and 2013 from human infection cases (40%) and from food sources (60%) was determined. Among the isolates that were analyzed, 81% were represented only by 13 MLST clones that we considered as major clones (Figure 3). Some of them were significantly associated to clinical or food origins.

As expected, at lineage level, the proportion of food and clinical isolates were significantly different between both lineages, with 56% of the lineage I isolates but only 24% of the lineage II isolates being of clinical origin (p < 1.10⁻¹⁵⁵). Similarly, significant differences were also observed between IVb and IIb serogroups, as 62% and 42% of the isolates were of clinical origin, respectively (p < 1.10⁻²²). These results confirm the common view that lineage I and serotype 4b are associated with infections.

As compared to the entire dataset, lineage I clones CC1, CC2, CC4, and CC6 (p < 1.10⁻⁴), as well as the lineage II clones CC101-90, CC207 (P-values < 1.10⁻³) and CC7 (p < 1.10⁻⁴) were significantly associated to a clinical origin. In contrast, the lineage II clones CC121, CC9, CC31 (P-values < 1.10⁻⁴) and CC204 (p < 1.10⁻³) were significantly associated to a food origin. In addition, CC1, CC2, CC4 and CC6 represented almost half of the clinical isolates, and CC9 and CC121 more than 40% of the food isolates (Figures 4A, 4B and 4C).

At the lineage level, the clones CC1 and CC4 (lineage I) as well as CC101-90, CC155, CC207, CC7, CC8-16 and ST504 (lineage II) were significantly associated to clinical origin (p < 1.10⁻⁴). In contrast, the clones CC5 and CC3 (lineage I) as well as CC121, CC31 and CC9 (lineage II) were significantly associated to a food origin (p < 1.10⁻⁴), as compared to other clones of their respective lineage.

Eleven of the thirteen major clones are the most frequently isolates from clinical infections, *i.e.,* clones CC1, CC2, CC4, CC6, CC3, CC5, CC121, CC155, CC7, CC8-16 and CC9 (Figure 1, top panel). Clones CC8 and CC16 have highly similar Pulsed Field Electrophoresis (PFGE) profiles; they were therefore merged for clone identification and were considered as a single clone (CC8-16).

Table 1 below shows the correspondence of the (eleven) CC clones with the lineage and serotype classifications as well as with the serogrouping profile (as established by the reference method of multiplex PCR serogrouping of Doumith *et al.* 2004).

**Table 1:**

| Clones [&] | Main serotypes [§] | Serogroup [‡] | Lineage [*] |
|---|---|---|---|
| CC1; CC2; CC4; CC6 | 4b ; 4d ; 4e | IVb | I |
| CC3; CC5 | 1/2b ; 3b | IIb | |
| CC7 ; CC8 and CC16 ; CC121 ; CC155 | 1/2a ; 3a | IIa | II |
| CC9 | 1/2c ; 3c | IIc | |

| | | | |
|---|---|---|---|
| [*] as determined by sequence-based studies (*cf. e.g.,* Ward *et al.* 2008, Ragon *et al*., 2008). [§] as determined by serotyping of somatic and flagellar antigens (*cf. e.g.,* Seeliger and Hohne 1979). [‡] as determined by multiplex PCR serogrouping of Doumith *et al.* 2004. [&] defined as MLST clonal complexes as described in Ragon *et al.* 2008. | | | |

Clones CC1, CC2, CC4, CC6, and CC7, and the group consisting of the other strains from serogroup IVb "group "other IVb") were significantly associated to clinical origin (p<1.10-4). These clones and the group "other IVb" have a high potentiality of causing an invasive infection in a human (and are therefore classified as hyperinfective) (Figure 2A and 2B).

Clones CC155, CC8-16, CC3, and CC5, and the group consisting of the other strains from serogroup IIb "group "other IIb") were not significantly associated to either clinical or food origin. These clones and the group "other IIb" still have a potentiality of causing an invasive infection in a human (Figure 2A and 2B).

Clones CC9 and CC121, and the group consisting of the other strains from lineage II ("group "other IIa") were significantly associated to food origin (p<1.10-5). These clones and the group "other IIa" have a low potentiality of causing an invasive infection in a human (and are therefore classified as hypoinfective) (Figure 2A and 2B).

### Example 2: Clone-specific target genes to identify major clones of Listeria monocytogenes

Clone-specific target genes to identify major clones of *L. monocytogenes* were identified based on a comparative genomics analysis. The objective was to identify genes that are specifically present in the clones of interest, and totally absent from all the other clones.

For this purpose, it was important to use a set of genomes that covers the diversity of *L. monocytogenes* clones. As no such set of genome existed in public databases, we constructed our own genome dataset. We therefore selected and sequenced by ILLUMINA® a set of 69 strains that represented the species diversity based on their MultiLocus Sequence Typing (MLST) and Pulsed Field Gel Electrophoresis (PFGE) types. A minimum of two strains with distinct frequent PFGE profiles (considering the two enzymes *AscI* and *ApaI*) and isolated from distinct sources have been selected per major clone, when it was possible. They comprised 37 strains that were isolated from human clinical infections, 16 from animal infections, 12 from food sources, 1 from the environment and 3 from unknown origin (*i.e.,* 69 isolated strains). We also included in the analysis 35 genomes from public databases that were of good quality, but did not represent all important clones of *L. monoccytogenes.*

The global view of the diversity covered by the 104 sequenced strains is illustrated in the attached minimum spanning tree (Figure 5). The 104 genomes covered the breadth of diversity of *L. monocytogenes* and comprised 41 genomes from lineage I, 57 from lineage II, 5 from lineage III and 1 from lineage IV strains. Clones CC8 and CC16 were considered as a unique clone (CC8-16) since strains belonging to these clones have such similar PFGE profiles that it is difficult to discriminate them only on the PFGE profile criteria. Moreover, no genome was available for CC16 at the time of the analysis.

Genes that are specific for eleven major clones CC1, CC2, CC4, CC6, CC3, CC5, CC7, CC8, CC121, CC155 and CC9, lineages or serogroups were identified, representing putative target genes for PCR identification. Those clone-specific genes that are not present on mobile genetic elements were selected: *cf.* Table 2 below. Some of these genes allowed the production of primers that are suitable for multiplex PCR (*cf.* example 3 below). These genes are listed in Table 4 below. Control genes, *i.e.,* genes specific for lineage I, serogroup IVb, serogroup IIb and lineage II, were selected: *cf.* Table 3 below. Table 5 below shows control genes that allowed the production of primers that are suitable for multiplex PCR (*cf.* example 3 below).

**Table 2:**

| CLONES | GENES (clone-specific genes that are not present on mobile elements) | |
|---|---|---|
| | NAME | SEQ ID NO: |
| CC1 | *LMOf2365*_*2702* | 1 |
| | *LMOf2365_0687* | 61 |
| | *LMOf2365*_*2701* | 62 |
| | *LMOf2365*_*2703* | 63 |
| | *LISMF_2745* | 64 |
| | *LMOf2365*_*2704* | 65 |
| | *LMOf2365*_*2705* | 66 |
| | *LMOf2365*_*2706* | 67 |
| | *LISMF_2749* | 68 |
| | *LISMF_2841* | 69 |
| | *LISMF_2842* | 70 |
| | *LMOf2365_2799* | 71 |
| CC2 | *LMOATCC19117 2417* | 2 |
| | *LMOATCC19117_0336* | 72 |
| | *LMOATCC19117_0355* | 73 |
| | *LMOATCC19117_0356* | 74 |
| | *LMOATCC19117 1127* | 75 |
| | *LMOATCC19117 1128* | 76 |
| | *LMA19117_1703* | 77 |
| | *LMOATCC19117 2382* | 78 |
| | *LMOATCC19117_2383* | 79 |
| CC4 | *LMOL312_1102* | 3 |
| | *Lm4b_00679* | 80 |
| | *Lm4b_00680* | 81 |
| | *Lm4b_00681* | 82 |
| | *Lm4b_01121* | 83 |
| | *LMOL312_0396* | 84 |
| | *LMOL312_0397* | 85 |
| CC6 | *LM1816_RS00050* | 4 |
| | *0600581v1_10346* | 86 |
| | *0600581v1_10347* | 87 |
| | *0600581v1_10348* | 88 |
| | *0600581v1_10349* | 89 |
| | *0600581v1_10350* | 90 |
| | *0600581v1_10351* | 91 |
| | *0600581v1_10352* | 92 |
| | *0600581v1_10353* | 93 |
| | *0600581v1_10355* | 94 |
| | *0600581v1_10356* | 95 |
| | *0600581v1_10357* | 96 |
| | *0600581v1_10358* | 97 |
| | *0600581v1_70288* | 98 |
| | *0600581v1_140005* | 99 |
| | *0600581v1_140006* | 100 |
| | *0600581v1_140042* | 101 |
| | *0600581v1_140049* | 102 |
| | *0600581v1_210015* | 103 |
| | *0600581v1_210016* | 104 |
| | *0600581v1_240129* | 105 |
| | *0600581v1_280080* | 106 |
| CC3 | *LMOSLCC2482_0307* | 5 |
| | *SLCC2482_0277* | 107 |
| | *LMOSLCC2482_0304* | 108 |
| | *LMOSLCC2482_0305* | 109 |
| | *LMOSLCC2482_0306* | 110 |
| | *LMOSLCC2482_1467* | 111 |
| CC5 | *M637_08170* | 6 |
| | *0500704v1_110453* | 112 |
| | *0500704v1_110642* | 113 |
| | *0500704v1_110643* | 114 |
| | *0500704v1_110644* | 115 |
| | *0500704v1_110646* | 116 |
| | *0500704v1_110647* | 117 |
| | *0500704v1_110648* | 118 |
| | *0500704v1_111156* | 119 |
| | *0500704v1_111157* | 120 |
| | *0500704v1_160076* | 121 |
| | *0500704v1_180010* | 122 |
| CC121 | *LM6179_0610* | 7 |
| | *070104v1_70003* | 123 |
| | *070104v1_110043* | 124 |
| | *070104v1_110044* | 125 |
| | *070104v1_190011* | 126 |
| | *070104v1_210032* | 127 |
| | *070104v1_490004* | 128 |
| CC155 | *LMLG_RS02400* | 8 |
| | *LISMN_0064* | 129 |
| | *LMLG_2609* | 130 |
| | *LISMN_0816* | 131 |
| | *LMLG_1649* | 132 |
| | *LMLG_1650* | 133 |
| | *LMLG_2951* | 134 |
| | *LMLG_1653* | 135 |
| | *LISMN_0842* | 136 |
| | *LISMN_2393* | 137 |
| | *LISMN_2481* | 138 |
| | *LMLG_3016* | 139 |
| | *LISMN_2757* | 140 |
| | *LMLG_0301* | 141 |
| CC7 | *LMRG_02577* | 9 |
| | *LM03S_0078* | 142 |
| | *LMRG_02892* | 143 |
| | *LMRG_02891* | 144 |
| | *LMRG_02937* | 145 |
| | *LM03S_0305* | 146 |
| | *LMRG_02934* | 147 |
| | *LMRG_02576* | 148 |
| | *LMRG_02575* | 149 |
| CC8 and/or (and) CC16 | *LM5578_1185* | 10 |
| | *LISM1_0269* | 150 |
| | *LISM1_1210* | 151 |
| | *LM5578_1182* | 152 |
| | *LM5578_1183* | 153 |
| | *LISM1_1214* | 154 |
| | *LISM1_1215* | 155 |
| CC9 | *lmo1118* | 11 |

**Table 3:**

| CONTROL GENES (genes with lineage/subgroup specificity) | | |
|---|---|---|
| SPECIFICITY | NAME | SEQ ID NO: |
| Lineage I | *LMOf2365_2638* | 12 |
| | *LMOf2365_2058* | 156 |
| | *LISMF_2812* | 157 |
| Lineage II | *lmo0525* | 13 |
| | *LISIN_0086* | 158 |
| | *LISIN_0633* | 159 |
| | *lmo0734* | 160 |
| | *lmo0735* | 161 |
| | *lmo0737* | 162 |
| | *lmo1060* | 163 |
| | *lmo1061* | 164 |
| | *lmo1062* | 165 |
| | *LISIN_1421* | 166 |
| | *mlo1968* | 167 |
| | *lmo1971* | 168 |
| | *lmo1973* | 169 |
| | *lmo1974* | 170 |
| | *LISIN_2953* | 171 |
| Serogroup IIb and lineage II | *lmo1077* | 14 |
| | *lmo1076* | 172 |
| | *lmo1079* | 173 |
| | *lmo1080* | 174 |
| | *lmo1081* | 175 |
| | *lmo1082* | 176 |
| | *lmo1083* | 177 |
| | *lmo1084* | 178 |
| | *lmo1085* | 179 |
| Serogroup IVb | *LMOf2365_1900* | 15 |
| | *LMOf2365_2740* | 180 |
| | *LMOf2365_2741* | 181 |

**Table 4:**

| CLONES | SELECTED GENES (clone-specific genes that are not present on mobile elements and that allowed the production of primers for multiplex PCR) | | |
|---|---|---|---|
| | NAME | GENBANK® accession number | SEQ ID NO: |
| CC1 | *LMOf2365*_*2702* | AE017262.2 ; complement(2749802..2751088) | 1 |
| CC2 | *LMOATCC19117_2417* | NC_018584.1 ; complement(2492236..2495424) | 2 |
| CC4 | *LMOL312_1102* | NC_018642.1 ; complement(1135287..1136126) | 3 |
| CC6 | *LM1816_RS00050* | NC_021829.1 ; complement(7752..8792) | 4 |
| CC3 | *LMOSLCC2482_0307* | NC_018591.1 ; (334781..336511) | 5 |
| CC5 | *M637_08170* | CP006592.1 ; (1638443..1639s34) | 6 |
| CC121 | *LM6179_0610* | NZ_HG813249.1 ; complement(614809..616449) | 7 |
| CC155 | *LMLG_RS02400* | NC_017547.1 ; (496745..497509) | 8 |
| CC7 | *LMRG_02577* | NC_017544.1 ; complement(317879..318991) | 9 |
| CC8 and/or (and) CC16 | *LM5578_1185* | NC_013766.2 ; complement(1175757..1177562) | 10 |
| CC9 | *lmo1118* | NC_003210.1 : complement(1154864..1156381) | 11 |

**Table 5:**

| SELECTED CONTROL GENES (genes with lineage/subgroup specificity that allowed the production of primers for multiplex PCR) | | | |
|---|---|---|---|
| SPECIFICITY | NAME | GENBANK® accession number | SEQ ID NO: |
| Lineage I | *LMOf2365_2638* | AE017262.2 ; (2684358..2685827) | 12 |
| Lineage II | *lmo0525* | NC_003210.1 ; (560443..561774) | 13 |
| Serogroup IIb and lineage II | *lmo1077* | NC_003210.1 ; (1107975..1109663) | 14 |
| Serogroup IVb | *LMOf2365_1900* | AE017262.2 ; complement(1924279..1925631) | 15 |

Sequences SEQ ID NOs: 1-15 are as follows (in the 5'-3' orientation):
SEQ ID NO: 1 
ATGGGGAAATTGTTTATTAATGCAACAAAAAATTTTGGCAATAAAATAGAAATAGCA
ATCAGCAACTCAAACGGAAGAATCGATGGAGTACACTATAGTATAGCTGATATACCA
ATAGAAAAAACTTTTGAATTGAGAAGTGAGGGTATTTACACTTTTCCTGAACTACCTC
TTTACTATCTAACCACAAAAAATGATTATATTGAAAAATATTTGGATCGATCACCAAT
TAATTTAGAGGATGATAAAAGCTTTATCCCTAACGAAAGAAATAACAAGCTTATCTGC
ATGGAAAAGCATATTGATTTATCATTTTTAAGTACGCTACCATTATCAACTTTTGAGTA
TTCTACAAAACGTGAAGAATTTTTAAATATGGTTAATACATTTTATGATACTTATTACG
AAGATGATTATCTTATGACGCCGACATTTATGATTAATAAAGTCGGTTCGTGGAAGA
GATTATGTTGCTAGATGTTTTAAAAAATACAAATGAATATCTAATTTCTAATCAGAAA
AGTAACGTTTTTTTGAGTATATTACTTGATCCAGTGCTTTTGGATTCTTCGGATATTGC
AGGTAATATCTTGAACTATATCGACATGTATCCTTTGATAAACAACTTCAGTTTAACT
ATAATTAGTGATGAGTCGATGTATTATCATACAGAAGATAATTATAAAAATATACTTA
TTTTTATAAAGGAGTTAAGAGCTCGAAATATAGAAGTCTACTTGCAATATTGTGGAAT
TAAAGATGTTGTTTTTTCAGTGTTGAATATAAAAAGATTTTCAGTAGGTTGGTTTGGA
AGTTATAGAAATTTTGATACTAATTCAAAGAGAATTTCAGAAGTGAGCTCCAGTTCCT
TTGGAAAGAGAATCAGAAAAATATTGACAGAAAATTTTATGTCAGAGGTGCCGTTAA
GCTATTTAAGTGTTTTAAATGAAAGTGAATGTAAGAAATATTTTTCTATTGAAAAAGA
TAAACTTGAAACTATTGACTATAAGGACCTGGAACAAAGATACTGGTGTGAGATGTT
AAAAATTATAGAAGAAGACACTTCTGACGAGAATCTTTATACTGGAGATGAGTTAAT
AAACTATAGATGTGAAAGAATTAAAATAAAATTTCACAATGCAGTTTCCAATTTGTCT
TTTTTGATAAGAAAACTAAAAGAAGCAGGACGATTTGCGGATGCTACGAAATTAGAA
AAAAATAATTTGAAACATGTAAAACTTTATGAAGAAGTTTTAAATGATTTTCAGGAAA
AATTATTCTTTTAA
SEQ ID NO: 2 
ATGGTTCAATATTATACAGATAAAGAATTTACAGACTTGACACATACACTTGCAAAGG
CAGTAAAGGATTTTAATCTAGTTGTTTCGTAGGAGCAGGAACTTCTTATCACAAGG
ATATCCAAATTGGAATGGTTATATTGAGAAGCTAATTCATTTCTGGCAGTTCAATATT
CAACATGTTGCTGGCGGAAAGTTACAGTGACAAATGAGTTGCTTTCGCAATTTGATG
AAATCTTGAATTCAAATACTACACCAAAAAGAAAAATTGATTTATTGCACACTTTGTT
ACAGGATATTTTGGAAGAAGACTTTAAAGAGGTAAAGCTTAATTTTGAAGAGTATTTT
TTCAAAGATGTTGTACCAGATTACATAGAAAATAGTGTGTTAGTTGAAATGATAAAAT
TAGATCCAATCTTTATCACTTCGAATTATGATTTTGAAATTGAAAGACATTTGAAGGTT
TCAAAACAAAAAGGAGCATTCAAACCAATAAATAATATTCGTGAGTTTGTAGAATCA
 
AATAATAAGTTACGTTCAGGCGATGTTTACATTTACATGGAACCACTCAGGGAAATT
GGGATTTTTTTGTCAATTCATCAGTTGATTATTCTCGACAGTATTTAAAAGAGTCAAGC
GACTTCAATAGTTTAAGTAAATGGTTTCAAGAAAGAAATCCAGTTGTTCTATTTATTG
GAAGTAGTATGGAAGAAGAGGAAATTTATCTCTACTTCCAGCCACTACTAAAAATTT
CGCACTGATGAAAGCAAATTCAAATGAGACACGTAGTTTAGAAAAATCTACAATAA
GACATATCAAAATAACAATAATACAACTATTTTTGGTACGGAGATAGCTATGATGAT
TTACCTGGAAAAGTATCTGAAATTGTAAAGGCTATCGAAAATGAATTAGAAATTCCAC
AAAGCATTGATGATTGGAATATACTCCATTCAGTATCTACTAAGGATAAAATATATAA
GGAAATTCTTGAAAAGCATATAGAAGATAAGAGATTTTATTTGATATTTTTAAAGCA
GAAGATCCTAATTTAGAAAAAAAGATTTTAAAGAATACATTTAGCAGTCATATTTTAT
TAGATAAAATTAGTGATATTAGTAGTTTTTGGACAATGATTGATAAAAATTTTGAAAC
TTTAGATGAAGAACAAGTTCGATCGATTATTAGCATTTTCCAAAAGCAGAGCTTGAGC
GTTTATTGGAAGGAAATCTTTAAAGTTTTTGAGAAATTAAAAGAATCAGAACCTATCG
GACAAGATGATATAAATAAAATGAGAAGAAATCTTTCCCAAGAGCAAGAAATTATTG
AAACAGCTTTCTCTAGTGATGCTGATTTAATGGGTTATTGGTTAATTGAACAACTTCA
AAAGGAAACTTCAAATCGTAGTAGTATTTTTTATGATGATGAGATACTTTCAATTAAT
TTGAAATCTGAAATGATTCCATTAATTGTTAAACTAATGACGGATGAAACTAGATATA
TTTATTGGTCTTTTAAAGAAATTATCTCTGATGAGCTGATAAGAATTATATATGTATCT
CTTCTGAATGACAAAATGTTGTTGGATAATAAGTCAATATTAGATAATTGTCCTGACT
TATTACTAGAATCTCATCCTTTCCAAAGAATTTTAGTAAGTATTGATAATGAGGTTGG
ATTGAATGATTCTATTAAAAATAAGTTGATAACTAAAATTGATTTTCCAATACTACCT
TTGGAAGCGAGCTAAACCTTTTTCAAGAAAACACAAAGATGAGATTGAAGAATTAG
GGATAGAAATTTCAAGAGATTATCAAGATATGATTTTTGGAGTTGAGTCTGGTTTTGT
ACATCAGAAATCATTTATTGATATTAATCAGATTCTTAGTGAAGATATGGATGCTATT
TTGGAAATTTTATTACCCAAACAAGATGAGCTTAGTTCAAAAAGAGAAGACTTTTTTT
CTGAAAGCACATATCAAGAAACATCTAATTTTTATTAAGCTTGTTGAATAAAAATGA
TGAAGTATCAAAAAAAGTAAAACAGATAATTCTTGAAAAAGGATTACTTTTGTATCCA
ATATATGACAAACTTTTCGTTGAGATTTTAGTAAATAATACCTATTGTACTGAATTAA
GAAATGAATCTCTGAATATCTTCTTAGAGAAGTTCAGTTTGAAGAGTTTTAGTTGGGA
AGAGAAGAAATTTTTCGAAGCATTAATTGATAAAGAAGAATTTGAGAACCTAGCTTTT
GAAAAGCTACTAAAAGTAAATGTTAATGAGTTGAACTATGATTATGTTTTTGTAGATA
AAACAAGACCAGAATTACTAGAAGTCAATGATTTTATTAACACGGAACTAGGAAGAT
ATTTAGAGATACTAATAAAATTGAATAAAAAAGAGTACTCTAGGCGGAGTGAAATTA
AAAATATTATATCTCAAGTAAATTCAAAACCATTTAAAGAATTTTCACAAGGTGCTTT
ATCATTAGTGAACAGTTCTGTAGACTTAGAAGAAATTACTATTAATACCTTCCAAGGC
TATTCTTATGTTGTTCGTGGATTTCAAGAAAAAAGTTTGGAAAAATTTAAATCTGTAG
GTCAAGAGTTATTAAAAAAAGGGCTTGTAAATGACTTGAATAAAGATAATCTATTTAT
 
TCTCTCTCTATCCATGATAAATCCGAGGGAGGAAGAAATGGAGATTAATTGGAGTGA
AATTAATTTTTCAAGGATTTTGATATCATTCTTCAAAATGAACTTGAGTTCGCTTTTG
AGTCACAATGGATTGAAAATATAATTTTAAAGGATGAAGATGGACAATATGGGGTGA
GTATCTTATATTCTATCAAAAGAGAGCAAGCTTTAATAGATAAGTCAAAAAAGCTTGT
TAATATATTCGAAAAAGAAATTAGTAATTATAGTGGGGAAATAAAAGTTGATTTGTTA
CCGCATGCTATTAAAAAACAAGACAATCCACAAAAAAAGGATTTATTGATTAGATTCT
TCTTTATATTACTAGATAATGCTAAACTTGCTCAGAGTTATTTTGGAAGTGGGATGTTA
GCAGATTTAATGATGCACTTGAATTCTAGTTTACAGAAAAAACTTGCCAAACATCCTA
AGTTATCGACTATTCTATCACCATTAGAAATTGAAAATTTGAAAAGAGAGATTGAATA
A
SEQ ID NO: 3 
ATGAATCAATTACAATACTCTGAAGATGCTTTAAAAAATAGGTATCTCTTTTTTAAAG
AAATAAATGAAATAAATATTTTTGTTGAAGATAAAGGAAAAGAATATGAATATGAGG
AAATATTGTCTAAAGTTTTTGCTGATGAATATCATATACAGACGATATATGCACTGGG
GGGTAAACCACAAGTTTTATCCGCATTTAGAGACTGTAAAGAACATGTTTCTAGCAAT
AATAATATAAAAAACATATATCTTTTAGATGGTGATTTTGATCAATATTTGGATAATA
TAGTAATGGAATCACATCCACATATTATTTATTTGCAATCATATAATATTGAAAATTAT
TTTTTGAATGAAGAATCGGTAGTAACTTTTATGCAAGGAAAAATGAAAATGCTGAAG
AATGATGTAAGGACTAAAATAGCTTATTCTGAATGGCTTTCTAATACATTATACAATA
TAGAAAACATTTTTATTTTATATTGTATAATTCAAGAAAAGGAATTAGGGATTCCTAA
TGTAGGGGATTCTGAATATAAAGTAATTAATATGATCGATGGATCTATCAATATGACG
AGATATGAGGATTTGAAAAAGCAAATAAGTAATTGTACAGTAAACATAGATTTAGTA
GAGCAAGAAATGAAAGCAAAAATTCACAATATAAATCCTAATGTGTTTGATTTGATTT
GTGGTAAACATTTGATTTGTAGTTTGTACCGTCATCTGTTAAAAATAACTAAAGGTTTC
AGCTATGATGAATTTAGATGGCATTTAGTTCAAAATGTTGAAGTATCTAGACTTAATT
TTATAAAGGAAAGAGTTGCTAATTTGTAA
SEQ ID NO: 4 
ATGAAGAAAAAGAATGTATTAAATTTAATTAAATATCATTCAGAAAAAAATGATTTTC
AGTTTAGAAATGAGGCTATCGAAATTGCTCGCTATTTTGATACAATAGGTGACAGTCA
ATTATCGGAATACATCATGAGTTTGTTATCTACAGCAAATACCTTTGTGCCGCAAGGT
GAGGCTTTTGAGAACTTATTTTTCAAAAAAATAGAACCTAATGTAGAACCATTACCAC
TCCCTAAAGCAATTAGTGAAGATATTCGAGGTATTATAAATGCTGTAAATCATCATGT
AGGAATAAATAAATTTCTCTTCGAGGGTGCACCTGGAACTGGAAAGACAGAAACGGT
TAAACAGATTGCTCGATTATTAGAACGCCAACTATATATGGTAGAATTTAGCGAATTA
ATTGATAGTAAAATGGGGCAAACAGCTAAGAACATTGTAGCAGTGTTCAACGAAATA
 
AATCGTGTATCAAATCCATCAAAATTATTAATACTTTTCGATGAAATAGATGCTATAG
CCCTAGATAGAGTAAACTCTAATGACTTAAGAGAAATGGACGGTAACATCTACAA
TCTTGAAAGAGTTGGATAAGTTAAATGAAGAAGTTGTTCTTATTGCCACTACTAATTT
GTATGAAAAATTTGATAGAGCCTTAATAAGAAGATTTGATTCAGTAATTAATTTTAAT
AGATATGAAAAGGAAGATTTAGTAGAAATTTCAGAAATCATATTGAATTCATTATTGA
AAAAATTTAAAGATGCTGGACGAGATATGAAGTTATTTAAAAAAGTAATCAGAAACA
TGGATGAAATTCCGAATCCTGGAGAATTAAAAAATTTGATTAGGGTATCATTAGCTTT
TAGTGATCCAACAAATGAATTTGATTATCTCAGGAGATTGCTGAGGTATGTAGTTGAA
AAACCTGGTAATATAGAATTGAAAGAGTTACAAAACATGGGGTTTACTGTTAGAGAG
ATAGAGATATTAACTGGTATATCAAAAAGCCAAGTCTCAAGAGAGTTGAAGGGAGAT
TGA
SEQ ID NO: 5 
ATGAGGAGAAATATAAGTAGCATTGATTATGATATTCTGGAGGAGTGTGCTCTAAAAT
ATTTTGAAAAAGTAGAAAAAAAAACAAAATCATTAGATAACAAAAATAAAGCTAGA
ATAGGATTTTATTACTTAGTTTTTGATTTACTCTATAATATGAATCAGGTGGAAGAAGT
GAATACGTGCATTACAGATCAGACTTTCAGTAATATAATGAGACAACAAGATAATAA
AGATTTAGGCATTGATGCTGTATACATAGATGAAGAGAATAAGGAAGTTAATTTTTTT
AATTTTAAATATCGAGAAAAATTTATAAAAGGTAAGACTAAAGCCCATGATGAATTA
AGAGCGTGTGAACCATTTTTACTTTATGTTCAGGATACAAATAGTTTTGAAAAAGATA
AGTCAGAATTAAGTAATTTTACTAGAGAAAAATTAAATGATATAGCTGATGTGAAAA
AAGATTCACAATACTCATATAAGCTATATATGGTAACAAATGATATGACTACTACAGA
TATTGAGGAGCCACAAATAACAGCGTTTAAAAAGAACTATAGTTGGTTAGAAGTTGA
GGATATCAATTTAGCTACTATAAGCGAATGGGTATCTATTCGTTCAAAGGAAAATGTT
TCTATATTAGTATTAGAGAACGAGTCTCTTTTACAACATGGTACGTCATACGAGACAG
CTAATTCATATGTTGCAGAGGTTTCTTTAGTTGAATTAATAAAGATGACAAGTCAAAA
TATCGATTTGCGAATGAAAGAAAATATTGATGATCTTAGCATTGAAGAACTTGCTGAT
TTAGAAATTGATTTAGATGTTTTGTTTGATAATGTCAGAGGGTATCTAGGAAACACAA
ATTATAATAAAAAAATTCTTGAAACTTTGAAAAAAGAACCTGGAAAATTCTTTTTATT
TAACAATGGTATAACAATTACTGCAGATGATATTAATGTGAAAGAAGTTAGACTGGG
TAAGGCTAGTCGAATTAAGCTAGATCGGTTTCAAGTTGTCAATGGGGGACAATCCTTA
CGAACTATATATAAATACAAAGATCAAAAAGACTCACCAATTGAAAATTTAGCTAAG
GCAAGTGTATTAGTGAGATTTTTTAAAACAGGGCAAGAAGAGGGATTAATTAATAAG
GTATCAGAATATACCAATAGTCAGAATGCTATCTCAGCTATTGATTTAAGGACAGTTG
ATCAATTACAATTAAAAATTGAGAAGCACTTAGAACTCAAGGGAATTCAATATATAA
GAAAGAGAAGTTCTGATAAAACTAAGTTGCAACTAGAAAATTCTATTACAATGGAAA
AACTAGGCCAATTGTTATTTGCATACGCGGGTTATCCTGAAAAAGCAGGGAATAATA
 
AGAGGAAAATATTTAGTGAATATTACGAGAAAGTATTTACTAATGAAAGTGATTTGAT
GGATAGAATTATTTCTACTATAGATACTTATCATAAAATAATTTCTTTATATAATGATG
ATGACTACAAATATTATGAGCAGAAAGTTATTATATAATTTATTTAAAAAGATTTTT
ATCAAATAAAAATTTGAAAGAGTGCTTAGAGCTTTTTGAGGATGTTTTGCTCAATTAT
GAAACCAAACAAGAAGTATCCCCCTCTAGAAAGCTATTACAATCTAAATTTAAAGAG
GAAATAAATAGAATAATTAAGTTGGATATAGGAGATATAAAGGGTCTAATTAATTTG
TAG
SEQ ID NO: 6 
GTGAAAAAAGTTGGGAGAAATGAATTATGTCCATGTGGTAGCGGAAGAAATTTAAA
AGATGCCACCTTGGTGTTAATGAAATAGATGAAGATAATATGGAAGACTTTGTTAAG
ACTTATAAACAATTAAATAAAGAAAGGCGAATAAAACAATGCTTATATCCAGAAAAA
TCTAAATGCAGTGATAAAATAATTAGAGCGCATTCTATACAAAATAGTAAAATAATTC
AACGAATATCTGAGAATGGGAATGTTTATATGAACAAAGCAAAACCAAATAATCCAT
TTGCTGTATTTACTAAGTGGGGGCGTAAAGAAGCAACGACATTTACTGGATTTTGTGA
TTATCATGATAAAGTTGTATTCCAGCCAATTGAAGATAGAGACTTTGAGTTAAGTAAT
GAACAGATCTTTTTGCATACTTATAGATGTTTGGTAGCTAACATGCATGATAAGCAAG
AACAAGTTAAGTTAGGGAAATCAACTACTGAAACTTTTTCATTATCCAGTGAATCTAG
TGTTGGATTATTGGGCTTAGGAAATGGCTTGGCAGTGGATGATTTCTTGCGAAAAA
ATGATTTTGGATAAGGCTTTAATAAATGGAGACTTTGATGTAGTTGATTCAATTGTAT
GGGAATTTGATTTTCCCATACAGTTTGCAGGTAGCGCATTTGAAGCTCCTACACATGA
CTTGAAAAATAGAAAGATACAATCTCTCGAAACCAATGATATTATAGCTCATTTATTT
ATTACTATTTTTCCAGAAAATGATAAATCATATTGTATCATTTCATGGCTAAAAAATTC
GAAAGAGATTCTTAGTGAGTTAGTTGAACAAATTATGGGGCTTAGTGCGAAGGAAAG
AAAAGATTATTTAAACACAACTTTAGTAAAAAATTCGGATAATCTTGTTTTTAGTCCT
AAATTGATTGAAAGAATGTCAAAAGATGAAGAGCATTACTTTGGGTATACTTCTCAGG
GAGCCTATCTATTCGAGGCATTACTTGAAGCGATGGATGATGAACTTCATATTGAATC
TAAGATCCCAGAGGTAAGTGATCCAGGTTATGATTTATTCAAGCAATGTTAA
SEQ ID NO: 7 
ATGGTCTCAAGTAAATTAAGAACTACCTGGTTTGTAACTCGTCCTCAACGTGATCCTC
AATTTTTTCACAAGCATTAGAAGCTTAAAAAAAGCAACGAGTAATTTAATGAAAA
ATGGTCCTCTAATAGAGAAATCCATAAAAAATATGAGAAATTTCTAGCAGAGAATGG
GCTAAAAAAAGAAAATATTTCTTCTGATGGATCCGGCGGTCGAACTTGGGTTGCTATG
TTAAGAACATATAGCCTGGTTTATATAGATCCGAACTCTGGTAAACTCATCCCCACTA
AAGTAGGTATAGCTTTACTTAACGGGAAAAAAAGATATGAAAATGTGACGAAACAAC
TACTTACTTTACAAATCCCAAATGCATATTTTGTTTCTTCAGGCTTTAGACCTAAGTAT
 
GATAAAGGCTTCTCAATTCGTCCTATTCGCTTTTTAATAAAGTTAACATGTCAAAGTTC
TTTAGAGTATTATATCACTAAAGAAGAAATTATTTTTTTGCTATGACCGCAAAACAA
GATTGTGAGCTTGATGCAGTAATAGAAAGAATTAAAGATTTTAGGCTTAGTGATTCGA
AAAAAAGAAATTATTTAAAACAAAGTATAGCTACAATTGATTATCGGGAACGATCAG
ATAAAGGAGCCAGAGGTTTTGAAGAAGCTAATGGAGATGTAGCTCACACTCTAATGT
TACAAGCTAGTTATACAGAATTGGTGAAATACTCTGCTGGAAAATTAATAATAGAAC
AGAGCCCTATTGAATTTAAAAAAGTGATGCAAACACTATCAGAATATGATGAGAGGT
ACCCATTCGATAGTCGTTATGAAATTTCTGAACCCAGATTTAGTGAACATGCTGGTTT
AGACATTGATAGTTATAAATCTAGGCCTATTGTAAATACTAAAGTTGCCAGCAATCAG
CAAAAAATAGACCTACAGTTAAAAAAAATCGTGTCTAAATCACCTAACTTAATTGGCC
AAGATTATGACGAGATTGTTAAGATTCTTTTTGTTAATTTTCCTCCAAAAAAAGCAAA
GGAACTTGCGCACAAAATTGTAGAAATGACTCCTAAACACAACCAAATGGATGAGGG
GTTTATAACTTCTTATCTAAACCAAACTAATGATTTAGAATTTGAAAAACAAACAGTT
GAGGTACTTCGAGCTTTGGGTTTTGAAACGGTCCTTCAACCTAAGCCTGTTGTTAAAG
GAGTAAGAACTAGTATTGAAATATTAATTCATCTAGATGCGCAAAATATTGCTATTAT
AGATGCAAAAAACTACAAAAAGAAATTCACTCTAACAGCAAACTTGGCCAATTATAT
GGCTACTGAATATATCCCCATATATCAAGGCTATGATGGAAAAAATGTTACACATTTT
GGATATATTACAGCTTCTAAAATTGGCGGTAGTAGCAATCTAAAAAAAATAGTACAT
ACAGCTAGAACATATAATGATAAATTCCGAGTTAATGGTTTTTAATTTCAGCGCCTG
CTTTACTAGGTCTTCTAGATTATTGTATAGAAAATAGTATTGATAATTCTCGAAGAAA
ACAATATTTCTTAACACTATTAAATGATAATAAAGCATATGAAACCTACAGTGAAGTA
TCACAACGATTAAATTTATCTTAA
SEQ ID NO: 8 
ATGAAAAAAATTCGTAATAGTTTAATTGTAATAATGAGTATTTTTGCGTTAACATTTG
GGATGATACCATCAGGAGTTGCTAATGCACAAGAGCCTAACGAAGAGTCTGATTTTA
AAATCACAGACCATGTTGTAACTGACAATAAAGAGTCTTTTACAATTGCTATGAACGG
TGAGGAAGTGGATTATAAGTCGACTAAAACGGAAGATTCATTCGAAATTGAAACTGT
AGAAGAAAATAATGAAGTACATACCTTTACATATACTAAAGGTGACGATTACATGAT
GATAGACGGCGAAAAAGTATCTGTTGAAATTGATACAGAAGTAGAAGCAGAATTAAC
AGATTCTTCTGCACCAAAACTATTAAAAGCTTCGGCATGGACACCTAAGTATATGTCG
ACAGGATCTATAAAGATAAGTAAGTCAGTAAAAACATTAGGAACTCTGGTTGGAGTA
ATTGGGGGAGCTATTTCTATTGCTGCACTATCAGGGGTGGCAATTGCAACATCAACAA
TTGCAAGTACAGTAGGACACTGGGCTAGTGCAGTAGGTTTAGGCACTATCGCTGCGG
GTTACGCATTTAATGGTAAAGTTCAATTTAAGCAGTATCGTACAACAGGATTAGTGCC
AACAAATTATGGCAAAAGATTATATGCTTTTAGATTTCAAGATGTGCGTGTTATAGGT
 
ACGCTTAAAGGCAAAAAAATAGATAAACAACTGAAAAATGTAGGAAATTGGTTCTTT
AATTCTAAACCGTATTAA
SEQ ID NO: 9 
ATGAGTAAATGGATTTCTACTTCTCTAGGTGAGGTAGCAAAAATAATCACTGGCCCAT
TCGGAACCCAACTTCACAAGTCAGATTATGTTGACTCTGGAGTTGCAGTTATAATGCC
TCAAAATATAGGTTCTAGACAAGTTAATTATGAGAAGATTTCTTACATTTCTGAGGAG
TTTGCAACGACTCTAAGGCGATACAAAGTATTAAAAAACGATATTGTTTTTGCTCGTC
GAGGTGATGTTGAAAAACATGCATTTATCACAGAATCTGAGGAAGGTGAACTCTGCG
GAACTGGATGTTTTCTCGTACGATTCACAAGCGAACATGTTTTACCAGAATTTATTTCT
TTGATTTTAAGTACACCTTTCGTGAAAAAATGGCTAGTCTTAAATGCTGTCGGTTCAA
ATATGCCAAATTTGAATACTGAAATATTAAAAAATGTTCCTATTAAATTTCCAGATTT
ATCTACTCAACAAAAGATTTTATCAACTATTTCATCGGTTGAATATAAAATTAGAATC
AATACAAAGATAAATACTAATTTATTAGACATGGCTAAGGCAATTTACATGCATTCTT
TTTTTGGAAAACATGAGAATGCAAAGATTTCAGATATACTTCTGGAAAACAGTAAGTC
CAACATCCAAGTTGGTGAAGCAAGAGAGGCTCGCGGCGATTATCCATTTTTTACAAGT
GGTGAAACAATTTATGAATGGGATAATTATCTTGTCAAAGATAGAAATATTTATCTTA
ATACTGGTGGAAATGCCGATGTCAAATTCTATATTGGTAAAGCCGCATATTCCACTGA
TACATGGTGCATTTCAGCTAAAAATGATTTACAGACTATCTCTATCTATTCCTTGATG
CAATAAGACCTGAATTAAATCAGAAATTCTTTCAAGGTACCGGGTTAAAACACCTACA
GAAAGCATTAGTTAAAGATAAAGAAATATACCTTCCTTCCAAAGAAATACTAACAGA
ATTTAACTCTATTGTGAAACCGATGATGGAACAGGTATCCTTCAATACTCGTAATAAC
CAATACTTATCTGACCTACGTGATTGGCTATTACTTGTCTTGATGAACGAACTAGCAA
CAATAGAATAG
SEQ ID NO: 10 
ATGACCAAGAAAACTACCATTTCGCATCCCGTGATAAAATCATATGTTGATAACTTTA
TTTCTGAACATGAGATATCAAAATCAAAAGCCAAAAACCAGCATGGTATATTTGAAC
AATATATTAATGATTTAATTTATCAATTTATTCAAACGATCCTAGTGTAACTTATCAA
GATATGGAGACAGGTTCTGCATTTGGAATTGATGGAGTTGCTATATTTGTTTCAGATC
GAGTTGCAACTAGTACGGAAGATGTTGATTCTATAATTGAAGGAGTAAAAAAATTTG
AAGTAAGCTTTTTCTTTACACAATCAAAAACAACAGATCAATTTGATCGAACAGATAT
TTCTGATTTTTTTACAGCTGTTAGACGTTTTTCAATTTTAATAAATGTGAAATACCTG
AACTTAGAGAATATTGGGATGTCGCCAAATATATCTACTCTAAAGGTTCAAAATTCAA
ACGGCAACCAAGTTTAAATATGAAATTCATTACTTTATCTCCTAAAGAAATAGATTTG
ACTGATATCCATATGAAATCAGAAATAGATTTGGGATTGAATGATTTAAATGACATGA
GCCTTTTCAATGAAGTGAAAGTTCCTGACTTTTTAGGAATCAAAGATATTATGTCATT
 
ACATGAGAAAAACACTTCCGGCTTAGAAGTTTCTGTGACTTTAACAAAACTACCCGTA
CCATATCCTAAGGATACTCATAATAAAATAAAAAATGGGTATTATGGATTAATTAAAC
TTGAAGAATTTATACTTTTACTAACAGATGAAGTTAATAATGAAAAGATTTTACGAAA
AGGTATCTTCAATGATAACATTCGTTACTATCTTGGCTCATCAGAAAAAATAGAGGTC
AATACTTCAATGAAAAATCAGCTGTTAGGTACAGAAGATTATTTGTTTGGATTATTAA
ATAATGGTATTACTATAATTGCAGATGAGATTTCATTAAGTTCTGATGACTTAACACTT
ACTAATTACCAAATAGTAAATGGGTGCCAAACAAGTAATGTTATATTTGAATGTTTAG
AGTCTATTGAGCAATCTAGTGACATATATATTCCTATTAGGTTGATTTCAACAGAGGA
CGAAGACACAAAGCACTCCATTATAAAAGCTACTAATAGTCAAACACAATTAAAGCC
AGAACAGTTGGTTGCTTTATCAGCAATTCAGAAAGCTATTGAGCAGTACTATAATACA
AAGGCAAAAGGAAACTCATGCCCTTTATACTATGAACGTAGAACTGAACAATACCGA
GATGAAAATATACCAAAGACAAAAATAATCAATATACCATTTCAAATCAAGGCTACT
TCTGCTTTATTTTTTGATTTACCTCATGAAGTTTCTGGTCAATATGGTAAAGTTGTTAA
ACAAACAGGTGATTTGTTATTTAAAGATACGCATCTATCTTTTCTTAACTCTTATTACG
TAAGTGGTCTTGCTTGGTATAAAGTTGAAAGATTTGTACTGAATAATGAAGATGGCAA
AAGATTCAGAAGAGCTAGATGGCACATTATAATGATTTTAAAATATCTCTGCTTCCCT
ATGGACAAAATAAAAATTTCTATTGATAAGCAATCTGAACAAAATAGTATGATATTA
GAAAAAATACTACTTGATGATGAAAAAACAAATGAACTTTTGAAAAAGGCAATAGAT
ATTATTGAGAAAAGTTTAAGTCAAAATGAATCTATTGAAGAAGTATTAGCGGATAGA
AAAACATTTGAAAGAAAAGAAACTACAACCACTATTATTGAATATGTGAAAACAAAT
AACAATAACTAA
SEQ ID NO: 11 
ATGGGTGTAAAATATGTAGAAATCGAAAAAATTGATAACTATATTGAAAATCCTAGG
CATGAAGTAGGTCTGAATGAATTAGATACTTTAAAAAGTTATTTGATGTTTCTGGAT
ATCAAAATATGATTAATTTGGCACAAGATATCTATACTAATGGTTTGGTAAATGCAAG
TCTAGTTACAATTGTAAAACTCAATAATGCCGATAGATATACTGTATATGAAGGTAAT
AGACGAGTTGCATGTATAAAACTAATTTTACATCCAGAAAAATTAGTTTTTTACCAA
AGAACCAAATTGATCGAATCAAAAAAATGAAAAGTGACACTCCTTCAAAAATAAATC
TCTCTCAAATTGAATGCTTAATAACAGATGAAGAGGATGCTTTTTCATTATGAGGCG
TATACACTCAGGAGAAGATAAAGGTAGGGGTCTTAAATCCTGGAATACTAAGGAACA
AGAAATCTTTAAACTTCGAACTAATCCAAAAAACAGTACATCTATTGCAAAGATTATT
AGTGACAAATATGAAGAATTTTTAAAGAAGATATTCAAGAAGAAATGGCATATACT
AATATCCAAAGATTATTTAATAATTTGGAAGTGAGAGAGTCACTAGGCATTGAAAAA
GACAATATAGATTCTTTTTCATGTGAAAGATTATATTTAATTAAAGGTGTTATTGAAA
AAGTAAACCAGATTGCTAAAATTGAAAATTTATCTATTTCTCGTAAATTTAATAAAAG
GAAAATAATTGAACAAATAGTAATACCCATTATAGAAGAATTAAAATCTAAACAGTT
 
GGAAAGTAAAGCAATAAATTCTATACATATTGAACAAAAAAATAAAAGTAATAAATT
ATCAAATTCTAAAGAACAACAAACACAAAATAAAGAAGCAACTTCAAACAATGGAAT
TAGTGTACGAGAAGATACTGGTTTGAAAACTGAATATAAAAGCATAGAATCTATATC
ATCCACTCTAAAACCAAAATTACCCCCAAAAATAGGGAGCGGAAAACTTCTAAGTAG
TACTATGCCCTTTACAAATTTATATCATTCTAATGTGCGTATAAATACTCTAGTAATTG
AGATTAATCAGATTAGATATAAGGATTTTCGTTTAGCTACACAATTCTTATTAAGGTCT
CTTATGGAAGTATATGGACATGAATATATTGATTACTTTGCAAATTTAGACTATCAAG
ATGTCCAGAAAATGAAGGCTATAGCAAAAGACCGTATAAAACGAAATCAAACAGTAA
ATGAAATTTATTCAGAGTTTATTAGTAACCACATAACTAAACATTTTCCTAAGTATGC
CGAACAAGCCGAATTAATTAATATATCATTATCAAAAAACAACAATAGTTCTCTAATG
AAAATTTTGAATTTTATGGTTCATTCTCAAAATCATATTCCTGATAGTATCGAATTAAT
AGAAGCTTGGTACAAAGTTAAATCTATCGTTGAAGCAATTGATAATATATTGCATGAG
GTTAAGCATAACTACTAA
SEQ ID NO: 12 
GTGTTACAAAGAAGATTTTGGGGTATTTTTTGTTTTGCCATATTCTTGTTTTTATTTCCA
ACTATTGGTTCTGCCGAAACATCTGGTGACTATGAATATACAATAAATGGAAATGAAG
CTACCATTACTGATTATACAGGACAGTCTACTGATATTACCATTCCTACAACACTTGGT
CCTAATAATGAATATACCGTTACTGCTATCGGGAATGGCGCATTCAAATCCAAGAGAT
TAACTAATGTAACGATTCCTAATACAGTTATAACTATTGGCGATGGTGCTTTTACCATC
AATTCACTTGAACAACTAGTCTTACCCAACTCAGTGCAGACTATAGGGAGGAACTCTT
TTAGTGTCAATAAATTAGAAAAAATAACTTATTCTACTGCTTTAAAAAATATTCCTTCC
CAAGCATTTTTAGCTAATAATTTAAAAACAGTGACTACACCAGCCACTGTTGAAAGTA
TTGATGCTTCTGCCTTTGAAAATAATTTTATTACTAATATTACAATTCAAAACCCAAAT
CTTCAAATGGCTTATCAAGCTTTTGCCGCTCAAACAGTTTTAAGTACACTCATTGTACC
AAGCAACCACATCCTACCTATAGAAAATTACATTCAGTTTCAAGATGCTTCGGCTCAT
TTGACTACAGATAATTTATTCATAACTGATTTAGCGAATGGTATTACGTATAATCAAG
CCGAAAAAGCATTAAACTTTTCAGCAGAGCCACTCGAATCTACTTTTTCACTTTTCACT
GGGACAAATCGATTTGATTCTTACTATGATATTTCTGAATATGGTCCTTCTGGAAAGC
CATTTATCTACTTCAAGTATACTAAACCAGTCCTAGTTTCCTATAAAGATGCGTCTGGG
AATGAATTAGCTACTTCTACTAGATTAGATGGTAGTATTGGTGAAAATTACGTCACTA
CTCCTAAAATAATAGATGGCTATACTTTAAAAGAAACACCCGGAAATGCGACAGGAC
AATTTTCTGAAACACTGCAAAATGTAACGTATATTTATGAAAAAACTGCTGTCCAAAA
TGGGACAGTCACTGTAAAATATCAAGATGAATCGGGTAAAACACTAGCAAAAGATAC
TGTTTTAACCGGTGAAGTAAATAATACCTACCAAACTAAAAGCAAAGACATAGCAGG
CTATAAACTTCAAAAAGTGGAAGGAAATGAATCGGGTACTTTTAGCACAACCCCTGC
AACTGTTACCTATATTTATGAAAAAATAGCAAACAGTGATAATACAAATACCAATGG
 
AGAAATGACCGACAATACTACACTGAGTACTAATGATACTGTGATTTCATCCGAAGCA
ACAAAAAAAGTTGATAAAAACACCTCTAATATCCTCCCAACTACTGGTGATTCAAAA
GATGCTCTCTTTTTTGCACTAGGAAGTTTATTGACGTTGTTATCTACTAGCTTCTTTTTC
TTTAAAAGAAGTTAA
SEQ ID NO: 13 
ATGGCAACGCAAAAGAATTTAGTTGATTTAGCAACAAAAGCTTATATTTACGGTTTTC
CTTTCATTTTTAATACACAACAAATCGAGCGTTATGTAACGGTAGGGATTGGTGGAAC
AAAGCAAGTTCCTTTCAATAATTTACTCATGCATCTCGCTTGGCAGAACCGTCTGAT
AAATTTGTCAGTGTGAACAACGATACTATTTATTCTAATGCACCAATTGATGTTAGTG
CCGGACCGGTCGTTTTAAGTGTACCCGATACAAGTGGTCGTTATTATGTACTTCAGTTT
GTTGATGCATGGTCAAACAATTTTGCTTATGTTGGAAAACGTGCAACGGGGACTTCAG
CTGGCAAATTTCTTTTAACTCCTCCAAATTGGAACGGTGATGTTCCTGCTGATATGATA
GAAATTAAATTTCCTACTAATATAGGGATTATTATTGGCCGACTTGCTTGTGACGGTG
AAGCTGATTTACCAACTGTAAGAGAATTACAAGAACAACTTAAGATAACTCCTTTAAA
TGAAGGGAAAGAAGTGGATGGTTTTCCTGAATATGATCGCTCACTAGGAAAAGAGCT
AGCTTTCTTTGAACAATTACGAGTTTATATGGCTCAATTTCCACCAGCAGAAAGAGAT
TTGGTTAAGCAAGAAAGTTTTGCGCCAATTGGTTTGATGGAAAAAGGGGTATCTCCTT
ATAGTAACCCGAGTGAAGAATTAAAAAATGCCCTTATAGAGGGAGCAAAAGCAGGTT
TAGCGAATATCAAAAAAGCAACAACCAATTTTAAATCAGAAAATGGCTGGGGATTAA
CACAACATTTATTTGATTATAATGCTGATTTTTTGAAATAGGAACGAAAAAATCTTT
AGACTGGGTAATTGAGGACCGTGAAGAAGCATACATAATTCGTGCAGTTTCAGCTATT
ACGGCATTATGGGGAAACCATGGCTATGAAGCGGTTTATTTGATGACATGGACAGAC
ACAGACGGTAATGCCTTAAATGGAAAAAATAAATATACATTGGAGCTAAATCCAATT
CCGCCAGTTGACTCATTTTGGTCGATTACGATGTATGACTTGCCTGAATACTTTTTATG
TGAGAACCCGATTAATCGTTACTCGATTGGAGACCGTACGCCAGGTATACAATATAAT
GATGATGGCGGTATCACTATAGTGGTTTCGAAAGATGAGCCAACTGACCCGAAAGAA
CGTGCGAATTGGTTGCCAGCTCCTGATGCTGAGTTCAGACCAATGTTCCGCTTATATA
ATCCGCAACAACCACTCGTGGATGGAAGCTATCATTTTCCGGGATTTGAAAAAAAGTA
A
SEQ ID NO: 14 
ATGAGCAACAGTAAATTTATTATTACAGAATTATACTGGGAAAGAATTCAACTACATG
TAAAGGGTAAAATAGAAAATATTTCGTTAGAGAACTATACGTTTTCATTTAGGACTTT
AACAGATGAATTTCATTTTTCACCAACGGGAATCAAATTGGAAGGAAACACATTTGAT
CTTCGTTTCAATATTGCTATTTTAAATGATGGGAATTATTTACCAAGTGGGGATTATCT
CTTATCCTTATATAACAGTGAAACAGCGGATGAAATTGTTGCCCAACCGGCTGCTGAA
 
CTATATAAATTTCCAGAAGATGAAGATCTTTTAGAAGAACTAAACGAAGCTAAAACC
GAAAACGAAAAAAACAATGTGCTTTTAGCAGGTTTAAGAAAAGAATTTAAACGCGGT
GGGGCGAATCTCAAGTATACGTATAAAGTCACCCCAATGATTTCTGCAGATGTAAATG
AATTTGTTTTCTCTGTTTCATTTACAATCCCTGTACCAAAACCAACAAAGTGGCAAGTA
TTCAAAAATAAAATTAAAGAAATGTATCATACTTTCAGTTTTAATTTTAGAACTGGTTT
GTTTAAATCTATTTTCTATACCTCACAAGCCCTAGTACCTAAAAACGGAAAACGAATT
CTTTTTTCTTCAGATTCCCGTGCAGAAATTGGTGGTAATTTTGAGTTCGTTCTAAATAA
AATGAAAGAGATGGGTATTGATAAAGATTATAAAATCAAAATGACCTTTAAACCCAA
CATTCGTTTGAGAAGAGCTTTTATTGAGAAATTAGATTTCCATTTTTGCTAGGGAGTT
CAGATATTATTTTTATTGATGATTATCATCCGATGATTTATACAATTGACTTCAGCGAA
AAAACACAAGTAATTCAATTATGGCATGCATGTGGGGCTTTTAAAACAGTTGGATTCA
GTCGTTCAGGTAAGCAAGGCGGGCCGTTCTTTGATGATAGAGCACATCGTAATTATAC
ACGTGCTATTGTTGCTTCAGATACAGACATACCATTTTATGCAGAAGCTTTTGGGATA
AAAGAATCTTCTATTTTACCAACTGGTGTACCAAGAACAGATATTTTCTTTGATCCAG
AGTACAAAAGAAATATTGTTACTACGATGGAGCAGTTGTTCCCAGAAACTCAAGGCA
AACAAGTTATTTTATTTGCTCCAACTTTCCGTGGAAATGGTGCGAATCAAGCTCATTA
CCCATATTTTAAAATTAATCTGGCTAAGTTTGCTGAATACTGTCGAAAAAATAATTCG
GTCGTAATCTTTAAAATGCATCCGTTTATTAAAAATGAATTTATTATTCCAGAACAAT
ATGCAGATGTTTTCATTGATGCTAGTTCTTATCGTGAAGTAAATGATATTCTATTCATT
ACGGATATATTGATTACGGATTATTCATCTGTTATTTTCGAATTCTCTACCTTACAACG
CAAAATGCTGTTCTATGCATTTGACTTGGAAGATTATGTTTCTACGCGTGACTTTTATG
AAGAATATGAAGGTTTTGTGCCAGGGAAAATTGTATATGATTTCGAAGCATTAATTAA
GGCGCTTGAAATGAATGATTTTGAACAAGAAAAGGTAAAACCATTTTTAGATAAGCA
CTTTAAATATCAAGATACTAATTCTGCAAAACGAATTGTGGAAGAAATCTTTAAAAAA
TAA
SEQ ID NO: 15

ATGAAAAAGCTTAGAATATTTACTTTGATGCTTGCTTTTGCTCTATTTTTATTAGGTGG
AGGAATTGTTGCACAAGCAGCAGAGGAAGCCCCAATCGATGAAAAGATAGTTGGGGA
AACGGTAACAAATGACGGAGAAGAGTTTATAGTCGATGAGATTAGTGATTTAGAAGA
TGTTAATTCAACAACGGGGACGTTAGATACATCTGATGAAGTTGAGGTTGATTTATCA
GGATTAACTATTGAAAATGAGGAAGCCGTTCTAACCCCAGGTTTAAAAACGATTTTTG
GCGGAGATGGTAGAAAACTTGTAACAAATACAACTCAATATCCATATTCAACGAGCG
CTTACCTTGTAATGGAATTTCCAAATGGAAAAACTTATATAGGTAGTGGACAATTGAT
TGGTGAAGATTCTGTACTTACAGCCGCTCATTGTTTATATGGTAAAAAAGATGGTGGA
TGGGCAAAAAAAGTGACTGTATATCCTGGATATAATGGCACGAAAGCTCCTTTTGGA
ACAGCAAAAGCAAGAAAAATGTATGTTCCAAAAGAATGGACAAAAAAAGAACCTTCT

ACAGAAGATTATGGTGTTATTAAATTAGATAAAAATATTGGGACAAAAACTGGAACA
ATGGGGTTAACAACTAATACATCTGGTGCAATTACTATTAGTGGTTATCATGGTGACA
AAAAAGGAAATTGTACACTCAAACTGGAAATATCTCTCAAGTCACTGCAAATAATG
TTTTTATAGATTAGATACAACAGGTGGTAGTAGTGGTAGTGGTGTTTATAATTCTAA
AAAACAGATTTTAGCAGTAAACGCATATGAATATTTAAATGGTACCGGGACAACTTT
GGTACAAGAATAACAAAAGAAAAACTAAATAATATTTATACTTGGGCGTTTGACAAT
AATCTTTCTGTAAGCAAACAAAAAGGATAAATTACGAGCTCCACGTCCAAAGTAAG
GGATGGATGGGAAACGTTGCTAATAGTATGACTTCGGGCACAGTTGGCCTTGGCTTAC
GAGCAGAAGCAATGAAAATTAGTTTATCAGGGATGCCGTATTCTGGAGATATTCAAT
ATCGCTCACATGTACAAGGTAGTGGATGGCAAGGTTGGTTGAAAAATGGGGAAATTT
CTGGAACTCGTGGACAGTCAAAACGTCTGGAAGCTTTCCAAGTAAAATTAACTGGAA
ATATGGCTAAAAATTATAGTGTGCAGTATAGAGCGCATGTCCAAAATAAAGGTTGGC
AAAGTTGGGTTAAAGATGGAGCAACAGCAGGAACAACAGGAGAAAGTTTAAGAATA
GAAGCTGTACAAATGCGACTAGTTGCAAAATAA

The sequences of SEQ ID NOs: 61-181 are shown in the sequence listing, which is filed together with the application (ST25.txt file), and which is herein incorporated by reference

### Example 3: Multiplex PCR method to identify major clones of L. monocytogenes,

Multiplex primers were then carefully designed (*cf.* Tables 6, 7 and 8 below) to produce amplification products with different sizes and to amplify DNA under a unique temperature.

Target genes were grouped into three 'clonogrouping' multiplex PCR assays. These clonogrouping multiplex PCR assays can be implemented on their own, or can be used downstream of the widely used PCR serogrouping assay. First, the IVb clonogrouping PCR assay was designed to identify IVb clones CC1, CC2, CC4 and CC6, or any other IVb isolates. Second, clonogrouping PCR IIb was designed to identify IIb clones CC3 and CC5, or any other serogroup IIb isolates. Third, clonogrouping PCR IIaIIc was designed to identify either serogroup IIa clones CC7, CC8, CC121 and CC155, or clone CC9, most isolates of which are of serogroup IIc.

Expected amplification results are given in Table 9 below.

**Table 6:**

| Target and primers for selected CLONE-SPECIFIC GENES | | | | | |
|---|---|---|---|---|---|
| | | SEQ ID NO: | | | |
| SEQ ID NO: of the genes | Clone specificity | Selected target | Forward primer | Reverse primer | Multiplex grouping |
| 1 | CC1 | 16 | 17 | 18 | Multiplex PCR **IVb** of the application |
| 2 | CC2 | 19 | 20 | 21 | |
| 3 | CC4 | 22 | 23 | 24 | |
| 4 | CC6 | 25 | 26 | 27 | |
| 5 | CC3 | 28 | 29 | 30 | Multiplex PCR **IIb** of the application |
| 6 | CC5 | 31 | 32 | 33 | |
| 7 | CC121 | 34 | 35 | 36 | Multiplex PCR **IIaIIc** of the application |
| 8 | CC155 | 37 | 38 | 39 | |
| 9 | CC7 | 40 | 41 | 42 | |
| 10 | CC8 and/or (and) CC16 | 43 | 44 | 45 | |
| 11 | CC9 | 46 | 47 | 48 | |

The primer pair of SEQ ID NO: 47 and SEQ ID NO: 48 has been disclosed in Doumith *et al.* 2004.

**Table 7:**

| Targets and primers for selected CONTROL GENES | | | | | |
|---|---|---|---|---|---|
| SEQ ID NO: of control genes | Lineage/subgroup specificity | SEQ ID NO: | | | |
| | | Selected target | Forward primer | Reverse primer | Suitable in multiplex with |
| 12 | Lineage I | 49 | 50 | 51 | Multiplex PCR **IVb** and **IIb** of the application |
| 13 | Lineage II | 52 | 53 | 54 | Multiplex PCR **IIaIIc** of the application |
| 14 | Serogroup IIb and lineage II | 55 | 56 | 57 | Multiplex PCR **IIb** of the application |
| 15 | Serogroup IVb (lineage I) | 58 | 59 | 60 | Multiplex PCR **IVb** of the application |

Sequences SEQ ID NOs: 16-60 are as follows (in the 5'-3' orientation):
SEQ ID NO: 16 
TCAGCAACTCAAACGGAAGAATCGATGGAGTACACTATAGTATAGCTGATATACCAA
TAGAAAAACTTTTGAATTGAGAAGTGAGGGTATTTACACTTTTCCTGAACTACCTCT
TTACTATCTAACCACAAAAAATGATTATATTGAAAATATTTGGATCGATCACCAATT
AATTTAGAGGATGATAAAAGCTTTATCCCTAACGAAAGAAATAACAAGCTTATCTGC
ATGGAAAAGCATATTGATTTATCATTTTTAAGTACGCTACCATTATCAACTTTTGAGTA
TTCTACAAAACGTGAAGAATTTTTAAATATGGTTAATACATTTTATGATACTTATTACG
AAGATGATTATCTTATGACGCCGACATTTATGATTAATAAAGTCGGTTCGTGGGAAG
SEQ ID NO: 17
   TCAGCAACTCAAACGGAAGA
SEQ ID NO: 18
   CTTCCCACGAACCGACTTTA
SEQ ID NO: 19 
GCATTTTCCAAAAGCAGAGCTTGAGCGTTTATTGGAAGGAAATCTTTAAAGTTTTTGA
GAAATTAAAAGAATCAGAACCTATCGGACAAGATGATATAAATAAAATGAGAAGAA
ATCTTTCCCAAGAGCAAGAAATTATTGAAACAGCTTTCTCTAGTGATGCTGATTTAAT
GGGTTATTGGTTAATTGAACAACTTCAAAAGGAAACTTCAAATCGTAGTAGTATTTTT
TATGATGATGAGATACTTTCAATTAATTTGAAATCTGAAATGATTCCATTAATTGTTAA
ACTAATGACGGATGAAACTAGATATATTTATTGGTCTTTTAAAGAAATTATCTCTGAT
GAGCTGATAAGAATTATATATGTATCTCTTCTGAATGACAAAATGTTGTTGGATAATA
AGTCAATATTAGATAATTGTCCTGACTTATTACTAGAATCTCATCCTTTCCAAAGAATT
TTAGTAAGTATTGATAATGAGGTTGGATTGAATGATTCTATTAAAAATAAGTTGATAA
CTAAAATTGATTTTTCCAATACTACCTTTGGAAGCGAGCTAAA
SEQ ID NO: 20
   GCATTTTCCAAAAGCAGAGC
SEQ ID NO: 21
   TTTAGCTCGCTTCCAAAGGT
SEQ ID NO: 22 
GGGGGTAAACCACAAGTTTTATCCGCATTTAGAGACTGTAAAGAACATGTTTCTAGCA
ATAATAATATAAAAAACATATATCTTTTAGATGGTGATTTTGATCAATATTTGGATAA
TATAGTAATGGAATCACATCCACATATTATTTATTTGCAATCATATAATATTGAAAATT
ATTTTTTGAATGAAGAATCGGTAGTAACTTTTATGCAAGGAAAAATGAAAATGCTGAA
GAATGATGTAAGGACTAAAATAGCTTATTCTGAATGGCTTTCTAATACATTATACAAT
ATAGAAAACATTTTTATTTTATATTGTATAATTCAAGAAAAGGAATTAGGGATTCCTA
 
ATGTAGGGGATTCTGAATATAAAGTAATTAATATGATCGATGGATCTATCAATATGAC
GAGATATGAGGATTTGAAAAAGCAAATAAGTAATTGTACAGTAAACATAGATTTAGT
AGAGCAAGAAATGAAAGCAAAAATTCACAATATAAATCCTAATGTGTTTGATTTGATT
TGTGGTAAACATTTGATTTGTAGTTTGTACCGTCATCTGTTAAAAATAACTAAAGGTTT
CAGCTATGATGAATTTAGATGGCATTTAGTTCAAAATGTTG
SEQ ID NO: 23
   GGGGGTAAACCACAAGTTTT
SEQ ID NO: 24
   CAACATTTTGAACTAAATGCCATC
SEQ ID NO: 25 
TGAGGCTATCGAAATTGCTCGCTATTTTGATACAATAGGTGACAGTCAATTATCGGAA
TACATCATGAGTTTGTTATCTACAGCAAATACCTTTGTGCCGCAAGGTGAGGCTTTTG
AGAACTTATTTTCAAAAAAATAGAACCTAATGTAGAACCATTACCACTCCCTAAAGC
AATTAGTGAAGATATTCGAGGTATTATAAATGCTGTAAATCATCATGTAGGAATAAAT
AAATTTCTCTTCGAGGGTGCACCTGGAACTGGAAAGACAGAAACGGTTAAACAGATT
GCTCGATTATTAGAACGCCAACTATATATGGTAGAATTTAGCGAATTAATTGATAGTA
AAATGGGGCAAACAGCTAAGAACATTGTAGCAGTGTTCAACGAAATAAATCGTGTAT
CAAATCCATCAAAATTATTAATACTTTTCGATGAAATAGATGCTATAGCCCTAGATAG
AGTAAACTCTAATGACTTAAGAGAAATGGGACGGGTAACATCTACAATCTTGAAAGA
GTTGGATAAGTTAAATGAAGAAGTTGTTCTTATTGCCACTACTAATTTGTATGAAAAA
TTTGATAGAGCCTTAATAAGAAGATTTGATTCAGTAATTAATTTTAATAGATATGAAA
AGGAAGATTTAGTAGAAATTTCAGAAATCATATTGAATTCATTATTGAAAAAATTTAA
AGATGCTGGACGAGATATGAAGTTATTTAAAAAAGTAATCAGAAACATGGATGAAAT
TCCGAATCCTGGAG
SEQ ID NO: 26
   TGAGGCTATCGAAATTGCTC
SEQ ID NO: 27
   CTCCAGGATTCGGAATTTCA
SEQ ID NO: 28 
TCGATTTGCGAATGAAAGAAAATATTGATGATCTTAGCATTGAAGAACTTGCTGATTT
AGAAATTGATTTAGATGTTTTGTTTGATAATGTCAGAGGGTATCTAGGAAACACAAAT
TATAATAAAAAAATTCTTGAAACTTTGAAAAAAGAACCTGGAAAATTCTTTTTATTTA
ACAATGGTATAACAATTACTGCAGATGATATTAATGTGAAAGAAGTTAGACTGGGTA
AGGCTAGTCGAATTAAGCTAGATCGGTTTCAAGTTGTCAATGGGGGACAATCCTTACG
AACTATATATAAATACAAAGATCAAAAAGACTCACCAATTGAAAATTTAGCTAAGGC
 
AAGTGTATTAGTGAGATTTTTAAAACAGGGCAAGAAGAGGGATTAATTAATAAGGT
ATCAGAATATACCAATAGTCAGAATGCTATCTCAGCTATTGATTTAAGGACAGTTGAT
CAATTACAATTAAAAATTGAGAAGCACTTAGAACTCAAGGGAATTCAATATATAAGA
AAGAGAAGTTCTGATAAAACTAAGTTGCAACTAGAAAATTCTATTACAATGGAAAAA
CTAGGCCAATTGTTATTTGCATACGCGGGTT
SEQ ID NO: 29
   TCGATTTGCGAATGAAAGAA
SEQ ID NO: 30
   AACCCGCGTATGCAAATAAC
SEQ ID NO: 31 
TGCCACCTTGGTGTTAATGAAATAGATGAAGATAATATGGAAGACTTTGTTAAGACTT
ATAAACAATTAAATAAAGAAAGGCGAATAAAACAATGCTTATATCCAGAAAAATCTA
AATGCAGTGATAAAATAATTAGAGCGCATTCTATACAAAATAGTAAAATAATTCAAC
GAATATCTGAGAATGGGAATGTTTATATGAACAAAGCAAAACCAAATAATCCATTTG
CTGTATTTACTAAGTGGGGGCGTAAAGAAGCAACGACATTTACTGGATTTTGTGATTA
TCATGATAAAGTTGTATTCCAGCCAATTGAAGATAGAGACTTTGAGTTAAGTAATGAA
CAGATCTTTTTGCATACTTATAGATGTTTGGTAGCTAACATGCATGATAAGCAAGAAC
AAGTTAAGTTAGGGAAATCAACTACTGAAACTTTTTCATTATCCAGTGAATCTAGTGT
TGGATTATTGGGCTTAGGAAATGGCT
SEQ ID NO: 32
   TGCCACCTTGGTGTTAATGA
SEQ ID NO: 33
   AGCCATTTCCTAAGCCCAAT
SEQ ID NO: 34 
TTTGAAACGGTCCTTCAACCTAAGCCTGTTGTTAAAGGAGTAAGAACTAGTATTGAAA
TATTAATTCATCTAGATGCGCAAAATATTGCTATTATAGATGCAAAAAACTACAAAAA
GAAATTCACTCTAACAGCAAACTTGGCCAATTATATGGCTACTGAATATATCCCCATA
TATCAAGGCTATGATGGAAAAAATGTTACACATTTTGGATATATTACAGCTTCTAAAA
TTGGCGGTAGTAGCAATCTAAAAAAAATAGTACATACAGCTAGAACATATAATGATA
AATTCCGAGTTAATGGTTTTTAATTTCAGCGCCTGCT
SEQ ID NO: 35
   TTTGAAACGGTCCTTCAACC
SEQ ID NO: 36
   AGCAGGCGCTGAAATTAAAA
SEQ ID NO: 37 
TGCGTTAACATTTGGGATGATACCATCAGGAGTTGCTAATGCACAAGAGCCTAACGA
AGAGTCTGATTTTAAAATCACAGACCATGTTGTAACTGACAATAAAGAGTCTTTTACA
ATTGCTATGAACGGTGAGGAAGTGGATTATAAGTCGACTAAAACGGAAGATTCATTC
GAAATTGAAACTGTAGAAGAAAATAATGAAGTACATACCTTTACATATACTAAAGGT
GACGATTACATGATGATAGACGGCGAAAAAGTATCTGTTGAAATTGATACAGAAGTA
GAAGCAGAATTAACAGATTCTTCTGCACCAAAACTATTAAAAGCTTCGGCATGGACA
CCTAAGTATATGTCGACAGGATCTATAAAGATAAGTAAGTCAGTAAAAACATTAGGA
ACTCTGGTTGGAGTAATTGGGGGAGCTATTTCTATTGCTGCACTATCAGGGGTGGCAA
TT
SEQ ID NO: 38
   TGCGTTAACATTTGGGATGA
SEQ ID NO: 39
   AATTGCCACCCCTGATAGTG
SEQ ID NO: 40 
AATAATCACTGGCCCATTCGGAACCCAACTTCACAAGTCAGATTATGTTGACTCTGGA
GTTGCAGTTATAATGCCTCAAAATATAGGTTCTAGACAAGTTAATTATGAGAAGATTT
CTTACATTTCTGAGGAGTTTGCAACGACTCTAAGGCGATACAAAGTATTAAAAAACGA
TATTGTTTTTGCTCGTCGAGGTGATGTTGAAAAACATGCATTTATCACAGAATCTGAG
GAAGGTGAACTCTGCGGAACTGGATGTTTTCTCGTACGATTCACAAGCGAACATGTTT
TACCAGAATTTATTTCTTTGATTTTAAGTACACCTTTCGTGAAAAAATGGCTAGTCTTA
AATGCTGTCGGTTCAAATATGCCAAATTTGAATACTGAAATATTAAAAAATGTTCCTA
TTAAATTTCCAGATTTATCTACTCAACAAAAGATTTTATCAACTATTTCATCGGTTGAA
TATAAAATTAGAATCAATACAAAGATAAATACTAATTTATTAGACATGGCTAAGGCA
ATTTACATGCATTCTTTTTTTGGAAAACATGAGAATGCAAAGATTTCAGATATACTTCT
GGAAAACAGTAAGTCCAACATCCAAGTTGGTGAAGCAAGAGAGGC
SEQ ID NO: 41
   AATAATCACTGGCCCATTCG
SEQ ID NO: 42
   GCCTCTCTTGCTTCACCAAC
SEQ ID NO: 43 
AAATTCAAACGGCAACCAAGTTTAAATATGAAATTCATTACTTTATCTCCTAAAGAAA
TAGATTTGACTGATATCCATATGAAATCAGAAATAGATTTGGGATTGAATGATTTAAA
TGACATGAGCCTTTTCAATGAAGTGAAAGTTCCTGACTTTTTAGGAATCAAAGATATT
ATGTCATTACATGAGAAAAACACTTCCGGCTTAGAAGTTTCTGTGACTTTAACAAAAC
TACCCGTACCATATCCTAAGGATACTCATAATAAAATAAAAAATGGGTATTATGGATT
 
AATTAAACTTGAAGAATTTATACTTTTACTAACAGATGAAGTTAATAATGAAAAGATT
TTACGAAAAGGTATCTTCAATGATAACATTCGTTACTATCTTGGCTCATCAGAAAAAA
TAGAGGTCAATACTTCAATGAAAAATCAGCTGTTAGGTACAGAAGATTATTTGTTTGG
ATTATTAAATAATGGTATTACTATAATTGCAGATGAGATTTCATTAAGTTCTGATGACT
TAACACTTACTAATTACCAAATAGTAAATGGGTGCCAAACAAGTAATGTTATATTTGA
ATGTTTAGAGTCTATTGAGCAATCTAGTGACATATATATTCCTATTAGGTTGATTTCAA
CAGAGGACGAAGACACAAAGCACTCCATTATAAAAGCTACTAATAGTCAAACACAAT
TAAAGCCAGAACAGTTGGTTGCTTTATCAGCA
SEQ ID NO: 44
   AAATTCAAACGGCAACCAAG
SEQ ID NO: 45
   TGCTGATAAAGCAACCAACTG
SEQ ID NO: 46 
AGGGGTCTTAAATCCTGGAATACTAAGGAACAAGAAATCTTTAAACTTCGAACTAATC
CAAAAAACAGTACATCTATTGCAAAGATTATTAGTGACAAATATGAAGAATTTTTAA
AGAAGATATTCAAGAAGAAATGGCATATACTAATATCCAAAGATTATTTAATAATTTG
GAAGTGAGAGAGTCACTAGGCATTGAAAAAGACAATATAGATTCTTTTTCATGTGAA
AGATTATATTTAATTAAAGGTGTTATTGAAAAAGTAAACCAGATTGCTAAAATTGAAA
ATTTATCTATTTCTCGTAAATTTAATAAAAGGAAAATAATTGAACAAATAGTAATACC
CATTATAGAAGAATTAAAATCTAAACAGTTGGAAAGTAAAGCAATAAATTCTATACA
TATTGAACAAAAAAATAAAAGTAATAAATTATCAAATTCTAAAGAACAACAAACACA
AAATAAAGAAGCAACTTCAAACAATGGAATTAGTGTACGAGAAGATACTGGTTTGAA
AACTGAATATAAAAGCATAGAATCTATATCATCCACTCTAAAACCAAAATTACCCCCA
AAAATAGGGAGCGGAAAACTTCTAAGTAGTACTATGCCCTTTACAAATTTATATCATT
CTAATGTGCGTATAAATACTCTAGTAATTGAGATTAATCAGATTAGATATAAGGATTT
TCGTTTAGCTACACAATTCTTATTAAGGTCTCTTATGGAAGTATATGGACATGAATAT
ATTGATTACTTTGCAAATTTAGACTATCAAGATGTCCAGAAAATGAAGGCTATAGCAA
AAGACCGTATAAAACGAAATCAAACAGTAAATGAAATTTATTCAGAGTTTATTAGTA
ACCACATAACTAAACATTTTCCTAAGTATGCCGAACAAGCCG
SEQ ID NO: 47
   AGGGGTCTTAAATCCTGGAA
SEQ ID NO: 48
   CGGCTTGTTCGGCATACTTA
SEQ ID NO: 49 
GGTCCTTCTGGAAAGCCATTTATCTACTTCAAGTATACTAAACCAGTCCTAGTTTCCTA
TAAAGATGCGTCTGGGAATGAATTAGCTACTTCTACTAGATTAGATGGTAGTATTGGT
GAAAATTACGTCACTACTCCTAAAATAATAGATGGCTATACTTTAAAAGAAACACCCG
GAAATGCGACAGGACAATT
SEQ ID NO: 50
   GGTCCTTCTGGAAAGCCATT
SEQ ID NO: 51
   AATTGTCCTGTCGCATTTCC
SEQ ID NO: 52 
CAGAAAATGGCTGGGGATTAACACAACATTTATTTGATTATAATGCTGATTTTTTTGA
AATAGGAACGAAAAAATCTTTAGACTGGGTAATTGAGGACCGTGAAGAAGCATACAT
AATTCGTGCAGTTTCAGCTATTACGGCATTATGGGGAAACCATGGCTA
SEQ ID NO: 53
   CAGAAAATGGCTGGGGATTA
SEQ ID NO: 54
   TAGCCATGGTTTCCCCATAA
SEQ ID NO: 55 
CGAAGCTAAAACCGAAAACGAAAAAAACAATGTGCTTTTAGCAGGTTTAAGAAAAGA
ATTTAAACGCGGTGGGGCGAATCTCAAGTATACGTATAAAGTCACCCCAATGATTTCT
GCAGATGTAAATGAATTTGTTTTCTCTGTTTCATTTACAATCCCTGTACCAAAACCAAC
AAAGTGGCAAGTATTCAAAAATAAAATTAAAGAAATGTATCATACTTTCAGTTTTAAT
TTTAGAACTGGTTTGTTTAAATCTATTTTCTATACCTCACAAGCCCTAGTACCTAAAAA
CGGAAAACGAATTCTTTTTCTTCAGATTCCCGTGCAGAAATT
SEQ ID NO: 56
   CGAAGCTAAAACCGAAAACG
SEQ ID NO: 57
   AATTTCTGCACGGGAATCTG
SEQ ID NO: 58 
TATGACTTCGGGCACAGTTGGCCTTGGCTTACGAGCAGAAGCAATGAAAATTAGTTTA
TCAGGGATGCCGTATTCTGGAGATATTCAATATCGCTCACATGTACAAGGTAGTGGAT
GGCAAGGTTGGTTGAAAAATGGGGAAATTTCTGGAACTCGTGGACAGTCAAAACGTC
TGGAAGCTTTCCAAGTAAAATTAACTGGAAATATGGCTAAAAATTATAGTGTGCAGTA
TAGAGCGCATGTCCAAAATAAAGGTTGGCAAAGTTGGGTTAAAGATGGAGCAACAGC
AGGAACAACAGGA
SEQ ID NO: 59
   TATGACTTCGGGCACAGTTG
SEQ ID NO: 60
   TCCTGTTGTTCCTGCTGTTG

**Table 8. Primers used in this study.**

| Multiplex PCR | Gene | Putative function | Genome | Primer name^{a} | Primer sequence (5' - 3') | PCR product size |
|---|---|---|---|---|---|---|
| PCR IVb | *LMOf2365_2638* | Putative peptidoglycan bound protein (LPXTG motif) | F2365 | *LMOf2365_*2638-F | GGT CCT TCT GGA AAG CCA TT | 194 |
| | | | | *LMOf2365_*2638-R | AAT TGT CCT GTC GCA TTT CC | |
| | *LMOf2365_1900* | Putative serine protease | F2365 | *LMOf2365_1900*-F | TAT GAC TTC GGG CAC AGT TG | 301 |
| | | | | *LMOf2365_1900*-R | TCC TGT TGT TCC TGC TGT TG | |
| | *LMOf2365-2702* | hypothetical protein | F2365 | *LMOf2365-2702*-F | TCA GCA ACT CAA ACG GAA GA | 405 |
| | | | | *LMOf2365_2702*-R | CTT CCC ACG AAC CGA CTT TA | |
| | *LMOATCC19117_2417* | hypothetical protein | *ATCC19117* | *LMOATCC19117_2417*-F | GCA TTT TCC AAA AGC AGA GC | 565 |
| | | | | *LMOATCC19117_2417-*R | TTT AGC TCG CTT CCA AAG GT | |
| | *LMOL312_1102* | hypothetical protein | *L312* | *LMOL312_1102*-F | GGG GGT AAA CCA CAA GTT TT | 622 |
| | | | | *LMOL312_1102*-R | CAA CAT TTT GAA CTA AAT GCC ATC | |
| | *LM1816_RS00050* | AAA ATPase family protein | *H7858* | *LM1816_RS00050*-F | TGA GGC TAT CGA AAT TGC TC | 764 |
| | | | | *LM1816 R500050-*R | CTC CAG GAT TCG GAA TTT CA | |
| PCR IIb | *LMOf2365_2638* | Putative peptidoglycan bound protein (PXTG motif) | F2365 | *LMOf2365_2638*-F | GGT CCT TCT GGA AAG CCA TT | 194 |
| | | | | *LMOf2365_2638-*R | AAT TGT CCT GTC GCA TTT CC | |
| | *lmo 1077* | Glycosyl transferase | *EGDe* | *lmo1077*-F | CGA AGC TAA AAC CGA AAA CG | 334 |
| | | | | *lmo1077*-R | AAT TTC TGC ACG GGA ATC TG | |
| | *M637_08170* | conserved protein of unknown function | *FSLJ2-064* | *M637_08170*-F | TGC CAC CTT GGT GTT AAT GA | 487 |
| | | | | *M637_08170-*R | AGC CAT TTC CTA AGC CCA AT | |
| | *LMOSLCC2482_0307* | hypothetical protein | *SLCC2482* | *LMOSLCC2482_0307*-F | TCG ATT TGC GAA TGA AAG AA | 606 |
| | | | | *LMOSLCC2482_0307*-R | AAC CCG CGT ATG CAA ATA AC | |
| PCR IIaIIc | *lmo0525* | hypothetical protein | *EGDe* | *lmo0525-*F | CAG AAA ATG GCT GGG GAT TA | 163 |
| | | | | *lmo0525-*R | TAG CCA TGG TTT CCC CAT AA | |
| | *LM6179*_*0610* | Predicted protein | *6179* | *LM6179_0610-* -F | TTT GAA ACG GTC CTT CAA CC | 327 |
| | | | | *LM6179_0610-* -R | AGC AGG CGC TGA AAT TAA AA | |
| | *LMLG_RS02400* | hypothetical protein | *Finland 1998* | *LMLG_R502400-*F | TGC GTT AAC ATT TGG GAT GA | 460 |
| | | | | *LMLG_R502400-*R | AAT TGC CAC CCC TGA TAG TG | |
| | *LMRG*_*02577* | hypothetical protein | *10403S* | *LMRG*_*02577-*F | AAT AAT CAC TGG CCC ATT CG | 627 |
| | | | | *LMRG_02577*-R | GCC TCT CTT GCT TCA CCA AC | |
| | *LM5578*_*1185* | hypothetical protein | *08-5578* | *LM5578_1185*-F | AAA TTC AAA CGG CAA CCA AG | 729 |
| | | | | *LM5578_1185*-R | TGC TGA TAA AGC AAC CAA CTG | |
| | *lmo1118 #* | hypothetical protein | *EGDe* | *lmo1118*-F | AGG GGT CTT AAA TCC TGG AA | 907 |
| | | | | *lmo1118*-R | CGG CTT GTT CGG CAT ACT TA | |

| | | | | | | |
|---|---|---|---|---|---|---|
| a :F, forward; R, reverse # : Doumith *et al.,* 2004 | | | | | | |

**Table 9. Expected amplification profiles.**

| | | Lineage I | | | | | | | | Lineage II | | | | | |
|---|---|---|---|---|---|---|---|---|---|---|---|---|---|---|---|
| | | IVb | | | | | IIb | | | IIa | | | | IIc^{#} | |
| Multiplex PCR assay | Gene target | CC1 | CC2 | CC4 | CC6 | Other | CC5 | CC3 | Other | CC121 | CC155 | CC7 | CC8 | CC9 | Other |
| PCR IVb | *LMOf2365_2638* | 1 | 1 | 1 | 1 | 1 | 1 | 1 | 1 | | | | | | |
| | *LMOf2365_1900* | 1 | 1 | 1 | 1 | 1 | | | | | | | | | |
| | *LMOf2365_ 2702* | 1 | | | | | | | | | | | | | |
| | *LMOATCC19117_2417* | | 1 | | | | | | | | | | | | |
| | *LMOL312_1102* | | | 1 | | | | | | | | | | | |
| | *LM1816_RS00050* | | | | 1 | | | | | | | | | | |
| | | | | | | | | | | | | | | | |
| PCR IIb | *LMOf2365_2638* | 1 | 1 | 1 | 1 | 1 | 1 | 1 | 1 | | | | | | |
| | *Imo1077* | | | | | | 1 | 1 | 1 | 1 | 1 | 1 | 1 | 1 | 1 |
| | *M637_08170* | | | | | | 1 | | | | | | | | |
| | *LMOSLCC2482_0307* | | | | | | | 1 | | | | | | | |
| | | | | | | | | | | | | | | | |
| PCR IIaIIc | *Imo0525* | | | | | | | | | 1 | 1 | 1 | 1 | 1 | 1 |
| | *LM6179*_*0610* | | | | | | | | | 1 | | | | | |
| | *LMLG_RS02400* | | | | | | | | | | 1 | | | | |
| | *LMRG_02577* | | | | | | | | | | | 1 | | | |
| | *LM5578_1185* | | | | | | | | | | | | 1 | | |
| | *Imo1118* | | | | | | | | | | | | | 1 | |

| | | | | | | | | | | | | | | | |
|---|---|---|---|---|---|---|---|---|---|---|---|---|---|---|---|
| CC: clonal complex; 1: band expected to be amplified # Except EGDe, a CC9 strain which is of serogroup IIc but belongs to serotype 1/2a | | | | | | | | | | | | | | | |

A total of 282 *L. monocytogenes* strains were used to validate the method (*cf.* Table 10 below). The three multiplex PCRs were performed with an initial denaturation step at 94°C for 30 s followed by 25 cycles of 30 s at 94°C, 30 s at 55°C and 75 s at 72°C, and a final elongation step at 72°C for 10 min. Eight microliters of the PCR products were mixed with 3 µl of loading buffer and separated on a 1.5% agarose gel in a TBE buffer 1x at 120 V for 90 min. The primers were concentrated at 20 µM for the PCRs IVb and IIb and at 10 µM for the PCR IIaIIc.

Among these, 185 were analyzed previously by MLST, whereas the MLST genotype of 97 remaining strains was determined in the present study. The latter strains were selected from the Collection of Listeria of Institut Pasteur (CLIP) maintained at the French NRCL and WHO Collaborating Center for Listeria (Centre National de Reference des Listeria; Institut Pasteur; 25-28 rue du Docteur Roux; 75724 Paris Cedex 15; France).

DNA extractions were performed using the PROMEGA WIZARD® Genomic DNA Purification kit (PROMEGA Corporation 2800 Woods Hollow Road, Madison, WI 53711-5399 USA). PCR serogrouping and MLST were performed as previously described (Doumith *et al.* 2004; Ragon *et al.* 2008). Clonal complexes were defined as groups of allelic profiles sharing at least 6 out of 7 alleles with at least one other member of the group (Ragon *et al.* 2008). Allelic profiles sharing less than 6 alleles with all the other allelic profiles were designated as singletons; these groups were defined based on MLST data from this study and previous ones (Ragon *et al.* 2008; Chenal-Franscisque *et al.*; 2001; Cantinelli *et al.* 2013; Haase *et al.* 2014; Chenal-Franscisque *et al.* 2013). Discrimination index was calculated using the web tool available at http://darwin.phyloviz.net/ComparingPartitions.

The 282 strains were analyzed using the three multiplex PCR assays (*cf.* Table 9 above). Figures 6A, 6B and 6C show PCR amplification patterns obtained for representatives of the eleven target clones. Amplification results were highly consistent with the expected profiles of presence/absence of clone-specific genes and lineage- or serogroup-specific control genes (*cf.* Table 9 above). Overall, 222 out of 232 (95.7%) strains that belonged to target clones, were correctly identified (*cf.* Table 11 below and Figure 7): 100% of strains belonging to CC3, CC4, CC5, CC7, CC8, CC121 and CC155 were successfully identified; for other clones, correct identification rates were 78.6% (CC1), 93.1% (CC2), 94.7% (CC6) and 96% (CC9). For nine strains (6 CC1 strains, 2 CC2 strains and 1 CC9 strain), identification was incomplete, being correct at the level of lineage and serogroup although the expected clone-specific PCR product was not amplified (Figure 7, Table 10 below). Only CC6 strain CLIP2009/01048 was misidentified, as CC1. Out of the 50 strains that did not belong to target clones, 47 (94%) yielded the expected lineage and serogroup specific PCR fragments, and three were misidentified as either CC1 or CC8, from which they differed by two or three MLST alleles. Therefore, in total, only four (1.4%) strains were misidentified, whereas 269 out of 282 (95.4%) were identified correctly.

In conclusion, we have developed a set of three multiplex PCRs enabling 'clonogrouping', *i.e.,* determining whether a strain belongs to one of the major clonal groups of *L. monocytogenes.* This novel tool can be used in a one-step strategy, which comprises the implementation of the three clonogrouping PCR assays (clonogrouping PCR assays IVb, IIb and IIaIIc) either simultaneously or sequentially.

Alternatively, this novel tool can be used in a two-step strategy, such as:
i. implementation of the PCR serogrouping method of Doumith *et al.* 2004 to determine the PCR serogrouping profile (IVb, IIb, IIa or IIc), and
ii. implementation of the clonogrouping PCR assay that corresponds to the PCR serogrouping profile determined at step i. (clonogrouping PCR assay IVb, IIb or IIaIIc), thereby allowing for identification at the MLST clone level.

Unlike MLST, our tools can provide clone identification in less than one day starting from a bacterial culture.

Based on the dataset of this study, the discriminatory power (Simpson's index) of clonogrouping was 92.3% (CI, 91.8-92.8), whereas PCR serogrouping of Doumith *et al.* 2004 had a discrimination level of only 77.5% (CI, 76.3-78.7).

This new widely applicable tool will therefore significantly refine, beyond PCR serogrouping, the genotypic characterization of *L. monocytogenes* strains or isolates. It will thus provide improved resolution for molecular surveillance, while providing rapid information to risk managers during the investigations of outbreaks and for the control of food safety and food production environment. Finally, the wide use of this novel method should contribute to better define the distribution of major clonal groups in clinical, food and environmental sources.

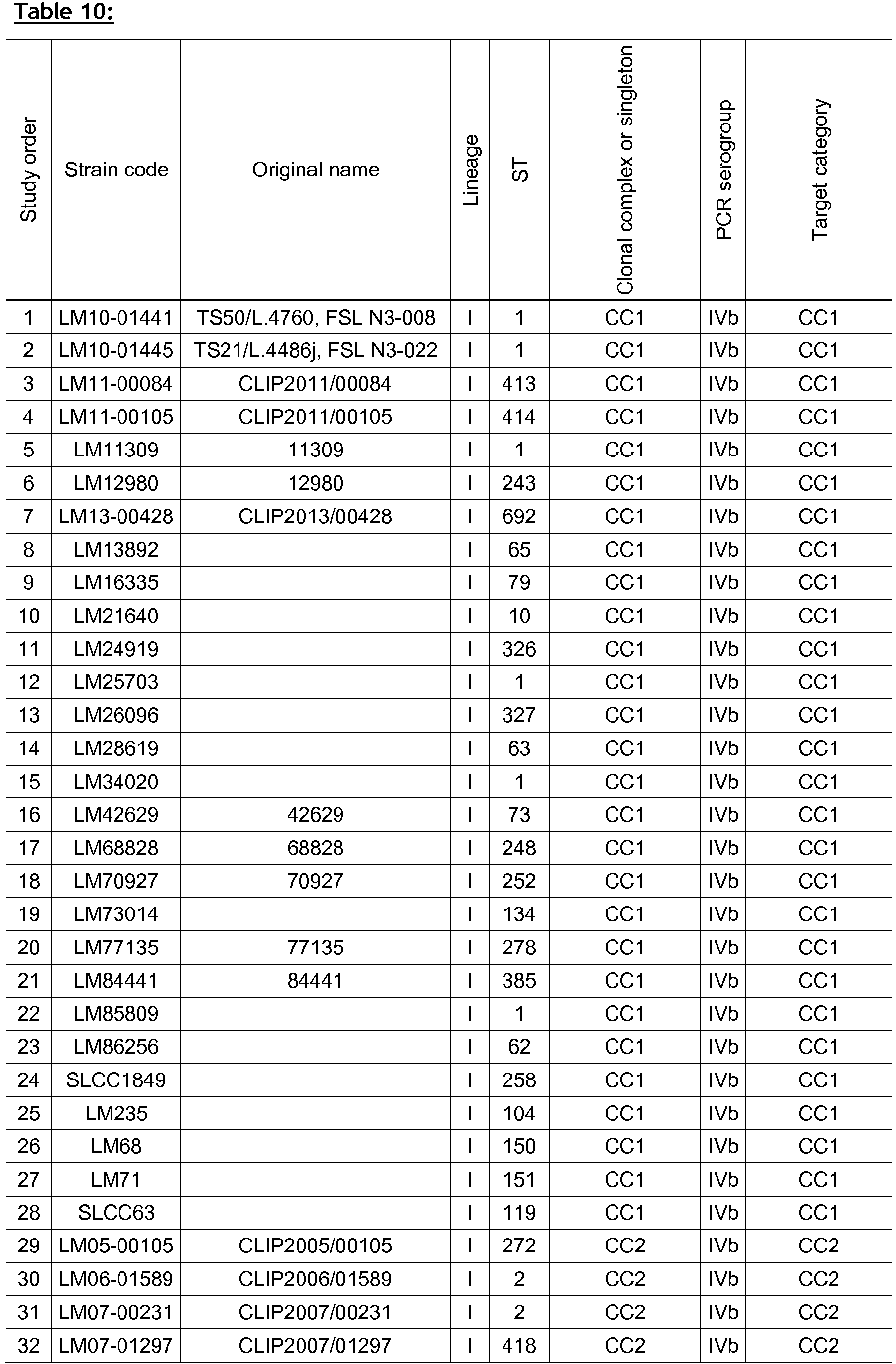

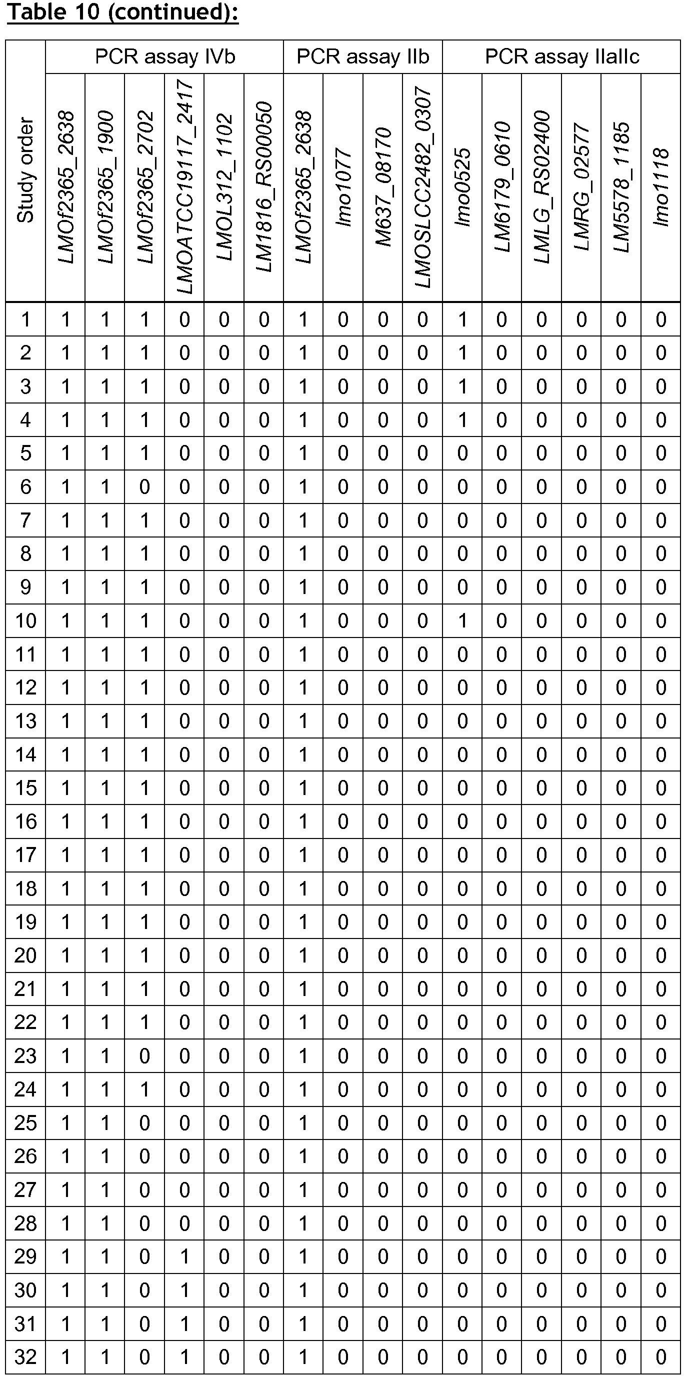

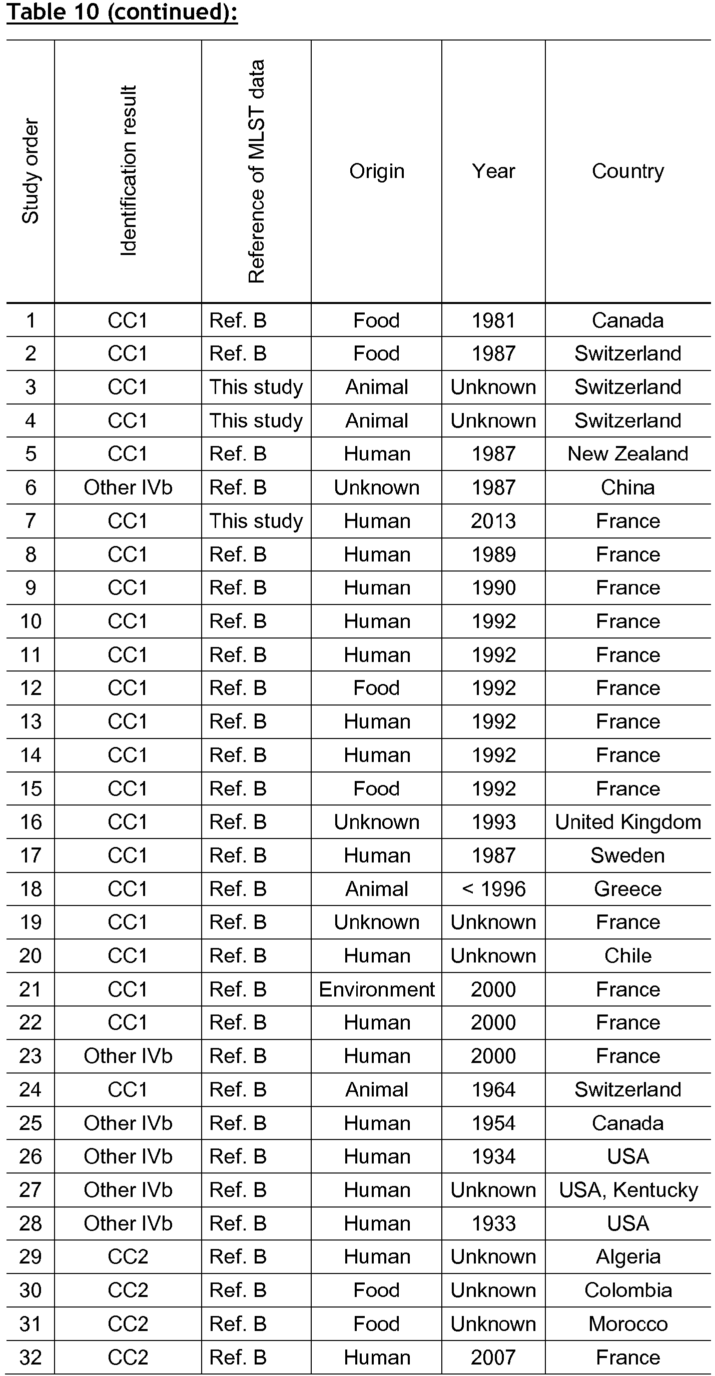

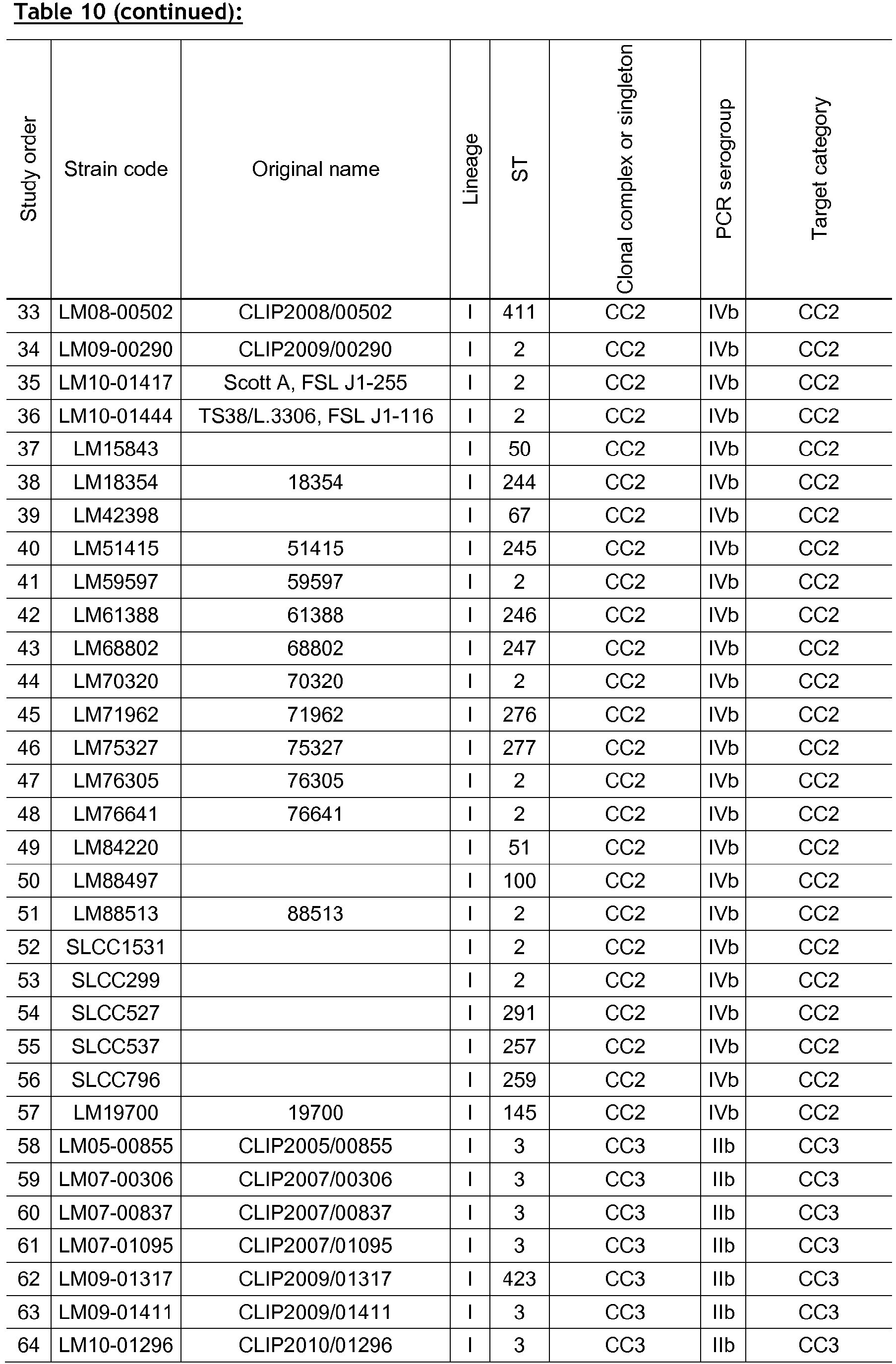

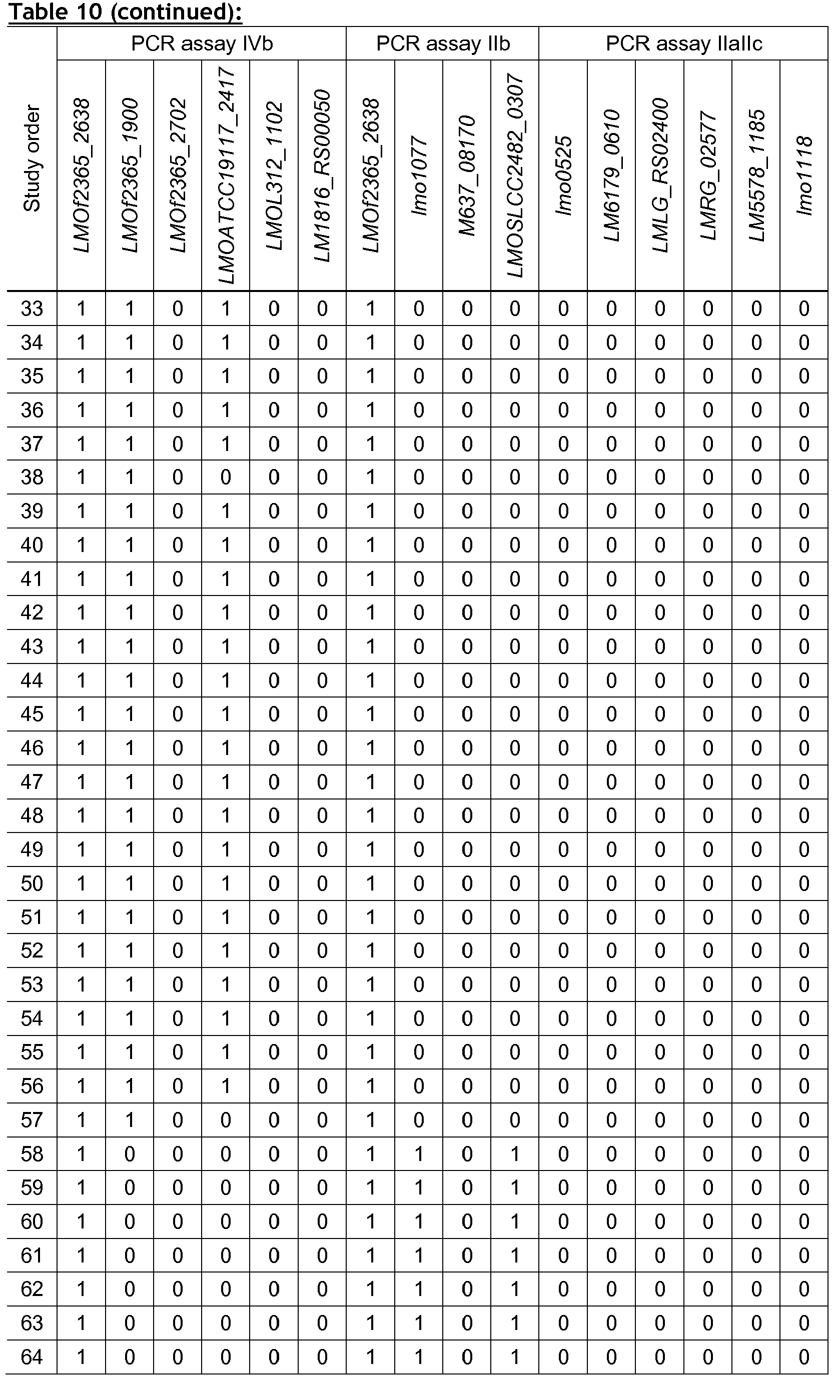

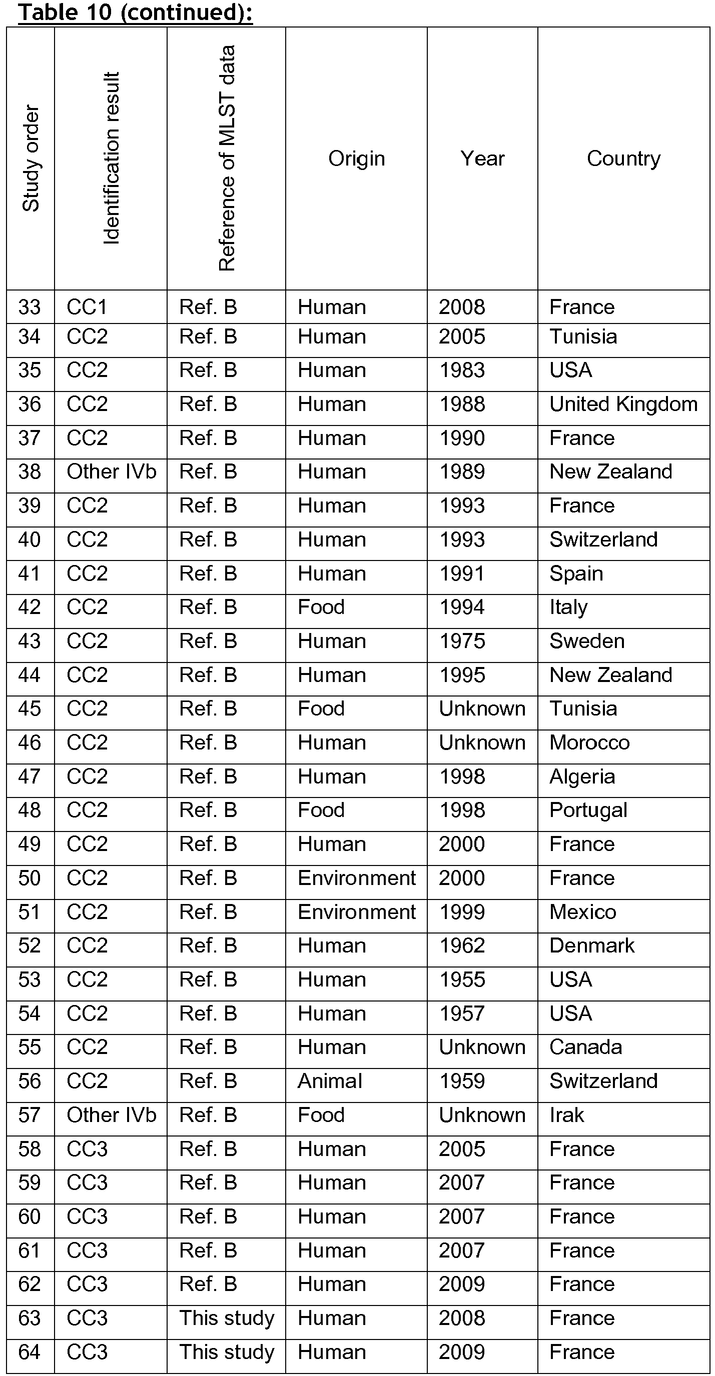

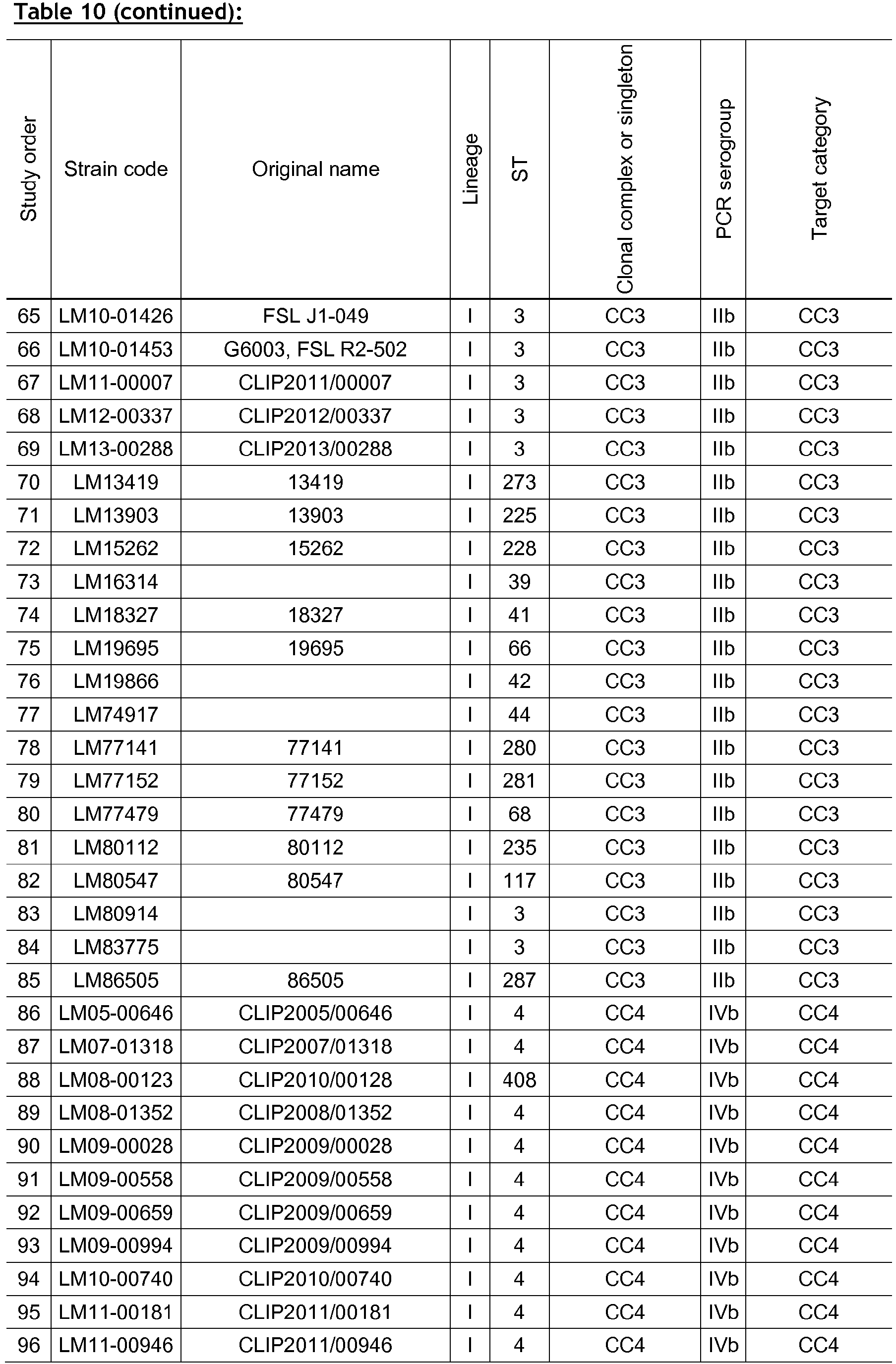

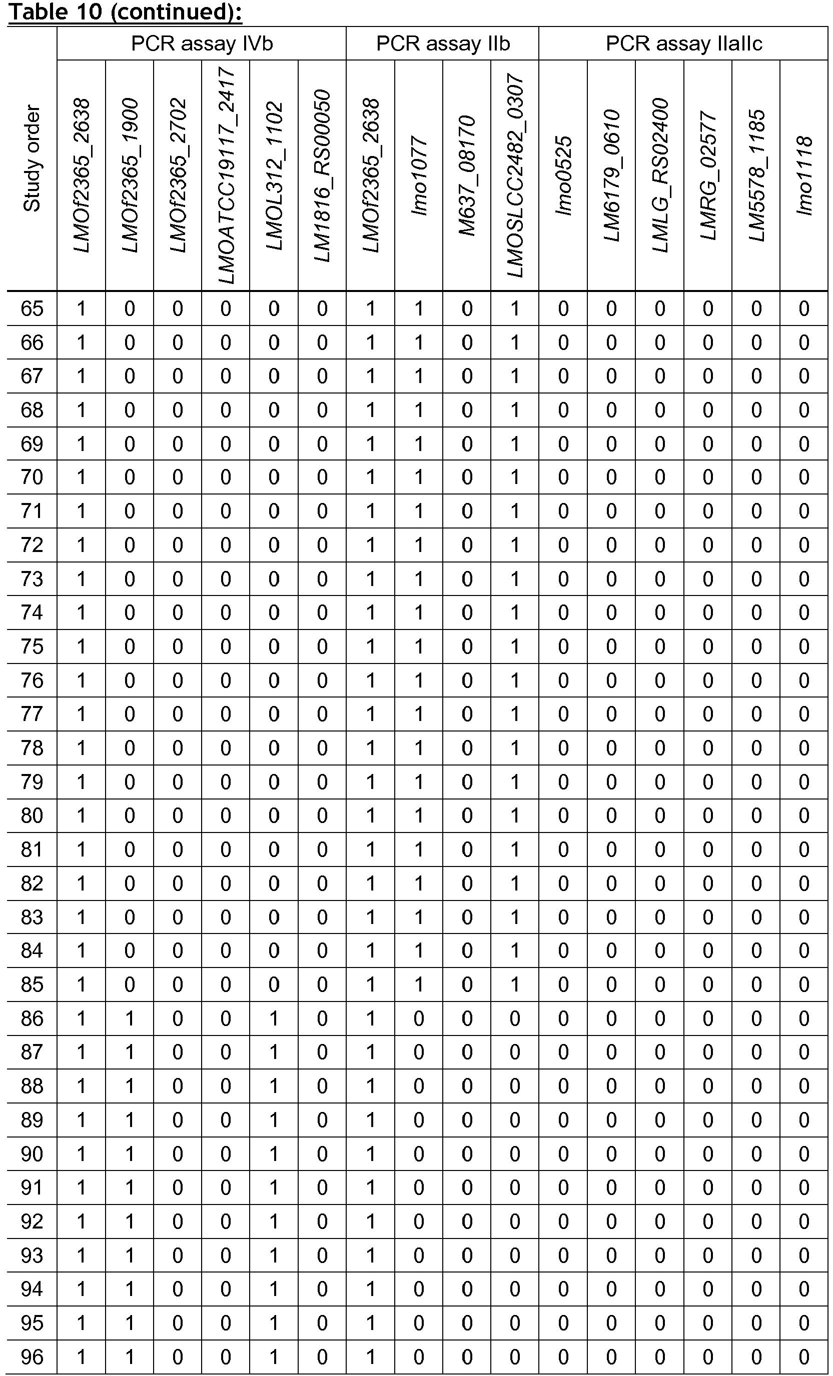

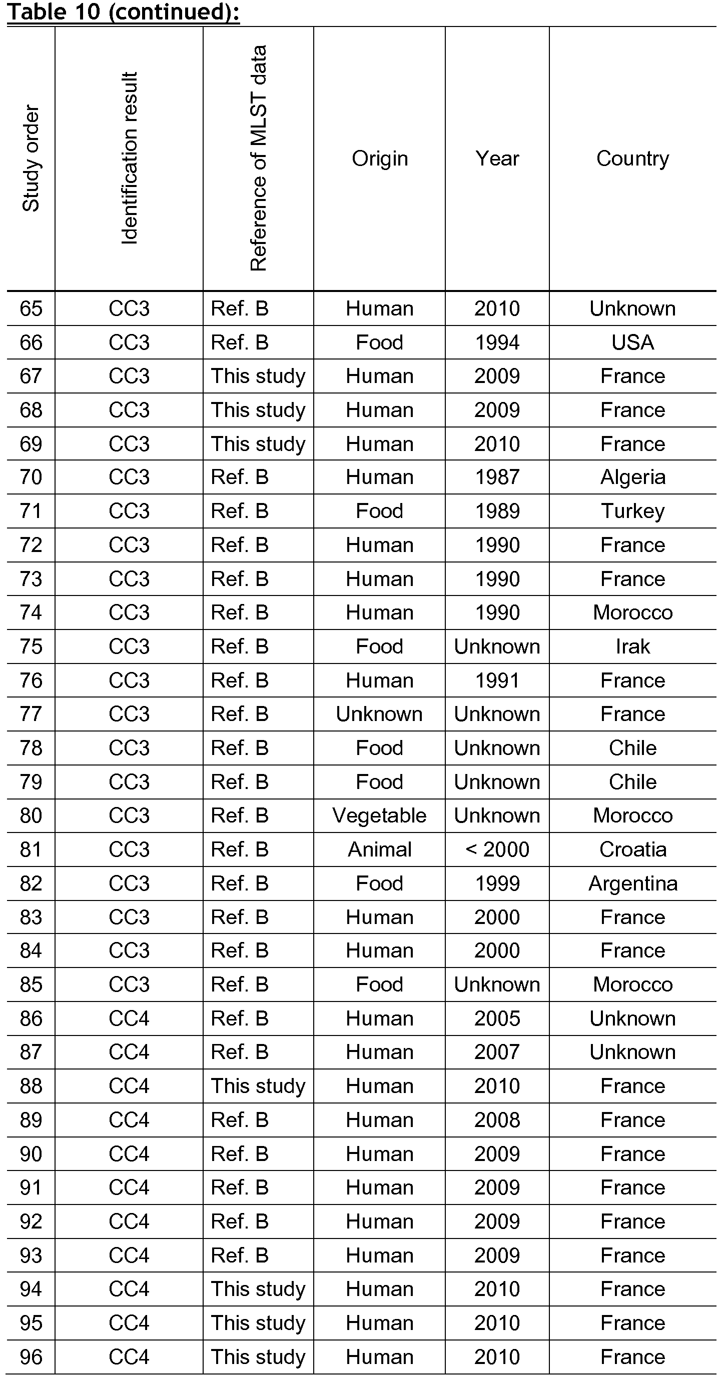

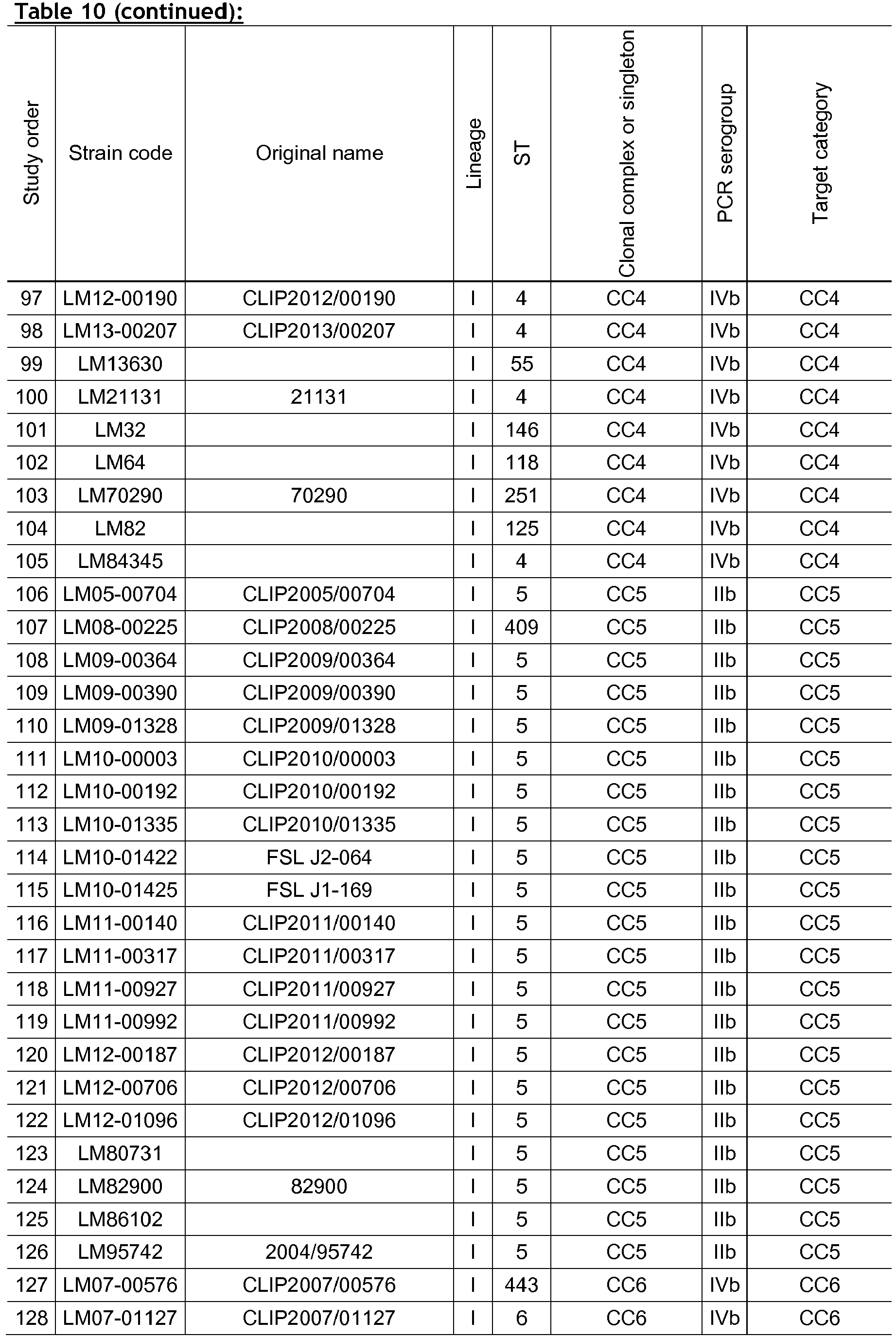

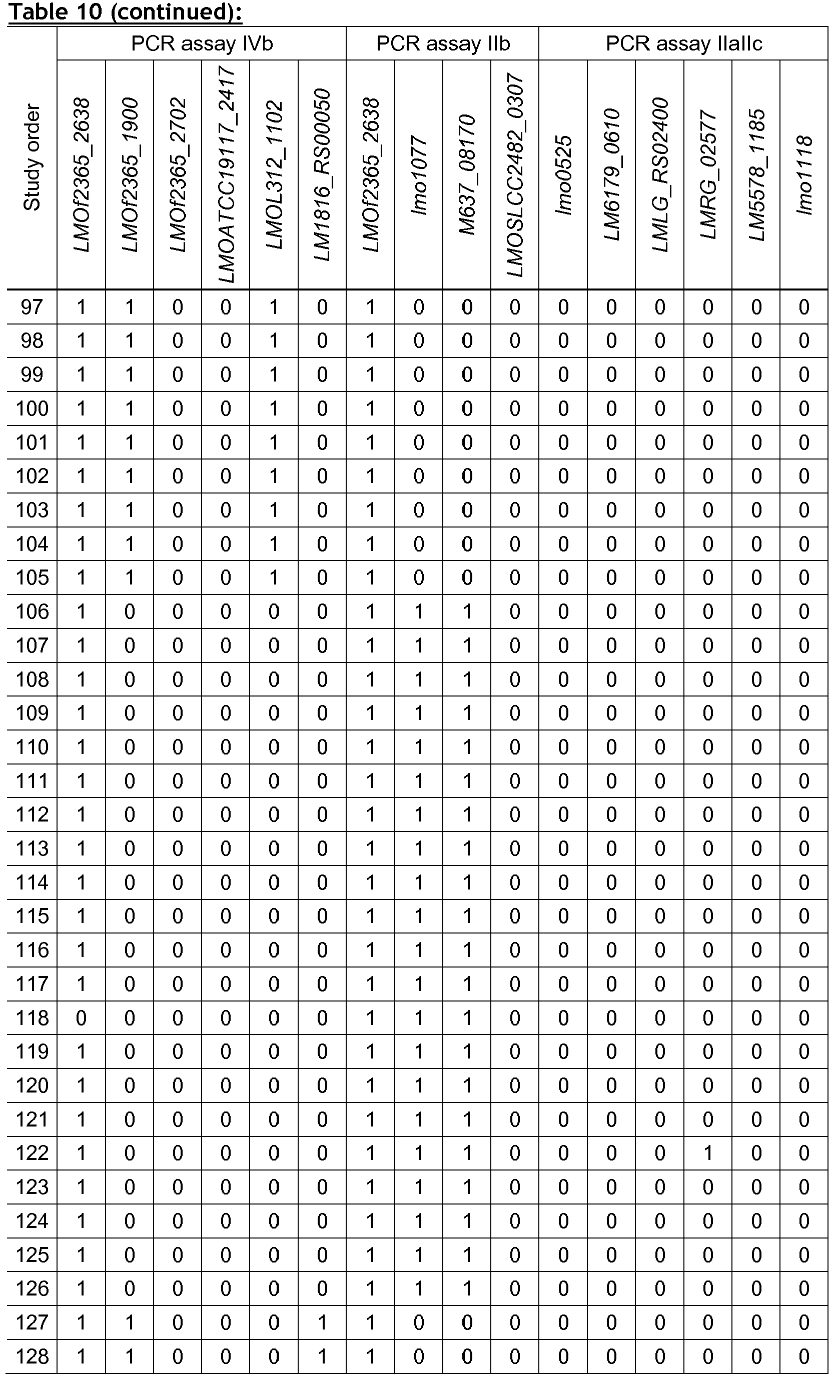

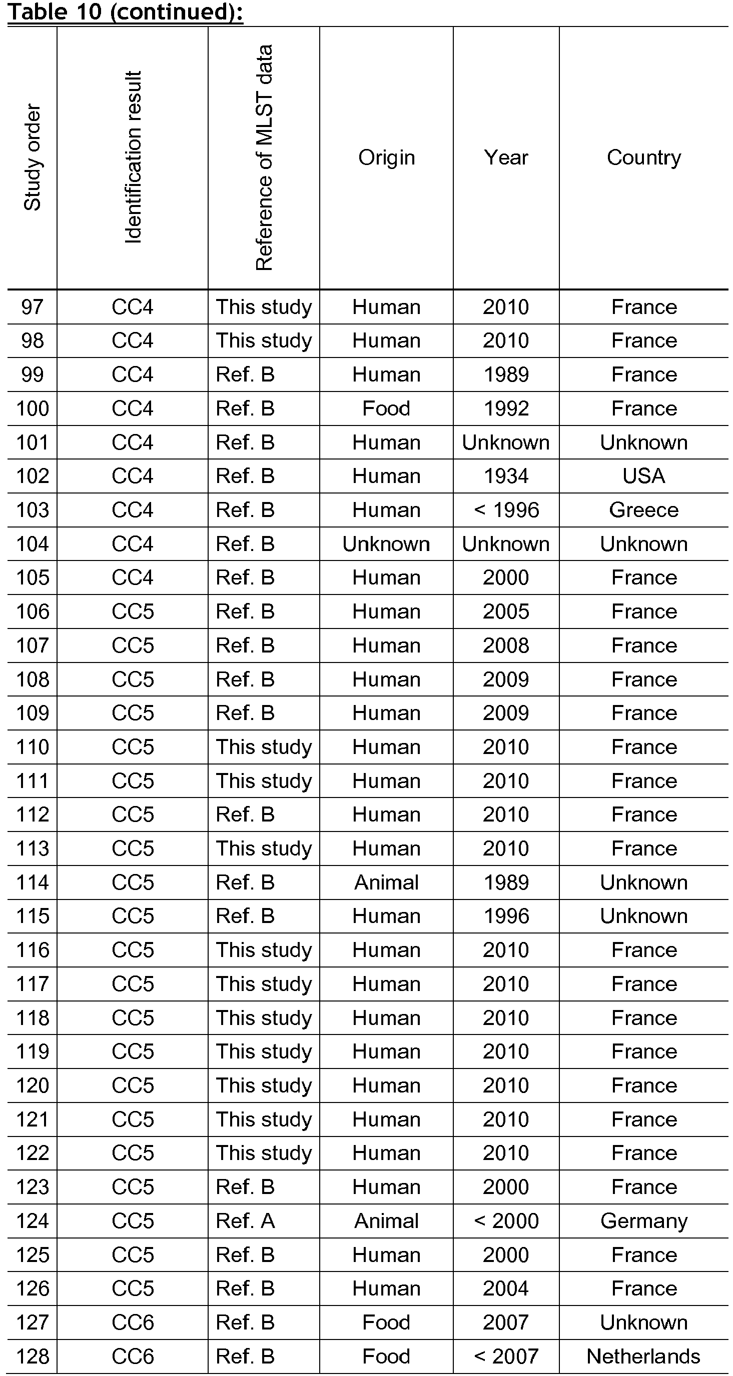

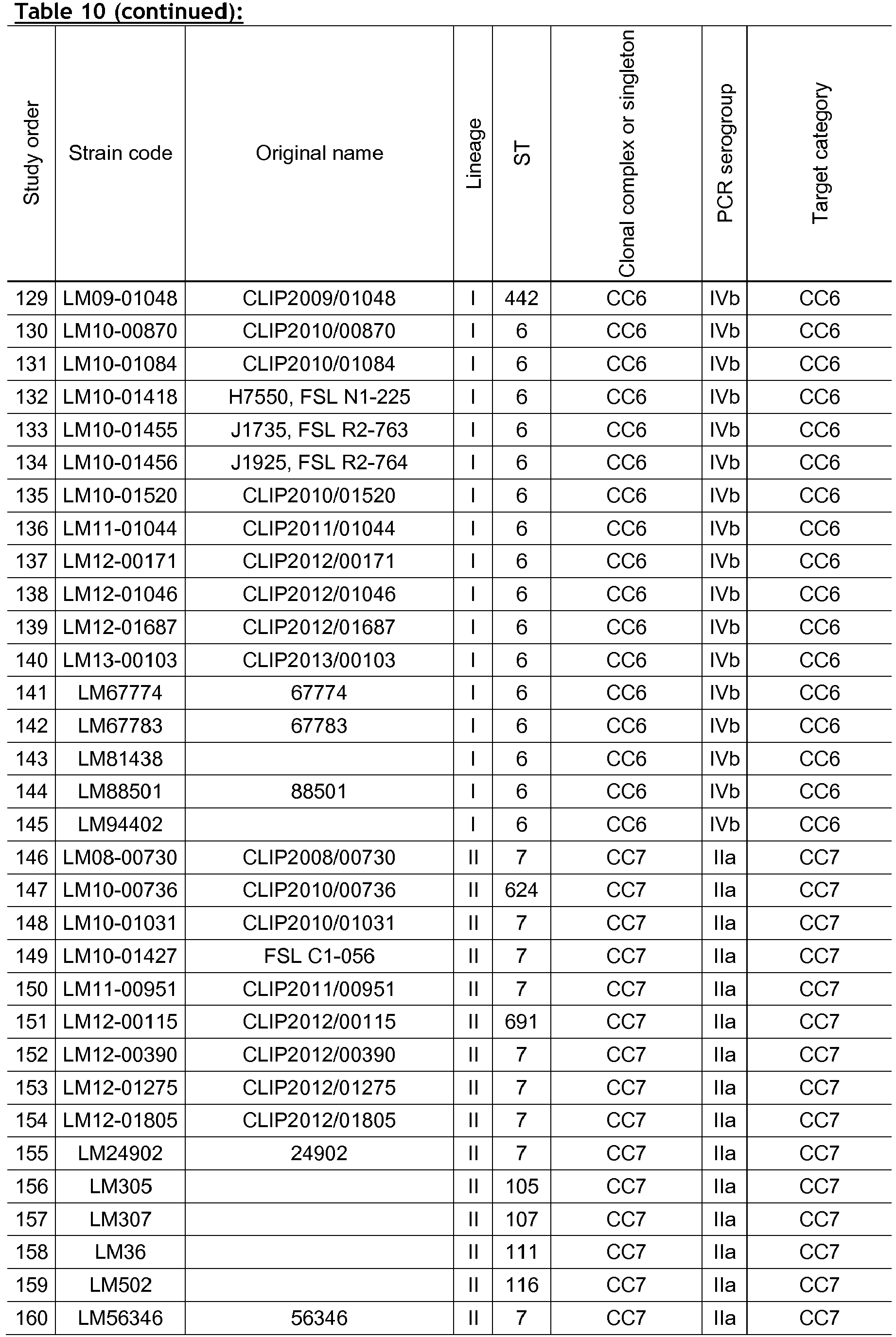

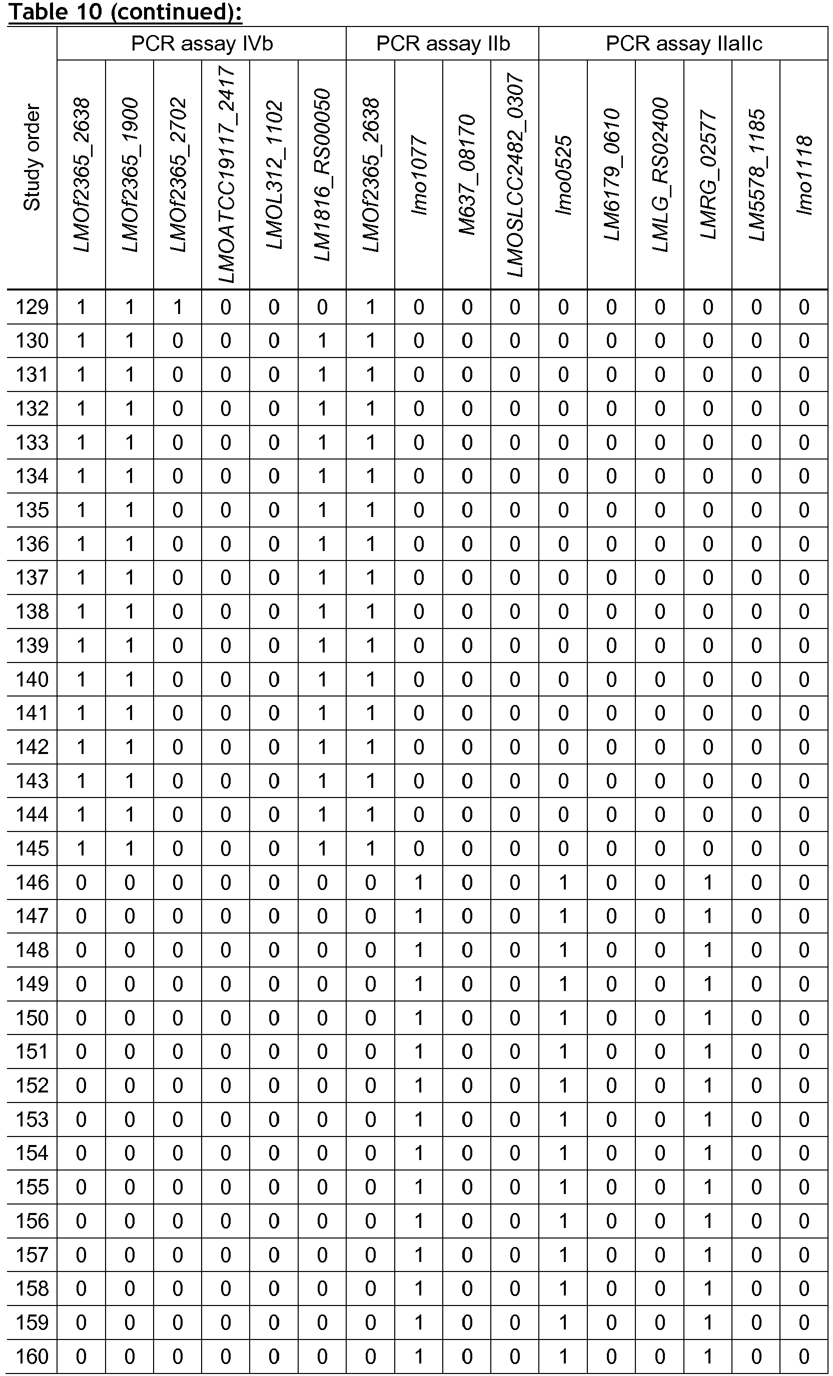

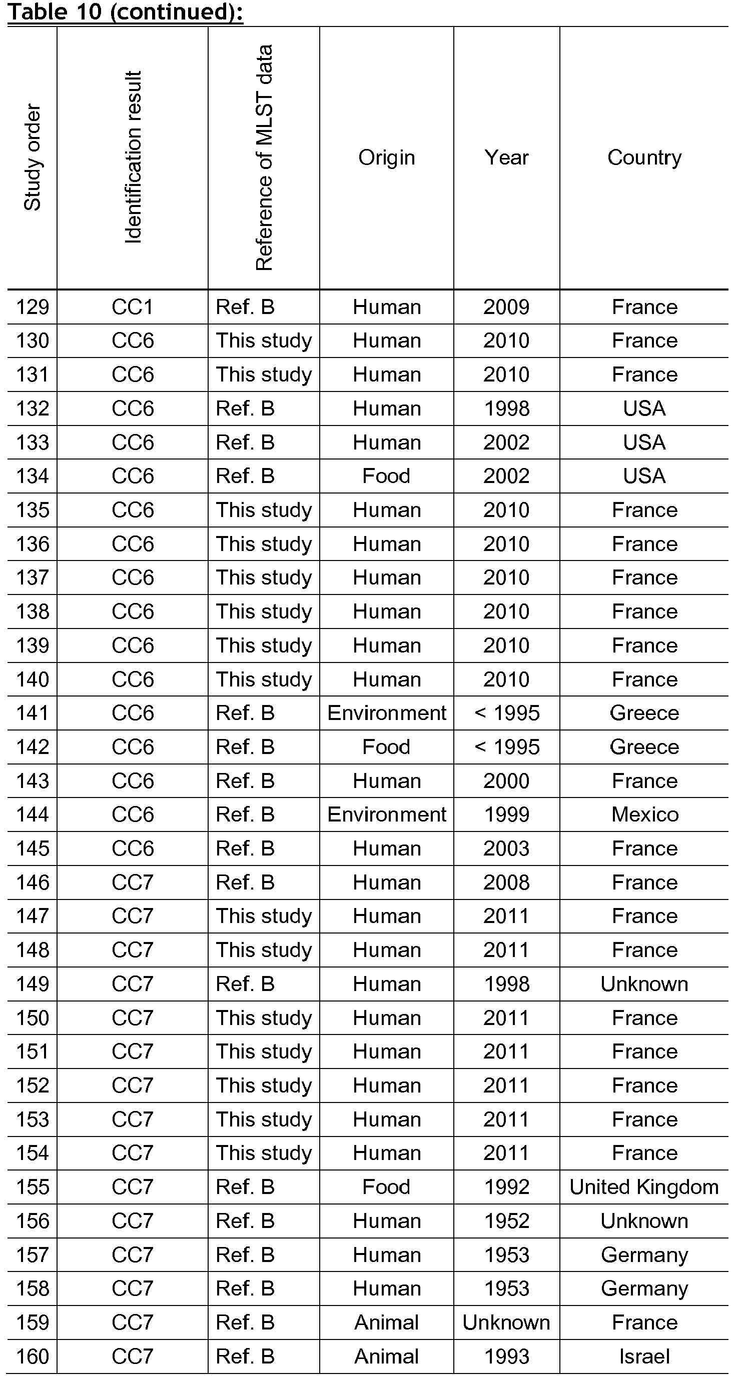

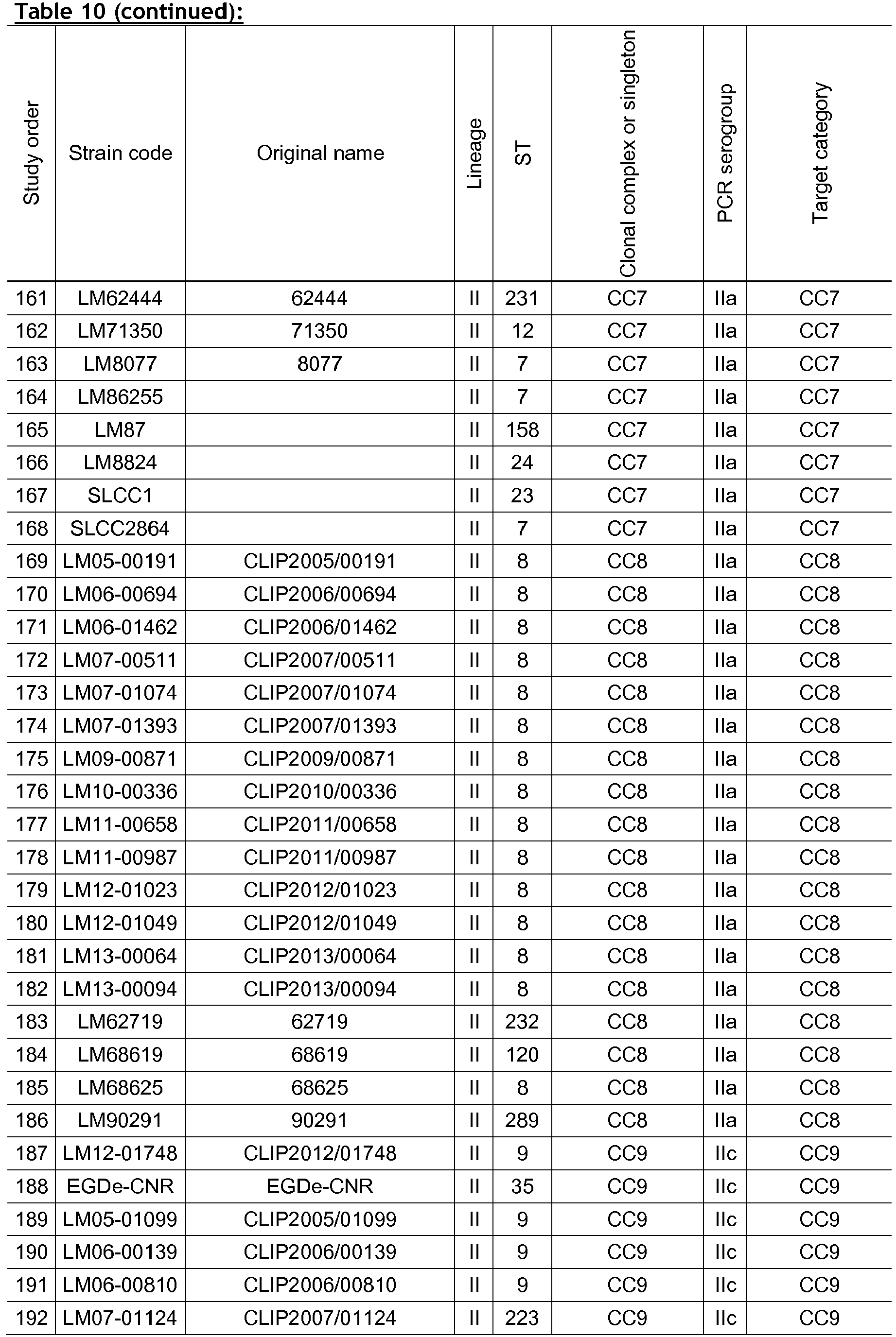

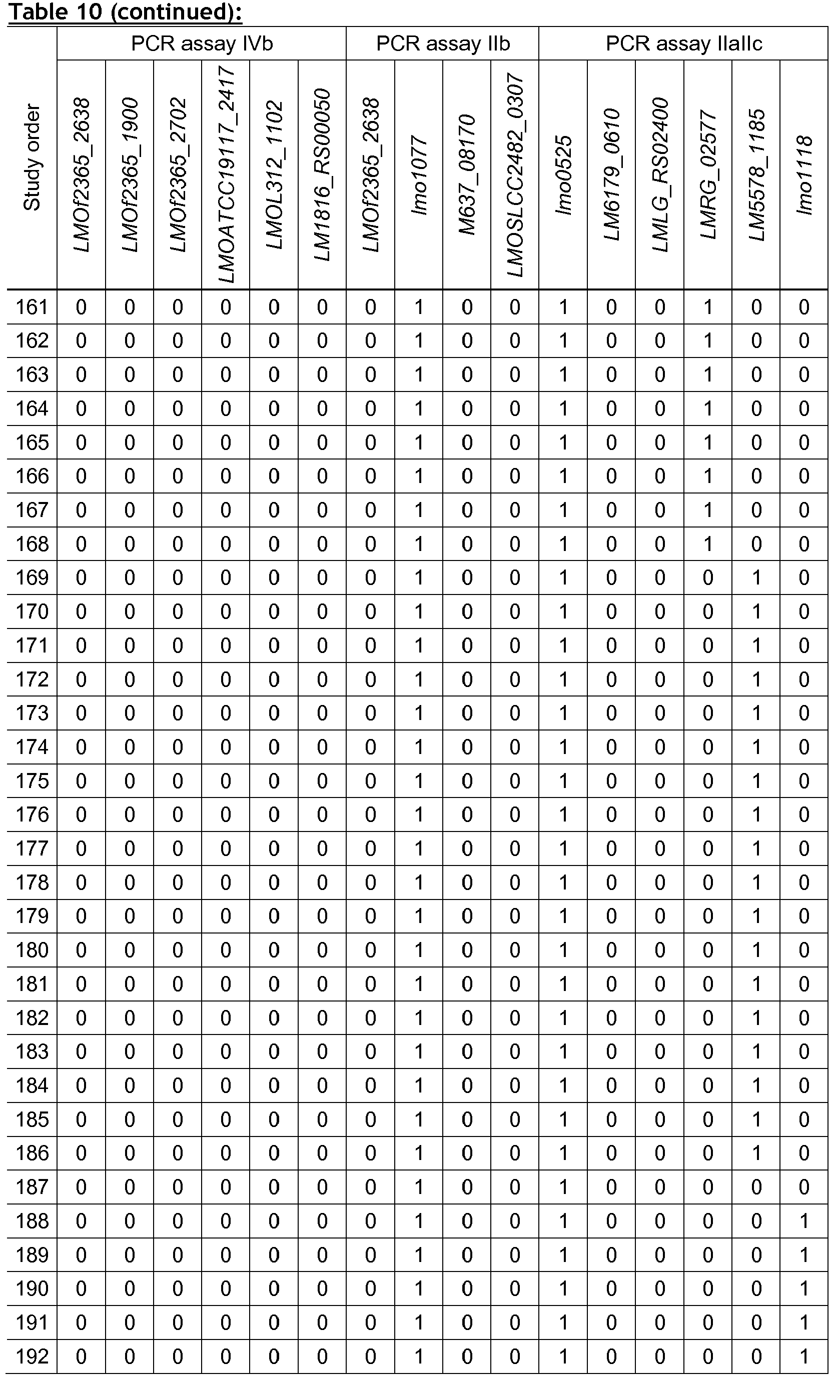

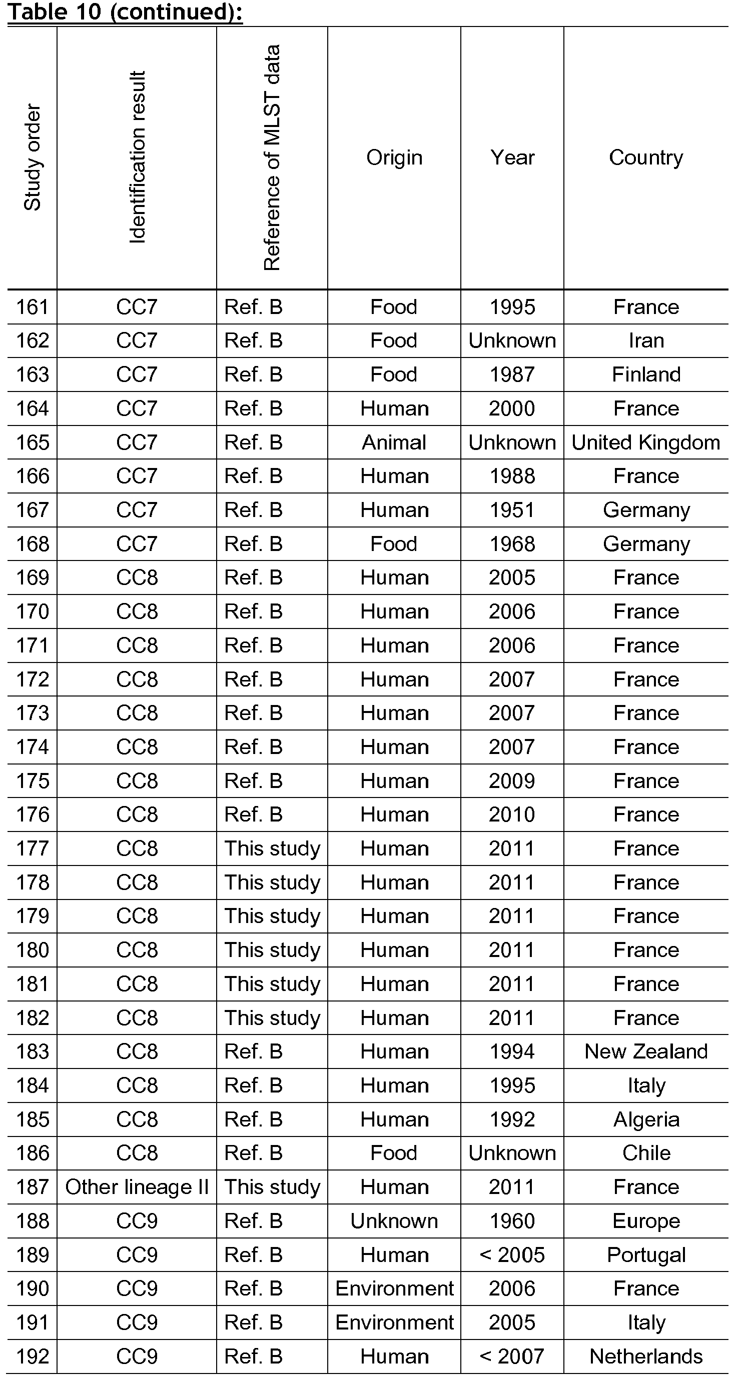

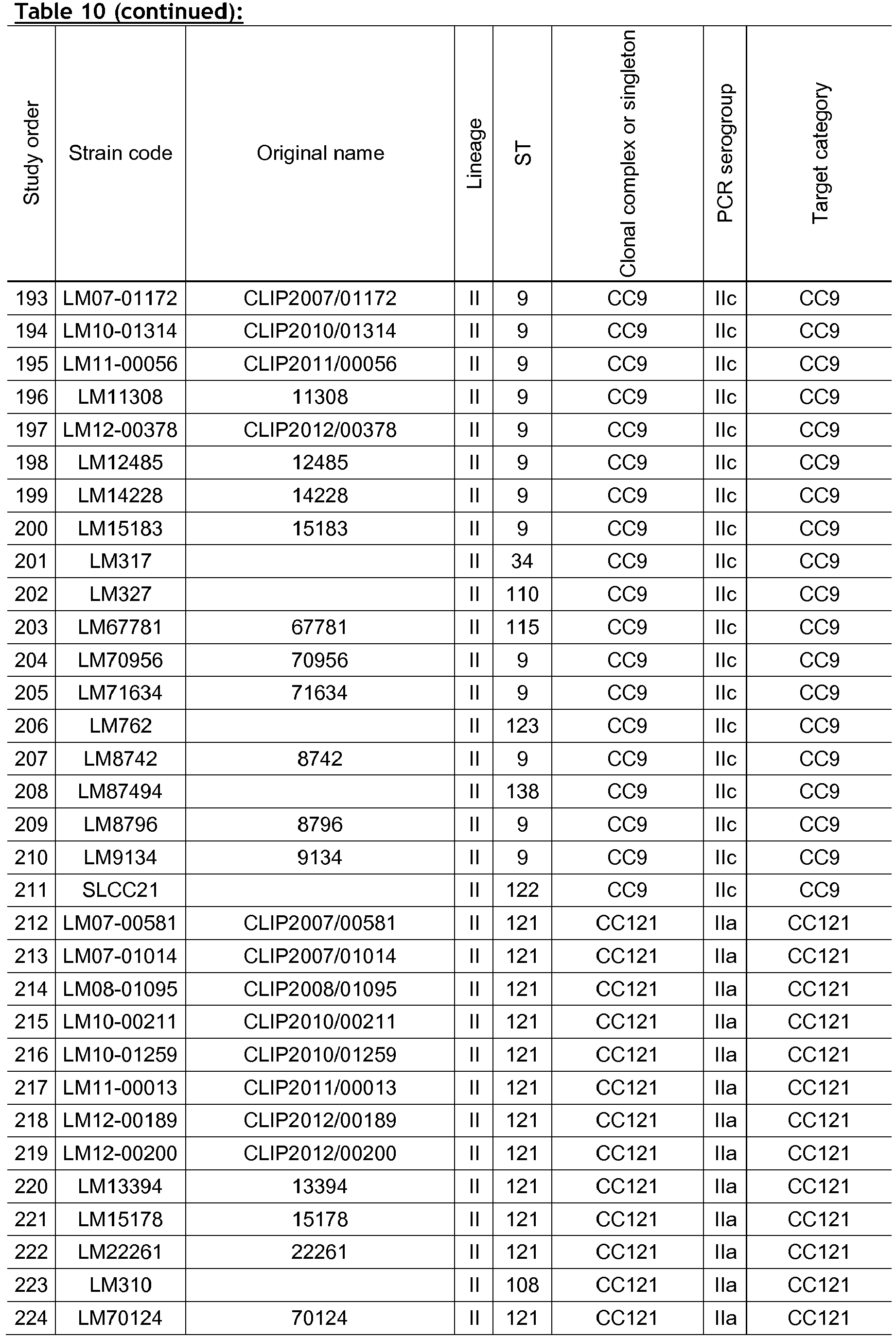

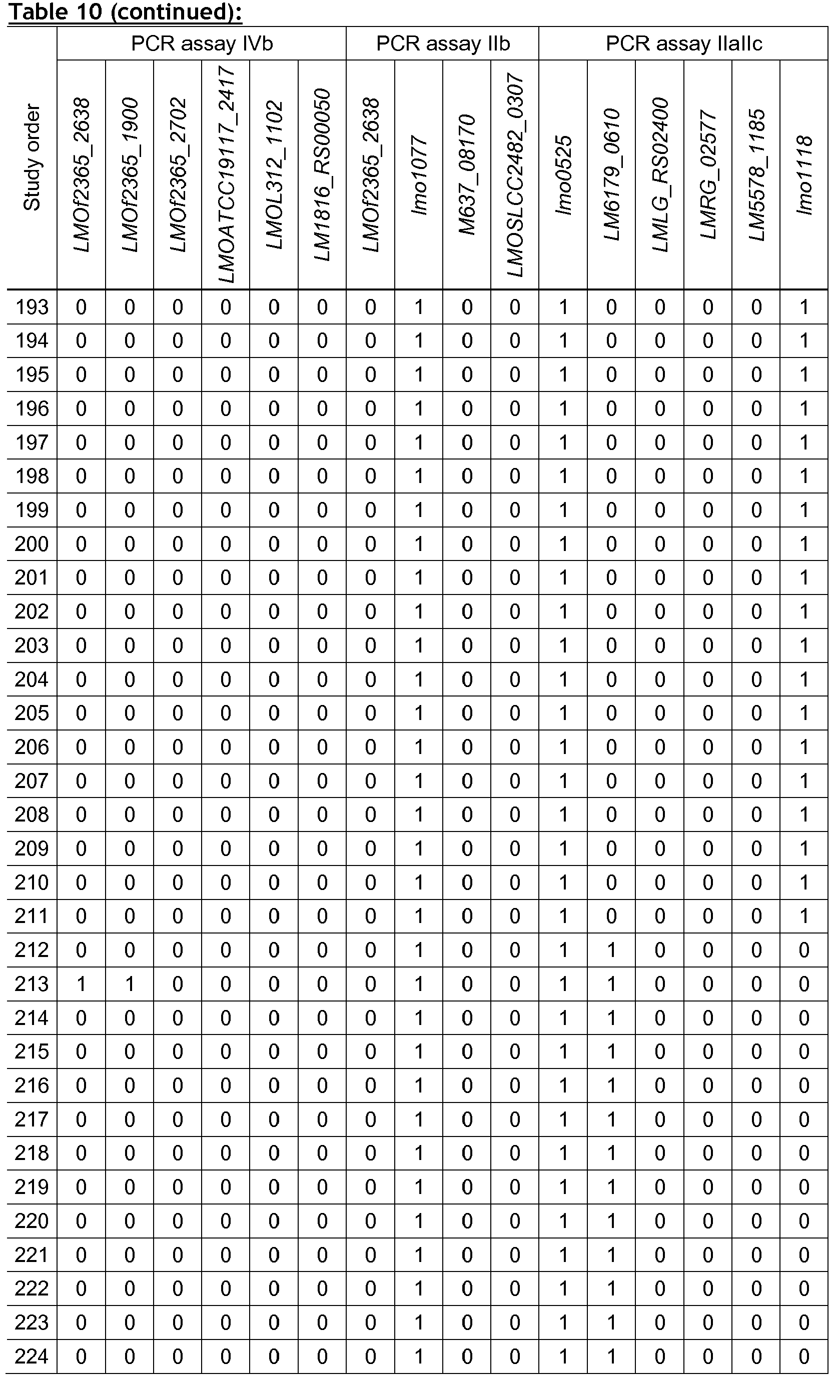

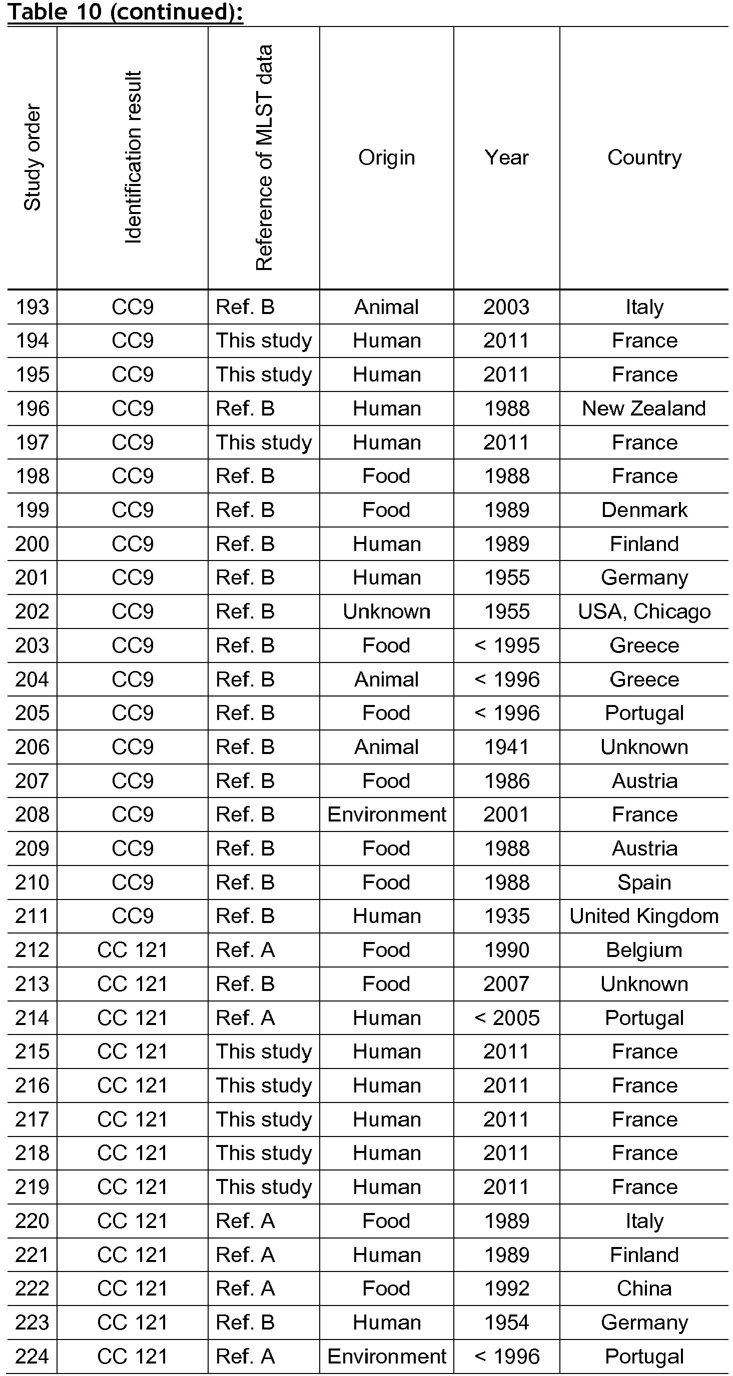

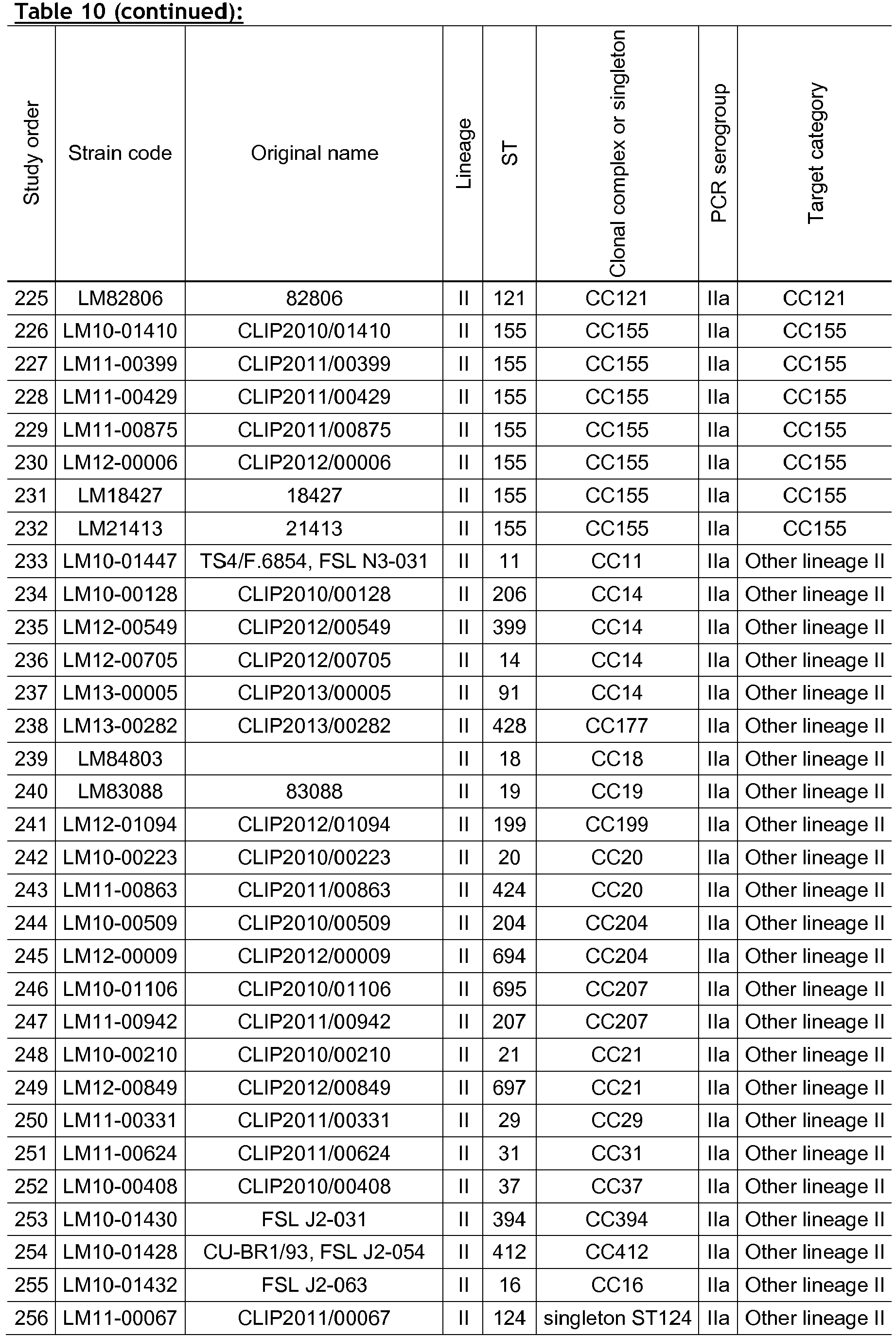

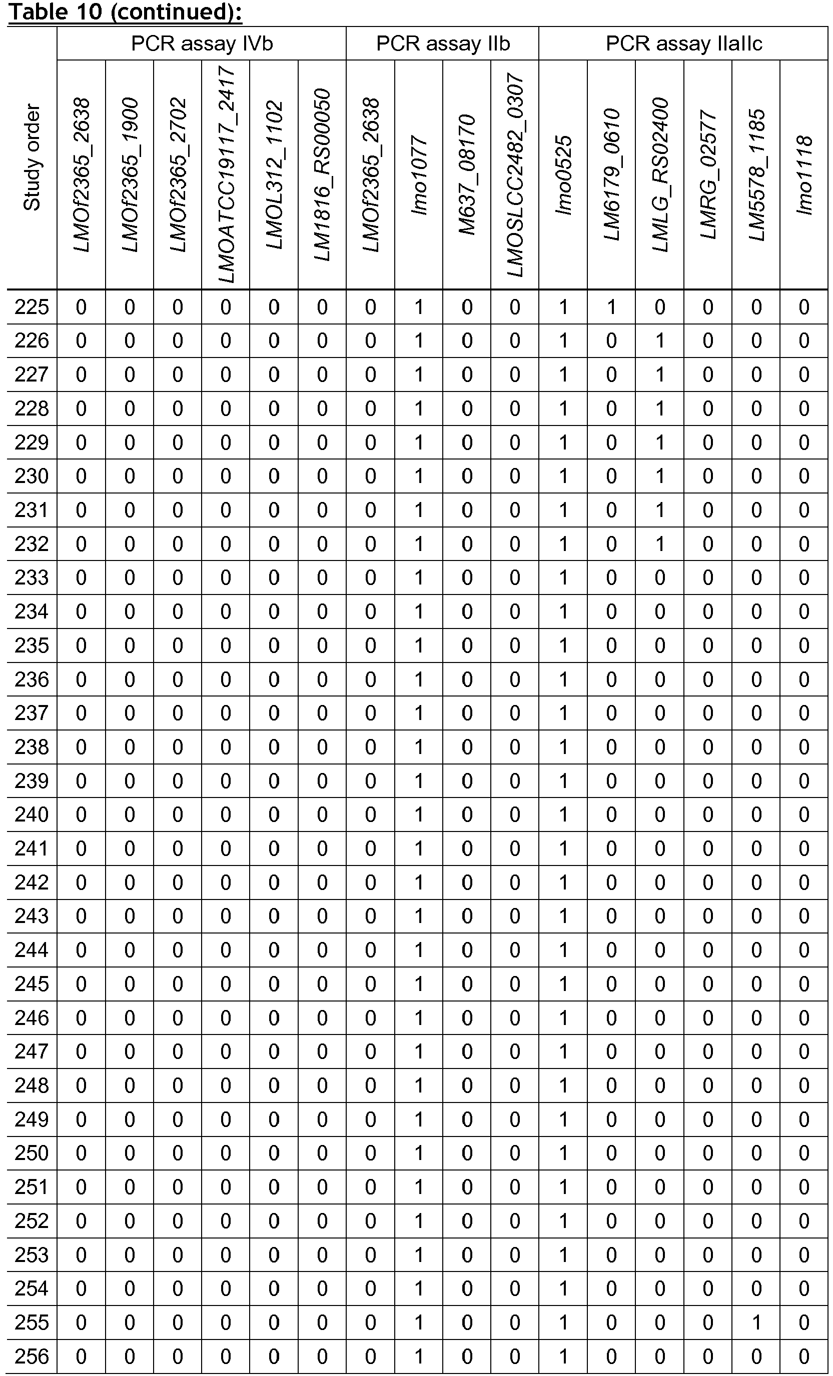

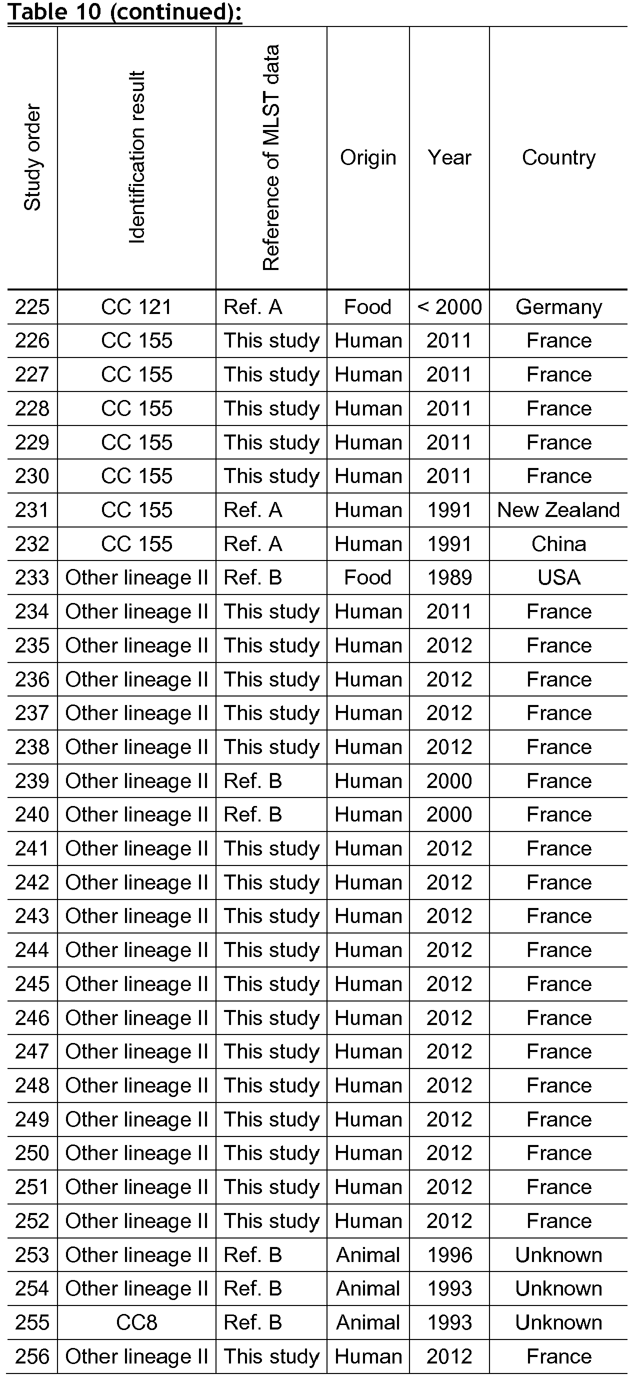

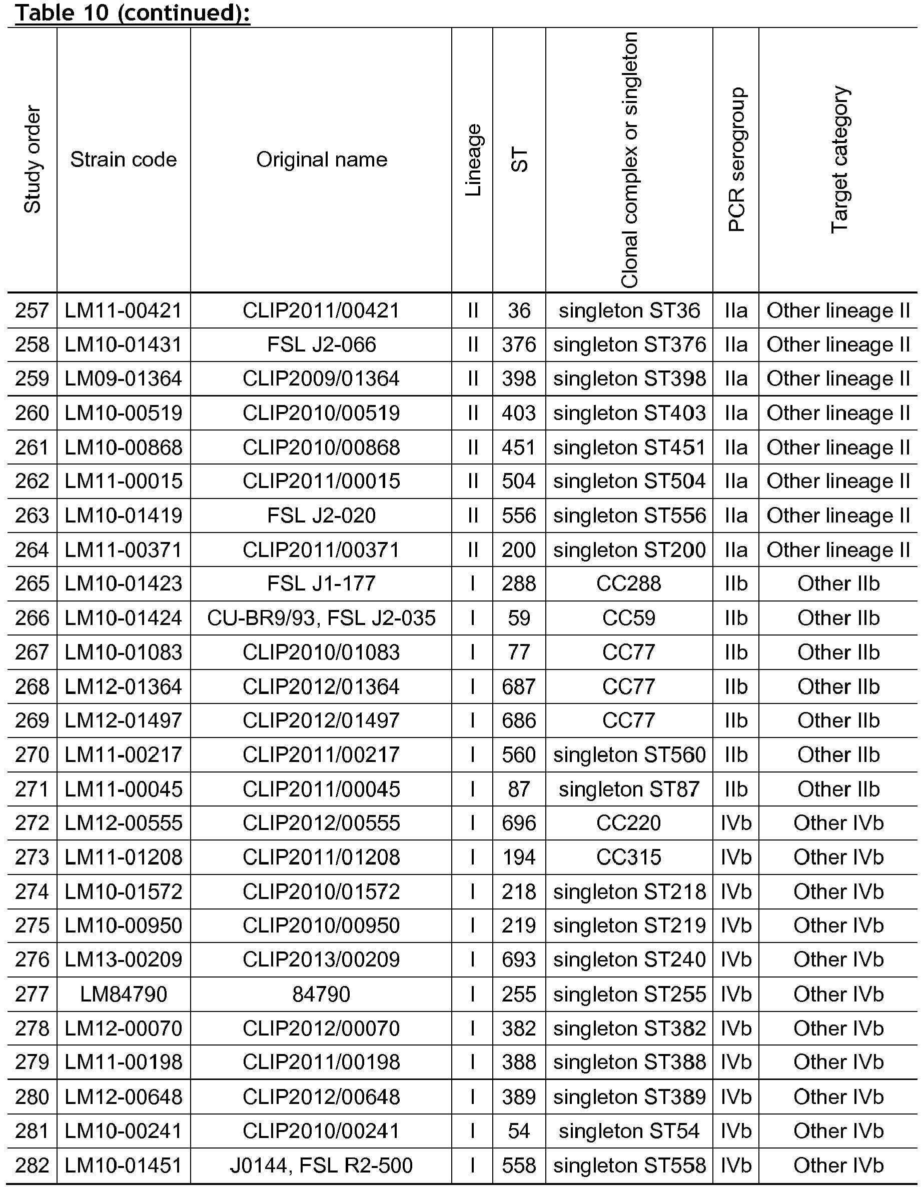

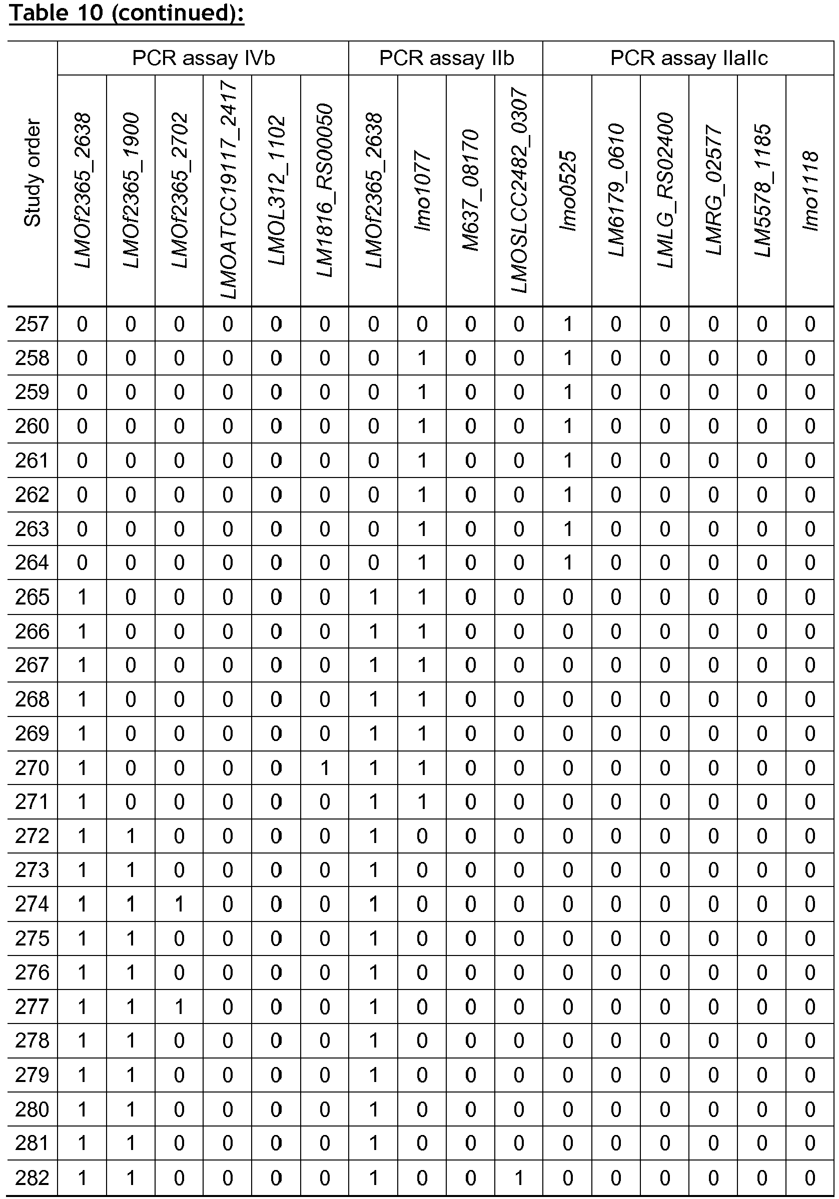

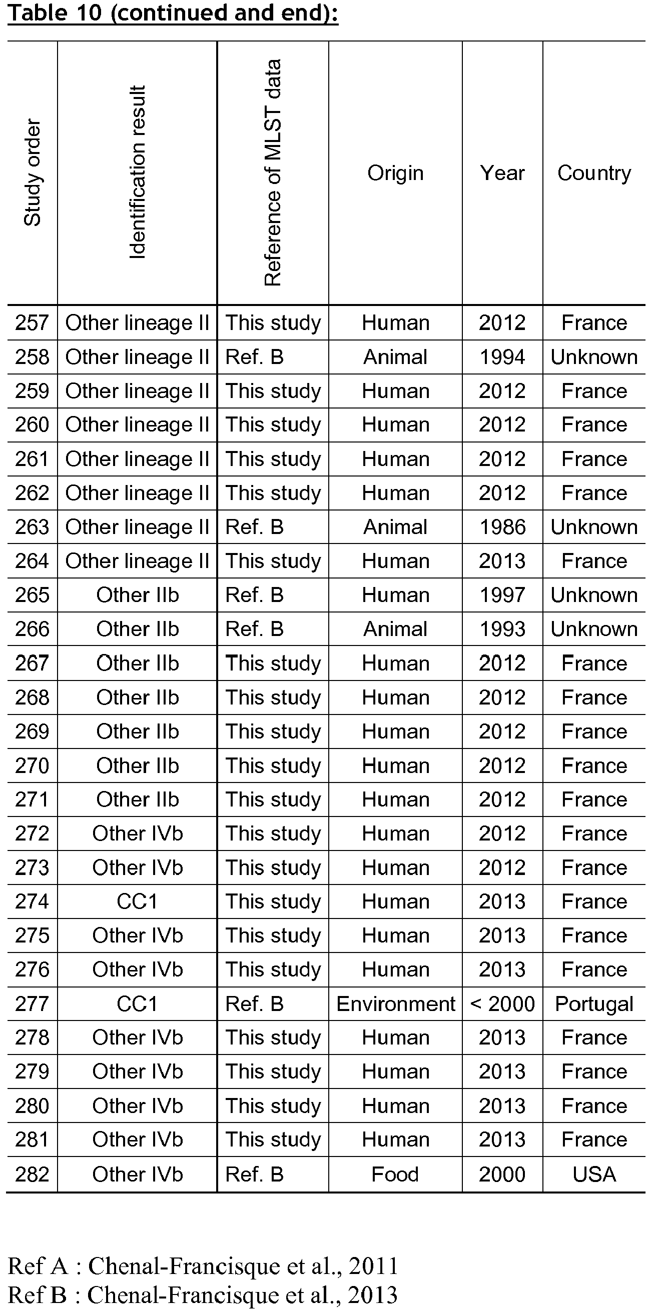

**Table 11. Identification results.**

| CC | No. isolates | No. of correctly identified strains (%) | No. of incomplete identification (%) | No. of incorrect identification (%) |
|---|---|---|---|---|
| CC1 | 28 | 22 (78.6) | 6 strains identified as other IVb (21.4%) | |
| CC2 | 29 | 27 (93.1) | 2 strains identified as other IVb (6.9%) | |
| CC3 | 28 | 28 (100) | | |
| CC4 | 20 | 20 (100) | | |
| CC5 | 21 | 21 (100) | | |
| CC6 | 19 | 18 (94.7) | | 1 strain identified as CC1 (5.3%) |
| CC7 | 23 | 23 (100) | | |
| CC8 | 18 | 18 (100) | | |
| CC9 | 25 | 24 (96) | 1 strain identified as other lineage II (4.0%) | |
| CC121 | 14 | 14 (100) | | |
| CC155 | 7 | 7 (100) | | |
| Other IVb | 11 | 9 (81.8) | | 2 strains identified as CC1 (18.2%) |
| Other lib | 7 | 7 (86) | | |
| Other lineage II | 32 | 31 (96.9) | | 1 strain identified as CC8 (3.1%) |
| Total | 282 | 269 (95.4%) | 9 (3.2%) | 4 (1.4%) |

### BIBLIOGRAPHIC REFERENCES

Brosch, Chen and Luchansky 1994. Pulsed-field fingerprinting of Listeriae: identification of genomic divisions for Listeria monocytogenes and their correlation with serovar. Appl. Environ. Microbiol. 60: 2584-2592.
Cantinelli T, Chenal-Francisque V, Diancourt L, Frezal L, Leclercq A, Wirth T, Lecuit M, Brisse S. 2013. "Epidemic clones" of Listeria monocytogenes are widespread and ancient clonal groups. J Clin Microbiol 51:3770-3779.
Chenal-Francisque V, Lopez J, Cantinelli T, Caro V, Tran C, Leclercq A, Lecuit M, Brisse S. 2011. Worldwide distribution of major clones of Listeria monocytogenes. Emerg Infect Dis 17:1110-1112.
Chenal-Francisque V, Diancourt L, Cantinelli T, Passet V, Tran-Hykes C, Bracq-Dieye H, Leclercq A, Pourcel C, Lecuit M, Brisse S. 2013. An optimized MLVA assay and its complementarity with PFGE and MLST for Listeria monocytogenes clone identification and surveillance. J Clin Microbiol 61.
Doumith, Buchrieser, Glaser, Jacquet and Martin 2004. Differentiation of the major Listeria monocytogenes serovars by multiplex PCR. J. Clin. Microbiol. 42:3819-3822.
Graves LM, Swaminathan B. PulseNet standardized protocol for subtyping Listeria monocytogenes by macrorestriction and pulsed-field gel electrophoresis. Int J Food Microbiol 2001 Apr 11; 65(1-2):55-62.
Haase JK, Didelot X, Lecuit M, Korkeala H, Group LmMS, Achtman M. 2014. The ubiquitous nature of Listeria monocytogenes clones: a large-scale Multilocus Sequence Typing study. Environ Microbiol 16:405-416.
Ragon, Wirth, Hollandt, Lavenir, Lecuit, Le Monnier and Brisse 2008. A new perspective on Listeria monocytogenes evolution. PLoS Pathog. 4:e1000146.
Seeliger and Hohne 1979. Serotyping of Listeria monocytogenes and related species. Methods Microbiol.: 31-49.
Ward, Ducey, Usgaard, Dunn, Bielawski 2008. Multilocus genotyping assays for single nucleotide polymorphism-based subtyping of Listeria monocytogenes isolates. Appl. Environ. Microbiol., 74 (2008), pp. 7629-7642.

## Claims

1. An *in vitro* method for determining that an isolate of *Listeria monocytogenes,* has a high probability of belonging to clone CC1, to clone CC2, to clone CC4, to clone CC6, to clone CC7, to clone CC3, to clone CC5, to clone CC8 or CC16, or to clone CC155 of *L. monocytogenes,* wherein a high probability of belonging to one of these clones is indicative that said *L. monocytogenes,* isolate has a potentiality, more particularly a high potentiality, of causing invasive infection in a human, wherein said method comprises detecting that one of the following genes i.-ix. is present in said *L.* monocytogenes isolate:
i. a gene, which is specific of *L. monocytogenes* clone CC1, wherein said CC1-specific gene is *LMOf2365_2702* (SEQ ID NO: 1),
ii. a gene, which is specific of *L. monocytogenes* clone CC2, wherein said CC2-specific gene is *LMOATCC19117_2417* (SEQ ID NO: 2),
iii. a gene, which is specific of *L. monocytogenes* clone CC4, wherein said CC4-specific gene is *LM0312_1102* (SEQ ID NO: 3),
iv. a gene, which is specific of *L. monocytogenes* clone CC6, wherein said CC6-specific gene is *LM1816_RS00050* (SEQ ID NO: 4),
v. a gene, which is specific of *L. monocytogenes* clone CC7, wherein said CC7-specific gene is *LMRG_02577* (SEQ ID NO: 9),
vi. a gene, which is specific of *L. monoccytogenes* clone CC3, wherein said CC3-specific gene is *LMOSLCC2482_0307* (SEQ ID NO: 5),
vii. a gene, which is specific of *L. monoccytogenes* clone CC5, wherein said CC5-specific gene is *M637_08170* (SEQ ID NO: 6),
viii. a gene, which is specific of *L. monoccytogenes* clones CC8 and CC16, wherein said CC8- and CC16-specific gene of vii. is *LM5578_1185* (SEQ ID NO: 10), and
ix. a gene, which is specific of *L. monoccytogenes* clone CC155, wherein said CC155-specific gene is *LMLG_RS02400* (SEQ ID NO: 8),
wherein the presence of one of said genes i.-ix. is indicative that said *L. monocytogenes* isolate has a high probability of belonging to the clone(s) that is(are) recited in i.-ix., respectively.

2. An *in vitro* method for determining that a *Listeria monocytogenes* isolate, which belongs to serogroup IVb or to serogroup IIb or to one of serogroups IIa and IIc, has a potentiality, more particularly a high potentiality, of causing invasive infection in a human, wherein said method comprises
when said *L. monocytogenes* isolate belongs to serogroup IVb, detecting that one of genes i.-iv. is present in said *L. monocytogenes* isolate,
when said *L. monocytogenes* isolate belongs to serogroup IIb, detecting that one of genes vi. and vii. is present in said *L. monocytogenes* isolate,
when said *L. monocytogenes,* isolate belongs to one of serogroups IIa and IIc, detecting that one of genes v., viii. and ix. is present in said *L. monocytogenes,* isolate;
said genes i.-iv., said genes vi. and vii., and said genes v., viii. and ix. being as defined in claim 1, wherein detecting that one of said genes i.-iv., or of said genes vi. and vii., or of said genes v., viii. and ix., is present in said *L. monocytogenes,* isolate is indicative that said *L. monocytogenes,* isolate has a potentiality, more particularly a high potentiality, of causing invasive infection in a human.

3. The *in vitro* method of claim 2, which further comprises, when said *L. monocytogenes* isolate belongs to one of serogroups IIa and IIc, determining whether one or none of the further following genes is or are present:
x. a gene, which is specific of *L. monocytogenes* clone CC121, wherein said CC121-specific gene is *LM6179_0610* (SEQ ID NO: 7); and
xi. a gene, which is specific of *L. monocytogenes* clone CC9, wherein said CC9-specific gene is *lmo1118* (SEQ ID NO: 11),
wherein detecting that one of said genes x. and xi., is present in said *L. monocytogenes* isolate is indicative that said *L. monocytogenes* isolate does not have a potentiality, more particularly a high potentiality, of causing invasive infection in a human, and
wherein detecting that one of said genes i.-iv., or of said genes vi. and vii., or of said genes v., viii. and ix., respectively, is present in said *L. monocytogenes,* isolate is indicative that said *L. monocytogenes,* isolate has a potentiality, more particularly a high potentiality, of causing invasive infection in a human.

4. The *in vitro* method of any one of claims 1-3, wherein detecting that one of said genes is present is performed by determining the presence or absence of a plurality of said genes.

5. The *in vitro* method of claim 4, wherein said plurality of genes comprises
- said genes i.-iv., or
- said genes vi.-vii., or
- said genes v., viii. and ix., or
- said genes v., and viii.-xi., or
- said genes i.-iv. and said genes vi.-vii., or
- said genes i.-iv. and said genes v., viii. and ix., or
- said genes i.-iv. and said genes v., and viii.-xi., or
- said genes vi.-vii. and said genes v., viii. and ix., or
- said genes vi.-vii. and said genes v., and viii.-xi., or
- said genes i.-iv., said genes vi.-vii. and said genes v., viii. and ix., or
- said genes i.-iv., said genes vi.-vii. and said genes v., and viii.-xi.

6. The *in vitro* method of claim 4 or 5, wherein primers and/or probes are used to determine the presence or absence of each gene of said plurality.

7. The *in vitro* method of claim 6, wherein said primers comprise
- a primer pair for specifically amplifying the sequence of SEQ ID NO: 16 from said gene i. of SEQ ID NO: 1, wherein said primer pair is
- the primer pair of SEQ ID NOs: 17 and 18, or
- a primer pair, which anneals to gene i. of SEQ ID NO: 1 to specifically amplify the sequence of SEQ ID NO: 16, wherein the first primer of said primer pair is at least 95% identical to the primer of SEQ ID NO: 17 and is of the same nucleotide length as SEQ ID NO: 17, and wherein the second primer of said primer pair is at least 95% identical to the primer of SEQ ID NO: 18 and is of the same nucleotide length as SEQ ID NO: 18;
- a primer pair for specifically amplifying the sequence of SEQ ID NO: 19 from said gene ii. of SEQ ID NO: 2, which is
- the primer pair of SEQ ID NOs: 20 and 21, or
- a primer pair, which anneals to gene ii. of SEQ ID NO: 2 to specifically amplify the sequence of SEQ ID NO: 19, wherein the first primer of said primer pair is at least 95% identical to the primer of SEQ ID NO: 20 and is of the same nucleotide length as SEQ ID NO: 20, and wherein the second primer of said primer pair is at least 95% identical to the primer of SEQ ID NO: 21 and is of the same nucleotide length as SEQ ID NO: 21;
- a primer pair for specifically amplifying the sequence of SEQ ID NO: 22 from said gene iii. of SEQ ID NO: 3, which is
- the primer pair of SEQ ID NOs: 23 and 24, or
- a primer pair, which anneals to gene iii. of SEQ ID NO: 3 to specifically amplify the sequence of SEQ ID NO: 22, wherein the first primer of said primer pair is at least 95% identical to the primer of SEQ ID NO: 23 and is of the same nucleotide length as SEQ ID NO: 23, and wherein the second primer of said primer pair is at least 95% identical to the primer of SEQ ID NO: 24 and is of the same nucleotide length as SEQ ID NO: 24; and
- a primer pair for specifically amplifying the sequence of SEQ ID NO: 25 from said gene iv. of SEQ ID NO: 4, which is
- the primer pair of SEQ ID NOs: 26 and 27, or
- a primer pair, which anneals to gene iv. of SEQ ID NO: 4 to specifically amplify the sequence of SEQ ID NO: 25, wherein the first primer of said primer pair is at least 95% identical to the primer of SEQ ID NO: 26 and is of the same nucleotide length as SEQ ID NO: 26, and wherein the second primer of said primer pair is at least 95% identical to the primer of SEQ ID NO: 27 and is of the same nucleotide length as SEQ ID NO: 27.

8. The *in vitro* method of claim 6, wherein said primers comprise
- a primer pair for specifically amplifying the sequence of SEQ ID NO: 28 from said gene vi. of SEQ ID NO: 5, which is
- the primer pair of SEQ ID NOs: 29 and 30, or
- a primer pair, which anneals to gene vi. of SEQ ID NO: 5 to specifically amplify the sequence of SEQ ID NO: 28, wherein the first primer of said primer pair is at least 95% identical to the primer of SEQ ID NO: 29 and is of the same nucleotide length as SEQ ID NO: 29, and wherein the second primer of said primer pair is at least 95% identical to the primer of SEQ ID NO: 30 and is of the same nucleotide length as SEQ ID NO: 30; and
- a primer pair for specifically amplifying the sequence of SEQ ID NO: 31 from said gene vii. of SEQ ID NO: 6, which is
- the primer pair of SEQ ID NOs: 32 and 33, or
- a primer pair, which anneals to gene vii. of SEQ ID NO: 6 to specifically amplify the sequence of SEQ ID NO: 31, wherein the first primer of said primer pair is at least 95% identical to the primer of SEQ ID NO: 32 and is of the same nucleotide length as SEQ ID NO: 32, and wherein the second primer of said primer pair is at least 95% identical to the primer of SEQ ID NO: 33 and is of the same nucleotide length as SEQ ID NO: 33.

9. The *in vitro* method of claim 6, wherein said primers comprise
- a primer pair for specifically amplifying the sequence of SEQ ID NO: 40 from said gene v. of SEQ ID NO: 9, which is
- the primer pair of SEQ ID NOs: 41 and 42, or
- a primer pair, which anneals to gene v. of SEQ ID NO: 9 to specifically amplify the sequence of SEQ ID NO: 40, wherein the first primer of said primer pair is at least 95% identical to the primer of SEQ ID NO: 41 and is of the same nucleotide length as SEQ ID NO: 41, and wherein the second primer of said primer pair is at least 95% identical to the primer of SEQ ID NO: 42 and is of the same nucleotide length as SEQ ID NO: 42;
- a primer pair for specifically amplifying the sequence of SEQ ID NO: 43 from said gene viii. of SEQ ID NO: 10, which is
- the primer pair of SEQ ID NOs: 44 and 45, or
- a primer pair, which anneals to gene viii. of SEQ ID NO: 10 to specifically amplify the sequence of SEQ ID NO: 43, wherein the first primer of said primer pair is at least 95% identical to the primer of SEQ ID NO: 44 and is of the same nucleotide length as SEQ ID NO: 44, and wherein the second primer of said primer pair is at least 95% identical to the primer of SEQ ID NO: 45 and is of the same nucleotide length as SEQ ID NO: 45; and
- a primer pair for specifically amplifying the sequence of SEQ ID NO: 37 from said gene ix. of SEQ ID NO: 8, which is
- the primer pair of SEQ ID NOs: 38 and 39, or
- a primer pair, which anneals to gene ix. of SEQ ID NO: 8 to specifically amplify the sequence of SEQ ID NO: 37, wherein the first primer of said primer pair is at least 95% identical to the primer of SEQ ID NO: 38 and is of the same nucleotide length as SEQ ID NO: 38, and wherein the second primer of said primer pair is at least 95% identical to the primer of SEQ ID NO: 39 and is of the same nucleotide length as SEQ ID NO: 39.

10. The *in vitro* method of any one of claims 4-9, wherein, in addition to determining the presence or absence of a plurality of said genes, said method further comprises determining the presence or absence of control marker gene(s), which is(are)
- a control marker gene or a combination of control marker genes, which is specific of *L. monocytogenes* serogroup IVb, and/or
- a control marker gene or a combination of control marker genes, which is specific of *L. monocytogenes* serogroup IIb, and/or
- a control marker gene or a combination of control marker genes, which is specific of *L. monocytogenes* serogroups IIa and IIc or of *L. monocytogenes,* lineage II.

11. The *in vitro* method of any one of claims 7-9, wherein said primer pairs are mixed together.

12. The *in vitro* method of any one of claims 6-11, wherein said primers comprise
- the primers as defined in claim 7 and the primers as defined in claim 8, or
- the primers as defined in claim 7 and the primers as defined in claim 9, or
- the primers as defined in claim 8 and the primers as defined in claim 9, or
- the primers as defined in claim 7, the primers as defined in claim 8 and the primers as defined in claim 9,
and wherein said primers are implemented simultaneously under the same PCR thermocycling conditions.

13. A set of oligonucleotides, which comprises the primers as defined in claim 7 wherein said primers are in mixture in said set, or which comprises the primers as defined in claim 8 wherein said primers are in mixture in said set, or which comprises the primers as defined in claim 9 wherein said primers are in mixture in said set.

14. A kit, which comprises
- the primers as defined in claim 7 and the primers as defined in claim 8, or which comprises
- the primers as defined in claim 7 and the primers as defined in claim 9, or which comprises
- the primers as defined in claim 8 and the primers as defined in claim 9, or which comprises
- the primers as defined in claim 7, the primers as defined in claim 8 and the primers as defined in claim 9.

15. The kit of claim 14, which comprises
- the primers as defined in claim 7 and the primers as defined in claim 8, wherein the primers as defined in claim 7 are mixed together in a first set of oligonucleotides and/or the primers as defined in claim 8 are mixed together in a second set of oligonucleotides, or which comprises
- the primers as defined in claim 7 and the primers as defined in claim 9, wherein the primers as defined in claim 7 are mixed together in a first set of oligonucleotides and/or the primers as defined in claim 9 are mixed together in a second set of oligonucleotides, or which comprises
- the primers as defined in claim 8 and the primers as defined in claim 9, wherein the primers as defined in claim 8 are mixed together in a first set of oligonucleotides and/or the primers as defined in claim 9 are mixed together in a second set of oligonucleotides, or which comprises
- the primers as defined in claim 7, the primers as defined in claim 8 and the primers as defined in claim 9, wherein the primers as defined in claim 7 are mixed together in a first set of oligonucleotides and/or the primers as defined in claim 8 are mixed together in a second set of oligonucleotides and/or the primers as defined in claim 9 are mixed together in a third set of oligonucleotides.

16. A computer device, which comprises a processing unit in the memory of which is stored a computer program product for storage in a memory of a processing unit or on a removable memory support for cooperation with a reader of said processing unit and which further comprises measurement values for the presence or absence of the genes as defined in any one of claims 1-12, wherein said computer program product comprises instructions for carrying out the method of any one of claims 1-12.
